# EUROPEAN PATENT SPECIFICATION

(11) **EP 2 704 800 B1**
(45) Date of publication and mention of the grant of the patent: **12.09.2018**
(21) Application number: 12776262.3
(22) Date of filing: 27.04.2012
(51) Int. Cl.: C12Q 1/6883

(54) **METHODS OF PREDICTING THE DEVELOPMENT OF COMPLEMENT-MEDIATED DISEASE**
VERFAHREN ZUR VORHERSAGE DER ENTWICKLUNG VON KOMPLEMENTVERMITTELTEN KRANKHEITEN
PROCÉDÉ DE PRÉDICTION DU DÉVELOPPEMENT D'UNE MALADIE MÉDIÉE PAR LE COMPLÉMENT

(30) Priority: 29.04.2011 US 201161480929 P
(43) Date of publication of application: 12.03.2014
(73) Proprietor: University of Utah Research Foundation, Salt Lake City, UT 84108 (US)
(72) Inventor: HAGEMAN, Gregory, S., Salt Lake City, UT 84105 (US); PAPPAS, Christian, Matthew, Salt Lake City, UT 84105 (US); BROWN, Eric, N., Overland Park, KS 66207 (US); HUTCHESON, David, Salt Lake City, UT 84105 (US)
(74) Representative: Michalski Hüttermann & Partner Patentanwälte mbB
(86) International application number: PCT/US2012/035467
(87) International publication number: WO 2012/149329

(56) References cited:
- WO-A2-2006/096561
- WO-A2-2008/008986
- WO-A2-2010/075519
- WO-A2-2012/082912
- US-A1- 2009 269 761
- US-A1- 2010 330 097
- US-B2- 7 745 389
- BAATZ H ET AL: "Komplementfaktor-Polymorphismen und altersassoziierte Makuladegeneration in einer Gruppe deutscher Patienten Polymorphisms of Complement Factor Genes and Age-Related Macular Degeneration in a German Population", KLINISCHE MONATSBLÄTTER FÜR AUGENHEILKUNDE, THIEME, DE, vol. 226, no. 8, 1 January 2009 (2009-01-01), pages 654-658, XP009181304, ISSN: 0344-6360, DOI: 10.1055/S-0028-1109318
- HUGHES ANNE E ET AL: "A common CFH haplotype, with deletion of CFHR1 and CFHR3, is associated with lower risk of age-related macular degeneration", NATURE GENETICS, vol. 38, no. 10, October 2006 (2006-10), pages 1173-1177, XP002732585, ISSN: 1061-4036
- ZAREPARSI SEPIDEH ET AL: "Strong association of the Y402H variant in complement factor H at 1q32 with susceptibility to age-related macular degeneration", AMERICAN JOURNAL OF HUMAN GENETICS, AMERICAN SOCIETY OF HUMAN GENETICS, CHICAGO, IL, US, vol. 77, no. 1, 13 May 2005 (2005-05-13), pages 149-153, XP002391910, ISSN: 0002-9297, DOI: 10.1086/431426
- ZHANG ET AL.: 'The NEI/NCBI dbGAP database: Genotypes and haplotypes that may specifically predispose to risk of neovascular age-related macular degeneration.' BMC MEDICAL GENETICS vol. 9, no. 51, 09 June 2008, pages 51 - 61, XP002611536
- BERGERON-SAWITZKE ET AL.: 'Multilocus analysis of age-related macular degeneration.' EUROPEAN JOUMAL OF HUMAN GENETICS vol. 17, no. 9, 04 March 2009, pages 1190 - 1199, XP055128812

### SEQUENCE LISTING

<110> University of Utah Research Foundation
   Hageman, Gregory S
   Pappas, Christian M
   Brown, Eric N
   Hutcheson, David
<120> Methods of predicting the development of complement-mediated disease
<130> 21101.0249P1
<150> 61/480,929
   <151> 2011-04-29
<160> 376
<170> PatentIn version 3.5
<210> 1
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 1
   accgnatcat cgcagnagag aacggccgca tgaccgaggg cggcgtaccg gggcaagcac 60
   cg 62
<210> 2
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 2
   accgnatcat cgcagnagag aacggccgca tgaccgaggg cggcgtaccg aatcaagtac 60
   cg 62
<210> 3
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 3
   actanatcca tattgngatt agtgaacgca tgaccgaaag tggcgtacct ggttaagcac 60
   cg 62
<210> 4
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 4
   atcgnatcca tatatnggat ggcggacttg attccaaagg caatgttcat aatcaagtac 60
   cg 62
<210> 5
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 5
   accgngctca tatagnaatt agttgacgcn nnnccgaagg cagctcacat gatcaaatgt 60
   ta 62
<210> 6
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 6
   actanatcca tattgngatt agtgaacgca tgaccgaaag tgatgttcat aatcaagtac 60
   cg 62
<210> 7
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 7
   gtcgnatcca tatatnagag aacggccgca tgaccgaggg cggcgtaccg aatcaagtac 60
   cg 62
<210> 8
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 8
   accgnatcat cgcagnagag aacggccgca tgaccgaggg cgatgttcat aatcaagtac 60
   cg 62
<210> 9
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 9
   atcgnatcca tatatnggat ggcggacttg attccgaagg cggcgtaccg gggcaagcac 60
   cg 62
<210> 10
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 10
   atcgnatcca tatatnggat ggcggacttg attccagagg cagcgtaccg gggcaagcac 60
   cg 62
<210> 11
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 11
   atcgnatcca tatatnaatt agttgacgcn nnnccaaagg caatgttcat aatcaagtac 60
   cg 62
<210> 12
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 12
   accgnatcat cgcagnagag aacggccgca tgaccgaaag tggcgtacct ggttaagcac 60
   cg 62
<210> 13
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 13
   accgngctca tatagnaatt agttgacgcn nnnccgaagg caatgttcat aatcaagtac 60
   cg 62
<210> 14
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 14
   accgnatcat cgcagnagag aacggccgca tgaccgaagg cggcgtaccg gggcaagcac 60
   cg 62
<210> 15
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 15
   accgngctca tatagnaatt agttgacgcn nnnccgaagg cagcgtaccg gggcaagcac 60
   cg 62
<210> 16
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 16
   accgnatcat cgcagnagag aacggctgca tgacagaggg cggcgtaccg gggcaagcac 60
   cg 62
<210> 17
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 17
   atcgnatcca tatatnggat ggcggacttg attccgaaga cggcgtaccg gggcaagcac     60
   cg                                                                    62
<210> 18
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 18
   atcgngctca tatagnaatt agttgacgcn nnnccgaagg cagctcacat gatcaaatgt     60
   ta                                                                    62
<210> 19
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 19
   atcgnatcat cgcagnagag aacggccgca tgaccgaggg cggcgtaccg gggcaagcac 60
   cg 62
<210> 20
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 20
   accgnatcat cgcagnagag aacggccgca tgaccgaggg cggcgtaccg gggctagcac 60
   cg 62
<210> 21
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 21
   atcgnatcca tatatnggat ggcggacttg attccaaagg cagcgttcat aatcaagtac 60
   cg 62
<210> 22
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 22
   gtcgnatcat cgcagnagag aacggccgca tgaccgaggg cggcgtaccg aatcaagtac 60
   cg 62
<210> 23
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 23
   accgnatcat cgcagnagag aacggccgca tgagcgaggg cggcgtaccg aatcaagtac 60
   cg 62
<210> 24
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 24
   accgnatcat cgcagnagag aacggccgca tgaccaaagg caatgttcat aatcaagtac 60
   cg 62
<210> 25
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 25
   actanatcca tatatnggat ggcggacttg attccgaaga cggcgtaccg gggcaagcac 60
   cg 62
<210> 26
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 26
   atcgnatcca tatatnaatt agttgacgcn nnnccaaagg caatgttcat aatcaggtac 60
   cg 62
<210> 27
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 27
   accgnatcat cgcagnagag aacggccgca tgaccgaggg cggcgtacct ggttaagcac 60
   cg 62
<210> 28
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 28
   atcgnatcca tatatnaatt agttgacgcn nnnccaaagg caatgtaccg gggcaagcac 60
   cg 62
<210> 29
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 29
   atcgnatcca tatatnggat ggcggacttg attccgaaag tggcgtacct ggttaagcac 60
   cg 62
<210> 30
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 30
   actanatcca tattgngatt agtgaacgca tgaccgaaag tggcgtaccg aatcaagtac 60
   cg 62
<210> 31
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 31
   atcgnatcca tatatnaatt agttgacgcn nnnccgaaag tggcgtacct ggttaagcac 60
   cg 62
<210> 32
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 32
   atcgnatcca tatatnggat ggcggacttg attccgaggg cggcgtaccg aatcaagtac 60
   cg 62
<210> 33
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 33
   actanatcca tattgngatt agtgaacgca tgaccgaaag tggcgtaccg gggcaagcac 60
   cg 62
<210> 34
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 34
   actanatcca tattgngatt agtgaacgca tgaccgaaag tggcgtacct gggcaagcac 60
   cg 62
<210> 35
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 35
   accgnatcat cgcagnagag aacggccgca tgaccgaggg cggcgtatcg gggcaagcac 60
   cg 62
<210> 36
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 36
   accgnatcca tatatnagag aacggccgca tgaccgaggg cggcgtaccg aatcaagtac 60
   cg 62
<210> 37
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 37
   actanatcca tattgngatt agtgaacgca tgaccgaggg cggcgtaccg gggcaagcac      60
   cg                                                                     62
<210> 38
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 38
   atcgnatcca tatatnaatt agttgacttg attccagagg cggcgtaccg gggcaagcac      60
   cg                                                                     62
<210> 39
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 39
   atcgnatcat cgcagnagag aacggccgca tgaccgaggg cggcgtaccg aatcaagtac 60
   cg 62
<210> 40
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 40
   actanatcca tattgngatt agtgaacgca tgaccgaagg cggcgtaccg aatcaagtac 60
   cg 62
<210> 41
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 41
   accgnatcca tattgngatt agtgaacgca tgaccgaaag tggcgtacct ggttaagcac 60
   cg 62
<210> 42
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 42
   atcgnatcat cgcagnagag aacggccgca tgaccgaggg cggcgtaccg aatcaagtac 60
   cg 62
<210> 43
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 43
   accgngctca tatagnaatt agttgacgca tgaccgaagg cagctcacat gatcaaatgt 60
   ta 62
<210> 44
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 44
   accgnatcat cgcagnagag aacggccgca tgaccgaggg cggctcacat gatcaaatgt    60
   ta                                                                   62
<210> 45
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 45
   accgnatcat cgcagnagag aacggccgca tgaccgaggg cggtgttcat aatcaagtac    60
   cg                                                                   62
<210> 46
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 46
   accgnatcat cgcagnagag aacggccgcn nnnccgaggg cggcgtaccg aatcaagtac 60
   cg 62
<210> 47
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 47
   gtcgnatcat cgcagnagag aacggccgca tgagcgaggg cggcgtaccg aatcaagtac 60
   cg 62
<210> 48
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 48
   atcgnatcca tatatnggat ggcggacttg attccaaagg cagcgtaccg gggcaagcac 60
   cg 62
<210> 49
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 49
   atcgnatcca tatatnaatt agttgacgcn nnnccgaggg cggcgtaccg gggcaagcac 60
   cg 62
<210> 50
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 50
   atcgnatcat cgcagnagag aacggccgca tgaccgaggg cggcgtaccg gggcaagcac 60
   cg 62
<210> 51
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 51
   actanatcca tattgngatt agtgaacgca tttccgaaag tggcgtacct ggttaagcac 60
   cg 62
<210> 52
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 52
   actanatcca tattgngatt agtgaacgca tttccgaaag tgatgttcat aatcaagtac 60
   cg 62
<210> 53
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 53
   gtcgnatcca tatatnagag aacggccgca tgaccgaaag tggcgtacct ggttaagcac 60
   cg 62
<210> 54
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 54
   atcgnatcca tatatnaatt agttgacgca tgaccaaagg caatgttcat aatcaagtac 60
   cg 62
<210> 55
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 55
   accgnatcca tatatnggat ggcggacttg attccaaagg caatgttcat aatcaagtac 60
   cg 62
<210> 56
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 56
   accgngttca tatagnaatt agttgacgcn nnnccgaagg cagcgtaccg gggcaagcac 60
   cg 62
<210> 57
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 57
   accgnatcca tatatnaatt agttgacgcn nnnccgaaag tggcgtacct ggttaagcac 60
   cg 62
<210> 58
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 58
   actanatcca tattgnggtt agtgaacgca tgaccgaaag tggcgtacct ggttaagcac 60
   cg 62
<210> 59
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 59
   atcgnatcat cgcagnagag aacggccgca tgagcgaggg cggcgtaccg aatcaagtac 60
   cg 62
<210> 60
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 60
   accgngctca tatagnaatt agttgacgcn nnnccgaggg cggcgtaccg aatcaagtac 60
   cg 62
<210> 61
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 61
   actanatcca tattgngatt agtgaacgca tgaccgagag tggcgtacct ggttaagcac 60
   cg 62
<210> 62
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 62
   accgnatcat cgcagnagag aacggccgca tgaccgaggg cggcgtaccg gggcaagtac 60
   cg 62
<210> 63
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 63
   accgnatcat cgcagnagag aacggccgca tgaccagagg cagcgtaccg gggcaagcac 60
   cg 62
<210> 64
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 64
   actanatcca tatatnggat ggcggacttg attccgaggg cggcgtaccg gggcaagcac 60
   cg 62
<210> 65
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 65
   actanatcat cgcagnagag aacggccgca tgaccgaggg cggcgtaccg gggcaagcac 60
   cg 62
<210> 66
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 66
   actanatcca tatatnggat ggcggacttg attccagagg cagcgtaccg gggcaagcac 60
   cg 62
<210> 67
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 67
   attanatcca tattgngatt agtgaacgca tgaccgaaag tggcgtacct ggttaagcac 60
   cg 62
<210> 68
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 68
   accgnatcat cgcagnagag aacggacgca tgagcgaggg cggcgtaccg aatcaagtac 60
   cg 62
<210> 69
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 69
   atcgnatcca tattgngatt agtgaacgca tgaccgaaag tggcgtacct ggttaagcac 60
   cg 62
<210> 70
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 70
   actanatcca tattgngatt agtgaacgca tttccgaagg cggcgtaccg aatcaagtac 60
   cg 62
<210> 71
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 71
   gtcgnatcca tatatnagag aacggccgca tgaccgaagg cggcgtaccg aatcaagtac 60
   cg 62
<210> 72
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 72
   gtcgnatcca tatatnagag aacggccgca tgaccgaggg cggcgtaccg gggcaagcac 60
   cg 62
<210> 73
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 73
   accgnatcat cgcagnagag aacggccgca tgaccgagag tggcgtacct ggttaagcac 60
   cg 62
<210> 74
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 74
   atcgnatcca tatatnggat ggcggacttg attccgaagg caatgttcat aatcaagtac 60
   cg 62
<210> 75
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 75
   accgnatcat catagnagag aacggccgca tgaccgaggg cggcgtaccg gggcaagcac 60
   cg 62
<210> 76
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 76
   accgnatcat cgcagnagag aacggccgca tgaccgaggg cgatgtaccg gggcaagcac 60
   cg 62
<210> 77
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 77
   actanatcca tatatnggat ggcggacttg attccaaagg caatgttcat aatcaagtac 60
   cg 62
<210> 78
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 78
   gtcgnatcca tatatnggat ggcggacttg attccaaagg caatgttcat aatcaagtac 60
   cg 62
<210> 79
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 79
   actanattca tatagnaatt agttgacgcn nnnccgaagg cagctcacat gatcaaatgt 60
   ta 62
<210> 80
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 80
   accgnatcca tatatnagag aacggccgca tgaccgaggg cggcgtaccg gggcaagcac 60
   cg 62
<210> 81
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 81
   actanatcca tatatnggat ggcggacttg attccgaaag tggcgtacct ggttaagcac 60
   cg 62
<210> 82
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 82
   actangctca tatagnaatt agttgacgcn nnnccaaagg caatgttcat aatcaagtac 60
   cg 62
<210> 83
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 83
   accgngctca tatagnaatt agttgacgcn nnnccaaagg caatgttcat aatcaagtac 60
   cg 62
<210> 84
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 84
   accgnatcat cgcagnagag aacggccgca tgaccgaaag tggcgtaccg gggcaagcac 60
   cg 62
<210> 85
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 85
   atcgnatcat cgcagnagag aacggccttg attccaaagg caatgttcat aatcaagtac 60
   cg 62
<210> 86
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 86
   accgnatcca tatatnggat ggcggacttg attccgaggg cagcgtaccg aatcaagtac 60
   cg 62
<210> 87
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 87
   accgnatcca tattgngatt agtgaacgca tgaccgaaag tgatgttcat aatcaagtac 60
   cg 62
<210> 88
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 88
   accgnatcca tatatnggat ggcggacttg attccgaaga cggcgtaccg aatcaagtac 60
   cg 62
<210> 89
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 89
   accgnatcca tatatnggat ggcggacttg attccaaagg caatgttcat gatcaaatgt 60
   ta 62
<210> 90
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 90
   accgnatcca tatatnaatt agttgacgcn nnnccgaaag tgatgttcat aatcaagtac 60
   cg 62
<210> 91
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 91
   accgnatcaa tgcatnggat agcggccttg attccgaagg caatgttcat aatcaagtac 60
   cg 62
<210> 92
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 92
   accgngctca tatagnaatt agttgccgcn nnnccgaagg cagctcacat gatcaaatgt 60
   ta 62
<210> 93
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 93
   atcgnatcca tatatnggat ggcggccttg attccagagg cagcgtaccg gggcaagcac 60
   cg 62
<210> 94
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 94
   accgnatcat cgcagnagag aacggccgca tgaccgaggg cggcgtaccg gggcaagcac 60
   cg 62
<210> 95
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 95
   accgnattca tatagnaatt agttgacgcn nnnccgaagg caatgttcat aatcaagtac 60
   cg 62
<210> 96
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 96
   atcgngctca tatagnaatt agttgacgcn nnnccgaagg caatgttcat aatcaagtac 60
   cg 62
<210> 97
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 97
   accgnatcca tatatnggat ggcggacttg attccgaaga cggcgtaccg gggcaagcac 60
   cg 62
<210> 98
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 98
   accgnattca tatagnagtt ggttgacgcn nnnccgaagg caatgttcat aatcaagtac 60
   cg 62
<210> 99
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 99
   atcgnatcca tatatnagag aacggccgca tgaccgaggg cggcgtaccg aatcaagtac 60
   cg 62
<210> 100
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 100
   actanatcca tattgnggtt agtgaacgca tgaccgaaag tgatgttcat aatcaagtac 60
   cg 62
<210> 101
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 101
   atcgngctca tatagnaatt agttgacgcn nnnccgaagg cagcgtaccg gggcaagcac 60
   cg 62
<210> 102
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 102
   accgngctca tatagnaatt agttgacgcn nnnccgaagg caatgttcat aatcaagtac 60
   cg 62
<210> 103
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 103
   actanatcca tattgngatt agtgaacgca tgaccgaaag tggcgtacct ggttaagcac 60
   cg 62
<210> 104
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 104
   accgnatcat cgcagnagag aacggacgca tgaccgaggg cggcgtaccg aatcaagtac 60
   cg 62
<210> 105
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 105
   actanatcca tattgngatt agtgaacgca tgaccgaagg cagctcacat gatcaaatgt 60
   ta 62
<210> 106
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 106
   actanatcat cgcagnagag aacggccgca tgaccgaggg cggcgtaccg aatcaagtac 60
   cg 62
<210> 107
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 107
   accgngttca tatagnaatt agttgacgcn nnnccgaagg cagctcacat gatcaaatgt 60
   ta 62
<210> 108
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 108
   gccgnatcca tatatnagag aacggccgca tgaccgaggg cggcgtaccg aatcaagtac 60
   cg 62
<210> 109
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 109
   atcgnatcca tatatngaat ggcggacttg attccaaagg caatgttcat aatcaagtac 60
   cg 62
<210> 110
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 110
   atcgngctca tatagnagtt agttgacgcn nnnccgaagg cagctcacat gatcaaatgt 60
   ta 62
<210> 111
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 111
   atcgnatcca tatatnagtt agttgacgcn nnnccgaaag tggcgtacct ggttaagcac 60
   cg 62
<210> 112
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 112
   accgnatcat cgcagnagag aacggctgca tgacagaggg cggcgtacct ggttaagcac 60
   cg 62
<210> 113
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 113
   accgnattca tgcagnagtt agttgccgcg attccaaagg caatgttcat aatcaagtac 60
   cg 62
<210> 114
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 114
   accgngctca tatagnagtt agttgacgcn nnnccgaagg cagctcacat gatcaaatgt 60
   ta 62
<210> 115
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 115
   atcgnatcca tatatnaatt agttgacgca tgaccaaagg caatgttcat aatcaggtac      60
   cg                                                                      62
<210> 116
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 116
   atcgnatcca tatatnggat ggcggacttg attccgaggg cggcgtaccg gggcaagcac      60
   cg                                                                      62
<210> 117
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 117
   atcgnatcca tatatnggat ggcggacttg agaccgaggg cggcgtaccg aatcaagtac 60
   cg 62
<210> 118
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 118
   atcgnatcca tatatnaatt agttgacgcn nnnccaaagg cagcgtaccg gggcaagcac 60
   cg 62
<210> 119
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 119
   accgngctca tatagnagtt agttgacgcn nnnccgaagg cggcgtaccg aatcaagtac 60
   cg 62
<210> 120
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 120
   accgnatcat cgcatnagag aacggccgca tgaccgaggg cggcgtaccg aatcaagtac 60
   cg 62
<210> 121
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 121
   gtcgnatcca tatatnagag aacggccgca tgaccgagag tggcgtacct ggttaagcac 60
   cg 62
<210> 122
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 122
   accgnatcat cgcagnagag aacggccgca tgaccgagag tggcgtaccg aatcaagtac 60
   cg 62
<210> 123
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 123
   accgnatcat cgcagnagag aatggccgca tgaccaaagg cagcgttcat aatcaagtac 60
   cg 62
<210> 124
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 124
   accgnatcca tatagngatt agtggacgca tgaccgaagg cggcgtaccg aatcaagtac 60
   cg 62
<210> 125
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 125
   accgngctca tatagnaatt agttgacgcn nnnccgaagg cagctcacat aatcaagtac 60
   cg 62
<210> 126
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 126
   atcgnatcca tattgngatt agtgaacttg attccaaagg caatgttcat aatcaagtac 60
   cg 62
<210> 127
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 127
   accgnatcat cgcagnagat agcggacgca attccaaagg cggcttacat gatcaagtac 60
   cg 62
<210> 128
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 128
   accgnatcat cgcagnagag aacggccgca tgaccaaagg caatgttcat aatcaggtac 60
   cg 62
<210> 129
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 129
   accgnatcat cgcagnagag aacggccgca tgaccaaagg cggcgtaccg aatcaagtac 60
   cg 62
<210> 130
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 130
   accgngctca tatagnaatt agttgacgcn nnnccgaagg cagctttcat aatcaagtac 60
   cg 62
<210> 131
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 131
   actanatcca tattgngatt agtgaacttg attccagagg cagcgtaccg gggcaagcac 60
   cg 62
<210> 132
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 132
   actangctca tatagnagat agttgacgcn nnnccgaagg cggcgtaccg aatcaagtac 60
   cg 62
<210> 133
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 133
   actanatcca tattgnggtt agtgaacgca tgaccgaagg cagctcacat gatcaaatgt 60
   ta 62
<210> 134
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 134
   actanatcca tattgngaag aacggccgca tgaccgaggg cggcgtaccg aatcaagtac 60
   cg 62
<210> 135
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 135
   atcgnatcca tatatnaatt agttgacgcg attccaaagg caatgttcat aatcaagtac 60
   cg 62
<210> 136
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 136
   atcgnatcca tatatnaatt agttgacgcn nnnccaaagg cggcgtaccg aatcaagtac 60
   cg 62
<210> 137
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 137
   accgngccat cgcagnagag aacggccgca tgaccgaggg cggcgtaccg gggcaagcac        60
   cg                                                                       62
<210> 138
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 138
   atcgnatcca tatatnggat ggcggacttg agaccgaaga cggcgtaccg gggcaagcac        60
   cg                                                                       62
<210> 139
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 139
   accgngctca tatagnaatt agttgacgcn nnnccgaagg cagctcacat gatcaagtac 60
   cg 62
<210> 140
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 140
   accgnatcat cgcagnagag aacggccgca tgaccgaggg cggcgtaccg aatcaggtac 60
   cg 62
<210> 141
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 141
   atcgnattca tatagnaatt agttgacgcn nnnccgaagg cagctcacat gatcaaatgt 60
   ta 62
<210> 142
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 142
   gtcgnatcat cgcagnagag aacggccgca tgaccaaagg caatgttcat aatcaagtac 60
   cg 62
<210> 143
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 143
   atcgnatcca tatatnaatt agttgacgca tgaccaaagg caatgtaccg gggcaagcac 60
   cg 62
<210> 144
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 144
   accgngctca tatagnaatt ggcggacttg attccaaagg caatgttcat aatcaagtac 60
   cg 62
<210> 145
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 145
   actanatcca tatatnagag aacggccgca tgaccgaggg cggcgtaccg aatcaagtac 60
   cg 62
<210> 146
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 146
   atcgnatcca tatatnggat ggcggacgca tgaccaaagg caatgttcat aatcaagtac 60
   cg 62
<210> 147
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 147
   accgnatcaa tgcatnggat ggcggacttg attccaaagg caatgttcat aatcaagtac 60
   cg 62
<210> 148
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 148
   accgngctca tatagnaatt agttgacgcn nnnccgaggg cagctcacat gatcaaatgt 60
   ta 62
<210> 149
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 149
   accgnatcat cgcagnaaag aacggccgca tgaccgaggg cggcgtaccg gggctagcac 60
   cg 62
<210> 150
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 150
   actanatcat cgcagnagag aacggccgca tgaccgaggg cggcgtaccg aatcaagtac 60
   cg 62
<210> 151
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 151
   actanatcca tattgngatt agttgacgcn nnnccgaagg cagctcacat gatcaaatgt 60
   ta 62
<210> 152
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 152
   actanatcca tatatnggat agcggacgta attccgaagg cagctcacat gatcaaatgt 60
   ta 62
<210> 153
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 153
   actanatcca tatatnggat ggcggacttg attccaaaag cgatgttcat aatcaagtac 60
   cg 62
<210> 154
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 154
   atcgnatcca tatatnggat ggcggacttg attccagagg cagcgtaccg gggcaagcac 60
   tg 62
<210> 155
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 155
   accgnatcat cgcagnagag aacggccgca tgaccgaggg cggcgtaccg aatcaagcac 60
   cg 62
<210> 156
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 156
   accgnatcat cgcagnagag aacggccgca tgaccgaggg cagcgtaccg aatcaagtac 60
   cg 62
<210> 157
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 157
   accgnatcca tatatnagag aacggccgca tgaccgaggg cgatgttcat aatcaagtac 60
   cg 62
<210> 158
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 158
   accgngccct catagnagag aacggacgca tgaccgaggg cggcgtaccg gggcaagcac 60
   cg 62
<210> 159
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 159
   actanatcca tattgngatt agtgaacttg attccgaggg cggcgtaccg gggcaagcac 60
   cg 62
<210> 160
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<220>
   <221> misc_feature
   <222> (30)..(33)
   <223> n is a, c, g, or t
<400> 160
   atcgnatcat cgcagnagag aacggccgcn nnnccgaagg cagctcacat gatcaaatgt 60
   ta 62
<210> 161
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 161
   gtcgnatcat cgcagnagag aacggccgca tgaccgaggg cggcttaccg aatcaagtac 60
   cg 62
<210> 162
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 162
   actangctca tatagngatt agtgaacgca tgaccgaaag tggcgtacct ggttaagcac 60
   cg 62
<210> 163
   <211> 62
   <212> DNA
   <213> Artificial Sequence
<220>
   <223> Description of Artificial Sequence; Note = Synthetic Construct
<220>
   <221> misc_feature
   <222> (5)..(5)
   <223> n is a, c, g, or t
<220>
   <221> misc_feature
   <222> (16)..(16)
   <223> n is a, c, g, or t
<400> 163
   accgnatcct catagnaaag gacggacgca tgaccgaggg cggcgtaccg gggcaagcac 60
   cg 62
<210> 164
   <211> 51
   <212> DNA
   <213> Artificial Sequence
<220>
   <223> Description of Artificial Sequence; Note = Synthetic Construct
<400> 164
   accgatcaac catgacaagg ccgccggacg cggggttgcg cggcttcgtc c 51
<210> 165
   <211> 51
   <212> DNA
   <213> Artificial Sequence
<220>
   <223> Description of Artificial Sequence; Note = Synthetic Construct
<400> 165
   accgatcaac catgacaagg ccgccggacg cggggttgcg tatcttcatc c 51
<210> 166
   <211> 51
   <212> DNA
   <213> Artificial Sequence
<220>
   <223> Description of Artificial Sequence; Note = Synthetic Construct
<400> 166
   actaatcctt ttcataagag tcgccggata cggggttgct cgttttcgtc c 51
<210> 167
   <211> 51
   <212> DNA
   <213> Artificial Sequence
<220>
   <223> Description of Artificial Sequence; Note = Synthetic Construct
<400> 167
   atcgatcctt tacgaagggg ccttcgaatg caaagtagat tatcttcatc c 51
<210> 168
   <211> 51
   <212> DNA
   <213> Artificial Sequence
<220>
   <223> Description of Artificial Sequence; Note = Synthetic Construct
<400> 168
   accggctctt tatataagat ccgccggatg caggtctgat catctttact t 51
<210> 169
   <211> 51
   <212> DNA
   <213> Artificial Sequence
<220>
   <223> Description of Artificial Sequence; Note = Synthetic Construct
<400> 169
   actaatcctt ttcataagag tcgccggata cgaagtagat tatcttcatc c 51
<210> 170
   <211> 51
   <212> DNA
   <213> Artificial Sequence
<220>
   <223> Description of Artificial Sequence; Note = Synthetic Construct
<400> 170
   gtcgatcctt tatgacaagg ccgccggacg cggggttgcg tatcttcatc c 51
<210> 171
   <211> 51
   <212> DNA
   <213> Artificial Sequence
<220>
   <223> Description of Artificial Sequence; Note = Synthetic Construct
<400> 171
   accgatcaac catgacaagg ccgccggacg cgaagtagat tatcttcatc c 51
<210> 172
   <211> 51
   <212> DNA
   <213> Artificial Sequence
<220>
   <223> Description of Artificial Sequence; Note = Synthetic Construct
<400> 172
   atcgatcctt tacgaagggg ccttcggatg cggggttgcg cggcttcgtc c 51
<210> 173
   <211> 51
   <212> DNA
   <213> Artificial Sequence
<220>
   <223> Description of Artificial Sequence; Note = Synthetic Construct
<400> 173
   atcgatcctt tatataagat ccgccgaatg caaagtagat tatcttcatc c 51
<210> 174
   <211> 51
   <212> DNA
   <213> Artificial Sequence
<220>
   <223> Description of Artificial Sequence; Note = Synthetic Construct
<400> 174
   atcgatcctt tacgaagggg ccttcgagtg cagggttgcg cggcttcgtc c 51
<210> 175
   <211> 51
   <212> DNA
   <213> Artificial Sequence
<220>
   <223> Description of Artificial Sequence; Note = Synthetic Construct
<400> 175
   accgatcaac catgacaagg ccgccggata cggggttgct cgttttcgtc c 51
<210> 176
   <211> 51
   <212> DNA
   <213> Artificial Sequence
<220>
   <223> Description of Artificial Sequence; Note = Synthetic Construct
<400> 176
   accgatcaac catgacaagg ccgccggatg cggggttgcg cggcttcgtc c 51
<210> 177
   <211> 51
   <212> DNA
   <213> Artificial Sequence
<220>
   <223> Description of Artificial Sequence; Note = Synthetic Construct
<400> 177
   accggctctt tatataagat ccgccggatg caaagtagat tatcttcatc c 51
<210> 178
   <211> 51
   <212> DNA
   <213> Artificial Sequence
<220>
   <223> Description of Artificial Sequence; Note = Synthetic Construct
<400> 178
   accggctctt tatataagat ccgccggatg cagggttgcg cggcttcgtc c 51
<210> 179
   <211> 51
   <212> DNA
   <213> Artificial Sequence
<220>
   <223> Description of Artificial Sequence; Note = Synthetic Construct
<400> 179
   atcgatcaac catgacaagg ccgccggacg cggggttgcg cggcttcgtc c 51
<210> 180
   <211> 51
   <212> DNA
   <213> Artificial Sequence
<220>
   <223> Description of Artificial Sequence; Note = Synthetic Construct
<400> 180
   accgatcaac catgacaagg ccgcgggacg cggggttgcg tatcttcatc c 51
<210> 181
   <211> 51
   <212> DNA
   <213> Artificial Sequence
<220>
   <223> Description of Artificial Sequence; Note = Synthetic Construct
<400> 181
   accgatcaac catgacaagg ctgcctgacg cggggttgcg cggcttcgtc c 51
<210> 182
   <211> 51
   <212> DNA
   <213> Artificial Sequence
<220>
   <223> Description of Artificial Sequence; Note = Synthetic Construct
<400> 182
   atcgatcctt tacgaagggg ccttcggatg tggggttgcg cggcttcgtc c 51
<210> 183
   <211> 51
   <212> DNA
   <213> Artificial Sequence
<220>
   <223> Description of Artificial Sequence; Note = Synthetic Construct
<400> 183
   accgatcaac catgacaagg ccgccgaatg caaagtagat tatcttcatc c 51
<210> 184
   <211> 51
   <212> DNA
   <213> Artificial Sequence
<220>
   <223> Description of Artificial Sequence; Note = Synthetic Construct
<400> 184
   atcggctctt tatataagat ccgccggatg caggtctgat catctttact t 51
<210> 185
   <211> 51
   <212> DNA
   <213> Artificial Sequence
<220>
   <223> Description of Artificial Sequence; Note = Synthetic Construct
<400> 185
   atcgatcctt tatataagat ccgccgaatg caaagtagat tatctccatc c 51
<210> 186
   <211> 51
   <212> DNA
   <213> Artificial Sequence
<220>
   <223> Description of Artificial Sequence; Note = Synthetic Construct
<400> 186
   atcgatcctt tacgaagggg ccttcgaatg cagggtagat tatcttcatc c 51
<210> 187
   <211> 51
   <212> DNA
   <213> Artificial Sequence
<220>
   <223> Description of Artificial Sequence; Note = Synthetic Construct
<400> 187
   gtcgatcaac catgacaagg ccgccggacg cggggttgcg tatcttcatc c 51
<210> 188
   <211> 51
   <212> DNA
   <213> Artificial Sequence
<220>
   <223> Description of Artificial Sequence; Note = Synthetic Construct
<400> 188
   atcgatcaac catgacaagg ccgccggacg cggggttgcg tatcttcatc c 51
<210> 189
   <211> 51
   <212> DNA
   <213> Artificial Sequence
<220>
   <223> Description of Artificial Sequence; Note = Synthetic Construct
<400> 189
   accgatcaac catgacaagg ccgccggacg cggggttgcg cggcatcgtc c 51
<210> 190
   <211> 51
   <212> DNA
   <213> Artificial Sequence
<220>
   <223> Description of Artificial Sequence; Note = Synthetic Construct
<400> 190
   atcgatcctt tatataagat ccgccgaatg caaagttgcg cggcttcgtc c 51
<210> 191
   <211> 51
   <212> DNA
   <213> Artificial Sequence
<220>
   <223> Description of Artificial Sequence; Note = Synthetic Construct
<400> 191
   accgatcaac catgacaagg ccgccggacg cggggttgct cgttttcgtc c 51
<210> 192
   <211> 51
   <212> DNA
   <213> Artificial Sequence
<220>
   <223> Description of Artificial Sequence; Note = Synthetic Construct
<400> 192
   actaatcctt tacgaagggg ccttcggatg tggggttgcg cggcttcgtc c 51
<210> 193
   <211> 51
   <212> DNA
   <213> Artificial Sequence
<220>
   <223> Description of Artificial Sequence; Note = Synthetic Construct
<400> 193
   atcgatcctt tacgaagggg ccttcggacg cggggttgcg tatcttcatc c 51
<210> 194
   <211> 51
   <212> DNA
   <213> Artificial Sequence
<220>
   <223> Description of Artificial Sequence; Note = Synthetic Construct
<400> 194
   actaatcctt ttcataagag tcgccggata cggggttgcg tatcttcatc c 51
<210> 195
   <211> 51
   <212> DNA
   <213> Artificial Sequence
<220>
   <223> Description of Artificial Sequence; Note = Synthetic Construct
<400> 195
   actaatcctt ttcataagag tcgccggata cggggttgcg cggcttcgtc c 51
<210> 196
   <211> 51
   <212> DNA
   <213> Artificial Sequence
<220>
   <223> Description of Artificial Sequence; Note = Synthetic Construct
<400> 196
   accgatcctt tatgacaagg ccgccggacg cggggttgcg tatcttcatc c 51
<210> 197
   <211> 51
   <212> DNA
   <213> Artificial Sequence
<220>
   <223> Description of Artificial Sequence; Note = Synthetic Construct
<400> 197
   atcgatcctt tatataagat ccgccggata cggggttgct cgttttcgtc c 51
<210> 198
   <211> 51
   <212> DNA
   <213> Artificial Sequence
<220>
   <223> Description of Artificial Sequence; Note = Synthetic Construct
<400> 198
   actaatcctt ttcataagag tcgccggata cggggttgct cggcttcgtc c 51
<210> 199
   <211> 51
   <212> DNA
   <213> Artificial Sequence
<220>
   <223> Description of Artificial Sequence; Note = Synthetic Construct
<400> 199
   actaatcctt ttcataagag tcgccggatg cggggttgcg tatcttcatc c 51
<210> 200
   <211> 51
   <212> DNA
   <213> Artificial Sequence
<220>
   <223> Description of Artificial Sequence; Note = Synthetic Construct
<400> 200
   atcgatcctt tacgaagggg ccttcggata cggggttgct cgttttcgtc c 51
<210> 201
   <211> 51
   <212> DNA
   <213> Artificial Sequence
<220>
   <223> Description of Artificial Sequence; Note = Synthetic Construct
<400> 201
   accgatcaac catgacaagg ccgccggacg cggggttacg cggcttcgtc c 51
<210> 202
   <211> 51
   <212> DNA
   <213> Artificial Sequence
<220>
   <223> Description of Artificial Sequence; Note = Synthetic Construct
<400> 202
   accgatcctt ttcataagag tcgccggata cggggttgct cgttttcgtc c 51
<210> 203
   <211> 51
   <212> DNA
   <213> Artificial Sequence
<220>
   <223> Description of Artificial Sequence; Note = Synthetic Construct
<400> 203
   actaatcctt ttcataagag tcgccggacg cggggttgcg cggcttcgtc c 51
<210> 204
   <211> 51
   <212> DNA
   <213> Artificial Sequence
<220>
   <223> Description of Artificial Sequence; Note = Synthetic Construct
<400> 204
   accgatcaac catgacaagg ccgccggacg cggagtagat tatcttcatc c 51
<210> 205
   <211> 51
   <212> DNA
   <213> Artificial Sequence
<220>
   <223> Description of Artificial Sequence; Note = Synthetic Construct
<400> 205
   gtcgatcaac catgacaagg ccgcgggacg cggggttgcg tatcttcatc c 51
<210> 206
   <211> 51
   <212> DNA
   <213> Artificial Sequence
<220>
   <223> Description of Artificial Sequence; Note = Synthetic Construct
<400> 206
   gtcgatcctt tatgacaagg ccgccggata cggggttgct cgttttcgtc c 51
<210> 207
   <211> 51
   <212> DNA
   <213> Artificial Sequence
<220>
   <223> Description of Artificial Sequence; Note = Synthetic Construct
<400> 207
   atcgatcctt tatataagat ccgccggacg cggggttgcg cggcttcgtc c 51
<210> 208
   <211> 51
   <212> DNA
   <213> Artificial Sequence
<220>
   <223> Description of Artificial Sequence; Note = Synthetic Construct
<400> 208
   atcgatcctt tatataagat ccttcgagtg cggggttgcg cggcttcgtc c 51
<210> 209
   <211> 51
   <212> DNA
   <213> Artificial Sequence
<220>
   <223> Description of Artificial Sequence; Note = Synthetic Construct
<400> 209
   accgatcctt tacgaagggg ccttcgaatg caaagtagat tatcttcatc c 51
<210> 210
   <211> 51
   <212> DNA
   <213> Artificial Sequence
<220>
   <223> Description of Artificial Sequence; Note = Synthetic Construct
<400> 210
   accgatcaac catgacaagg ccgccggacg cgggtctgat catctttact t 51
<210> 211
   <211> 51
   <212> DNA
   <213> Artificial Sequence
<220>
   <223> Description of Artificial Sequence; Note = Synthetic Construct
<400> 211
   accggttctt tatataagat ccgccggatg cagggttgcg cggcttcgtc c 51
<210> 212
   <211> 51
   <212> DNA
   <213> Artificial Sequence
<220>
   <223> Description of Artificial Sequence; Note = Synthetic Construct
<400> 212
   actaatcctt tacgaagggg ccttcgagtg cagggttgcg cggcttcgtc c 51
<210> 213
   <211> 51
   <212> DNA
   <213> Artificial Sequence
<220>
   <223> Description of Artificial Sequence; Note = Synthetic Construct
<400> 213
   atcgatcctt tacgaagggg ccttcgaatg cagggttgcg cggcttcgtc c 51
<210> 214
   <211> 51
   <212> DNA
   <213> Artificial Sequence
<220>
   <223> Description of Artificial Sequence; Note = Synthetic Construct
<400> 214
   gtcgatcctt tatgacaagg ccgccggatg cggggttgcg tatcttcatc c 51
<210> 215
   <211> 51
   <212> DNA
   <213> Artificial Sequence
<220>
   <223> Description of Artificial Sequence; Note = Synthetic Construct
<400> 215
   accgatcctt tatgacaagg ccgccggacg cggggttgcg cggcttcgtc c 51
<210> 216
   <211> 51
   <212> DNA
   <213> Artificial Sequence
<220>
   <223> Description of Artificial Sequence; Note = Synthetic Construct
<400> 216
   accgatcctt tatataagat ccgccggata cggggttgct cgttttcgtc c 51
<210> 217
   <211> 51
   <212> DNA
   <213> Artificial Sequence
<220>
   <223> Description of Artificial Sequence; Note = Synthetic Construct
<400> 217
   actaatcctt tacgaagggg ccttcggacg cggggttgcg cggcttcgtc c 51
<210> 218
   <211> 51
   <212> DNA
   <213> Artificial Sequence
<220>
   <223> Description of Artificial Sequence; Note = Synthetic Construct
<400> 218
   accgatcctt tacgaagggg ccttcggatg tggggttgcg tatcttcatc c 51
<210> 219
   <211> 51
   <212> DNA
   <213> Artificial Sequence
<220>
   <223> Description of Artificial Sequence; Note = Synthetic Construct
<400> 219
   actaatcctt ttcataagag tcgccggaca cggggttgct cgttttcgtc c 51
<210> 220
   <211> 51
   <212> DNA
   <213> Artificial Sequence
<220>
   <223> Description of Artificial Sequence; Note = Synthetic Construct
<400> 220
   accgatcctt ttcataagag tcgccggata cgaagtagat tatcttcatc c 51
<210> 221
   <211> 51
   <212> DNA
   <213> Artificial Sequence
<220>
   <223> Description of Artificial Sequence; Note = Synthetic Construct
<400> 221
   atcgatcaac catgacaagg ccttcgaatg caaagtagat tatcttcatc c 51
<210> 222
   <211> 51
   <212> DNA
   <213> Artificial Sequence
<220>
   <223> Description of Artificial Sequence; Note = Synthetic Construct
<400> 222
   accgatcctt tacgaagggg ccttcgaatg caaagtagat catctttact t 51
<210> 223
   <211> 51
   <212> DNA
   <213> Artificial Sequence
<220>
   <223> Description of Artificial Sequence; Note = Synthetic Construct
<400> 223
   accgatcaac tatgacaagg ccgccggacg cggggttgcg cggcttcgtc c 51
<210> 224
   <211> 51
   <212> DNA
   <213> Artificial Sequence
<220>
   <223> Description of Artificial Sequence; Note = Synthetic Construct
<400> 224
   actagctctt tatataagat ccgccgaatg caaagtagat tatcttcatc c 51
<210> 225
   <211> 51
   <212> DNA
   <213> Artificial Sequence
<220>
   <223> Description of Artificial Sequence; Note = Synthetic Construct
<400> 225
   actaatcctt ttcgtaagag tcgccggata cggggttgct cgttttcgtc c 51
<210> 226
   <211> 51
   <212> DNA
   <213> Artificial Sequence
<220>
   <223> Description of Artificial Sequence; Note = Synthetic Construct
<400> 226
   atcgatcctt ttcataagag tcgccggata cggggttgct cgttttcgtc c 51
<210> 227
   <211> 51
   <212> DNA
   <213> Artificial Sequence
<220>
   <223> Description of Artificial Sequence; Note = Synthetic Construct
<400> 227
   actaatcctt tacgaagggg ccttcggata cggggttgct cgttttcgtc c 51
<210> 228
   <211> 51
   <212> DNA
   <213> Artificial Sequence
<220>
   <223> Description of Artificial Sequence; Note = Synthetic Construct
<400> 228
   atcgatcaac catgacaagg ccgcgggacg cggggttgcg tatcttcatc c 51
<210> 229
   <211> 51
   <212> DNA
   <213> Artificial Sequence
<220>
   <223> Description of Artificial Sequence; Note = Synthetic Construct
<400> 229
   accgatcaac catgacaagg ccgccggacg cggggttgcg cggcttcgtc c 51
<210> 230
   <211> 51
   <212> DNA
   <213> Artificial Sequence
<220>
   <223> Description of Artificial Sequence; Note = Synthetic Construct
<400> 230
   accgatcaac catgacaagg ccgccggacg cggggttgcg tatcttcatc c 51
<210> 231
   <211> 51
   <212> DNA
   <213> Artificial Sequence
<220>
   <223> Description of Artificial Sequence; Note = Synthetic Construct
<400> 231
   actaatcctt ttcataagag tcgccggata cggggttgct cgttttcgtc c 51
<210> 232
   <211> 51
   <212> DNA
   <213> Artificial Sequence
<220>
   <223> Description of Artificial Sequence; Note = Synthetic Construct
<400> 232
   atcgatcctt tacgaagggg ccttcgaatg caaagtagat tatcttcatc c 51
<210> 233
   <211> 51
   <212> DNA
   <213> Artificial Sequence
<220>
   <223> Description of Artificial Sequence; Note = Synthetic Construct
<400> 233
   accggctctt tatataagat ccgccggatg caggtctgat catctttact t 51
<210> 234
   <211> 51
   <212> DNA
   <213> Artificial Sequence
<220>
   <223> Description of Artificial Sequence; Note = Synthetic Construct
<400> 234
   actaatcctt ttcataagag tcgccggata cgaagtagat tatcttcatc c 51
<210> 235
   <211> 51
   <212> DNA
   <213> Artificial Sequence
<220>
   <223> Description of Artificial Sequence; Note = Synthetic Construct
<400> 235
   gtcgatcctt tatgacaagg ccgccggacg cggggttgcg tatcttcatc c 51
<210> 236
   <211> 51
   <212> DNA
   <213> Artificial Sequence
<220>
   <223> Description of Artificial Sequence; Note = Synthetic Construct
<400> 236
   accgatcaac catgacaagg ccgccggacg cgaagtagat tatcttcatc c 51
<210> 237
   <211> 51
   <212> DNA
   <213> Artificial Sequence
<220>
   <223> Description of Artificial Sequence; Note = Synthetic Construct
<400> 237
   atcgatcctt tacgaagggg ccttcgagtg cagggttgcg cggcttcgtc c 51
<210> 238
   <211> 51
   <212> DNA
   <213> Artificial Sequence
<220>
   <223> Description of Artificial Sequence; Note = Synthetic Construct
<400> 238
   atcgatcaac catgacaagg ccgccggacg cggggttgcg cggcttcgtc c 51
<210> 239
   <211> 51
   <212> DNA
   <213> Artificial Sequence
<220>
   <223> Description of Artificial Sequence; Note = Synthetic Construct
<400> 239
   atcgatcctt tacgaagggg ccttcggatg cggggttgcg cggcttcgtc c 51
<210> 240
   <211> 51
   <212> DNA
   <213> Artificial Sequence
<220>
   <223> Description of Artificial Sequence; Note = Synthetic Construct
<400> 240
   atcgatcctt tatataagat ccgccgaatg caaagtagat tatcttcatc c 51
<210> 241
   <211> 51
   <212> DNA
   <213> Artificial Sequence
<220>
   <223> Description of Artificial Sequence; Note = Synthetic Construct
<400> 241
   accgatcaac catgacaagg ctgcctgacg cggggttgcg cggcttcgtc c 51
<210> 242
   <211> 51
   <212> DNA
   <213> Artificial Sequence
<220>
   <223> Description of Artificial Sequence; Note = Synthetic Construct
<400> 242
   accggctctt tatataagat ccgccggatg caaagtagat tatcttcatc c 51
<210> 243
   <211> 51
   <212> DNA
   <213> Artificial Sequence
<220>
   <223> Description of Artificial Sequence; Note = Synthetic Construct
<400> 243
   accggctctt tatataagat ccgccggatg cagggttgcg cggcttcgtc c 51
<210> 244
   <211> 51
   <212> DNA
   <213> Artificial Sequence
<220>
   <223> Description of Artificial Sequence; Note = Synthetic Construct
<400> 244
   atcggctctt tatataagat ccgccggatg caggtctgat catctttact t 51
<210> 245
   <211> 51
   <212> DNA
   <213> Artificial Sequence
<220>
   <223> Description of Artificial Sequence; Note = Synthetic Construct
<400> 245
   accgatcaac catgacaagg ccgccggata cggggttgct cgttttcgtc c 51
<210> 246
   <211> 51
   <212> DNA
   <213> Artificial Sequence
<220>
   <223> Description of Artificial Sequence; Note = Synthetic Construct
<400> 246
   atcgatcctt tacgaagggg ccttcggata cggggttgct cgttttcgtc c 51
<210> 247
   <211> 51
   <212> DNA
   <213> Artificial Sequence
<220>
   <223> Description of Artificial Sequence; Note = Synthetic Construct
<400> 247
   accgatcaac catgacaagg ccgccggatg cggggttgcg cggcttcgtc c 51
<210> 248
   <211> 51
   <212> DNA
   <213> Artificial Sequence
<220>
   <223> Description of Artificial Sequence; Note = Synthetic Construct
<400> 248
   accgatcaac catgacaagg ccgccgaatg caaagtagat tatcttcatc c 51
<210> 249
   <211> 51
   <212> DNA
   <213> Artificial Sequence
<220>
   <223> Description of Artificial Sequence; Note = Synthetic Construct
<400> 249
   atcgatcctt tacgaagggg ccttcggatg tggggttgcg cggcttcgtc c 51
<210> 250
   <211> 51
   <212> DNA
   <213> Artificial Sequence
<220>
   <223> Description of Artificial Sequence; Note = Synthetic Construct
<400> 250
   accgatcaac catgacaagg ccgccggacg cggggttacg cggcttcgtc c 51
<210> 251
   <211> 51
   <212> DNA
   <213> Artificial Sequence
<220>
   <223> Description of Artificial Sequence; Note = Synthetic Construct
<400> 251
   atcgatcaac catgacaagg ccgccggacg cggggttgcg tatcttcatc c 51
<210> 252
   <211> 51
   <212> DNA
   <213> Artificial Sequence
<220>
   <223> Description of Artificial Sequence; Note = Synthetic Construct
<400> 252
   accgatcaac catgacaagg ccgcgggacg cggggttgcg tatcttcatc c 51
<210> 253
   <211> 51
   <212> DNA
   <213> Artificial Sequence
<220>
   <223> Description of Artificial Sequence; Note = Synthetic Construct
<400> 253
   atcgatcctt tacgaagggg ccttcgaatg cagggtagat tatcttcatc c 51
<210> 254
   <211> 51
   <212> DNA
   <213> Artificial Sequence
<220>
   <223> Description of Artificial Sequence; Note = Synthetic Construct
<400> 254
   actaatcctt tacgaagggg ccttcggatg tggggttgcg cggcttcgtc c 51
<210> 255
   <211> 51
   <212> DNA
   <213> Artificial Sequence
<220>
   <223> Description of Artificial Sequence; Note = Synthetic Construct
<400> 255
   actaatcctt ttcataagag tcgccggata cggggttgcg cggcttcgtc c 51
<210> 256
   <211> 51
   <212> DNA
   <213> Artificial Sequence
<220>
   <223> Description of Artificial Sequence; Note = Synthetic Construct
<400> 256
   atcgatcctt tacgaagggg ccttcggacg cggggttgcg tatcttcatc c 51
<210> 257
   <211> 51
   <212> DNA
   <213> Artificial Sequence
<220>
   <223> Description of Artificial Sequence; Note = Synthetic Construct
<400> 257
   gtcgatcaac catgacaagg ccgccggacg cggggttgcg tatcttcatc c 51
<210> 258
   <211> 51
   <212> DNA
   <213> Artificial Sequence
<220>
   <223> Description of Artificial Sequence; Note = Synthetic Construct
<400> 258
   atcgatcctt tatataagat ccgccggata cggggttgct cgttttcgtc c 51
<210> 259
   <211> 51
   <212> DNA
   <213> Artificial Sequence
<220>
   <223> Description of Artificial Sequence; Note = Synthetic Construct
<400> 259
   accgatcaac catgacaagg ccgccggacg cggggttgcg cggcatcgtc c 51
<210> 260
   <211> 51
   <212> DNA
   <213> Artificial Sequence
<220>
   <223> Description of Artificial Sequence; Note = Synthetic Construct
<400> 260
   atcgatcctt tatataagat ccgccgaatg caaagtagat tatctccatc c 51
<210> 261
   <211> 51
   <212> DNA
   <213> Artificial Sequence
<220>
   <223> Description of Artificial Sequence; Note = Synthetic Construct
<400> 261
   accgatcctt tacgaagggg ccttcgaatg caaagtagat tatcttcatc c 51
<210> 262
   <211> 51
   <212> DNA
   <213> Artificial Sequence
<220>
   <223> Description of Artificial Sequence; Note = Synthetic Construct
<400> 262
   atcgatcctt tatataagat ccgccgaatg caaagttgcg cggcttcgtc c 51
<210> 263
   <211> 51
   <212> DNA
   <213> Artificial Sequence
<220>
   <223> Description of Artificial Sequence; Note = Synthetic Construct
<400> 263
   gtcgatcaac catgacaagg ccgcgggacg cggggttgcg tatcttcatc c 51
<210> 264
   <211> 51
   <212> DNA
   <213> Artificial Sequence
<220>
   <223> Description of Artificial Sequence; Note = Synthetic Construct
<400> 264
   accgatcaac catgacaagg ccgccggacg cggggttgct cgttttcgtc c 51
<210> 265
   <211> 51
   <212> DNA
   <213> Artificial Sequence
<220>
   <223> Description of Artificial Sequence; Note = Synthetic Construct
<400> 265
   actaatcctt ttcataagag tcgccggatg cggggttgcg tatcttcatc c 51
<210> 266
   <211> 51
   <212> DNA
   <213> Artificial Sequence
<220>
   <223> Description of Artificial Sequence; Note = Synthetic Construct
<400> 266
   actaactctt tacataagag tcgccggata cggggttgct cgttttcgtc c 51
<210> 267
   <211> 51
   <212> DNA
   <213> Artificial Sequence
<220>
   <223> Description of Artificial Sequence; Note = Synthetic Construct
<400> 267
   accgatcctt tatgacaagg ccgccggacg cggggttgcg tatcttcatc c 51
<210> 268
   <211> 51
   <212> DNA
   <213> Artificial Sequence
<220>
   <223> Description of Artificial Sequence; Note = Synthetic Construct
<400> 268
   atcggctctt tatataagat ccgccggatg cagggttgcg cggcttcgtc c 51
<210> 269
   <211> 51
   <212> DNA
   <213> Artificial Sequence
<220>
   <223> Description of Artificial Sequence; Note = Synthetic Construct
<400> 269
   actaatcctt ttcataagag tcgccggata cggggttgcg tatcttcatc c 51
<210> 270
   <211> 51
   <212> DNA
   <213> Artificial Sequence
<220>
   <223> Description of Artificial Sequence; Note = Synthetic Construct
<400> 270
   actagctctt tatataagat ccgccgaatg caaagtagat tatcttcatc c 51
<210> 271
   <211> 51
   <212> DNA
   <213> Artificial Sequence
<220>
   <223> Description of Artificial Sequence; Note = Synthetic Construct
<400> 271
   actaatcctt ttcataagag tcgccggata cggggttgct cggcttcgtc c 51
<210> 272
   <211> 51
   <212> DNA
   <213> Artificial Sequence
<220>
   <223> Description of Artificial Sequence; Note = Synthetic Construct
<400> 272
   gccgatcaac catgacaagg ccgccggacg cggggttgcg cggcttcgtc c 51
<210> 273
   <211> 51
   <212> DNA
   <213> Artificial Sequence
<220>
   <223> Description of Artificial Sequence; Note = Synthetic Construct
<400> 273
   accgatcctt ttcataagag tcgccggata cggggttgct cgttttcgtc c 51
<210> 274
   <211> 51
   <212> DNA
   <213> Artificial Sequence
<220>
   <223> Description of Artificial Sequence; Note = Synthetic Construct
<400> 274
   atcgatcctt tacgaagggg ccttcggatg tggggttgcg tatcttcatc c 51
<210> 275
   <211> 51
   <212> DNA
   <213> Artificial Sequence
<220>
   <223> Description of Artificial Sequence; Note = Synthetic Construct
<400> 275
   atcgatcaac catgacaagg ccttcgaatg caaagtagat tatcttcatc c 51
<210> 276
   <211> 51
   <212> DNA
   <213> Artificial Sequence
<220>
   <223> Description of Artificial Sequence; Note = Synthetic Construct
<400> 276
   atcgatcctt tatataagat ccttcgagtg cggggttgcg cggcttcgtc c 51
<210> 277
   <211> 51
   <212> DNA
   <213> Artificial Sequence
<220>
   <223> Description of Artificial Sequence; Note = Synthetic Construct
<400> 277
   actaatcaac catgacaagg ccgccggacg cggggttgcg cggcttcgtc c 51
<210> 278
   <211> 51
   <212> DNA
   <213> Artificial Sequence
<220>
   <223> Description of Artificial Sequence; Note = Synthetic Construct
<400> 278
   accgatcaac catgacaagg ccgccggacg cgggtctgat catctttact t 51
<210> 279
   <211> 51
   <212> DNA
   <213> Artificial Sequence
<220>
   <223> Description of Artificial Sequence; Note = Synthetic Construct
<400> 279
   gtcgatcctt tatgacaagg ccgccggata cggggttgct cgttttcgtc c 51
<210> 280
   <211> 51
   <212> DNA
   <213> Artificial Sequence
<220>
   <223> Description of Artificial Sequence; Note = Synthetic Construct
<400> 280
   gccgatcaac catgacaagg ccgccggacg cggggttgcg tatcttcatc c 51
<210> 281
   <211> 51
   <212> DNA
   <213> Artificial Sequence
<220>
   <223> Description of Artificial Sequence; Note = Synthetic Construct
<400> 281
   actaatcctt ttcataagag tcgccggacg cggggttgcg cggcttcgtc c 51
<210> 282
   <211> 51
   <212> DNA
   <213> Artificial Sequence
<220>
   <223> Description of Artificial Sequence; Note = Synthetic Construct
<400> 282
   accgatcctt tacgaagggg ccttcggatg tggggttgcg cggcttcgtc c 51
<210> 283
   <211> 51
   <212> DNA
   <213> Artificial Sequence
<220>
   <223> Description of Artificial Sequence; Note = Synthetic Construct
<400> 283
   atcgatcctt tatataagat ccgccgaatg cagggttgcg tatcttcatc c 51
<210> 284
   <211> 51
   <212> DNA
   <213> Artificial Sequence
<220>
   <223> Description of Artificial Sequence; Note = Synthetic Construct
<400> 284
   atcgatcctt ttcataagag tcgccggata cggggttgct cgttttcgtc c 51
<210> 285
   <211> 22
   <212> DNA
   <213> Artificial Sequence
<220>
   <223> Description of Artificial Sequence; Note = Synthetic Construct
<400> 285
   tctgctcctg ctcttctttt cc 22
<210> 286
   <211> 25
   <212> DNA
   <213> Artificial Sequence
<220>
   <223> Description of Artificial Sequence; Note = Synthetic Construct
<400> 286
   ctctatgctt gctacaagag acaca 25
<210> 287
   <211> 16
   <212> DNA
   <213> Artificial Sequence
<220>
   <223> Description of Artificial Sequence; Note = Synthetic Construct
<400> 287
   aacagcatgg aatatc 16
<210> 288
   <211> 17
   <212> DNA
   <213> Artificial Sequence
<220>
   <223> Description of Artificial Sequence; Note = Synthetic Construct
<400> 288
   cgatccacgt cgcggtc 17
<210> 289
   <211> 17
   <212> DNA
   <213> Artificial Sequence
<220>
   <223> Description of Artificial Sequence; Note = Synthetic Construct
<400> 289
   cgatccacgt cgtattc 17
<210> 290
   <211> 17
   <212> DNA
   <213> Artificial Sequence
<220>
   <223> Description of Artificial Sequence; Note = Synthetic Construct
<400> 290
   cacctcatgt ctcgttc 17
<210> 291
   <211> 17
   <212> DNA
   <213> Artificial Sequence
<220>
   <223> Description of Artificial Sequence; Note = Synthetic Construct
<400> 291
   tgccctatat attattc 17
<210> 292
   <211> 17
   <212> DNA
   <213> Artificial Sequence
<220>
   <223> Description of Artificial Sequence; Note = Synthetic Construct
<400> 292
   cgctccatac atcattt 17
<210> 293
   <211> 17
   <212> DNA
   <213> Artificial Sequence
<220>
   <223> Description of Artificial Sequence; Note = Synthetic Construct
<400> 293
   cacctcatgt attattc 17
<210> 294
   <211> 17
   <212> DNA
   <213> Artificial Sequence
<220>
   <223> Description of Artificial Sequence; Note = Synthetic Construct
<400> 294
   tgctccacgt cgtattc 17
<210> 295
   <211> 17
   <212> DNA
   <213> Artificial Sequence
<220>
   <223> Description of Artificial Sequence; Note = Synthetic Construct
<400> 295
   cgatccacgt attattc 17
<210> 296
   <211> 17
   <212> DNA
   <213> Artificial Sequence
<220>
   <223> Description of Artificial Sequence; Note = Synthetic Construct
<400> 296
   tgccctgtat cgcggtc 17
<210> 297
   <211> 17
   <212> DNA
   <213> Artificial Sequence
<220>
   <223> Description of Artificial Sequence; Note = Synthetic Construct
<400> 297
   tgatccacgt cgcggtc 17
<210> 298
   <211> 17
   <212> DNA
   <213> Artificial Sequence
<220>
   <223> Description of Artificial Sequence; Note = Synthetic Construct
<400> 298
   tgccctatgt cgcggtc 17
<210> 299
   <211> 17
   <212> DNA
   <213> Artificial Sequence
<220>
   <223> Description of Artificial Sequence; Note = Synthetic Construct
<400> 299
   tgctccatat attattc 17
<210> 300
   <211> 17
   <212> DNA
   <213> Artificial Sequence
<220>
   <223> Description of Artificial Sequence; Note = Synthetic Construct
<400> 300
   cgatccacgt cgcggtc 17
<210> 301
   <211> 17
   <212> DNA
   <213> Artificial Sequence
<220>
   <223> Description of Artificial Sequence; Note = Synthetic Construct
<400> 301
   cgctccatat attattc 17
<210> 302
   <211> 17
   <212> DNA
   <213> Artificial Sequence
<220>
   <223> Description of Artificial Sequence; Note = Synthetic Construct
<400> 302
   cgctccatat cgcggtc 17
<210> 303
   <211> 17
   <212> DNA
   <213> Artificial Sequence
<220>
   <223> Description of Artificial Sequence; Note = Synthetic Construct
<400> 303
   tgctccatac atcattt 17
<210> 304
   <211> 17
   <212> DNA
   <213> Artificial Sequence
<220>
   <223> Description of Artificial Sequence; Note = Synthetic Construct
<400> 304
   cgatccatgt ctcgttc 17
<210> 305
   <211> 17
   <212> DNA
   <213> Artificial Sequence
<220>
   <223> Description of Artificial Sequence; Note = Synthetic Construct
<400> 305
   tgccctatgt ctcgttc 17
<210> 306
   <211> 17
   <212> DNA
   <213> Artificial Sequence
<220>
   <223> Description of Artificial Sequence; Note = Synthetic Construct
<400> 306
   cgatccatgt cgcggtc 17
<210> 307
   <211> 17
   <212> DNA
   <213> Artificial Sequence
<220>
   <223> Description of Artificial Sequence; Note = Synthetic Construct
<400> 307
   cgatccatat attattc 17
<210> 308
   <211> 17
   <212> DNA
   <213> Artificial Sequence
<220>
   <223> Description of Artificial Sequence; Note = Synthetic Construct
<400> 308
   tgccctatgt cgcggtc 17
<210> 309
   <211> 17
   <212> DNA
   <213> Artificial Sequence
<220>
   <223> Description of Artificial Sequence; Note = Synthetic Construct
<400> 309
   cgatccacgt cgcggtc 17
<210> 310
   <211> 17
   <212> DNA
   <213> Artificial Sequence
<220>
   <223> Description of Artificial Sequence; Note = Synthetic Construct
<400> 310
   tgatccacgt cgtattc 17
<210> 311
   <211> 17
   <212> DNA
   <213> Artificial Sequence
<220>
   <223> Description of Artificial Sequence; Note = Synthetic Construct
<400> 311
   cgatccacgt cgtattc 17
<210> 312
   <211> 17
   <212> DNA
   <213> Artificial Sequence
<220>
   <223> Description of Artificial Sequence; Note = Synthetic Construct
<400> 312
   tgccctatat attattc 17
<210> 313
   <211> 17
   <212> DNA
   <213> Artificial Sequence
<220>
   <223> Description of Artificial Sequence; Note = Synthetic Construct
<400> 313
   caccctatgt cgcggtc 17
<210> 314
   <211> 17
   <212> DNA
   <213> Artificial Sequence
<220>
   <223> Description of Artificial Sequence; Note = Synthetic Construct
<400> 314
   cacctcatgt cgcggtc 17
<210> 315
   <211> 17
   <212> DNA
   <213> Artificial Sequence
<220>
   <223> Description of Artificial Sequence; Note = Synthetic Construct
<400> 315
   tgccctacgt cgtattc 17
<210> 316
   <211> 17
   <212> DNA
   <213> Artificial Sequence
<220>
   <223> Description of Artificial Sequence; Note = Synthetic Construct
<400> 316
   tgatccacgt cgtattc 17
<210> 317
   <211> 17
   <212> DNA
   <213> Artificial Sequence
<220>
   <223> Description of Artificial Sequence; Note = Synthetic Construct
<400> 317
   tgctccatgt ctcgttc 17
<210> 318
   <211> 17
   <212> DNA
   <213> Artificial Sequence
<220>
   <223> Description of Artificial Sequence; Note = Synthetic Construct
<400> 318
   cgatccacgt cgcggtc 17
<210> 319
   <211> 17
   <212> DNA
   <213> Artificial Sequence
<220>
   <223> Description of Artificial Sequence; Note = Synthetic Construct
<400> 319
   tgctccatat attatcc 17
<210> 320
   <211> 17
   <212> DNA
   <213> Artificial Sequence
<220>
   <223> Description of Artificial Sequence; Note = Synthetic Construct
<400> 320
   cgccctatat attattc 17
<210> 321
   <211> 17
   <212> DNA
   <213> Artificial Sequence
<220>
   <223> Description of Artificial Sequence; Note = Synthetic Construct
<400> 321
   tgctccatat cgcggtc 17
<210> 322
   <211> 17
   <212> DNA
   <213> Artificial Sequence
<220>
   <223> Description of Artificial Sequence; Note = Synthetic Construct
<400> 322
   tgatccacgt cgtattc 17
<210> 323
   <211> 17
   <212> DNA
   <213> Artificial Sequence
<220>
   <223> Description of Artificial Sequence; Note = Synthetic Construct
<400> 323
   cgatccacgt ctcgttc 17
<210> 324
   <211> 17
   <212> DNA
   <213> Artificial Sequence
<220>
   <223> Description of Artificial Sequence; Note = Synthetic Construct
<400> 324
   cacctcatgt cgtattc 17
<210> 325
   <211> 17
   <212> DNA
   <213> Artificial Sequence
<220>
   <223> Description of Artificial Sequence; Note = Synthetic Construct
<400> 325
   cacctcatgt ctcgttc 17
<210> 326
   <211> 17
   <212> DNA
   <213> Artificial Sequence
<220>
   <223> Description of Artificial Sequence; Note = Synthetic Construct
<400> 326
   cgctccacgt cgtattc 17
<210> 327
   <211> 17
   <212> DNA
   <213> Artificial Sequence
<220>
   <223> Description of Artificial Sequence; Note = Synthetic Construct
<400> 327
   tgctccatat cgcggtc 17
<210> 328
   <211> 17
   <212> DNA
   <213> Artificial Sequence
<220>
   <223> Description of Artificial Sequence; Note = Synthetic Construct
<400> 328
   cacctcatgt cgtattc 17
<210> 329
   <211> 17
   <212> DNA
   <213> Artificial Sequence
<220>
   <223> Description of Artificial Sequence; Note = Synthetic Construct
<400> 329
   cactccatat attattc 17
<210> 330
   <211> 17
   <212> DNA
   <213> Artificial Sequence
<220>
   <223> Description of Artificial Sequence; Note = Synthetic Construct
<400> 330
   cacctcatgt ctcggtc 17
<210> 331
   <211> 17
   <212> DNA
   <213> Artificial Sequence
<220>
   <223> Description of Artificial Sequence; Note = Synthetic Construct
<400> 331
   cgatccacgt cgcggtc 17
<210> 332
   <211> 17
   <212> DNA
   <213> Artificial Sequence
<220>
   <223> Description of Artificial Sequence; Note = Synthetic Construct
<400> 332
   cgcctcatgt ctcgttc 17
<210> 333
   <211> 17
   <212> DNA
   <213> Artificial Sequence
<220>
   <223> Description of Artificial Sequence; Note = Synthetic Construct
<400> 333
   tgccctatgt cgtattc 17
<210> 334
   <211> 17
   <212> DNA
   <213> Artificial Sequence
<220>
   <223> Description of Artificial Sequence; Note = Synthetic Construct
<400> 334
   tgatctatat attattc 17
<210> 335
   <211> 17
   <212> DNA
   <213> Artificial Sequence
<220>
   <223> Description of Artificial Sequence; Note = Synthetic Construct
<400> 335
   tgctctgtgt cgcggtc 17
<210> 336
   <211> 17
   <212> DNA
   <213> Artificial Sequence
<220>
   <223> Description of Artificial Sequence; Note = Synthetic Construct
<400> 336
   caatccacgt cgcggtc 17
<210> 337
   <211> 17
   <212> DNA
   <213> Artificial Sequence
<220>
   <223> Description of Artificial Sequence; Note = Synthetic Construct
<400> 337
   cgatccacgc atcattt 17
<210> 338
   <211> 17
   <212> DNA
   <213> Artificial Sequence
<220>
   <223> Description of Artificial Sequence; Note = Synthetic Construct
<400> 338
   tgctccatgt ctcgttc 17
<210> 339
   <211> 17
   <212> DNA
   <213> Artificial Sequence
<220>
   <223> Description of Artificial Sequence; Note = Synthetic Construct
<400> 339
   cgatccacgt cgtattc 17
<210> 340
   <211> 17
   <212> DNA
   <213> Artificial Sequence
<220>
   <223> Description of Artificial Sequence; Note = Synthetic Construct
<400> 340
   cacctcacgt cgcggtc 17
<210> 341
   <211> 17
   <212> DNA
   <213> Artificial Sequence
<220>
   <223> Description of Artificial Sequence; Note = Synthetic Construct
<400> 341
   cgccctatgt cgcggtc 17
<210> 342
   <211> 17
   <212> DNA
   <213> Artificial Sequence
<220>
   <223> Description of Artificial Sequence; Note = Synthetic Construct
<400> 342
   tgctccatat cgtattc 17
<210> 343
   <211> 17
   <212> DNA
   <213> Artificial Sequence
<220>
   <223> Description of Artificial Sequence; Note = Synthetic Construct
<400> 343
   tgcctcatgt ctcgttc 17
<210> 344
   <211> 17
   <212> DNA
   <213> Artificial Sequence
<220>
   <223> Description of Artificial Sequence; Note = Synthetic Construct
<400> 344
   cgatccacgt cgcggtc 17
<210> 345
   <211> 17
   <212> DNA
   <213> Artificial Sequence
<220>
   <223> Description of Artificial Sequence; Note = Synthetic Construct
<400> 345
   cgatccacgt cgcggtc 17
<210> 346
   <211> 17
   <212> DNA
   <213> Artificial Sequence
<220>
   <223> Description of Artificial Sequence; Note = Synthetic Construct
<400> 346
   cacctcatgt ctcgttc 17
<210> 347
   <211> 17
   <212> DNA
   <213> Artificial Sequence
<220>
   <223> Description of Artificial Sequence; Note = Synthetic Construct
<400> 347
   tgccctatat attattc 17
<210> 348
   <211> 17
   <212> DNA
   <213> Artificial Sequence
<220>
   <223> Description of Artificial Sequence; Note = Synthetic Construct
<400> 348
   cgatccacgt cgtattc 17
<210> 349
   <211> 17
   <212> DNA
   <213> Artificial Sequence
<220>
   <223> Description of Artificial Sequence; Note = Synthetic Construct
<400> 349
   cacctcatgt attattc 17
<210> 350
   <211> 17
   <212> DNA
   <213> Artificial Sequence
<220>
   <223> Description of Artificial Sequence; Note = Synthetic Construct
<400> 350
   cgctccatac atcattt 17
<210> 351
   <211> 17
   <212> DNA
   <213> Artificial Sequence
<220>
   <223> Description of Artificial Sequence; Note = Synthetic Construct
<400> 351
   cacctcatgt ctcgttc 17
<210> 352
   <211> 17
   <212> DNA
   <213> Artificial Sequence
<220>
   <223> Description of Artificial Sequence; Note = Synthetic Construct
<400> 352
   tgctccacgt cgtattc 17
<210> 353
   <211> 17
   <212> DNA
   <213> Artificial Sequence
<220>
   <223> Description of Artificial Sequence; Note = Synthetic Construct
<400> 353
   tgccctgtat cgcggtc 17
<210> 354
   <211> 17
   <212> DNA
   <213> Artificial Sequence
<220>
   <223> Description of Artificial Sequence; Note = Synthetic Construct
<400> 354
   tgccctatgt cgcggtc 17
<210> 355
   <211> 17
   <212> DNA
   <213> Artificial Sequence
<220>
   <223> Description of Artificial Sequence; Note = Synthetic Construct
<400> 355
   cgatccacgt cgtattc 17
<210> 356
   <211> 17
   <212> DNA
   <213> Artificial Sequence
<220>
   <223> Description of Artificial Sequence; Note = Synthetic Construct
<400> 356
   cgctccatac atcattt 17
<210> 357
   <211> 17
   <212> DNA
   <213> Artificial Sequence
<220>
   <223> Description of Artificial Sequence; Note = Synthetic Construct
<400> 357
   tgctccatat attattc 17
<210> 358
   <211> 17
   <212> DNA
   <213> Artificial Sequence
<220>
   <223> Description of Artificial Sequence; Note = Synthetic Construct
<400> 358
   cgctccatat attattc 17
<210> 359
   <211> 17
   <212> DNA
   <213> Artificial Sequence
<220>
   <223> Description of Artificial Sequence; Note = Synthetic Construct
<400> 359
   cgatccacgt attattc 17
<210> 360
   <211> 17
   <212> DNA
   <213> Artificial Sequence
<220>
   <223> Description of Artificial Sequence; Note = Synthetic Construct
<400> 360
   tgctccatat cgcggtc 17
<210> 361
   <211> 17
   <212> DNA
   <213> Artificial Sequence
<220>
   <223> Description of Artificial Sequence; Note = Synthetic Construct
<400> 361
   cgctccatat cgcggtc 17
<210> 362
   <211> 17
   <212> DNA
   <213> Artificial Sequence
<220>
   <223> Description of Artificial Sequence; Note = Synthetic Construct
<400> 362
   cgatccacgt cgtattc 17
<210> 363
   <211> 17
   <212> DNA
   <213> Artificial Sequence
<220>
   <223> Description of Artificial Sequence; Note = Synthetic Construct
<400> 363
   cgatccatat attattc 17
<210> 364
   <211> 17
   <212> DNA
   <213> Artificial Sequence
<220>
   <223> Description of Artificial Sequence; Note = Synthetic Construct
<400> 364
   cgatccatgt cgcggtc 17
<210> 365
   <211> 17
   <212> DNA
   <213> Artificial Sequence
<220>
   <223> Description of Artificial Sequence; Note = Synthetic Construct
<400> 365
   cacctcatgt ctcggtc 17
<210> 366
   <211> 17
   <212> DNA
   <213> Artificial Sequence
<220>
   <223> Description of Artificial Sequence; Note = Synthetic Construct
<400> 366
   cgatccatgt ctcgttc 17
<210> 367
   <211> 17
   <212> DNA
   <213> Artificial Sequence
<220>
   <223> Description of Artificial Sequence; Note = Synthetic Construct
<400> 367
   cgatccatgt ctcgttc 17
<210> 368
   <211> 17
   <212> DNA
   <213> Artificial Sequence
<220>
   <223> Description of Artificial Sequence; Note = Synthetic Construct
<400> 368
   cacctcatgt cgtattc 17
<210> 369
   <211> 17
   <212> DNA
   <213> Artificial Sequence
<220>
   <223> Description of Artificial Sequence; Note = Synthetic Construct
<400> 369
   cgctccacgt cgtattc 17
<210> 370
   <211> 17
   <212> DNA
   <213> Artificial Sequence
<220>
   <223> Description of Artificial Sequence; Note = Synthetic Construct
<400> 370
   tgccctatat attattc 17
<210> 371
   <211> 17
   <212> DNA
   <213> Artificial Sequence
<220>
   <223> Description of Artificial Sequence; Note = Synthetic Construct
<400> 371
   cgctccatat cgcggtc 17
<210> 372
   <211> 17
   <212> DNA
   <213> Artificial Sequence
<220>
   <223> Description of Artificial Sequence; Note = Synthetic Construct
<400> 372
   tgatccacgt cgcggtc 17
<210> 373
   <211> 17
   <212> DNA
   <213> Artificial Sequence
<220>
   <223> Description of Artificial Sequence; Note = Synthetic Construct
<400> 373
   tgatccacgt cgcggtc 17
<210> 374
   <211> 17
   <212> DNA
   <213> Artificial Sequence
<220>
   <223> Description of Artificial Sequence; Note = Synthetic Construct
<400> 374
   cgctccatgt cgcggtc 17
<210> 375
   <211> 17
   <212> DNA
   <213> Artificial Sequence
<220>
   <223> Description of Artificial Sequence; Note = Synthetic Construct
<400> 375
   tgctccatat attatcc 17
<210> 376
   <211> 17
   <212> DNA
   <213> Artificial Sequence
<220>
   <223> Description of Artificial Sequence; Note = Synthetic Construct
<400> 376
   cgctccatac atcattt 17

## Description

### FIELD OF THE INVENTION

The invention relates generally to the diagnosis and treatment of complement-mediated disease in a human subject. Specifically, the invention relates to the prediction or diagnosis of age-related macular degeneration through the detection of a collection of polymorphisms and haplotypes comprised of multiple variations in the CFH-to-F13B locus.

### BACKGROUND

Age related macular degeneration (AMD), a complement-mediated disease, is the leading cause of blindness in the elderly, a problem that will grow worse with an aging population. Accurate identification of risk for developing complement mediated diseases such as AMD is essential for both starting treatment early in disease progression and identifying causal genetic variants to better target therapeutic strategies and development. Previously, loci have been implicated in risk for AMD on Chr1, Chr10, Chr6 (CFB), and Chr19 (C3).

The RCA gene cluster, sometimes referred to below as the "CFH-to-F13B locus," is located on chromosome 1q32, and includes the genes for complement factor H (CFH), five Factor H-related genes (CFHR1, CFHR2, CFHR3, CFHR4 and CFHR5), and the gene encoding the beta subunit of coagulation factor XIII. See US Pat. Pub. US 2007/0020647). CFH is a significant regulator of complement activity and is thought to be essential to prevent injury to self-tissues by inappropriate C3 activation. Additionally, alterations in CFH activity have been associated with altered binding to membrane bound glycoproteins, other complement inhibitors, and the surface of pathogenic bacteria.

Previously, haplotype studies have focused on CFH and only rarely extended as far as CFHR2. However, the full CFH locus is an area of the genome that contains additional variants that associate with complement-mediated diseases such as AMD. Therefore, what is needed are more effective methods of describing risk for having or developing complement-mediated disease at this locus. Additionally, what is needed are methods to place known risk and protective variants into the broader genetic background in which they exist, that is the entire CHF-to-F13B locus.

International patent application WO 2006/096561 A2 discloses a haplotype strongly associated with AMD, as well as a coding change in LOC387715 as the second major AMD susceptibility allele. Baatz, H. et al., Klinische Monatsblätter für Augenheilkunde (2009) 226, 8, 654-658, provide an analysis of SNPs associated with AMD. Huges, A.E., et al, Nature Genetics (2006) 38, 10, 1173-1177, disclose a CFH haplotype associated with decreased risk of AMD. International patent application WO 2008/008986 A2 discloses that persons carrying a deletion of the CFHR-1 and/or CFHR-3 gene are at reduced risk of developing AMD. Zareparsi, S., American Journal of Human Genetics (2005) 77, 149-153, discloses that a CFH substitution is strongly associated with susceptibility to AMD.

### SUMMARY

The present invention relates to an in vitro method of (i) determining a Caucasian subject's susceptibility to having or developing age-related macular degeneration, of (ii) identifying a Caucasian subject in need of treatment for age-related macular degeneration, or of (iii) identifying a Caucasian subject in need of a prophylactic treatment for age-related macular degeneration. The method is defined in independent claim 1.
The present invention furthermore relates to an in vitro method of identifying a Caucasian subject appropriate for an age-related macular degeneration clinical trial. The method is defined in independent claim 2.
The present invention furthermore relates to the use of the haplotype H2 62 A, or a complement thereof, in a method of identifying a Caucasian subject appropriate for an age-related macular degeneration clinical trial. The use is defined in independent claim 3.

Described herein are methods for determining a Caucasian subject's susceptibility to having or developing a complement-mediated disease comprising determining in the Caucasian subject the identity of one or more haplotypes, wherein the one or more haplotypes are H1_62_A, H2_62A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, or a complement thereof, and wherein the presence of one or more of the haplotypes indicates the subject's susceptibility for having or developing a complement-mediated disease.

Also described herein are methods for determining a Caucasian subject's susceptibility to having or developing a complement-mediated disease comprising determining in the Caucasian subject the identity of one or more haplotypes, wherein the one or more haplotypes are H1_51_A, H2_51_A, H3_51_A, H4_51_A, H5_51_A, H6_51_A, H7_51_A, H8_51_A, H9_51_A, H10_51_A, H11_51_A, H12_51_A, H13_51_A, H14_51_A, H15_51_A, H16_51_A, H17_51_A, or a complement thereof, and wherein the presence of one or more of the haplotypes indicates the subject's susceptibility for having or developing a complement-mediated disease.

Also described herein are methods for determining a Caucasian subject's susceptibility to having or developing a complement-mediated disease comprising determining in the Caucasian subject the identity of one or more haplotypes, wherein the one or more haplotypes are H1_51_A, H2_51_A, H3_51_A, H4_51_A, H5_51_A, H6_51_A, H7_51_A, H8_51_A, H9_51_A, H10_51_A, H11_51_A, H12_51_A, H13_51_A, H14_51_A, H15_51_A, H16_51_A, H17_51_A, or a complement thereof, and wherein the presence of one or more of the haplotypes indicates the subject's susceptibility for having or developing a complement-mediated disease.

Also described herein are methods for determining a Caucasian subject's susceptibility to having or developing a complement-mediated disease comprising determining in the Caucasian subject the identity of at least six SNPs in the CFH-to-F13B locus, wherein the SNPs are: (i) rs35928059, rs800292, rs1061170, rs12144939, rs7546940, rs1409153, rs10922153, or rs698859, or (ii) a SNP in linkage disequilibrium with the SNPs of (i), and wherein the presence of at least six of the SNPs indicates the subject's susceptibility for having or developing a complement-mediated disease.

Also described herein are methods for determining a Caucasian subject's susceptibility to having or developing a complement-mediated disease comprising determining in the subject the identity of one or more SNPs in the CFH-to-F13B locus, wherein the SNPs is: (i) rs1061170, rs1410996, rs2274700, rs3753395, rs403846, or rs3753396 or (ii) a SNP in linkage disequilibrium with the SNPs of (i), and wherein the presence of one or more of the SNPs indicates the subject's susceptibility for having or developing a complement-mediated disease.

Also described herein are methods for determining a Caucasian subject's susceptibility to having or developing a complement-mediated disease comprising determining in the subject the identity of a deletion tagging SNP in the CFH-to-F13B locus, wherein the SNP is: (i) rs12144939 or (ii) a SNP in linkage disequilibrium with rs12144939, and wherein the presence of the SNP indicates the subject's susceptibility for having or developing a complement-mediated disease.

Also described herein are methods of identifying a Caucasian subject in need of treatment for a complement-mediated disease comprising determining in the Caucasian subject the identity of one or more haplotypes, wherein the one or more haplotypes are H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, or a complement thereof, and wherein the presence of one or more of the haplotypes indicates whether the subject is in need of treatment for a complement-mediated disease.

Also described herein are methods of identifying a Caucasian subject in need of treatment for a complement-mediated disease comprising determining in the Caucasian subject the identity of one or more haplotypes, wherein the one or more haplotypes are H1_51_A, H2_51_A, H3_51_A, H4_51_A, H5_51_A, H6_51_A, H7_51_A, H8_51_A, H9_51_A, H10_51_A, H11_51_A, H12_51_A, H13_51_A, H14_51_A, H15_51_A, H16_51_A, H17_51_A, or a complement thereof, and wherein the presence of one or more of the haplotypes indicates whether the subject is in need of treatment for a complement-mediated disease.

Also described herein are methods of identifying a Caucasian subject in need of treatment for a complement-mediated disease comprising determining in the Caucasian subject the identity of one or more haplotypes, wherein the one or more haplotypes are H1_51_A, H2_51_A, H3_51_A, H4_51_A, H5_51_A, H6_51_A, H7_51_A, H8_51_A, H9_51_A, H10_51_A, H11_51_A, H12_51_A, H13_51_A, H14_51_A, H15_51_A, H16_51_A, H17_51_A, or a complement thereof, and wherein the presence of one or more of the haplotypes indicates whether the subject is in need of treatment for a complement-mediated disease.

Also described herein are methods of identifying a Caucasian subject in need of treatment for a complement-mediated disease comprising determining in the Caucasian subject the identity of at least six SNPs in the CFH-to-F13B locus, wherein the SNPs are: (i) rs35928059, rs800292, rs1061170, rs12144939, rs7546940, rs1409153, rs10922153, or rs698859, or (ii) a SNP in linkage disequilibrium with the SNPs of (i), and wherein the presence of at least six of the SNPs indicates whether the subject is in need of treatment for a complement-mediated disease.

Also described herein are methods of identifying a Caucasian subject in need of treatment for a complement-mediated disease comprising determining in the subject the identity of one or more SNPs in the CFH-to-F13B locus, wherein the SNPs is: (i) rs1061170, rs1410996, rs2274700, rs3753395, rs403846, or rs3753396 or (ii) a SNP in linkage disequilibrium with the SNPs of (i), and wherein the presence of one or more of the SNPs indicates whether the subject is in need of treatment for a complement-mediated disease.

Also described herein are methods of identifying a Caucasian subject in need of treatment for a complement-mediated disease comprising determining in the subject the identity of a deletion tagging SNP in the CFH-to-F13B locus, wherein the SNPs are: (i) rs12144939 or (ii) a SNP in linkage disequilibrium with rs12144939, and wherein the presence of one or more of the SNPs indicates whether the subject is in need of treatment for a complement-mediated disease.

Also described herein are methods of identifying a Caucasian subject in need of a prophylactic treatment for a complement-mediated disease comprising determining in the Caucasian subject the identity of one or more haplotypes, wherein the one or more haplotypes are H1_62_A, H2_62_A, H3_62_A, H4_62_A, H5_62_A , H6_62_A, H7_62_A, H8_62_A, H9_62_A, H10_62_A, H11_62_A, H12_62_A, H13_62_A, H14_62_A, H15_62_A, or a complement thereof, and wherein the presence of one or more of the haplotypes indicates whether the subject is in need of prophylactic treatment for a complement-mediated disease.

Also described herein are methods of identifying a Caucasian subject in need of a prophylactic treatment for a complement-mediated disease comprising determining in the Caucasian subject the identity of one or more haplotypes, wherein the one or more haplotypes are H1_51_A, H2_51_A, H3_51_A, H4_51_A, H5_51_A, H6_51_A, H7_51_A, H8_51_A, H9_51_A, H10_51_A, H11_51_A, H12_51_A, H13_51_A, H14_51_A, H15_51_A, H16_51_A, H17_51_A, or a complement thereof, and wherein the presence of one or more of the haplotypes indicates whether the subject is in need of prophylactic treatment for a complement-mediated disease.

Also described herein are methods of identifying a Caucasian subject in need of a prophylactic treatment for a complement-mediated disease comprising determining in the Caucasian subject the identity of one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, and wherein the presence of one or more of the haplotypes indicates whether the subject is in need of prophylactic treatment for a complement-mediated disease.

Also described herein are methods of identifying a Caucasian subject in need of a prophylactic treatment for a complement-mediated disease comprising determining in the Caucasian subject the identity of one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, and wherein the presence of one or more of the haplotypes indicates whether the subject is in need of prophylactic treatment for a complement-mediated disease.

Also described herein are methods of identifying a Caucasian subject in need of a prophylactic treatment for a complement-mediated disease comprising determining in the subject the identity of one or more SNPs in the CFH-to-F13B locus, wherein the SNPs is: (i) rs1061170, rs1410996, rs2274700, rs3753395, rs403846, or rs3753396 or (ii) a SNP in linkage disequilibrium with the SNPs of (i), and wherein the presence of one or more of the SNPs indicates whether the subject is in need of prophylactic treatment for a complement-mediated disease.

Also described herein are methods of identifying a Caucasian subject in need of a prophylactic treatment for a complement-mediated disease comprising determining in the subject the identity of a deletion tagging SNP in the CFH-to-F13B locus, wherein the SNPs are: (i) rs12144939 or (ii) a SNP in linkage disequilibrium with rs12144939, and wherein the presence of one or more of the SNPs indicates whether the subject is in need of prophylactic treatment for a complement-mediated disease.

Also described herein are methods of identifying a Caucasian subject appropriate for an a complement-mediated disease clinical trial comprising determining in the Caucasian subject the identity of one or more haplotypes, wherein the one or more haplotypes are H1_62_A, H2_62_A, H3_62_A, H4_62_A, H5_62_A , H6_62_A, H7_62_A, H8_62_A, H9_62_A, H10_62_A, H11_62_A, H12_62_A, H13_62_A, H14_62_A, H15_62_A, or a complement thereof, and wherein the presence of one or more of the haplotypes indicates whether the subject is appropriate for the clinical trial.

Also described herein are methods of of identifying a Caucasian subject appropriate for an a complement-mediated disease clinical trial comprising determining in the Caucasian subject the identity of one or more haplotypes, wherein the one or more haplotypes are H1_51_A, H2_51_A, H3_51_A, H4_51_A, H5_51_A, H6_51_A, H7_51_A, H8_51_A, H9_51_A, H10_51_A, H11_51_A, H12_51_A, H13_51_A, H14_51_A, H15_51_A, H16_51_A, H17_51_A, or a complement thereof, and wherein the presence of one or more of the haplotypes indicates whether the subject is appropriate for the clinical trial.

Also described herein are methods of of identifying a Caucasian subject appropriate for an a complement-mediated disease clinical trial comprising determining in the Caucasian subject the identity of one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, and wherein the presence of one or more of the haplotypes indicates whether the subject is appropriate for the clinical trial.

Also described herein are methods of identifying a Caucasian subject appropriate for an a complement-mediated disease clinical trial comprising determining in the Caucasian subject the identity of at least six SNPs in the CFH-to-F13B locus, wherein the SNPs are: (i) rs35928059, rs800292, rs1061170, rs12144939, rs7546940, rs1409153, rs10922153, or rs698859, or (ii) a SNP in linkage disequilibrium with the SNPs of (i), and wherein the presence of at least six of the SNPs indicates whether the subject is appropriate for the clinical trial.

Also described herein are methods of of identifying a Caucasian subject appropriate for an a complement-mediated disease clinical trial comprising determining in the subject the identity of one or more SNPs in the CFH-to-F13B locus, wherein the SNPs is: (i) rs1061170, rs1410996, rs2274700, rs3753395, rs403846, or rs3753396 or (ii) a SNP in linkage disequilibrium with the SNPs of (i), and wherein the presence of one or more of the SNPs indicates whether the subject is appropriate for the clinical trial.

Also described herein are methods of of identifying a Caucasian subject appropriate for an a complement-mediated disease clinical trial comprising determining in the subject the identity of a deletion tagging SNP in the CFH-to-F13B locus, wherein the SNPs are: (i) rs12144939 or (ii) a SNP in linkage disequilibrium with rs12144939, and wherein the presence of one or more of the SNPs indicates whether the subject is appropriate for the clinical trial.

Also described herein are kits kit comprising an assay for detecting one or more haplotypes in a nucleic acid sample of a subject, wherein the one or more haplotypes are H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H1_51_A, H2_51_A, H3_51_A, H4_51_A, H5_51_A, H6_51_A, H7_51_A, H8_51_A, H9_51_A, H10_51_A, H11_51_A, H12_51_A, H13_51_A, H14_51_A, H15_51_A, H16_51_A, H17_51_A, H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof.

Also descibed herein are methods for determining a Caucasian subject's susceptibility to having or developing age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H1851_B, H19_51_B, H20_51_B, H21_51_B, H22_51_B, or a complement thereof, and wherein the presence of one or more of the diplotypes indicates the subject's susceptibility for having or developing age-related macular degeneration.

Also descibed herein are methods of identifying a Caucasian subject in need of treatment for age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_551_B, H19_51_B, H20_51_B, H21_51_B, H22_51_B, or a complement thereof, and wherein the presence of one or more of the diplotypes indicates whether the subject is in need of treatment for age-related macular degeneration.

Also descibed herein are methods of identifying a Caucasian subject in need of a prophylactic treatment for age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H1751_B, H18_51_B, H1951_B, H20_51_B, H21_51_B, H22_51_B, or a complement thereof, and wherein the presence of one or more of the diplotypes indicates whether the subject is in need of prophylactic treatment for age-related macular degeneration.

Also descibed herein are methods of identifying a Caucasian subject appropriate for an age-related macular degeneration clinical trial comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51B, H13_51_B, H14_51_B, H15_51_B, H16_51_B, H17_51_B, H18_51_B, H19_51_B, H20_51_B, H21_51_B, H22_51_B, or a complement thereof, and wherein the presence of one or more of the diplotypes indicates whether the subject is appropriate for the clinical trial.

### BRIEF DESCRIPTION OF THE FIGURES

The accompanying figures, which are incorporated in and constitute a part of this specification, illustrate several aspects and together with the description serve to explain the principles of the invention.
Figure 1 shows major haplotypes H1_61_A through H15_62_A.
Figure 2 shows the 63 SNPs utilized in the preliminary haplotype studies.
Figure 3 shows minor haplotypes H16_62_A through H163_62_A.
Figure 4 shows the verification of halotypes derived from the GSH - 1073 cohort and the GSH - 927 cohort.
Figure 5 shows the single SNP association data for the 62 SNP panel in the GSH cohort.
Figure 6 shows disease associations that were analyzed for the entire extended haplotypes using Haploview 4.2.
Figure 7 shows the haplotype tagging SNPs for 62 SNP haplotypes.
Figure 8 shows the 51_A haplotype tagging SNPS (51_A htSNPs).

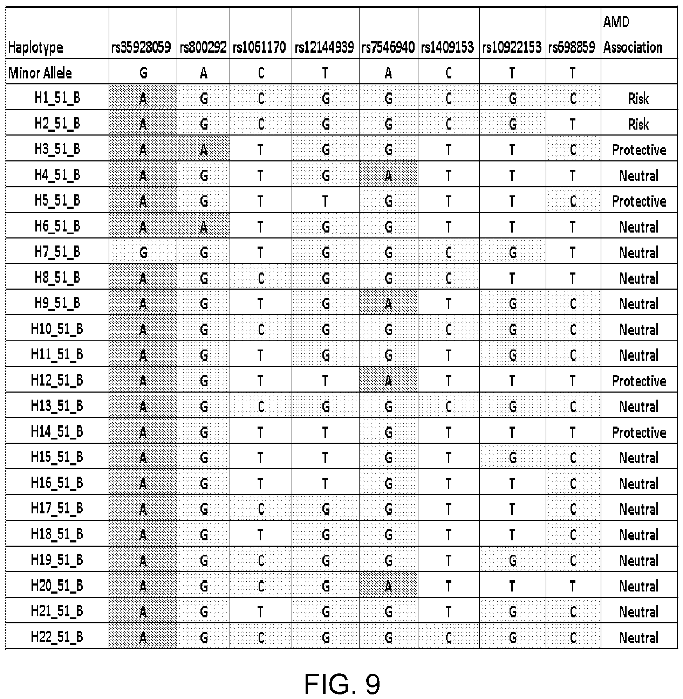

Figure 9 shows the 51_B haplotype tagging SNPS (51_A htSNPs).
Figure 10 shows the effect of generating haplotypes using fewer and fewer SNPs has on association values for haplotypes.
Figure 11 shows the qPCR assay for R3-1 deletion, regular PCR assay for homozygous deletion or non-deletion.
Figure 12 shows the asessment of 51 SNP haplotypes in the Sib-pair cohort.
Figure 13 shows the 62 single marker (SNPs) and haplotypes association with gender in the GSH case-control cohort the male and female gender association in the '927 cohort.
Figure 14 shows the haplotype boundaries extended beyond that covered by 63 SNPs. Provided in the figure is a list of all Hapmap SNPs.
Figure 15 shows the CFH locus and the haplotypes in the 51_B cohort.
Figure 16 shows a comparison of 51_A and 62_A haplotypes.
Figure 17 shows shows the frequences of the 62_A haplotypes.
Figure 18 shows the 62_A SNP haplotype schema regrouped with P values.
Figure 19 shows a comparison of the 51 SNP haplotypes in the 1073 cohort and combined cohorts.
Figure 20 shows the '927 cohort and the 62_A to 51_A conversion.
Figure 21 shows a haplotype tree for the 62_A cohort. Figure 21(A) shows the hierarchical relationships between the 163 62_A SNP-based haplotypes.
Figure 22 shows the association of haplotypes in the '927 cohort using a 51 SNP-based haplotype.
Figure 23 shows the gender associations of the 62_A haplotypes.
Figure 24 shows the gender associations of the 51_B haplotypes.

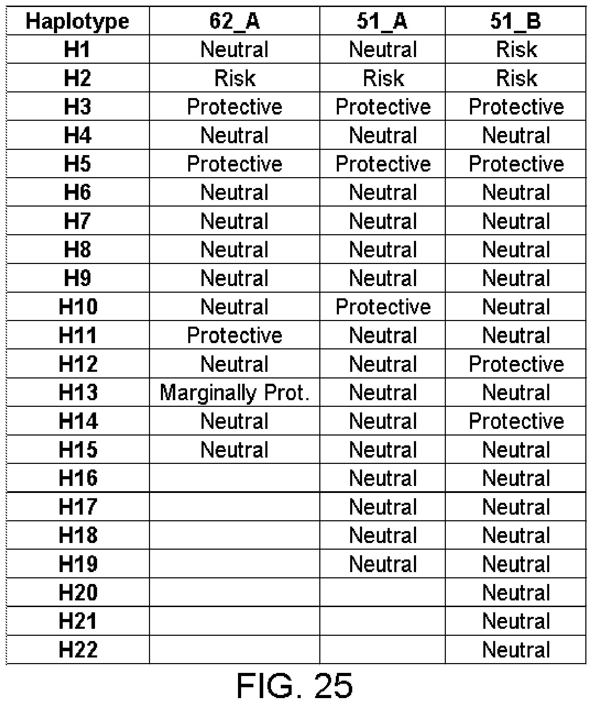

Figure 25 shows haplotype disease association.
Figure 26 shows haplotype disease association based on gender.
Figure 27 shows the SNPs used in a haplotype reconstruction.
Figure 28 shows Taqman qPCR copy number variation data.
Figure 29 shows the frequency of subjects with varying number of SNPs screened.
Figure 30 shows a deletion decision tree made to predict deletion status from three SNPs.
Figure 31 shows the results of the real-time quantitative PCR (qPCR) used to determine the copy number state of the CFHR3 and CFHR1 genes.
Figure 32 shows the H1-H19 haplotypes based on 1073 cohort screened for 63 SNPs.
Figure 33 shows haplotype frequencies calculated on 136 unrelated UGRP individuals, who were mostly first generation grandparents, using Haploview program's phased haplotype input mode, Haps format.
Figure 34 shows association of 63 SNP-based CFH-to-F13B Haplotypes with AMD.
Figure 35 shows the diplotype analysis of the overall 51_B cohort.
Figure 36 shows diplotype analysis for males in the 51_B cohort.
Figure 37 shows diplotype analysis for females in the 51_B cohort.
Figure 38 shows the combined cohort with all haplotypes, 0vs1b-4, highlighted to match major HapMap haplotypes.
Figure 39 shows the major CFH-to-F13B HapMap haplotypes with >0.5% frequencies for CEU.
Figure 40 shows the marker-rs number IDs shown in Figure 39.
Figure 41 shows a comparison of the 51_B-based haplotypes to HapMap haplotypes >0.05% frequencies.
Figure 42 shows single marker associations; Controls (0) vs all AMD in the 51_B cohort.
Figure 43 shows single marker associations; Controls (0) vs ealy AMD (1b-3) in the 51_B cohort.
Figure 44 shows single marker associations; Controls (0) vs geographic atrophy (4A) in the 51B cohort.
Figure 45 shows the single marker associations; Controls (0) vs CNV (4B) in the 51_B cohort.
Figure 46 shows the haplotype associations; Controls (0) vs all AMD, early AMD (1B-3), GA only (4A) & CNV only (4B) in the 51_B cohort.
Figure 47 shows the diplotype associations; Controls (0) vs early AMD (1B-3) in the 51_B cohort.
Figure 48 shows the diplotype associations; Controls (0) vs CNV (4B) in the 51_B cohort.

Additional advantages of the invention will be set forth in part in the description which follows, and in part will be obvious from the description, or can be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### DESCRIPTION

The present invention can be understood more readily by reference to the following detailed description of the invention and the Examples included therein.

Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is in no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

### Definitions

As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values described herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10" as well as "greater than or equal to 10" is also disclosed. It is also understood that throughout the application, data are provided in a number of different formats, and that these data, represent endpoints, starting points, and ranges for any combination of the data points. For example, if a particular data point "10" and a particular data point 15 are disclosed, it is understood that greater than, greater than or equal to, less than, less than or equal to, and equal to 10 and 15 are considered disclosed as well as between 10 and 15. It is also understood that each unit between two particular units is also disclosed. For example, if 10 and 15 are disclosed, then 11, 12, 13, and 14 are also disclosed.

"Optional" or "optionally" means that the subsequently described event or circumstance may or may not occur and that the description includes instances where said event or circumstance occurs and instances where it does not.

The word "or" as used herein means any one member of a particular list and also includes any combination of members of that list.

"Complement-Mediated Disease" as used herein refers to a disease caused by or resulting from abnormal complement activation or function of its four major pathways. The complement pathway consists of three stages: recognition, enzymatic activation and membrane attack leading to cell death. "Complement-Mediated Disease" as used herein also refers to a disease caused by or resulting from alterations of the classical, exogenous, mannan-binding lectin (MBL) or alternative pathways. Examples of Complement-Mediated Disease include, but are not limited to, atypical haemolytic uraemic syndrome (aHUS), membranoproliferative glomerulonephritis type II (MPGN II; also known as dense deposit disease), systemic lupus erythematosus (SLE), pyogenic infections, hemolysis and thrombosis, partial lipodystrophy, hereditary angioedema, paroxysmal nocturnal haemoglobinuria (PNH), age-related macular degeneration (AMD), rheumatois arthritis, myocarditis, multiple sclerosis, IgA nephropathy, Henoch-Schoenlein purpura, glaucoma, post-streptococcal disease, anti-phospholipid antibody syndrome, recurrent pregnancy loss, asthma, antibody-mediated cutaneous disease and anti-nuclear cytoplasmic antigen-associated pauci-immune vasculitis.

"Haplotype" as used herein refers to a DNA sequence or a combination of DNA sequences present at various loci on a chromosome that are transmitted together; a haplotype may be one locus, several loci, or an entire chromosome depending on the number of recombination events that have occurred between a given set of loci.

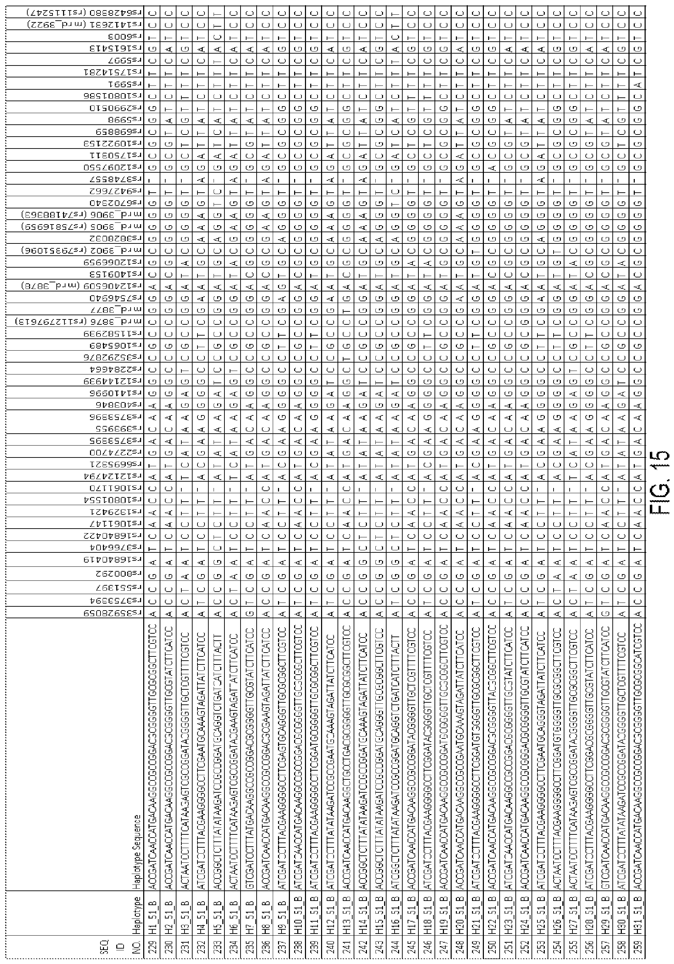

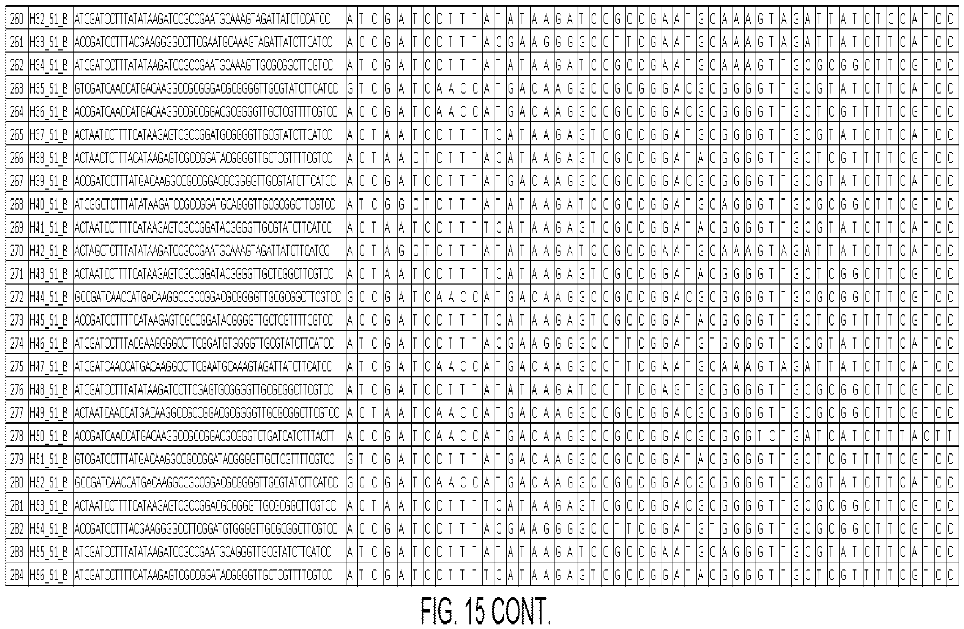

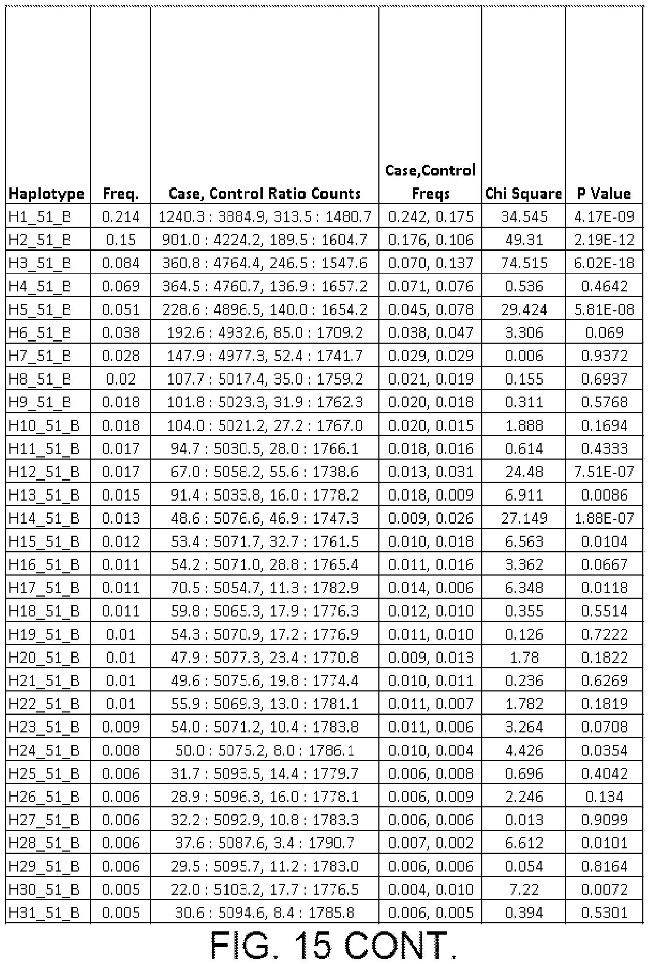

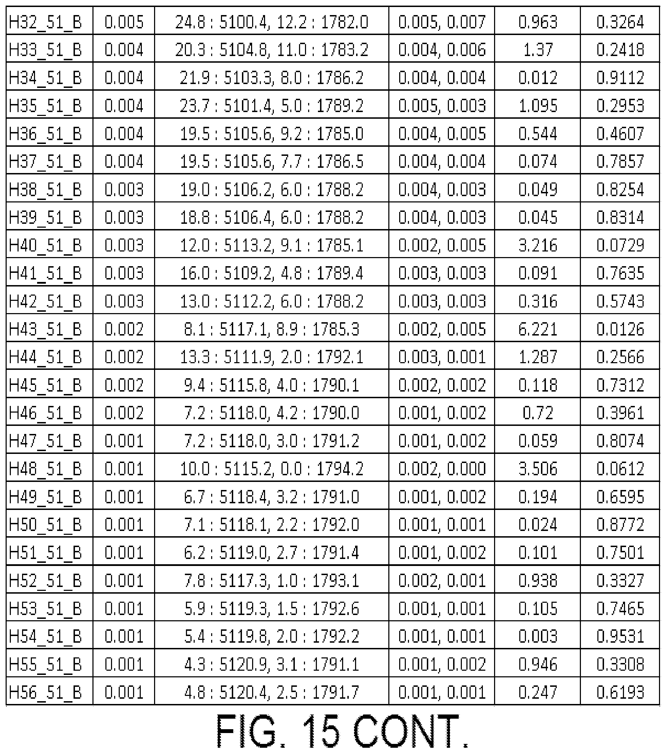

"Risk Haplotype" as used herein refers to a haplotype of a subject wherein the presence of the haplotype indicates the subject's increased risk of developing a complement-mediated disease (e.g. AMD) when compared to a subject without said risk haplotype. A "Risk Haplotype" also refers to a haplotype that occurs more often in subjects with Complement-Mediated Disease (e.g. AMD) (cases) than in subjects without Complement-Mediated Disease (e.g. AMD) (controls) in a given cohort. In other words, the case versus control percentage is different in cases than in controls as illustrated in the tables herein (see FIG. 15). Although not required, the difference between the number of subjects with Complement-Mediated Disease (e.g. AMD) (case) and the number of subjects without Complement-Mediated Disease (e.g. AMD) (control) can be statistically significant. For example, H2_62_A, H2_51_A, and H2_51_B are examples of "Risk Haplotypes" as they relate to AMD.

"Protective Haplotype" as used herein refers to a haplotype of a subject wherein the presence of the haplotype indicates the subject's decreased risk of developing Complement-Mediated Disease (e.g. AMD) when compared to a subject without said protective haplotype. A "Protective Haplotype" also refers to a haplotype that occurs less often in subjects with Complement-Mediated Disease (e.g. AMD) (cases) than in subjects without Complement-Mediated Disease (e.g. AMD) (controls), in other words, the case versus control percentage is lower in cases than in controls as illustrated in the tables herein (See FIG. 15). Although not required, the difference between the number of subjects with Complement-Mediated Disease (e.g. AMD) (case) and the number of subjects without Complement-Mediated Disease (e.g. AMD) (control) can be statistically significant. For example, H5_62_A, H5_51_A, and H5_51_B are examples of "protective haplotypes" as they relate to AMD.

"Neutral Haplotype" as used herein refers to a haplotype of a subject wherein the presence of the haplotype does not indicate the subject's increased or decreased risk of developing Complement-Mediated Disease (e.g. AMD). A "Neutral Haplotype" also refers to a haplotype that occurs about equally in subjects with Complement-Mediated Disease (e.g. AMD) (cases) and in subjects without Complement-Mediated Disease (e.g. AMD) (controls), in other words, the case versus control percentage is about equal in cases and controls as illustrated in the tables herein (See FIG. 15). Although not required, the difference between the number of subjects with Complement-Mediated Disease (e.g. AMD) (case) and the number of subjects without Complement-Mediated Disease (e.g. AMD) (control) can be non-statistically significant. For example, H4_62_A, H4_51_A, and H4_51_B are examples of "neutral haplotypes" as they relate to AMD.

Haplotypes as described herein can be referred to by the generic nomenclature: "Hx_Y_Z", wherein "Hx" indicates the numbering of the specific haplotype as determined by the frequency of the specific haplotype in a given population or cohort. For example, H2 indicates a specific haplotype that occurs with the second highest frequency in the given data set, population or cohort. "Y", in the context of this nomenclature, specifies the SNP panel from which the overall haplotype was determined. For example, Hx_62_Z would refer to the 62 SNP Panel as described herein, whereas Hx_51_Z refers to the 51 SNP Panel as described herein. "Z", in the context of this nomenclature, refers to the specific cohort used in ascertaining the specific haplotype. For example, Hx_Y_A would refer to the '927 cohort' as described herein, whereas Hx_Y_B refers to the combined cohort as described herein.

"Haplotype Tagging Haplotype" refers to haplotypes that can tag one or more of the haplotypes described herein. Such haplotypes can be referred to as "tagging haplotypes". Such tagging haplotypes can be identified based on HapMap Analysis as described herein. When such tagging haplotypes are referenced herein, the generic nomenclature: "Hx_Y_Z_t" can be used wherein "t" represents a "tagging haplotype" and Hx, Y, and Z represent the same categories as described above for the generic nomenclature "Hx_Y_Z". For example, H2_62_A_1 would represent a first haplotype that tags the H2_62_A haplotype.

"Major Haplotype" as used herein refers to a haplotype that occurs in greater than 1% of the population, data set or cohort being examined.

"Minor Haplotype" as used herein refers to a haplotype that occurs in 1% or less than 1% of the population, data set or cohort being examined.

"CFH-to-F13B Locus" as used herein refers to a region of chromosome 1q25-q31 (approximately 196,580,000 to 197,053,000; hg19) containing the following genes and pseudogenes: complement factor H (CFH) and its truncated isoform (CFHT), the complement factor H related genes 3, 1, 4, 2 and 5 (CFHR3, CFHR1, CFHR4, CFHR2 and CFHR5), a CFHR pseudogene () and factor 13B (F13B). The CFH-to-F13B Locus includes all non-genic DNA, including that extending proximal to CFH and distal to F13B. Blocks of linkage disequilibrium (LD) that initiate within the CFH-to-F13B and extend proximal to CFH and/or distal to F13b are also included.

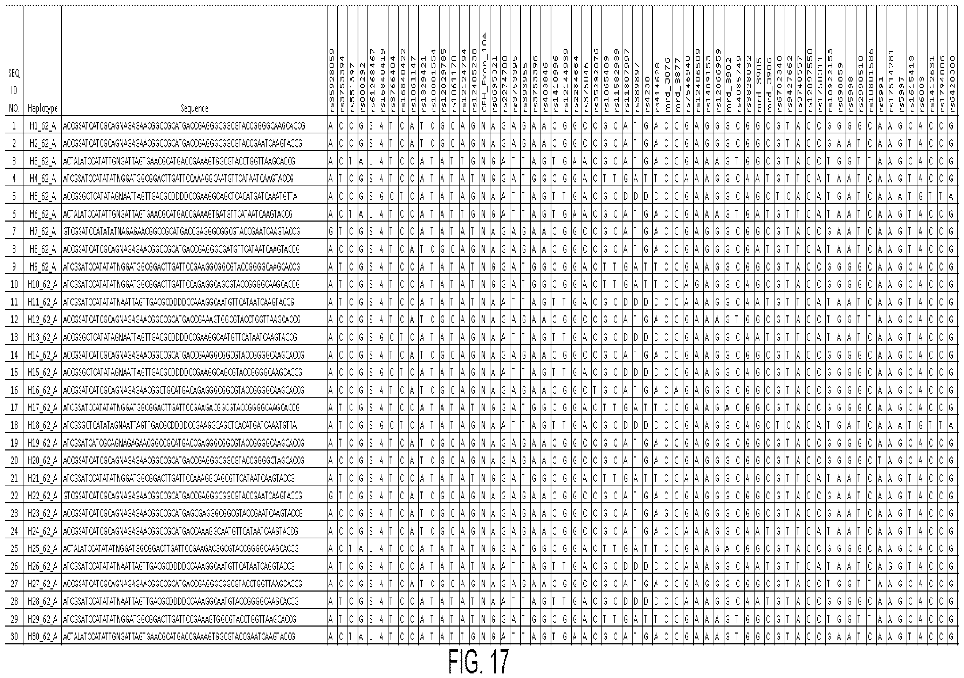

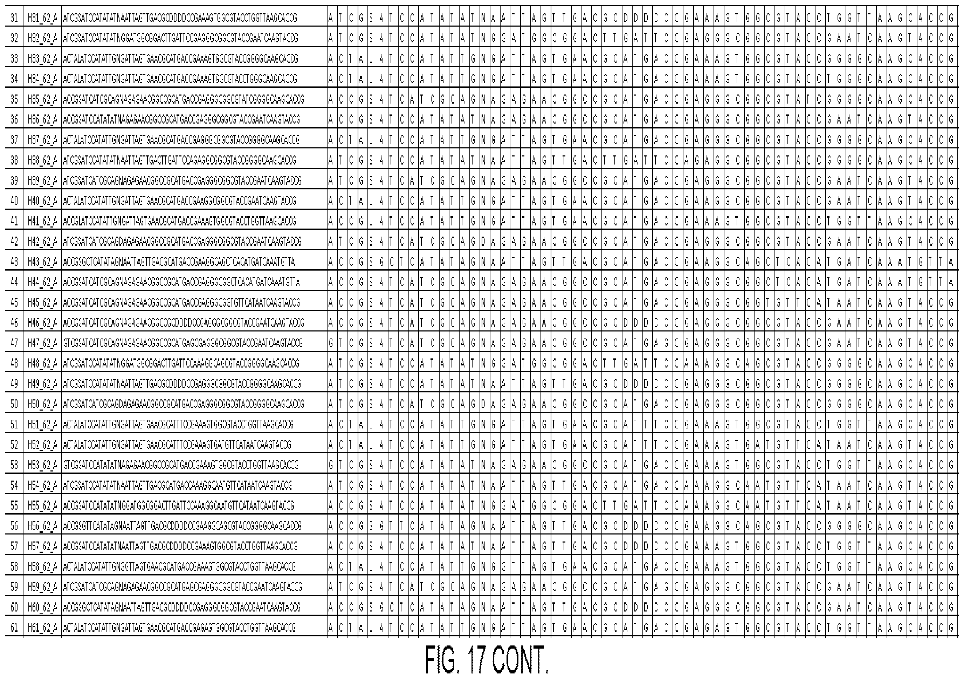

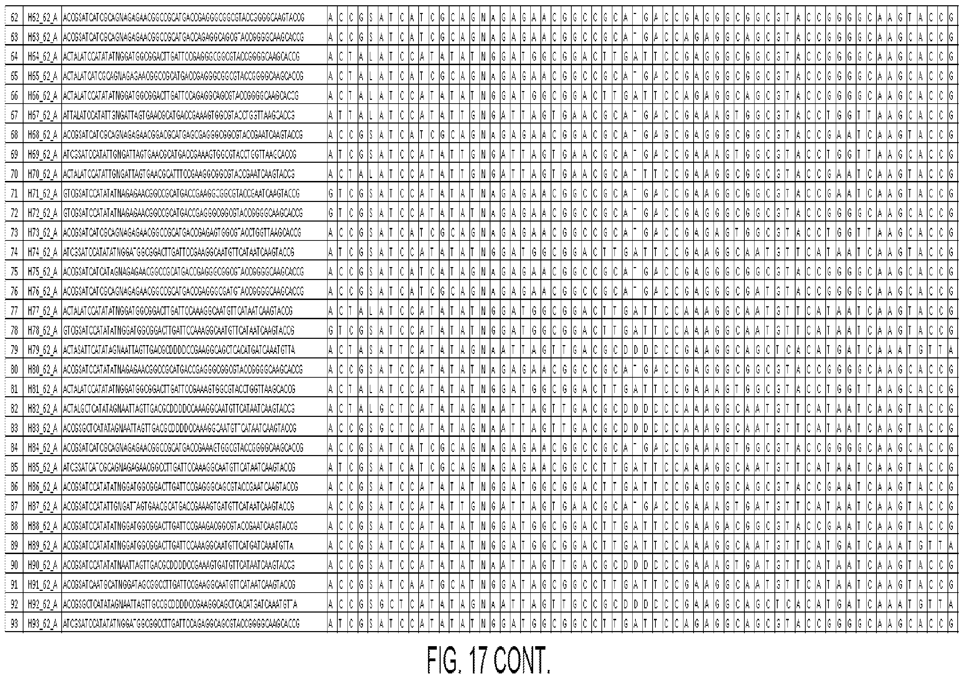

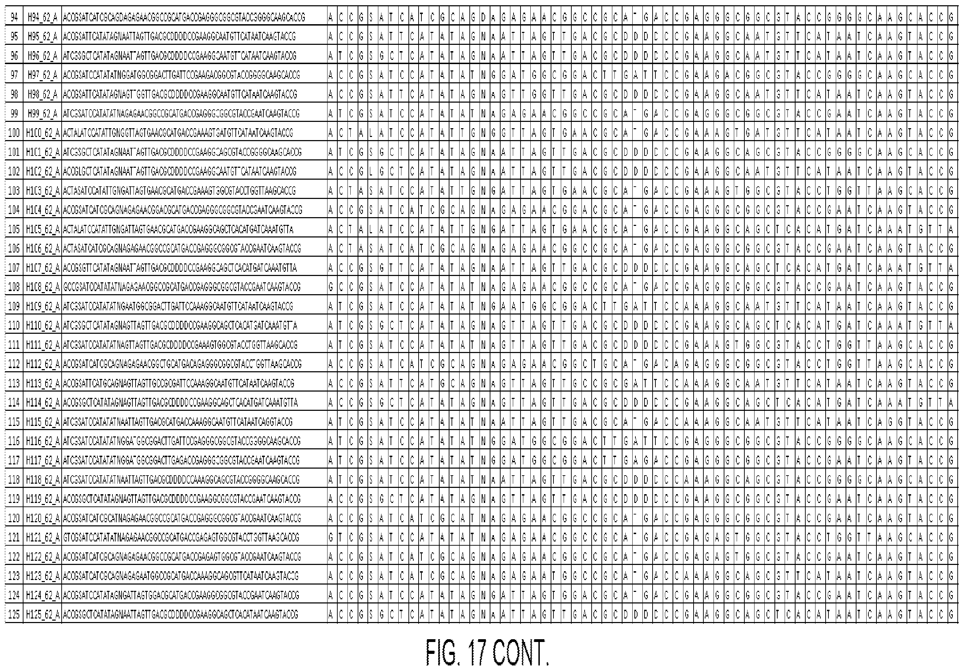

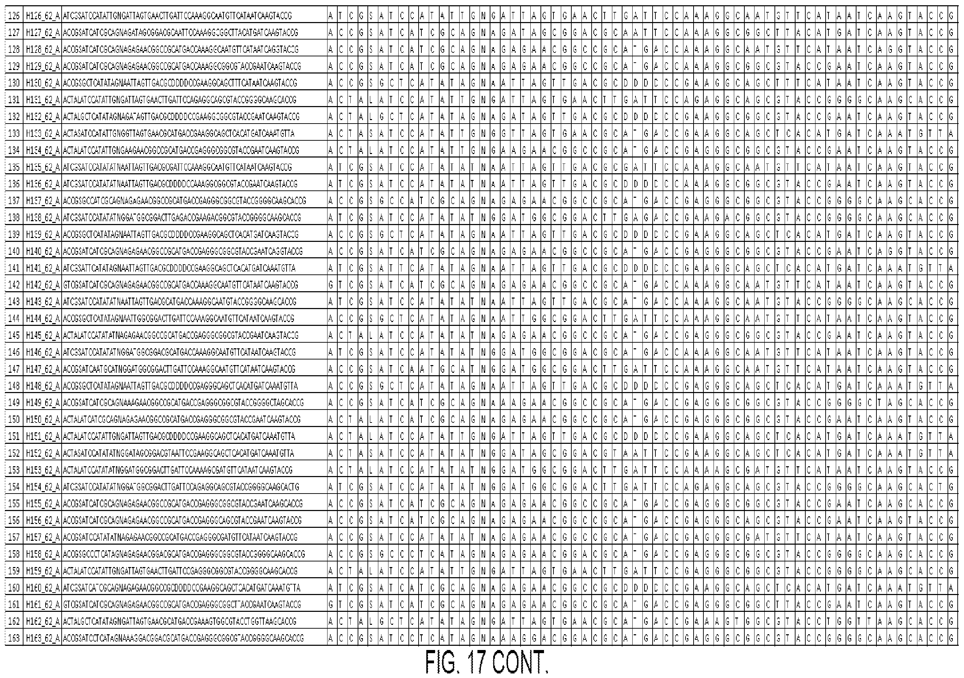

"62 SNP Panel" as used herein refers to 62 SNPs that were selected based on their significance of association as well as locations selected to provide coverage across the entire CFH-to-F13B Locus and its LD blocks. The 62 SNP Panel consists of the SNPs shown in FIG. 17.

"51 SNP Panel" as used herein refers to 51 SNPs of the 62 SNP Panel described herein. The 51 SNP Panel consists of the SNPs shown in FIG. 15.

"HapMap" as used herein refers to a haplotype map of the human genome that is a catalog of common genetic variants that occur in human beings. HapMap describes what these variants are, where they occur in DNA, how they distribute in haplotypes, and their frequencies among people within populations and among populations in different parts of the world. (The International HapMap Consortium. The International HapMap Project. Nature 426, 789-796 (2003)).

"HapMap Analysis" as used herein refers to an analysis that can be conducted using Haploview software (Barrett JC, Fry B, Maller J, Daly MJ. Haploview: analysis and visualization of LD and haplotype maps. Bioinformatics. 2005 Jan 15 [PubMed ID: 15297300]).

"Linkage Disequilibrium" as used herein refers to the non-random association between two or more alleles at two or more loci (not necessarily on the same chromosome) such that certain combinations of alleles are more likely to occur together on a chromosome than other combinations of alleles than would be expected from a random formation of haplotypes from alleles based on their frequencies.

### A. METHODS OF PREDICTING A CAUCASIAN SUBJECT'S RISK FOR HAVING OR DEVELOPING A COMPLEMENT-MEDIATED DISEASE

Described herein are methods for determining a Caucasian subject's susceptibility to having or developing a complement-mediated disease comprising determining in the Caucasian subject the identity of one or more major or minor haplotypes. The haplotypes can include, but are not limited to H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H1_51_A, H2_51_A, H3_51_A, H4_51_A, H5_51_A, H6_51_A, H7_51_A, H8_51_A, H9_51_A, H10_51_A, H11_51_A, H12_51_A, H13_51_A, H14_51_A, H15_51_A, H16_51_A, H17_51_A, H1_51_B, H2_51_B,H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof. As used herein, "complement thereof' means a SNP, haplotype, or diplotype that is located on the complement DNA or RNA strand of any SNP, haplotype, or diplotype described herein. For example, and not to be limiting, the haplotype sequence "ATGC" can be converted to its complement thereof "TACG". Therefore, by way of example, if "ATGC" represents a haplotype indicative of a subject's susceptibility to having or developing a complement-mediated disease, its complement haplotype sequence, "TACG", also represents a haplotype indicative of a subject's susceptibility to having or developing a complement-mediated disease. As described herein, the presence of one or more of the haplotypes disclosed herein can indicate a subject's susceptibility for having or developing a complement-mediated disease. In one aspect, the complement-mediated disease can be age-related macular degeneration (AMD). In another aspect, the complement-mediated disease can include, but is not limited to atypical haemolytic uraemic syndrome (aHUS), membranoproliferative glomerulonephritis type II (MPGN II; also known as dense deposit disease), systemic lupus erythematosus (SLE), pyogenic infections, hemolysis and thrombosis, partial lipodystrophy, hereditary angioedema, paroxysmal nocturnal haemoglobinuria (PNH), rheumatois arthritis, myocarditis, multiple sclerosis, IgA nephropathy, Henoch-Schoenlein purpura, glaucoma, post-streptococcal disease, anti-phospholipid antibody syndrome, recurrent pregnancy loss, asthma, antibody-mediated cutaneous disease, or anti-nuclear cytoplasmic antigen-associated pauci-immune vasculitis.

In one aspect, determining the identity of a certain haplotype or certain haplotypes in a subject can indicate that the subject has an increased risk of having or developing a complement-mediated disease. Such hapltoypes can be risk haplotypes. For example, and not to be limiting, a risk haplotype can be H2_62_A, H2_51_A, H1_51_B, H2_51_B, or a complement thereof. Thus, described herein are methods for determining a Caucasian subject's susceptibility to having or developing a complement-mediated disease comprising determining in the Caucasian subject the identity of one or more haplotypes described herein, wherein a risk haplotype is indicative of the subject's increased risk for having or developing a complement-mediated disease. In one aspect, also disclosed are methods of determining a Caucasian subject's increased risk susceptibility to having or developing complement-mediated disease, wherein the method further comprises administering a therapeutic composition to the subject when an increased risk is determined. In one aspect, also disclosed are methods of determining a Caucasian subject's increased risk susceptibility to having or developing complement-mediated disease, wherein the subject has an increased risk of having or developing complement-mediated disease and further comprises administering a therapeutic composition to the subject when an increased risk is determined.

In another aspect, determining the identity of a certain haplotype or certain haplotypes in a subject can indicate that the subject has a deacreased risk of having or developing a complement-mediated disease. Such haplotypes can be protective haplotypes. For example, and not to be limiting, a protective haplotype can be H3_62_A, H5_62_A, H11_62_A, H3_51_A, H5_51_A, H10_51_A, H3_51_B, H5_51_B, H12_51_B, H14_51_B, or a complement thereof. Thus, described herein are methods for determining a Caucasian subject's susceptibility to having or developing a complement-mediated disease comprising determining in the Caucasian subject the identity of one or more haplotypes described herein, wherein a protective haplotype is indicative of the subject's decreased risk for having or developing a complement-mediated disease.

In yet another aspect, determining the identity of a certain haplotype or certain haplotypes in a subject can indicate that the subject does not have an increased risk or a decreased risk of having or developing a complement-mediated disease. Such haplotypes can be neutral haplotypes. For example, and not to be limiting, a neutral haplotype can be H1_62_A, H4_62_A, H6_62_A, H7_62_A, H8_62_A, H9_62_A, H10_62_A, H12_62_A, H13_62_A, H14_62_A, H15_62_A, H1_51_A, H4_51_A, H6_51_A, H7_51_A, H8_51_A, H9_51_A, H11_51_A, H12_51_A, H13_51_A, H14_51_A, H15_51_A, H16_51_A, H17_51_A, H4_51_B, H6_51_B, H7_51_B, H8_51_B, H9_51_B, H10_51_B, H11_51_B, H13_51_B, H15_51_B, H_16-51_B, H17_51_B, H18_51_B, H19_51_B, H20_51_B, H21_51_B, H22_51_B, or a complement thereof. Thus, described herein are methods for determining a Caucasian subject's susceptibility to having or developing a complement-mediated disease comprising determining in the Caucasian subject the identity of one or more haplotypes described herein, wherein a neutral haplotype does not indicate that the subject has an increased risk or a decreased risk for having or developing a complement-mediated disease.

As used herein, the term "subject" means an individual. In one aspect, a subject is a mammal such as a human. In another aspect, the subject can be a Caucasian subject. In a further aspect a subject can be a non-human primate. Non-human primates include marmosets, monkeys, chimpanzees, gorillas, orangutans, and gibbons, to name a few. The term "subject" also includes domesticated animals, such as cats, dogs, etc., livestock (for example, cattle (cows), horses, pigs, sheep, goats, etc.), laboratory animals (for example, ferret, chinchilla, mouse, rabbit, rat, gerbil, guinea pig, etc.) and avian species (for example, chickens, turkeys, ducks, pheasants, pigeons, doves, parrots, cockatoos, geese, etc.). Subjects can also include, but are not limited to fish (for example, zebrafish, goldfish, tilapia, salmon, and trout), amphibians and reptiles. As used herein, a "subject" is the same as a "patient," and the terms can be used interchangeably.

A haplotype can refer to a set of polymorphisms in a single gene, an intergenic sequence, or in larger sequences including both gene and intergenic sequences, e.g., a collection of genes, or of genes and intergenic sequences. For example, a haplotype can refer to a set of polymorphisms in the CFH-to-F13B locus. The CFH-to-F13B locus comprises the chromosome region of chromosome 1q25-q31 (approximately 196,580,000 to 197,053,000; hg19) that contains the following genes and pseudogenes: complement factor H (CFH) and its truncated isoform (CFHT), the complement factor H related genes 3, 1, 4, 2, and 5 (CFHR3, CFHR1, CFHR4, CFHR2 and CFHR5), a CFHR pseudogene, and factor 13B (F13B). The locus can also incude all non-genic DNA, including that extending proximal to CFH and distal to F13B. Blocks of linkage disequilibrium (LD) that initiate within the CFH-to-F13B and extend proximal to CFH and/or distal to F13b are also included in the locus.

As used herein the term "haplotype" can also refer to a set of single nucleotide polymorphisms (SNPs) found to be statistically associated with each other on a single chromosome. A haplotype can also refer to a combination of polymorphisms (e.g., SNPs) and other genetic markers (e.g., an insertion or a deletion) found to be statistically associated with each other on a single chromosome.

The term "polymorphism" refers to the occurrence of one or more genetically determined alternative sequences or alleles in a population. A "polymorphic site" is the locus at which sequence divergence occurs. Polymorphic sites have at least one allele. A diallelic polymorphism has two alleles. A triallelic polymorphism has three alleles. Diploid organisms may be homozygous or heterozygous for allelic forms. A polymorphic site can be as small as one base pair. Examples of polymorphic sites include: restriction fragment length polymorphisms (RFLPs), variable number of tandem repeats (VNTRs), hypervariable regions, minisatellites, dinucleotide repeats, trinucleotide repeats, tetranucleotide repeats, and simple sequence repeats. As used herein, reference to a "polymorphism" can encompass a set of polymorphisms (i.e., a haplotype).

A "single nucleotide polymorphism (SNP)" can occur at a polymorphic site occupied by a single nucleotide, which is the site of variation between allelic sequences. The site can be preceded by and followed by highly conserved sequences of the allele. A SNP can arise due to substitution of one nucleotide for another at the polymorphic site. Replacement of one purine by another purine or one pyrimidine by another pyrimidine is called a transition. Replacement of a purine by a pyrimidine or vice versa is called a transversion. A synonymous SNP refers to a substitution of one nucleotide for another in the coding region that does not change the amino acid sequence of the encoded polypeptide. A non-synonymous SNP refers to a substitution of one nucleotide for another in the coding region that changes the amino acid sequence of the encoded polypeptide. A SNP may also arise from a deletion or an insertion of a nucleotide or nucleotides relative to a reference allele.

A "set" of polymorphisms means one or more polymorphism, e.g., at least 1, at least 2, at least 3, at least 4, at least 5, at least 6, or more than 6 polymorphisms known, for example, in the CFH-to-F13B locus.

As used herein, a "nucleic acid," "polynucleotide," or "oligonucleotide" can be a polymeric form of nucleotides of any length, can be DNA or RNA, and can be single- or double-stranded. Nucleic acids can include promoters or other regulatory sequences. Oligonucleotides can be prepared by synthetic means. Nucleic acids include segments of DNA, or their complements spanning or flanking any one of the polymorphic sites known in the CFH-to-F13B locus. The segments can be between 5 and 100 contiguous bases and can range from a lower limit of 5, 10, 15, 20, or 25 nucleotides to an upper limit of 10, 15, 20, 25, 30, 50, or 100 nucleotides (where the upper limit is greater than the lower limit). Nucleic acids between 5-10, 5-20, 10-20, 12-30, 15-30, 10-50, 20-50, or 20-100 bases are common. The polymorphic site can occur within any position of the segment. A reference to the sequence of one strand of a double-stranded nucleic acid defines the complementary sequence and except where otherwise clear from context, a reference to one strand of a nucleic acid also refers to its complement.

"Nucleotide" as described herein refers to molecules that, when joined, make up the individual structural units of the nucleic acids RNA and DNA. A nucleotide is composed of a nucleobase (nitrogenous base), a five-carbon sugar (either ribose or 2-deoxyribose), and one phosphate group. "Nucleic acids" are polymeric macromolecules made from nucleotide monomers. In DNA, the purine bases are adenine (A) and guanine (G), while the pyrimidines are thymine (T) and cytosine (C). RNA uses uracil (U) in place of thymine (T). As used in the figures, "S" represents short; "L" represents "long, and "D" represents deleted in the context of the SNP analysis. For purposes for the sequence listing "S", "L" and "D" are represented as "N" which refers to any specific nucleotide in the posotion identified.

Exemplary polymorphic sites in the CFH-to-F13B locus are described herein as examples and are not intended to be limiting. These polymorphic sites, SNPs, or haplotypes can also be used in carrying out methods described herein. Moreover, it will be appreciated that these CFH-to-F13B locus polymorphisms are useful for linkage and association studies, genotyping clinical populations, correlation of genotype information to phenotype information, loss of heterozygosity analysis, identification of the source of a cell sample, and can also be useful to target potential therapeutics to cells.

It will be appreciated that additional polymorphic sites in the CFH-to-F13B locus, which are not explicitly described herein, may further refine this analysis. A SNP analysis using non-synonymous polymorphisms in the CFH-to-F13B locus can be useful to identify variant polypeptides. Other SNPs associated with risk may encode a protein with the same sequence as a protein encoded by a neutral or protective SNP but contain an allele in a promoter or intron, for example, changes the level or site of gene expression. It will also be appreciated that a polymorphism in the CFH-to-F13B locus may be linked to a variation in a neighboring gene. The variation in the neighboring gene may result in a change in expression or form of an encoded protein and have detrimental or protective effects in the carrier.

As used herein, the term "variant" can also refer to a nucleotide sequence in which the sequence differs from the sequence most prevalent in a population, for example by one nucleotide, in the case of the SNPs described herein. For example, some variations or substitutions in the nucleotide sequence of the CFH-to-F13B locus alter a codon so that a different amino acid is encoded resulting in a variant polypeptide. The term "variant," can also refer to a polypeptide in which the sequence differs from the sequence most prevalent in a population at a position that does not change the amino acid sequence of the encoded polypeptide (i.e., a conserved change). Variant polypeptides can be encoded by a risk haplotype, encoded by a protective haplotype, or can be encoded by a neutral haplotype. Variant polypeptides can be associated with risk, associated with protection, or can be neutral.

The methods and materials described herein can be used to determine whether the nucleic acid of a subject (e.g., human) contains a polymorphism, such as a single nucleotide polymorphism (SNP). For example, methods and materials provided herein can be used to determine whether a subject has a variant SNP. Any method can be used to detect a polymorphism in the CFH-to-F13B locus. For example, polymorphisms can be detected by sequencing exons, introns, or untranslated sequences, denaturing high performance liquid chromatography (DHPLC), allele-specific hybridization, allele-specific restriction digests, mutation specific polymerase chain reactions, single-stranded conformational polymorphism detection, and combinations of such methods.

In one aspect, polymorphisms can be detected in a target nucleic acid isolated from a subject. Typically genomic DNA is analyzed. For assay of genomic DNA, virtually any biological sample containing genomic DNA or RNA, e.g., nucleated cells, is suitable. For example, in the experiments described in the Examples section herein, genomic DNA was obtained from peripheral blood leukocytes collected from case and control subjects (QIAamp DNA Blood Maxi kit, Qiagen, Valencia, Calif.). Other suitable samples include, but are not limited to, saliva, cheek scrapings, biopsies of retina, kidney or liver or other organs or tissues; skin biopsies; amniotic fluid or CNS samples; and the like. In one aspect RNA or cDNA can be assayed. In one aspect, the assay can detect variant proteins encoded by one or more genes present in the CFH-to-F13B. Methods for purification or partial purification of nucleic acids or proteins from patient samples for use in diagnostic or other assays are known in the art.

By "isolated nucleic acid" or "purified nucleic acid" is meant DNA that is free of the genes that, in the naturally-occurring genome of the organism from which the DNA disclosed herein is derived, flank the gene. The term therefore includes, for example, a recombinant DNA which is incorporated into a vector, such as an autonomously replicating plasmid or virus; or incorporated into the genomic DNA of a prokaryote or eukaryote (e.g., a transgene); or which exists as a separate molecule (for example, a cDNA or a genomic or cDNA fragment produced by PCR, restriction endonuclease digestion, or chemical or in vitro synthesis). It also includes a recombinant DNA which is part of a hybrid gene encoding additional polypeptide sequence. The term "isolated nucleic acid" also refers to RNA, e.g., an mRNA molecule that is encoded by an isolated DNA molecule, or that is chemically synthesized, or that is separated or substantially free from at least some cellular components, for example, other types of RNA molecules or polypeptide molecules.

The identity of bases occupying the polymorphic sites in CFH-to-F13B locus can be determined in a subject, e.g., in a patient being analyzed, using any of several methods known in the art. For example, and not to be limiting use of allele-specific probes, use of allele-specific primers, direct sequence analysis, denaturing gradient gel electrophoresis (DGGE) analysis, single-strand conformation polymorphism (SSCP) analysis, and denaturing high performance liquid chromatography (DHPLC) analysis. Other well known methods to detect polymorphisms in DNA include use of: Molecular Beacons technology (see, e.g., Piatek et al., 1998; Nat. Biotechnol. 16:359-63; Tyagi, and Kramer, 1996, Nat. Biotechnology 14:303-308; and Tyagi, et al., 1998, Nat. Biotechnol. 16:49-53), Invader technology (see, e.g., Neri et al., 2000, Advances in Nucleic Acid and Protein Analysis 3826:117-125 and U.S. Pat. No. 6,706,471), nucleic acid sequence based amplification (Nasba) (Compton, 1991), Scorpion technology (Thelwell et al., 2000, Nuc. Acids Res, 28:3752-3761 and Solinas et al., 2001, "Duplex Scorpion primers in SNP analysis and FRET applications" Nuc. Acids Res, 29:20.), restriction fragment length polymorphism (RFLP) analysis, and the like. Additional methods will be apparent to one of skill in the art.

The term "primer" refers to a single-stranded oligonucleotide capable of acting as a point of initiation of template-directed DNA synthesis under appropriate conditions, in an appropriate buffer and at a suitable temperature. The appropriate length of a primer depends on the intended use of the primer but typically ranges from 15 to 30 nucleotides. A primer sequence need not be exactly complementary to a template but must be sufficiently complementary to hybridize with a template. The term "primer site" refers to the area of the target DNA to which a primer hybridizes. The term "primer pair" means a set of primers including a 5' upstream primer, which hybridizes to the 5' end of the DNA sequence to be amplified and a 3' downstream primer, which hybridizes to the complement of the 3' end of the sequence to be amplified.

Exemplary hybridization conditions for short probes and primers is about 5 to 12 degrees C, below the calculated Tm. Formulas for calculating Tm are known and include: Tm = 4°C x (number of G's and C's in the primer) + 2°C x (number of A's and T's in the primer) for oligos <14 bases and assumes a reaction is carried out in the presence of 50 mM monovalent cations. For longer oligos, the following formula can be used: Tm = 64.9°C + 41°C x (number of G's and C's in the primer-16.4)/N, where N is the length of the primer. Another commonly used formula takes into account the salt concentration of the reaction (Rychlik, supra, Sambrook, supra, Mueller, supra.): Tm = 81.5°C + 16.6°C x (log 10[Na+]+[K+]) + 0.41°C x (% GC) - 675/N, where N is the number of nucleotides in the oligo. The aforementioned formulas provide a starting point for certain applications; however, the design of particular probes and primers may take into account additional or different factors. Methods for design of probes and primers for use in the methods of the invention are well known in the art.

The design and use of allele-specific probes for analyzing polymorphisms are described by e.g., Saiki et al., 1986; Dattagupta, EP 235,726, Saiki, WO 89/11548. Briefly, allele-specific probes are designed to hybridize to a segment of target DNA from one individual but not to the corresponding segment from another individual, if the two segments represent different polymorphic forms. Hybridization conditions are chosen that are sufficiently stringent so that a given probe essentially hybridizes to only one of two alleles. Typically, allele-specific probes can be designed to hybridize to a segment of target DNA such that the polymorphic site aligns with a central position of the probe.

As used herein, "probes" are nucleic acids capable of binding in a base-specific manner to a complementary strand of nucleic acid. Such probes include nucleic acids and peptide nucleic acids (Nielsen et al., 1991). Hybridization may be performed under stringent conditions which are known in the art. For example, see Berger and Kimmel (1987) Methods In Enzymology, Vol. 152: Guide To Molecular Cloning Techniques, San Diego: Academic Press, Inc.; Sambrook et al. (1989) Molecular Cloning: A Laboratory Manual, 2nd Ed., Vols. 1-3, Cold Spring Harbor Laboratory; Sambook (2001) 3rd Edition; Rychlik, W. and Rhoads, R. E., 1989, Nucl. Acids Res. 17, 8543; Mueller, P. R. et al. (1993) In: Current Protocols in Molecular Biology 15.5, Greene Publishing Associates, Inc. and John Wiley and Sons, New York; and Anderson and Young, Quantitative Filter Hybridization in Nucleic Acid Hybridization (1985)). As used herein, the term "probe" includes primers. Probes and primers are sometimes referred to as "oligonucleotides."

Allele-specific probes can be used in pairs, one member of a pair designed to hybridize to the reference allele of a target sequence and the other member designed to hybridize to the variant allele. Several pairs of probes can be immobilized on the same support for simultaneous analysis of multiple polymorphisms within the same target gene sequence.

The design and use of allele-specific primers for analyzing polymorphisms are described by, e.g., WO 93/22456 and Gibbs, 1989. Briefly, allele-specific primers are designed to hybridize to a site on target DNA overlapping a polymorphism and to prime DNA amplification according to standard PCR protocols only when the primer exhibits perfect complementarity to the particular allelic form. A single-base mismatch prevents DNA amplification and no detectable PCR product is formed. The method works best when the polymorphic site is at the extreme 3'-end of the primer, because this position is most destabilizing to elongation from the primer.

In some embodiments, genomic DNA can be used to detect polymorphisms in the CFH-to-F13B locus. Genomic DNA can be extracted from a sample, such as a peripheral blood sample or a tissue sample. Standard methods can be used to extract genomic DNA from a sample, such as phenol extraction. In some cases, genomic DNA can be extracted using a commercially available kit (e.g., from Qiagen, Chatsworth, Calif.; Promega, Madison, Wis.; or Gentra Systems, Minneapolis, Minn.).

Other methods for detecting polymorphisms can involve amplifying a nucleic acid from a sample obtained from a subject (e.g., amplifying the segments of nucleic acids from the CFH-to-F13B locus using CFH-to-F13B locus-specific primers) and analyzing the amplified nucleic acids. This can be accomplished by standard polymerase chain reaction (PCR, qPCT, & RT-PCR) protocols or other methods known in the art. The amplifying can result in the generation of CFH/F13B allele-specific oligonucleotides, which span the single nucleotide polymorphic sites in the CFH-to-F13B locus. The CFH/F13B specific primer sequences and CFH/F13B allele-specific oligonucleotides can be derived from the coding (exons) or non-coding (promoter, 5' untranslated, introns or 3' untranslated) regions of the CFH-to-F13B locus. In one aspect Genomic DNA from all subjects can be isolated from peripheral blood leukocytes with QIAamp DNA Blood Maxi kits (Qiagen, Valencia, CA). DNA samples can be screened for SNPs in the genes contained within the CFH-to-F13B locus. Genotyping can be performed byTaqMan assays (Applied Biosystems, Foster City, California) using 10 ng of template DNA in a 5uL reaction. The thermal cycling conditions in the 384-well thermocycler (PTC-225, MJ Research) can consist of an initial hold at 95°C for 10 minutes, followed by 40 cycles of a 15-second 95°C denaturation step and a 1-minute 60°C annealing and extension step. Plates can be read in the 7900HT Fast Real-Time PCR System (Applied Biosystems).

Amplification products generated using PCR can be analyzed by the use of denaturing gradient gel electrophoresis (DGGE). Different alleles can be identified based on sequence-dependent melting properties and electrophoretic migration in solution. See Erlich, ed., PCR Technology, Principles and Applications for DNA Amplification, Chapter 7 (W.H. Freeman and Co, New York, 1992).

Alleles of target sequences can be differentiated using single-strand conformation polymorphism (SSCP) analysis. Different alleles can be identified based on sequence- and structure-dependent electrophoretic migration of single stranded PCR products (Orita et al., 1989). Amplified PCR products can be generated according to standard protocols and heated or otherwise denatured to form single stranded products, which may refold or form secondary structures that are partially dependent on base sequence.

Alleles of target sequences can be differentiated using denaturing high performance liquid chromatography (DHPLC) analysis. Different alleles can be identified based on base differences by alteration in chromatographic migration of single stranded PCR products (Frueh and Noyer-Weidner, 2003). Amplified PCR products can be generated according to standard protocols and heated or otherwise denatured to form single stranded products, which may refold or form secondary structures that are partially dependent on the base sequence.

Direct sequence analysis of polymorphisms can be accomplished using DNA sequencing procedures that are well-known in the art. See Sambrook et al., Molecular Cloning, A Laboratory Manual (2nd Ed., CSHP, New York 1989) and Zyskind et al., Recombinant DNA Laboratory Manual (Acad. Press, 1988).

A wide variety of other methods are known in the art for detecting polymorphisms in a biological sample. See, e.g., Ullman et al. "Methods for single nucleotide polymorphism detection" U.S. Pat. No. 6,632,606; Shi, 2002, "Technologies for individual genotyping: detection of genetic polymorphisms in drug targets and disease genes" Am J Pharmacogenomics 2:197-205; Kwok et al., 2003, "Detection of single nucleotide polymorphisms" Curr Issues Biol. 5:43-60).

Also described herein are methods methods for determining a female Caucasian subject's susceptibility to having or developing a complement-mediated disease comprising determining in the female Caucasian subject the identity of one or more major or minor haplotypes. The haplotypes can include, but are not limited to H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, or a complement thereof.

In one aspect, determining the identity of a certain haplotype or certain haplotypes in a female subject can indicate that the female subject has an increased risk of having or developing a complement-mediated disease. Such hapltoypes can be risk haplotypes. For example, and not to be limiting, a risk haplotype can be H1_62_A, H2_62_A, H1_51_B, H2_51_B,or a complement thereof. Thus, described herein are methods for determining a female Caucasian subject's susceptibility to having or developing a complement-mediated disease comprising determining in the female Caucasian subject the identity of one or more haplotypes described herein, wherein a risk haplotype is indicative of the female subject's increased risk for having or developing a complement-mediated disease.

In another aspect, determining the identity of a certain haplotype or certain haplotypes in a female subject can indicate that the female subject has a deacreased risk of having or developing a complement-mediated disease. Such haplotypes can be protective haplotypes. For example, and not to be limiting, a protective haplotype can be H3_62_A, H5_62_A, H11_62_A, H3_51_B, H5_51_B, H12_51_B, H14_51_B, or a complement thereof. Thus, described herein are methods for determining a female Caucasian subject's susceptibility to having or developing a complement-mediated disease comprising determining in the female Caucasian subject the identity of one or more haplotypes described herein, wherein a protective haplotype is indicative of the female subject's decreased risk for having or developing a complement-mediated disease.

In yet another aspect, determining the identity of a certain haplotype or certain haplotypes in a female subject can indicate that the female subject does not have an increased risk or a decreased risk of having or developing a complement-mediated disease. Such haplotypes can be neutral haplotypes. For example, and not to be limiting, a neutral haplotype can be H4_62_A, H6_62_A, H7_62_A, H8_62_A, H9_62_A, H10_62_A, H12_62_A, H13_62_A, H14_62_A, H15_62_A, H4_51_B, H6_51_B, H7_51_B, H8_51_B, H9_51_B, H10_51_B, H11_51_B, H13_51_B, H15_51_B, H16_51_B, H17_51_B, H18_51_B, H19_51_B, H20_51_B,or a complement thereof. Thus, described herein are methods for determining a female Caucasian subject's susceptibility to having or developing a complement-mediated disease comprising determining in the female Caucasian subject the identity of one or more haplotypes described herein, wherein a neutral haplotype does not indicate that the female subject has an increased risk or a decreased risk for having or developing a complement-mediated disease.

Also described herein are methods methods for determining a male Caucasian subject's susceptibility to having or developing a complement-mediated disease comprising determining in the male Caucasian subject the identity of one or more major or minor haplotypes. The haplotypes can include, but are not limited to H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, or a complement thereof.

In one aspect, determining the identity of a certain haplotype or certain haplotypes in a male subject can indicate that the male subject has a increased risk of having or developing a complement-mediated disease. Such hapltoypes can be risk haplotypes. For example, and not to be limiting, a risk haplotype can be H2_62_A, H1_51_B, H2_51_B, or a complement thereof. Thus, described herein are methods for determining a male Caucasian subject's susceptibility to having or developing a complement-mediated disease comprising determining in the male Caucasian subject the identity of one or more haplotypes described herein, wherein a risk haplotype is indicative of the male subject's increased risk for having or developing a complement-mediated disease.

In another aspect, determining the identity of a certain haplotype or certain haplotypes in a male subject can indicate that the male subject has a deacreased risk of having or developing a complement-mediated disease. Such haplotypes can be protective haplotypes. For example, and not to be limiting, a protective haplotype can be H11_62_A, H3_51_B, H14_51_B, or a complement thereof. Thus, described herein are methods for determining a male Caucasian subject's susceptibility to having or developing a complement-mediated disease comprising determining in the male Caucasian subject the identity of one or more haplotypes described herein, wherein a protective haplotype is indicative of the male subject's decreased risk for having or developing a complement-mediated disease.

In yet another aspect, determining the identity of a certain haplotype or certain haplotypes in a male subject can indicate that the male subject does not have an increased risk or a decreased risk of having or developing a complement-mediated disease. Such haplotypes can be neutral haplotypes. For example, and not to be limiting, a neutral haplotype can be H1_62_A, H3_62_A, H4_62_A, H5_62_A, H6_62_A, H7_62_A, H8_62_A, H9_62_A, H10_62_A, H12_62_A, H13_62_A, H14_62_A, H15_62_A H4_51_B, H5_51_B, H6_51_B, H7_51_B, H8_51_B, H9_51_B, H10_51_B, H11_551_B, H12_51_B, H13_51_B, H15_51_B, H16_51_B, H17_51_B, H18_51_B, H19_51_B, H20_51_B, H21_51_B, H22_51_B, or a complement thereof. Thus, described herein are methods for determining a male Caucasian subject's susceptibility to having or developing a complement-mediated disease comprising determining in the male Caucasian subject the identity of one or more haplotypes described herein, wherein a neutral haplotype does not indicate that the male subject has an increased risk or a decreased risk for having or developing a complement-mediated disease.

In one aspect, the SNPs, haplotypes, or diplotypes described herein can be determined from a sample obtained from the subject. In another aspect, the subject's SNPs, haplotype, or diplotype can be determined by amplifying or sequencing a nucleic acid sample obtained from the subject. In yet a further aspect, the methods descrined herein can further comprise administering a therapeutic composition to the subject or treating the subject with an effective amount of a therapeutic composition.

As used herein, "treating" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. In various aspects, the term covers any treatment of a subject, including a mammal (e.g., a human), and includes: (i) preventing the disease from occurring in a subject that can be predisposed to the disease but has not yet been diagnosed as having it; (ii) inhibiting the disease, i.e., arresting its development; or (iii) relieving the disease, i.e., causing regression of the disease.

The terms "administering" and "administration" refer to any method of providing a pharmaceutical preparation to a subject. Such methods are well known to those skilled in the art and include, but are not limited to, oral administration, sublingual administration, trans-buccal mucosa administration, transdermal administration, administration by inhalation, nasal administration, topical administration, intravaginal administration, ophthalmic administration, intraaural administration, intracerebral administration, intrathecal administration, rectal administration, intraperitoneal administration, and parenteral administration, including injectable such as intravenous administration, intra-arterial administration, intramuscular administration, intradermal administration, and subcutaneous administration. Ophthalmic administration can include topical administration, subconjunctival administration, sub-Tenon's administration, epibulbar administration, retrobulbar administration, intra-orbital administration, and intraocular administration, which includes intra-vitreal administration. Administration can be continuous or intermittent. In various aspects, a preparation can be administered therapeutically; that is, administered to treat an existing disease or condition. In further various aspects, a preparation can be administered prophylactically; that is, administered for prevention of a disease or condition.

As used herein, the term "therapeutically effective amount" refers to an amount that is sufficient to achieve the desired result or to have an effect on an undesired condition.

In yet another aspect, the methods for determining a Caucasian subject's susceptibility to having or developing a complement-mediated disease described herein can further comprise examining the subject with an ophthalmological procedure.

Various ophthalmological procedures known to persons of ordinary skill in the art can be performed to examine the subject including, but not limited to, autofluorescent imaging techniques, infrared imaging techniques, optical coherence tomography (OCT), Stratus optical coherence tomography (Stratus OCT), Fourier-domain optical coherence tomography (Fd-OCT), two-photon-excited fluorescence (TPEF) imaging, adaptive optics scanning laser ophthalmoscopy (AOSLO), scanning laser ophthalmoscopy, near-infrared imaging combined with spectral domain optical coherence tomography (SD-OCT), color fundus photography, fundus autofluorescence imaging, red-free imaging, fluorescein angiography, indocyanin green angiography, multifocal electroretinography (ERG) recording, microperimetry, color Doppler optical coherence tomography (CDOCT), and visual field assessment. Additionally, the subject can be examined by using the Heidelberg Spectralis, the Zeiss Cirrus, the Topcon 3D OCT 2000, the Optivue RTVue SD-OCT, the Opko OCT SLO, the NIDEK F-10, or the Optopol SOCT Copernicus HR.

### 1. PREDICTING PROGRESSION OF DISEASE

Also described herein are methods for predicting progression of a complement-mediated disease in a Caucasian subject. The complement-mediated disease can be, but is not limited to, age-related macular degeneration. In one aspect the methods comprise determining in the Caucasian subject the identity of one or more major or minor haplotypes. The haplotypes can include, but are not limited to H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H1_51_A, H2_51_A, H3_51_A, H4_51_A, H5_51_A, H6_51_A, H7_51_A, H8_51_A, H9_51_A, H10_51_A, H11_51_A, H12_51_A, H13_51_A, H14_51_A, H15_51_A, H16_51_A, H17_51_A, H1_51_B, H2_51_B,H3_51_B, H4_5_B, H5_51_B, H6_51_B, H7_51_B, H8_51_B, H9_51_B, H10_51_B, H11_551_B, H12_51_B, H13_51_B, H14_51_B, H15_51_B, H16_51_B, H17_51_B, H18_51_B, H19_51_B, H20_51_B, H21_51_B, H22_51_B, or a complement thereof. As described herein, the presence of one or more of the haplotypes disclosed herein can indicate a subject's susceptibility for having or developing late-stage age-related macular degeneration.

In one aspect, determining the identity of a certain haplotype or certain haplotypes in a subject can indicate that the subject has a increased risk of having or developing late-stage age-related macular degeneration. Such hapltoypes can be risk haplotypes. For example, and not to be limiting, a risk haplotype can be H2_62_A, H2_51_A, H1_51_B, H2_51_B, or a complement thereof. Thus, described herein are methods for determining a Caucasian subject's susceptibility to having or developing late-stage age-related macular degeneration comprising determining in the Caucasian subject the identity of one or more haplotypes described herein, wherein a risk haplotype is indicative of the subject's increased risk for having or developing late-stage age-related macular degeneration.

In another aspect, determining the identity of a certain haplotype or certain haplotypes in a subject can indicate that the subject has a deacreased risk of having or developing late-stage age-related macular degeneration. Such haplotypes can be protective haplotypes. For example, and not to be limiting, a protective haplotype can be H3_62_A, H5_62_A, H11_62_A, H3_51_A, H5_51_A, H10_51_A, H3_51_B, H5_51_B, H12_51_B, H14_51_B, or a complement thereof. Thus, described herein are methods for determining a Caucasian subject's susceptibility to having or developing late-stage age-related macular degeneration comprising determining in the Caucasian subject the identity of one or more haplotypes described herein, wherein a protective haplotype is indicative of the subject's decreased risk for having or developing late-stage age-related macular degeneration.

In yet another aspect, determining the identity of a certain haplotype or certain haplotypes in a subject can indicate that the subject does not have an increased risk or a decreased risk of having or developing late-stage age-related macular degeneration. Such haplotypes can be neutral haplotypes. For example, and not to be limiting, a neutral haplotype can be H1_62_A, H4_62_A, H6_62_A, H7_62_A, H8_62_A, H9_62_A, H10_62_A, H12_62_A, H13_62_A, H14_62_A, H15_62_A, H1_51_A, H4_51_A, H6_51_A, H7_51_A, H8_51_A, H9_51_A, H11_51_A, H12_51_A, H13_51_A, H14_51_A, H15_51_A, H16_51_A, H17_51_A, H4_51_B, H6_51_B, H7_51_B, H8_51_B, H9_51_B, H10_51_B, H11_51_B, H13_51_B, H15_51_B, H16_51_B, H17_51_B, H18_51_B, H19_51_B, H20_51_B, H21_51_B, H22_51_B, or a complement thereof. Thus, described herein are methods for determining a Caucasian subject's susceptibility to having or developing late-stage age-related macular degeneration comprising determining in the Caucasian subject the identity of one or more haplotypes described herein, wherein a neutral haplotype does not indicate that the subject has an increased risk or a decreased risk for having or developing late-stage age-related macular degeneration.

Also described herein are methods methods for determining a female Caucasian subject's susceptibility to having or developing late-stage age-related macular degeneration comprising determining in the female Caucasian subject the identity of one or more major or minor haplotypes. The haplotypes can include, but are not limited to H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, or a complement thereof.

In one aspect, determining the identity of a certain haplotype or certain haplotypes in a female subject can indicate that the female subject has a increased risk of having or developing late-stage age-related macular degeneration. Such hapltoypes can be risk haplotypes. For example, and not to be limiting, a risk haplotype can be H1_62_A, H2_62_A, H1_51_B, H2_51_B, or a complement thereof. Thus, described herein are methods for determining a female Caucasian subject's susceptibility to having or developing late-stage age-related macular degeneration comprising determining in the female Caucasian subject the identity of one or more haplotypes described herein, wherein a risk haplotype is indicative of the female subject's increased risk for having or developing late-stage age-related macular degeneration.

In another aspect, determining the identity of a certain haplotype or certain haplotypes in a female subject can indicate that the female subject has a deacreased risk of having or developing late-stage age-related macular degeneration. Such haplotypes can be protective haplotypes. For example, and not to be limiting, a protective haplotype can be H3_62_A, H5_62_A, H11_62_A, H3_51_B, H5_51_B, H12_51_B, H14_51_B, or a complement thereof. Thus, described herein are methods for determining a female Caucasian subject's susceptibility to having or developing late-stage age-related macular degeneration comprising determining in the female Caucasian subject the identity of one or more haplotypes described herein, wherein a protective haplotype is indicative of the female subject's decreased risk for having or developing late-stage age-related macular degeneration.

In yet another aspect, determining the identity of a certain haplotype or certain haplotypes in a female subject can indicate that the female subject does not have an increased risk or a decreased risk of having or developing late-stage age-related macular degeneration. Such haplotypes can be neutral haplotypes. For example, and not to be limiting, a neutral haplotype can be H4_62_A, H6_62_A, H7_62_A, H8_62_A, H9_62_A, H10_62_A, H12_62_A, H13_62_A, H14_62_A, H15_62_A, H4_51_B, H6_51_B, H7_51_B, H8_51_B, H9_51_B, H10_51_B, H11_51_B, H13_51_B, H15_51_B, H16_51_B, H17_51_B, H18_51_B, H19_51_B, H20_51_B, or a complement thereof. Thus, described herein are methods for determining a female Caucasian subject's susceptibility to having or developing late-stage age-related macular degeneration comprising determining in the female Caucasian subject the identity of one or more haplotypes described herein, wherein a neutral haplotype does not indicate that the female subject has an increased risk or a decreased risk for having or developing late-stage age-related macular degeneration.

Also described herein are methods methods for determining a male Caucasian subject's susceptibility to having or developing late-stage age-related macular degeneration comprising determining in the male Caucasian subject the identity of one or more major or minor haplotypes. The haplotypes can include, but are not limited to H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, or a complement thereof.

In one aspect, determining the identity of a certain haplotype or certain haplotypes in a male subject can indicate that the male subject has a increased risk of having or developing late-stage age-related macular degeneration. Such hapltoypes can be risk haplotypes. For example, and not to be limiting, a risk haplotype can be H2_62_A, H1_51_B, H2_51_B, or a complement thereof. Thus, described herein are methods for determining a male Caucasian subject's susceptibility to having or late-stage age-related macular degeneration comprising determining in the male Caucasian subject the identity of one or more haplotypes described herein, wherein a risk haplotype is indicative of the male subject's increased risk for having or developing late-stage age-related macular degeneration.

In another aspect, determining the identity of a certain haplotype or certain haplotypes in a male subject can indicate that the male subject has a deacreased risk of having or developing late-stage age-related macular degeneration. Such haplotypes can be protective haplotypes. For example, and not to be limiting, a protective haplotype can be H11_62_A, H3_51_B, H14_51_B, or a complement thereof. Thus, described herein are methods for determining a male Caucasian subject's susceptibility to having or developing late-stage age-related macular degeneration comprising determining in the male Caucasian subject the identity of one or more haplotypes described herein, wherein a protective haplotype is indicative of the male subject's decreased risk for having or developing late-stage age-related macular degeneration.

In yet another aspect, determining the identity of a certain haplotype or certain haplotypes in a male subject can indicate that the male subject does not have an increased risk or a decreased risk of having or developing late-stage age-related macular degeneration. Such haplotypes can be neutral haplotypes. For example, and not to be limiting, a neutral haplotype can be H1_62_A, H3_62_A, H4_62_A, H5_62_A, H6_62_A, H7_62_A, H8_62_A, H9_62_A, H10_62_A, H12_62_A, H13_62_A, H14_62_A, H15_62_A H4_51_B, H5_51_B, H6_51_B, H7_51_B, H8_51_B, H9_51_B, H10_51_B, H11_51_B, H12_51_B, H13_51_B, H15_51_B, H16_51_B, H17_51_B, H18_51_B, H19_51_B, H20_51_B, H21_51_B, H22_51_B, or a complement thereof. Thus, described herein are methods for determining a male Caucasian subject's susceptibility to having or developing late-stage age-related macular degeneration comprising determining in the male Caucasian subject the identity of one or more haplotypes described herein, wherein a neutral haplotype does not indicate that the male subject has an increased risk or a decreased risk for having or developing late-stage age-related macular degeneration.

### 2. DIPLOTYPES

Also described herein are methods for determining a Caucasian subject's susceptibility to having or developing a complement-mediated disease comprising determining in the Caucasian subject the identity of one or more diplotypes described herein. Human autosomal chromosomes normally come in pairs, and the combination in one individual of these two haplotypes is referred to herein as a "Diplotype". As such, "based on" when used in the context of a diplotype "based on" a haplotype, can refer to identifying the combination in one individual of these two haplotypes.

Any haplotype or haplotypes described herein can be used to create a diplotype. For example, a diplotype can comprise a protective and a risk haplotype, two protective haplotypes, two risk haplotypes, a neutral and a risk haplotype, a neutral and a protective haplotype, or two neutral haplotypes. In an aspect, one haplotype may be dominant or one haplotype may be recessive.

For example, and not to be limiting, a single H1 haplotype when combined with another H1 haplotype can be an H1_Y_Z:H1_Y_Z diplotype; a single H1 haplotype when combined a H2, H8, or H13 haplotype can be an H1_Y_Z:H2_Y_Z diplotype, an H1_Y_Z:H8_Y_Z diplotype, or an H1_Y_Z:H13_Y_Z diplotype. In one aspect, the diplotypes described herein can be associated with increased risk for developing AMD. In another aspect, the diplotypes described herein can be associated with a decreased risk for developing AMD.

Described hereis are methods for determining a Caucasian subject's susceptibility to having or developing age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, and wherein the presence of one or more of the diplotypes indicates the subject's susceptibility for having or developing age-related macular degeneration. In some aspects, the subject's haplotype can be determined from a sample obtained from the subject. In some aspects the subject's haplotype can be determined by amplifying or sequencing a nucleic acid sample obtained from the subject.

Described hereis are methods for determining a Caucasian subject's susceptibility to having or developing age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_5_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, and wherein the presence of one or more of the diplotypes indicates the subject's susceptibility for having or developing age-related macular degeneration,

Described hereis are methods for determining a Caucasian subject's susceptibility to having or developing age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, wherein the diplotype H1_51_B: H1_51_B, H1_51_B:H2_51_B, H1_51_B:H8_51_B, H1_51_B:H13_51_B, H2_51_B:H2_51_B, H2_51_B:H10_51_B, H1_51_B:any minor 51_B haplotype, H2_51_B:any minor 51_B haplotype, or a complement thereof, is indicative of the subject's increased risk for having or developing age-related macular degeneration.

In some aspects wherein an increased risk is identified, the method can further comprise administering a therapeutic composition to the subject.

Described hereis are methods for determining a Caucasian subject's susceptibility to having or developing age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_5_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, wherein diplotype H1_51_B: H3_51_B, H1_51_B:H5_51_B, H1_51_B:H14__51_B, H3_51_B: H3_51_B, H3_51_B: H4_51_B, H3_51_B: H5_51_B, H3_51_B: H6_51_B, H3_51_B: any 51_B minor haplotype, H4_51_B: H5_51_B, H5_51_B: H5_51_B, H5_51_B: any 51_B minor haplotype, H6_51_B: H6_51_B, H6_51_B: any 51_B minor haplotype, or a complement thereof, is indicative of the subject's decreased risk for having or developing age-related macular degeneration.

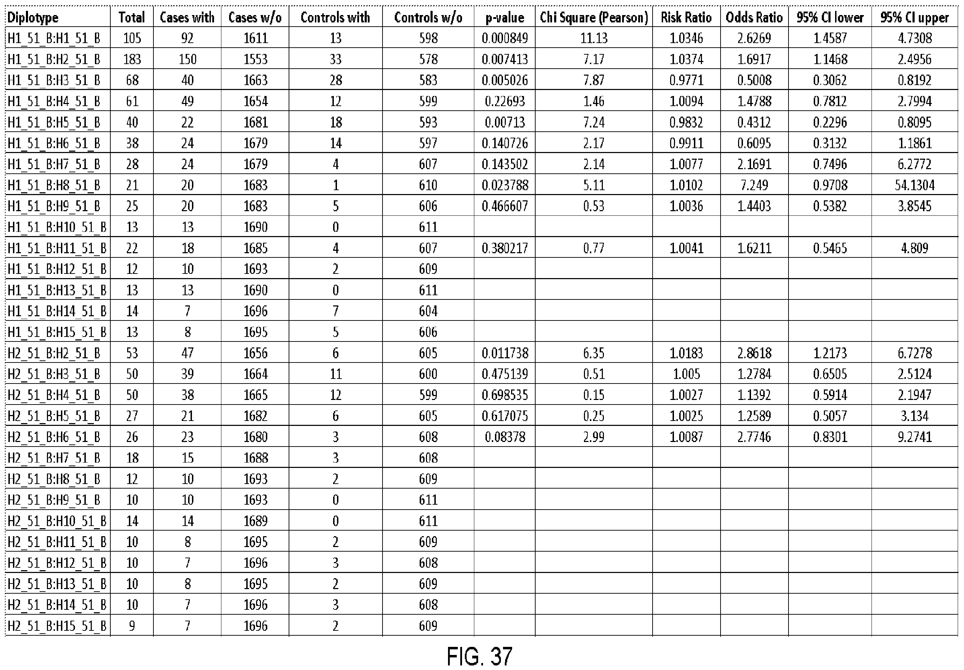

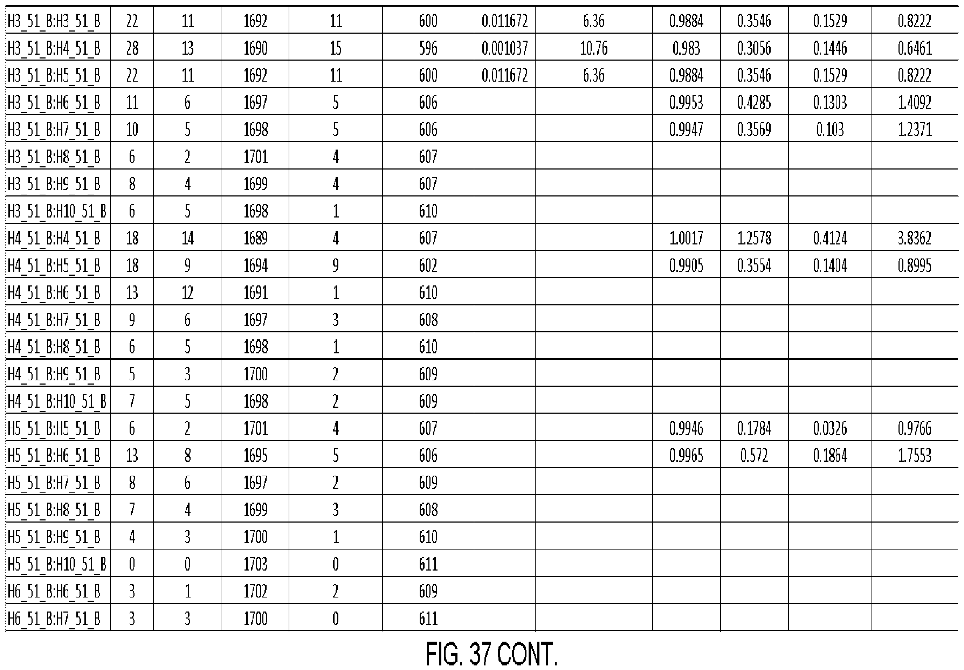

In some aspects the subject can be female. In some aspects wherein the subject is female, one or more of the diplotypes identified in Figure 37 can be used to indicate the subject's risk for having or developing age-related macular degeneration.

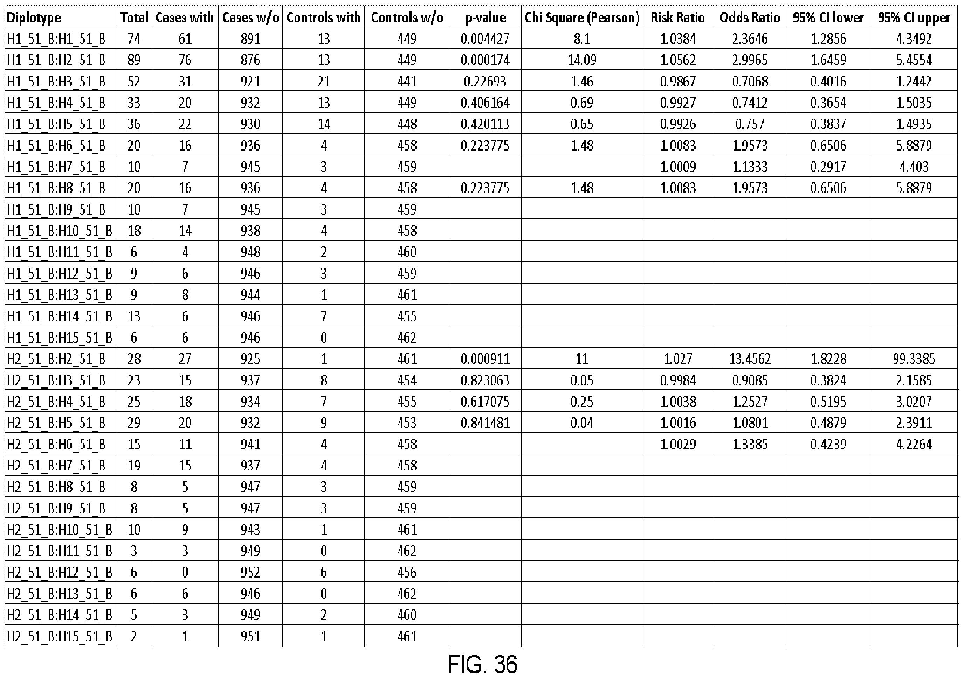

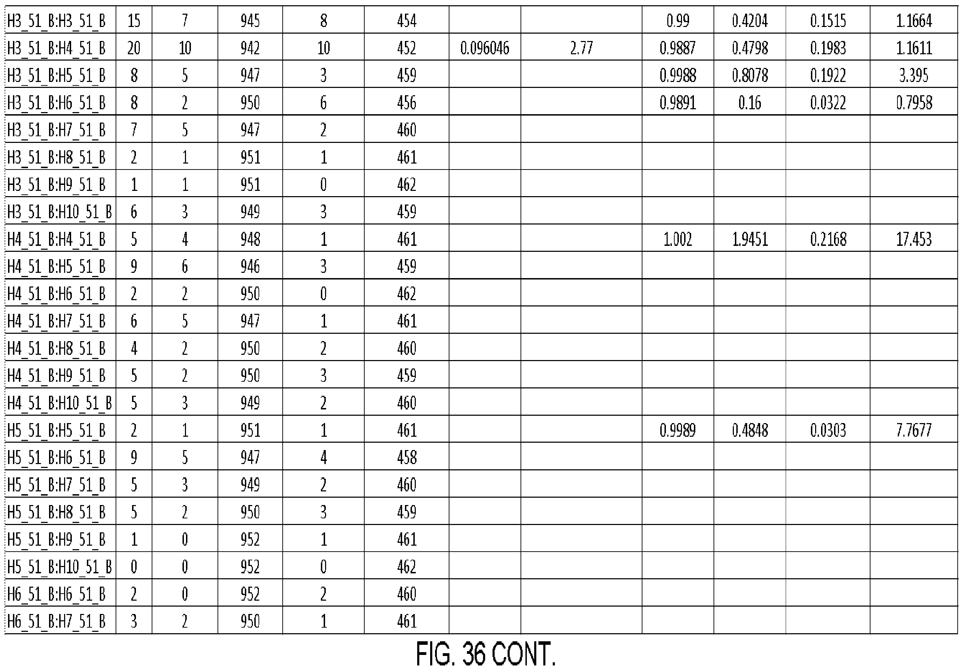

In some aspects the subject can be male. In some aspects wherein the subject is male, one or more of the diplotypes identified in Figure 36 can be used to indicate the subject's risk for having or developing age-related macular degeneration.

Described herein are methods of identifying a Caucasian subject in need of treatment for age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, and wherein the presence of one or more of the diplotypes indicates whether the subject is in need of treatment for age-related macular degeneration.

In some aspects, the subject's haplotype can be determined from a sample obtained from the subject. In some aspects the subject's haplotype can be determined by amplifying or sequencing a nucleic acid sample obtained from the subject.

Described herein are methods of identifying a Caucasian subject in need of treatment for age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, wherein diplotype H1_51_B: H1_51_B, H1_51_B:H2_51_B, H1_51_B:H8_51_B, H1_51_B:H13_51_B, H2_51_B:H2_51_B, H2_51_B:H10_51_B, H1_51_B:any minor 51_B haplotype, H2_51_B:any minor 51_B haplotype, or a complement thereof, indicates the subject is in need of treatment for age-related macular degeneration.

Described herein are methods of identifying a Caucasian subject in need of treatment for age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, wherein diplotype H1_51_B: H3_51_B, H1_51_B:H5_51_B, H1_51_B:H14_51_B, H3_51_B: H3_51_B, H3_51_B: H4_51_B, H3_51_B: H5_51_B, H3_51_B: H6_51_B, H3_51_B: any 51_B minor haplotype, H4_51_B: H5_51_B, H5_51_B: H5_51_B, H5_51_B: any 51_B minor haplotype, H6_51_B: H6_51_B, H6_51_B: any 51_B minor haplotype, or a complement thereof, indicates the subject is not in need of treatment for age-related macular degeneration.

Described herein are methods of identifying a Caucasian subject in need of treatment for age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_5_51_B, H12_51_B, H13_51_B, H14_51_B, H15_51_B, H16_51_B, H17_51_B, H18_51B, H19_51_B, H20_51_B, H21_51_B, H22_51_B, or a complement thereof, wherein the presence of one or more of the diplotypes identified in Figure 36 can be used to determine whether, or a complement thereof, can indicates the subject may be in need of treatment for age-related macular degeneration. In some aspects the subject can be female. In some aspects wherein the subject is female, one or more of the diplotypes identified in Figure 37 can be used to deterime whether the subject is in need of treatment for age-related macular degeneration. In some aspects the subject can be male. In some aspects wherein the subject is male, one or more of the diplotypes identified in Figure 36 can be used to determine whether the subject is in need of treatment for age-related macular degeneration.

Described herein are methods of identifying a Caucasian subject in need of a prophylactic treatment for age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_5_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, and wherein the presence of one or more of the diplotypes indicates whether the subject is in need of prophylactic treatment for age-related macular degeneration.

Described herein are methods of identifying a Caucasian subject in need of a prophylactic treatment for age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11__51_B, H12_51_B, H13_51_B, H14_51_B, H15_51_B, H16_51_B, H17_51_B, H18_51_B, H19_51_B, H20_51_B, H21_51_B, H22_51_B, or a complement thereof, wherein diplotype H1_51_B: H1_51_B, H1_51_B:H2_51_B, H1_51_B:H8_51_B, H1_51_B:H13_51_B, H2_51_B:H2_51_B, H2_51_B:H10_51_B, H1_51_B:any minor 51_B haplotype, H2_51_B:any minor 51_B haplotype, or a complement thereof, indicates the subject is in need of prophylactic treatment for age-related macular degeneration.

Described herein are methods of identifying a Caucasian subject in need of a prophylactic treatment for age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, wherein diplotype H1_51_B: H3_51_B, H1_51_B:H5_51_B, H1_51_B:H14_51_B, H3_51_B: H3_51_B, H3_51_B: H4_51_B, H3_51_B: H5_51_B, H3_51_B: H6_51_B, H3_51_B: any 51_B minor haplotype, H4_51_B: H5_51_B, H5_51_B: H5_51_B, H5 51_B: any 51_B minor haplotype, H6_51_B: H6_51_B, H6_51_B: any 51_B minor haplotype, or a complement thereof, indicates the subject is not in need of prophylactic treatment for age-related macular degeneration.

Described herein are methods of identifying a Caucasian subject in need of a prophylactic treatment for age-related macular degeneration comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_5_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, wherein the presence of one or more of the diplotypes identified in Figure 36, or a complement thereof, can be used to determine whether, the subject may be in need of prophylactic treatment for age-related macular degeneration.

Described herein are methods of identifying a Caucasian subject appropriate for an age-related macular degeneration clinical trial comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, and wherein the presence of one or more of the diplotypes indicates whether the subject is appropriate for the clinical trial.

Described herein are methods of identifying a Caucasian subject appropriate for an age-related macular degeneration clinical trial comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20 51_B, H21_51_B, H22 51_B, or a complement thereof, wherein diplotype H1_51_B: H1_51_B, H1_51_B:H2_51_B, H1_51_B:H8_51_B, H1_51_B:H13_51_B, H2_51_B:H2_51_B, H2_51_B:H10_51_B, H1_51_B:any minor 51_B haplotype, H2_51_B:any minor 51_B haplotype, or a complement thereof, indicates the subject is appropriate for the clinical trial.

Described herein are methods of identifying a Caucasian subject appropriate for an age-related macular degeneration clinical trial comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, wherein diplotype H1_51_B: H3_51_B, H1_51_B:H5_51_B, H1_51_B:H14_51_B, H3_51_B: H3_51_B, H3_51_B: H4_51_B, H3_51_B: H5_51_B, H3_51_B: H6_51_B, H3_51_B: any 51_B minor haplotype, H4_51_B: H5_51_B, H5_51_B: H5_51_B, H5_51_B: any 51_B minor haplotype, H6_51_B: H6_51_B, H6_51_B: any 51_B minor haplotype, or a complement thereof, indicates the subject is not appropriate for the clinical trial.

Described herein are methods of identifying a Caucasian subject appropriate for an age-related macular degeneration clinical trial comprising determining in the Caucasian subject the identity of one or more diplotypes based on one or more haplotypes, wherein the one or more haplotypes are H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof, wherein the presence of one or more of the diplotypes identified in Figure 36, or a complement thereof, indicates the subject may be appropriate for the clinical trial.

### 3. HAPLOTYPE TAGGING SNPs

Described herein are methods for determining a Caucasian subject's susceptibility to having or developing a complement-mediated disease comprising determining in the Caucasian subject the identity of at least six SNPs in the CFH-to-F13B locus. The SNPs can be, but are not limited to: (i) rs35928059, rs800292, rs1061170, rs12144939, rs7546940, rs1409153, rs10922153, or rs698859, or (ii) a SNP in linkage disequilibrium with the SNPs of (i). In one aspect, the presence of at least six of the SNPs can indicate the subject's susceptibility for having or developing age-related macular degeneration. In another aspect, the SNPs can be haplotype tagging SNPs (htSNPs). As used herein, haplotype tagging SNP means a SNP or multiple SNPs that can identify or represent a haplotype or multiple haplotypes described herein. A tagging SNP or multiple tagging SNPs can be used to identify or depict a haplotype or multiple haplotypes in a subject, and, therefore, can be used to determine a subject's susceptibility to having or developing a complement-mediated disease.

In one aspect, an A at rs35928059, a G at rs800292, a C at rs1061170, a G at rs12144939, a G at rs7546940, a G at rs1409153, a G at rs10922153, or an A at rs698859 can indicate the subject's increased risk for having or developing age-related macular degeneration. In another aspect, an A at rs35928059, a G at rs800292, a C at rs1061170, a G at rs12144939, a G at rs7546940, a C at rs1409153, a G at rs10922153, or a T at rs698859 can indicate the subject's increased risk for having or developing age-related macular degeneration. In another aspect, an A at rs35928059, a G at rs800292, a C at rs1061170, a G at rs12144939, a G at rs7546940, a C at rs1409153, a G at rs10922153, or a C at rs698859 can indicate the subject's increased risk for having or developing age-related macular degeneration. In yet another aspect, an A at rs35928059, an A at rs800292, a T at rs1061170, a G at rs12144939, a G at rs7546940, an A at rs1409153, a T at rs10922153, or a G at rs698859 can indicate the subject's decreased risk for having or developing age-related macular degeneration. In still another aspect, an A at rs35928059, a G at rs800292, a T at rs1061170, a T at rs12144939, a G at rs7546940, an A at rs1409153, a T at rs10922153, or a G at rs698859 can indicate the subject's decreased risk for having or developing age-related macular degeneration. In another aspect, an A at rs35928059, a G at rs800292, a T at rs1061170, a T at rs12144939, an A at rs7546940, an A at rs1409153, a T at rs10922153, or an A at rs698859 can indicate the subject's decreased risk for having or developing age-related macular degeneration. In yet another aspect, an A at rs35928059, an A at rs800292, a T at rs1061170, a G at rs12144939, a G at rs7546940, a T at rs1409153, a T at rs10922153, or a C at rs698859 can indicate the subject's decreased risk for having or developing age-related macular degeneration. In still another aspect, an A at rs35928059, a G at rs800292, a T at rs1061170, a T at rs12144939, a G at rs7546940, a T at rs1409153, a T at rs10922153, or a C at rs698859 can indicate the subject's decreased risk for having or developing age-related macular degeneration. In another aspect, an A at rs35928059, a G at rs800292, a T at rs1061170, a T at rs12144939, an A at rs7546940, a T at rs1409153, a T at rs10922153, or a T at rs698859 can indicate the subject's decreased risk for having or developing age-related macular degeneration. In yet another aspect, an A at rs35928059, a G at rs800292, a T at rs1061170, a T at rs12144939, a G at rs7546940, a T at rs1409153, a T at rs10922153, or a T at rs698859 can indicate the subject's decreased risk for having or developing age-related macular degeneration.

Also described herein are methods for determining a female Caucasian subject's susceptibility to having or developing a complement-mediated disease comprising determining in the female Caucasian subject the identity of at least six SNPs in the CFH-to-F13B locus. The SNPs can be, but are not limited to: (i) rs35928059, rs800292, rs1061170, rs12144939, rs7546940, rs1409153, rs10922153, or rs698859, or (ii) a SNP in linkage disequilibrium with the SNPs of (i). In one aspect, the presence of at least six of the SNPs can indicate the subject's susceptibility for having or developing a complement-mediated disease.

In one aspect, an A at rs35928059, a G at rs800292, a C at rs1061170, a G at rs12144939, a G at rs7546940, a G at rs1409153, a G at rs10922153, or an G at rs698859 can indicate the subject's increased risk for having or developing age-related macular degeneration. In another aspect, an A at rs35928059, a G at rs800292, a C at rs1061170, a G at rs12144939, a G at rs7546940, a G at rs1409153, a G at rs10922153, or an A at rs698859 can indicate the subject's increased risk for having or developing age-related macular degeneration. In yet another aspect, an A at rs35928059, a G at rs800292, a C at rs1061170, a G at rs12144939, a G at rs7546940, a C at rs1409153, a G at rs10922153, or a C at rs698859 can indicate the subject's increased risk for having or developing age-related macular degeneration. In still another aspect, an A at rs35928059, a G at rs800292, a C at rs1061170, a G at rs12144939, a G at rs7546940, a C at rs1409153, a G at rs10922153, or a T at rs698859 can indicate the subject's increased risk for having or developing age-related macular degeneration. In another aspect, an A at rs35928059, an A at rs800292, a T at rs1061170, a G at rs12144939, a G at rs7546940, an A at rs1409153, a T at rs10922153, or a G at rs698859 can indicate the subject's decreased risk for having or developing age-related macular degeneration. In yet another aspect, an A at rs35928059, a G at rs800292, a T at rs1061170, a T at rs12144939, a G at rs7546940, an A at rs1409153, a T at rs10922153, or a G at rs698859 can indicate the subject's decreased risk for having or developing age-related macular degeneration. In still another aspect, an A at rs35928059, a G at rs800292, a T at rs1061170, a T at rs12144939, an A at rs7546940, an A at rs1409153, a T at rs10922153, or an A at rs698859 can indicate the subject's decreased risk for having or developing age-related macular degeneration. In another aspect, an A at rs35928059, an A at rs800292, a T at rs1061170, a G at rs12144939, a G at rs7546940, a T at rs1409153, a T at rs10922153, or a C at rs698859 can indicate the subject's decreased risk for having or developing age-related macular degeneration. In yet another aspect, an A at rs35928059, a G at rs800292, a T at rs1061170, a T atrs12144939, a G at rs7546940, a T at rs1409153, a T at rs10922153, or a C at rs698859 can indicate the subject's decreased risk for having or developing age-related macular degeneration. In yet another aspect, an A at rs35928059, a G at rs800292, a T at rs1061170, a T at rs12144939, an A at rs7546940, a T at rs1409153, a T at rs10922153, or a T at rs698859 can indicate the subject's decreased risk for having or developing age-related macular degeneration. In sill another aspect, an A at rs35928059, a G at rs800292, a T at rs1061170, a T atrs12144939, a G at rs7546940, a T atrs1409153, a T at rs10922153, or a T at rs698859 can indicate the subject's decreased risk for having or developing age-related macular degeneration.

Also described herein are methods for determining a male Caucasian subject's susceptibility to having or developing a complement-mediated disease comprising determining in the male Caucasian subject the identity of at least six SNPs in the CFH-to-F13B locus. The SNPs can include, but are not limited to: (i) rs35928059, rs800292, rs1061170, rs12144939, rs7546940, rs1409153, rs10922153, or rs698859, or (ii) a SNP in linkage disequilibrium with the SNPs of (i). In one aspect, the presence of at least six of the SNPs indicates the subject's susceptibility for having or developing a complement-mediated disease.

In one aspect, an A at rs35928059, a G at rs800292, a C at rs1061170, a G at rs12144939, a G at rs7546940, a G at rs1409153, a G at rs10922153, or an A at rs698859 can indicate the subject's increased risk for having or developing age-related macular degeneration. In another aspect, an A at rs35928059, a G at rs800292, a C at rs1061170, a G at rs12144939, a G at rs7546940, a C at rs1409153, a G at rs10922153, or a C at rs698859 can indicate the subject's increased risk for having or developing age-related macular degeneration. In yet another aspect, an A at rs35928059, a G at rs800292, a C at rs1061170, a G at rs12144939, a G at rs7546940, a C at rs1409153, a G at rs10922153, or a T at rs698859 can indicate the subject's increased risk for having or developing age-related macular degeneration. In still another aspect, an A at rs35928059, a G at rs800292, a T at rs1061170, a T at rs12144939, an A at rs7546940, an A at rs1409153, a T at rs10922153, or an A at rs698859 can indicate the subject's decreased risk for having or developing age-related macular degeneration. In another aspect, an A at rs35928059, an A at rs800292, a T at rs1061170, a G at rs12144939, a G at rs7546940, a T at rs1409153, a T at rs10922153, or a C at rs698859 can indicate the subject's decreased risk for having or developing age-related macular degeneration. In yet another aspect, an A at rs35928059, a G at rs800292, a T at rs1061170, a T atrs12144939, a G at rs7546940, a T at rs1409153, a T at rs10922153, or a T at rs698859 can indicate the subject's decreased risk for having or developing age-related macular degeneration.

Also described herein are methods for predicting progression of a complement-mediated disease in a Caucasian subject comprising determining in the Caucasian subject the identity of at least six SNPs in the CFH-to-F13B locus. The SNPs can be, but are not limited to: (i) rs35928059, rs800292, rs1061170, rs12144939, rs7546940, rs1409153, rs10922153, rs698859, or (ii) a SNP in linkage disequilibrium with the SNPs of (i). In one aspect, the presence of at least six of the SNPs can indicate the subject's risk for having or developing late-stage age-related macular degeneration.

In one aspect, an A at rs35928059, a G at rs800292, a C at rs1061170, a G at rs12144939, a G at rs7546940, a G at rs1409153, a G at rs10922153, or an A at rs698859 can indicate the subject's increased risk for having or developing late-stage age-related macular degeneration. In another aspect, an A at rs35928059, a G at rs800292, a C at rs1061170, a G at rs12144939, a G at rs7546940, a C at rs1409153, a G at rs10922153, or a T at rs698859 can indicate the subject's increased risk for having or developing late-stage age-related macular degeneration. In yet another aspect, an A at rs35928059, a G at rs800292, a C at rs1061170, a G at rs12144939, a G at rs7546940, a C at rs1409153, a G at rs10922153, or a C at rs698859 can indicate the subject's increased risk for having or developing late-stage age-related macular degeneration. In still another aspect, an A at rs35928059, an A at rs800292, a T at rs1061170, a G at rs12144939, a G at rs7546940, an A at rs1409153, a T at rs10922153, or a G at rs698859 can indicate the subject's decreased risk for having or developing late-stage age-related macular degeneration. In another aspect, an A at rs35928059, a G at rs800292, a T at rs1061170, a T atrs12144939, a G at rs7546940, an A at rs1409153, a T at rs10922153, or a G at rs698859 can indicate the subject's decreased risk for having or developing late-stage age-related macular degeneration. In yet another aspect, an A at rs35928059, a G at rs800292, a T at rs1061170, a T at rs12144939, an A at rs7546940, an A at rs1409153, a T at rs10922153, or an A at rs698859 can indicate the subject's decreased risk for having or developing late-stage age-related macular degeneration. In still another aspect, an A at rs35928059, an A at rs800292, a T at rs1061170, a G at rs12144939, a G at rs7546940, a T at rs1409153, a T at rs10922153, or a C at rs698859 can indicate the subject's decreased risk for having or developing late-stage age-related macular degeneration. In another aspect, an A at rs35928059, a G at rs800292, a T at rs1061170, a T atrs12144939, a G at rs7546940, a T at rs1409153, a T at rs10922153, or a C at rs698859 can indicate the subject's decreased risk for having or developing late-stage age-related macular degeneration. In yet another aspect, an A at rs35928059, a G at rs800292, a T at rs1061170, a T at rs12144939, an A at rs7546940, a T atrs1409153, a T at rs10922153, or a T at rs698859 can indicate the subject's decreased risk for having or developing late-stage age-related macular degeneration. In still another aspect, an A at rs35928059, a G at rs800292, a T atrs1061170, a T atrs12144939, a G at rs7546940, a T at rs1409153, a T at rs10922153, or a T at rs698859 can indicate the subject's decreased risk for having or developing late-stage age-related macular degeneration.

Also described herein are methods for predicting progression of complement-mediated disease in a female Caucasian subject comprising determining in the female Caucasian subject the identity of at least six SNPs in the CFH-to-F13B locus. The SNPs can be, but are not limited to: (i) rs35928059, rs800292, rs1061170, rs12144939, rs7546940, rs1409153, rs10922153, or rs698859, or (ii) a SNP in linkage disequilibrium with the SNPs of (i). In one aspect, the presence of at least six of the SNPs is indicative of the subject's risk for having or developing late-stage age-related macular degeneration.

In one aspect, an A at rs35928059, a G atrs800292, a C at rs1061170, a G at rs12144939, a G at rs7546940, a G at rs1409153, a G at rs10922153, or an G at rs698859 can indicate the subject's increased risk for having or developing late-stage age-related macular degeneration. In another aspect, an A at rs35928059, a G at rs800292, a C at rs1061170, a G at rs12144939, a G at rs7546940, a G at rs1409153, a G at rs10922153, or an A at rs698859 can indicate the subject's increased risk for having or developing late-stage age-related macular degeneration. In yet another aspect, an A at rs35928059, a G at rs800292, a C at rs1061170, a G at rs12144939, a G at rs7546940, a C at rs1409153, a G at rs10922153, or a C at rs698859 can indicate the subject's increased risk for having or developing late-stage age-related macular degeneration. In still another aspect, an A at rs35928059, a G at rs800292, a C at rs1061170, a G at rs12144939, a G at rs7546940, a C at rs1409153, a G at rs10922153, or a T at rs698859 can indicate the subject's increased risk for having or developing late-stage age-related macular degeneration. In another aspect, an A at rs35928059, an A at rs800292, a T atrs1061170, a G at rs12144939, a G at rs7546940, an A at rs1409153, a T at rs10922153, or a G at rs698859 can indicate the subject's decreased risk for having or developing late-stage age-related macular degeneration. In yet another aspect, an A at rs35928059, a G at rs800292, a T at rs1061170, a T atrs12144939, a G at rs7546940, an A at rs1409153, a T at rs10922153, or a G at rs698859 can indicate the subject's decreased risk for having or developing late-stage age-related macular degeneration. In still another aspect, an A at rs35928059, a G at rs800292, a T at rs1061170, a T at rs12144939, an A at rs7546940, an A at rs1409153, a T at rs10922153, or an A at rs698859 can indicate the subject's decreased risk for having or developing late-stage age-related macular degeneration. In another aspect, an A at rs35928059, an A at rs800292, a T at rs1061170, a G at rs12144939, a G at rs7546940, a T at rs1409153, a T at rs10922153, or a C at rs698859 can indicate the subject's decreased risk for having or developing late-stage age-related macular degeneration. In yet another aspect, an A at rs35928059, a G at rs800292, a T atrs1061170, a T at rs12144939, a G at rs7546940, a T at rs1409153, a T at rs10922153, or a C at rs698859 can indicate the subject's decreased risk for having or developing late-stage age-related macular degeneration. In still another aspect, an A at rs35928059, a G at rs800292, a T at rs1061170, a T at rs12144939, an A at rs7546940, a T at rs1409153, a T at rs10922153, or a T at rs698859 can indicate the subject's decreased risk for having or developing late-stage age-related macular degeneration. In another aspect, an A at rs35928059, a G at rs800292, a T at rs1061170, a T atrs12144939, a G at rs7546940, a T at rs1409153, a T at rs10922153, or a T at rs698859 can indicate the subject's decreased risk for having or developing late-stage age-related macular degeneration.

Also described herein are methods for predicting progression of a complement-mediated disease in a male Caucasian subject comprising determining in the male Caucasian subject the identity of at least six SNPs in the CFH-to-F13B locus. The SNPs can include, but are not limited to: (i) rs35928059, rs800292, rs1061170, rs12144939, rs7546940, rs1409153, rs10922153, or rs698859, or (ii) a SNP in linkage disequilibrium with the SNPs of (i). In one aspect, the presence of at least six of the SNPs can indicate the subject's risk for having or developing late-stage age-related macular degeneration.

In another aspect, an A at rs35928059, a G at rs800292, a C at rs1061170, a G at rs12144939, a G at rs7546940, a G at rs1409153, a G at rs10922153, or an A at rs698859 can indicate the subject's increased risk for having or developing late-stage age-related macular degeneration. In yet another aspect, an A at rs35928059, a G at rs800292, a C at rs1061170, a G at rs12144939, a G at rs7546940, a C at rs1409153, a G at rs10922153, or a C at rs698859 can indicate the subject's increased risk for having or developing late-stage age-related macular degeneration. In still another aspect, an A at rs35928059, a G at rs800292, a C at rs1061170, a G at rs12144939, a G at rs7546940, a C at rs1409153, a G at rs10922153, or a T at rs698859 can indicate the subject's increased risk for having or developing late-stage age-related macular degeneration. In another aspect, an A at rs35928059, a G at rs800292, a T at rs1061170, a T at rs12144939, an A at rs7546940, an A at rs1409153, a T at rs10922153, or an A at rs698859 can indicate the subject's decreased risk for having or developing late-stage age-related macular degeneration. In yet another aspect, an A at rs35928059, an A at rs800292, a T at rs1061170, a G at rs12144939, a G at rs7546940, a T at rs1409153, a T at rs10922153, or a C at rs698859 can indicate the subject's decreased risk for having or developing late-stage age-related macular degeneration. In still another aspect, an A at rs35928059, a G at rs800292, a T at rs1061170, a T at rs12144939, a G at rs7546940, a T atrs1409153, a T atrs10922153, or a T at rs698859 can indicate the subject's decreased risk for having or developing late-stage age-related macular degeneration.

### 4. SINGLE SNPs

Described herein are a collection of polymorphisms and haplotypes comprised of multiple variations in the CFH-to-F13B locus. One or more of these polymorphisms or haplotypes can be associated with complement-mediated disease. Detection of these and other polymorphisms and sets of polymorphisms (e.g., haplotypes) can be useful in designing and performing diagnostic assays for complement-mediated disease. Polymorphisms and sets of polymorphisms can be detected by analysis of nucleic acids, by analysis of polypeptides encoded the CFH-to-F13B locus coding sequences (including polypeptides encoded by splice variants), by analysis of the CFH-to-F13B locus non-coding sequences, or by other means known in the art. Analysis of such polymorphisms and haplotypes can also be useful in designing prophylactic and therapeutic regimes for complement-mediated disease.

More specifically, described herein are methods for determining a Caucasian subject's susceptibility to having or developing a complement-mediated disease comprising determining in the subject the identity of one or more SNPs in the CFH-to-F13B locus. In one aspect, the the SNPs can include, but are not limited to: (i) rs1061170, rs1410996, rs2274700, rs3753395, rs403846, or rs3753396 or (ii) a SNP in linkage disequilibrium with the SNPs of (i). In another aspect, the presence of one or more of the SNPs described herein can indicate the subject's susceptibility for having or developing age-related macular degeneration.

In one aspect, an A at the rs1061170 SNP can be indicative of the subject's increased risk for having or developing a complement-mediated disease. In another aspect, an A at the rs1410996 SNP, an A at the rs2274700 SNP, a T at the rs3753395 SNP, or a G at the rs403846 SNP can be indicative of the subject's decreased risk for having or developing age-related macular degeneration. In yet another aspect, a G at the rs3753396 SNP does not indicate that the subject has an increased risk or a decreased risk for having or developing age-related macular degeneration.

Also described herein are methods for predicting progression of age-related macular degeneration in a Caucasian subject comprising determining in the subject the identity of one or more SNPs in the CFH-to-F13B locus. In one aspect, the SNPs can include, but are not limited to: (i) rs1061170, rs1410996, rs2274700, rs3753395, rs403846, or rs3753396 or (ii) a SNP in linkage disequilibrium with the SNPs of (i). In another aspect, the presence of one or more of the SNPs described herein can be indicative of the subject's risk for having or developing late-stage age-related macular degeneration.

In one aspect, an A at the rs1061170 SNP can be indicative of the subject's increased risk for having or developing late-stage age-related macular degeneration. In another aspect, an A at the rs1410996 SNP, an A at the rs2274700 SNP, a T at the rs3753395 SNP, or a G at the rs403846 SNP can be indicative of the subject's decreased risk for having or developing late-stage age-related macular degeneration. In yet another aspect, a G at the rs3753396 SNP does not indicate whether a subject has an increased risk or a decreased risk for having or developing late-stage age-related macular degeneration.

Also described herein are methods of screening for polymorphic sites in other genes that are in linkage disequilibrium (LD) with a risk, protective, or otherwise informative SNP or genetic marker described herein, including but not limited to the polymorphic sites in the CFH-to-F13B locus. These methods can involve identifying a polymorphic site in a gene that is in linkage disequilibrium with a polymorphic site in the CFH-to-F13B locus, wherein the polymorphic form of the polymorphic site in the CFH-to-F13B locus is associated with complement-mediated disease, (e.g., increased or decreased risk), and determining haplotypes in a population of individuals to indicate whether the linked polymorphic site has a polymorphic form in linkage disequilibrium with the polymorphic form of the CFH/F13B gene that correlates with the complement-mediated disease phenotype. SNPs in LD with the SNPs described herein include, but are not limited to rs10737680, rs7535263, rs10922106, rs395998, rs1329428 and rs7540032. In another aspect, SNPs in LD with the SNPs described herein can be, but are not limited to rs10489456, rs70620, rs742855, rs11799380, rs1065489, rs11582939, rs385390, rs421820, rs426736, rs370953, rs371075.

As used herein, "linkage disequilibrium" is the non-random association of alleles at two or more loci, not necessarily on the same chromosome. It is not the same as linkage, which describes the association of two or more loci on a chromosome with limited recombination between them. Linkage disequilibrium describes a situation in which some combinations of alleles or genetic markers occur more or less frequently in a population than would be expected from a random formation of haplotypes from alleles based on their frequencies. Non-random associations between polymorphisms at different loci are measured by the degree of linkage disequilibrium (LD). The level of linkage disequilibrium can be influenced by a number of factors including genetic linkage, the rate of recombination, the rate of mutation, random drift, non-random mating, and population structure. "Linkage disequilibrium" or "allelic association" thus means the non-random association of a particular allele or genetic marker with another specific allele or genetic marker more frequently than expected by chance for any particular allele frequency in the population. A marker in linkage disequilibrium with an informative marker, such as one of the CFH/F13B SNPs or haplotypes described herein can be useful in detecting susceptibility to complement-mediated disease. A SNP that is in linkage disequilibrium with a risk, protective, or otherwise informative SNP, haplotype, or genetic marker described herein can be referred to as a "proxy" or "surrogate" SNP. A proxy SNP may be in at least 50%, 60%, or 70% in linkage disequilibrium with risk, protective, or otherwise informative SNP or genetic marker described herein, and in one aspect is at least about 80%, 90%, and in another aspect 91%, 92%, 93%, 94%, 95%, 96%, 97%, 98%, 99%, or about 100% in LD with a risk, protective, or otherwise informative SNP, haplotype, or genetic marker described herein.

Publicly available databases such as the HapMap database (http://ftp.hapmap.org/ld_data/latest/) and Haploview (Barrett, J. C. et al., Bioinformatics 21, 263 (2005)) may be used to calculate linkage disequilibrium between two SNPs. The frequency of identified alleles in disease versus control populations can be determined using the methods described herein. Statistical analyses can be employed to determine the significance of a non-random association between the two SNPs (e.g., Hardy-Weinberg Equilibrium, Genotype likelihood ratio (genotype p value), Chi Square analysis, Fishers Exact test). A statistically significant non-random association between the two SNPs indicates that they are in linkage disequilibrium and that one SNP can serve as a proxy for the second SNP.

In another aspect, polymorphisms in the CFH-to-F13B locus, such as those described herein, can be used to establish physical linkage between a genetic locus associated with a trait of interest and polymorphic markers that are not associated with the trait, but are in physical proximity with the genetic locus responsible for the trait and co-segregate with it. Mapping a genetic locus associated with a trait of interest facilitates cloning the gene(s) responsible for the trait following procedures that are well-known in the art.

As used herein, "physical linkage" describes the tendency of genes, alleles, loci or genetic markers to be inherited together as a result of their location on the same chromosome. Linkage can be measured by percent recombination between the two genes, alleles, loci or genetic markers. Typically, loci occurring within a 50 centimorgan (cM) distance of each other are linked. Linked markers may occur within the same gene or gene cluster.

### 5. DELETION TAGGING SNPs

As disclosed herein, are SNPs that tag the deletion status of the CFHR-3/CFHR-1 genes. Therefore, also described herein are methods for determining a Caucasian subject's susceptibility to having or developing a complement-mediated disease comprising determining in the subject the identity of a deletion tagging SNP in the CFH-to-F13B locus. The SNP can be, but is not limited to: (i) rs12144939 or (ii) a SNP in linkage disequilibrium with rs12144939. In one aspect, the presence of the SNP can indicate the subject's susceptibility for having or developing age-related macular degeneration. In another aspect, a T at the rs12144939 SNP can indicate the subject's decreased risk for having or developing age-related macular degeneration. In another aspect, also described herein are methods determining for determining a Caucasian subject's susceptibility to having or developing a complement-mediated disease comprising determining in the subject the identity of at lease one SNP that tags the deletion tagging SNPs described herein. The tagging SNPs can be, but are not limited to, rs6689009, rs35253683, rs731557, and rs60642321. In another aspect, also described herein are methods determining for determining a Caucasian subject's susceptibility to having or developing a complement-mediated disease comprising determining in the subject the identity of at lease one SNP in LD with the deletion tagging SNPs described herein. The SNPs can be, but are not limited to, rs6677604, rs16840522 and rs2019727.

Also described herein are methods for predicting progression of a complement-mediated disease in a Caucasian subject comprising determining in the subject the identity of a deletion tagging SNP in the CFH-to-F13B locus. The SNP can include, but is not limited to: (i) rs1214493 9 or (ii) a SNP in linkage disequilibrium with rs12144939. In one aspect, the presence of the deletion tagging SNP can indicate the subject's risk for having or developing late-stage age-related macular degeneration. In another aspect, a T at the rs12144939 SNP can indicate the subject's decreased risk for having or developing late-stage age-related macular degeneration.

### 6. HAPLOTYPE TAGGING HAPLOTYPES

In one aspect, the haplotypes described herein can be compared to haplotypes generated using a genetic variation database, for example, HapMap, in order to further refine haplotype association with complement-mediated diseases, such as AMD. The International HapMap Project, available at http://hapmap.ncbi.nlm.nih.gov/, contains over 500 SNPs in the region encompassing the CFH-to-F13B locus (Hg18 chr1: 194869137-195303491). Thus, in one aspect, genotypes from Caucasian individuals (referred to as the CEU population) can be downloaded for each of these 500 plus SNPs. Phased genotype data from the CEU set can then be downloaded, including the most informative SNPs (*i.e.* those that segregate in the CEU population) in this region of interest. In one aspect, Haploview (www.broadinstitute.org) can be used to construct haplotypes for the CEU dataset and their frequencies can be calculated. The overlapping SNPs obtiained from that analysis can be used as 'tagging' SNPs to match the case/control haplotypes described herein to their equivalent HapMap CEU haplotypes, thereby allowing the disease associations described herein to be extended to the equivalent HapMap haplotypes.

In one aspect, the methods can identify HapMap SNPs that were not included in the 51 SNP-based haplotypes described herein. These newly identified SNPs can further refine the discriminative value of the 51 SNP-based haplotypes described herein, and the association of those haplotypes with complement-mediated disease.

Decribed herein are methods for identifying a single nucleotide polymorphism or haplotype that tags one or more haplotypes in the CFH-to-F13B locus. The one or more haplotypes can be any haplotype described herein as being associated with having or developing a complement-mediated disease. In one aspect, the methods can comprise entering the SNP sequence of one or more CFH/F13B haplotypes into a genetic variation database and determining the identity of one or more tagging SNPs or haplotypes. In one aspect, the one or more CFH/F13B haplotypes can be H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H1_51_A, H2_51_A, H3_51_A, H4_51_A, H5_51_A, H6_51_A, H7_51_A, H8_51_A, H9_51_A, H10_51_A, H11_51_A, H12_51_A, H13_51_A, H14_51_A, H15_51_A, H16_51_A, H17_51_A, H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H171_B, H18_51_B, H191_B, H20_51_B, H21_51_B, H22_51_B, or a complement thereof.

In another aspect, the genetic variation database can be HapMap. In yet another aspect, the one or more CFH/F13B haplotypes can be risk haplotypes, including, but not limited to, H2_62_A, H2_51_A, H1_51_B, H2_51_B, or a complement thereof. In still another aspect, the one or more CFH/F13B haplotypes can be protective haplotypes, including, but not limited to, H3_62_A, H5_62_A, H11_62_A, H3_51_A, H5_51_A, H10_51_A, H3_51_B, H5_51_B, H12_51_B, H14_51_B, or a complement thereof.

### B. METHODS OF IDENTIFYING A CAUCASIAN SUBJECT IN NEED OF TREATMENT FOR AGE-RELATED MACULAR DEGENERATION

Described herein are methods of identifying a Caucasian subject in need of treatment for complement-mediated disease comprising determining in the Caucasian subject the identity of one or more major or minor haplotypes. The haplotypes can include, but are not limited to H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H1_51_A, H2_51_A, H3_51_A, H4_51_A, H5_51_A, H6_51_A, H7_51_A, H8_51_A, H9_51_A, H10_51_A, H11_51_A, H12_51_A, H13_51_A, H14_51_A, H15_51_A, H16_51_A, H17_51_A, H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof. As described herein, the presence of one or more of the haplotypes disclosed herein can indicate whether the subject is in need of treatment for a complement-mediated disease. As used herein, "treatment" can also mean prophylactic, or preventative treatment. In one aspect, the complement-mediated disease can be age-related macular degeneration (AMD). Thus, the methods described herein can be used to identify a Caucasian subject in need of treatment for age-related macular degeneration.

In one aspect, determining the identity of a certain haplotype or certain haplotypes in a subject can indicate that the subject is in need of treatment for a complement-mediated disease. Such hapltoypes can be risk haplotypes. For example, and not to be limiting, a risk haplotype can be H2_62_A, H2_51_A, H1_51_B, H2_51_B, or a complement thereof. Thus, described herein are methods for identifying a Caucasian subject in need of treatment for a complement-mediated disease comprising determining in the Caucasian subject the identity of one or more haplotypes described herein, wherein a risk haplotype can indicate that the subject is in need of treatment. In one aspect, the method of identifying a subject in need of treatment can further comprise administering a therapeutic composition to the subject identified as being in need of treatment. In another aspect, the methods described herein can be used to identify a Caucasian subject in need of more frequent screening for complement-mediated diseases, including, but not limited to, age-related macular degeneration. For example, identifying a subject with a risk haplotype can indicate that the subject needs to be more closely and frequently monitored for the development of a complement-mediated disease such as AMD.

In another aspect, determining the identity of a certain haplotype or certain haplotypes in a subject can indicate that the subject is not in need of treatment for a complement-mediated disease. Such haplotypes can be protective haplotypes. For example, and not to be limiting, a protective haplotype can be H3_62_A, H5_62_A, H11_62_A, H3_51_A, H5_51_A, H10_51_A, H3_51_B, H5_51_B, H12_51_B, H14_51_B, or a complement thereof. Thus, described herein are methods for identifying a Caucasian subject in need of treatment for a complement-mediated disease comprising determining in the Caucasian subject the identity of one or more haplotypes described herein, wherein a protective haplotype can indicate that the subject is not in need of treatment for a complement-mediated disease.

In yet another aspect, determining the identity of a certain haplotype or certain haplotypes in a subject can indicate that the subject is in need of continued screening for the development of a complement-mediated disease. Such haplotypes can be neutral haplotypes. For example, and not to be limiting, a neutral haplotype can be H1_62_A, H4_62_A, H6_62_A, H7_62_A, H8_62_A, H9_62_A, H10_62_A, H12_62_A, H13_62_A, H14_62_A, H15_62_A, H1_51_A, H4_51_A, H6_51_A, H7_51_A, H8_51_A, H9_51_A, H11_51_A, H12_51_A, H13_51_A, H14_51_A, H15_51_A, H16_51_A, H17_51_A, H4_51_B, H6_51_B, H7_51_B, H8_51_B, H9_51_B, H10_51_B, H11_51_B, H13_51_B, H15_51_B, H16_51_B, H17_51_B, H18_51_B, H19_51_B, H20_51_B, H21_51_B, H22_51_B, or a complement thereof. Thus, described herein are methods identifying a Caucasian subject in need of screening for the development of a complement-mediated disease comprising determining in the Caucasian subject the identity of one or more haplotypes described herein, wherein a neutral haplotype can indicate a subject in need of screening for the development of a complement-mediated disease. For example, and not to be limiting, identifying a subject with a neutral haplotype can indicate that the subject should be screened for the development of a complement-mediated disease, such as AMD, at an earlier age, and more often than would typically be done in the general population.

Also described herein are methods for identifying a female Caucasian subject in need of treatment for complement-mediated disease comprising determining in the female Caucasian subject the identity of one or more major or minor haplotypes. The haplotypes can include, but are not limited to H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, or a complement thereof.

In one aspect, determining the identity of a certain haplotype or certain haplotypes in a female subject can indicate that the female subject is in need of treatment for a complement-mediated disease. Such hapltoypes can be risk haplotypes. For example, and not to be limiting, a risk haplotype can be H1_62_A, H2_62_A, H1_51_B, H2_51_B, or a complement thereof. Thus, described herein are methods of identifying a female Caucasian subject in need of treatment for a complement-mediated disease comprising determining in the female Caucasian subject the identity of one or more haplotypes described herein, wherein a risk haplotype can indicate that the subject is in need of treatment. In one aspect, the method of identifying a female subject in need of treatment can further comprise administering a therapeutic composition to the female subject identified as being in need of treatment. In another aspect, the methods described herein can be used to identify a female Caucasian subject in need of more frequent screening for complement-mediated diseases, including, but not limited to, age-related macular degeneration. For example, identifying a female subject with a risk haplotype can indicate that the female subject needs to be more closely and frequently monitored for the development of a complement-mediated disease such as AMD.

In another aspect, determining the identity of a certain haplotype or certain haplotypes in a female subject can indicate that the female subject is not in need of treatment for complement-mediated disease. Such haplotypes can be protective haplotypes. For example, and not to be limiting, a protective haplotype can be H3_62_A, H5_62_A, H11_62_A, H3_51_B, H5_51_B, H12_51_B, H14_51_B, or a complement thereof. Thus, described herein are methods for identifying a female Caucasian subject in need of treatment for a complement-mediated disease comprising determining in the female Caucasian subject the identity of one or more haplotypes described herein, wherein a protective haplotype can indicate that the female subject is not in need of treatment for a complement-mediated disease.

In yet another aspect, determining the identity of a certain haplotype or certain haplotypes in a female subject can indicate that the female subject is in need of continued screening for the development of a complement-mediated disease. Such haplotypes can be neutral haplotypes. For example, and not to be limiting, a neutral haplotype can be H4_62_A, H6_62_A, H7_62_A, H8_62_A, H9_62_A, H10_62_A, H12_62_A, H13_62_A, H14_62_A, H15_62_A, H4_51_B, H6_51_B, H7_51_B, H8_51_B, H9_51_B, H10_51_B, H11_51_B, H13_51_B, H15_51_B, H16_51_B, H17_51_B, H18_51_B, H19_51_B, H20_51_B, or a complement thereof. Thus, described herein are methods identifying a female Caucasian subject in need of screening for the development of a complement-mediated disease comprising determining in the female Caucasian subject the identity of one or more haplotypes described herein, wherein a neutral haplotype can indicate a female subject in need of screening for the development of a complement-mediated disease. For example, and not to be limiting, identifying a female subject with a neutral haplotype can indicate that the female subject should be screened for the development of a complement-mediated disease, such as AMD, at an earlier age, and more often than would typically be done in the general population.

Also described herein are methods methods identifying a male Caucasian subject in need of treatment for complement-mediated disease comprising determining in the male Caucasian subject the identity of one or more major or minor haplotypes. The haplotypes can include, but are not limited to H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, or a complement thereof.

In one aspect, determining the identity of a certain haplotype or certain haplotypes in a male subject can indicate that the male subject is in need of treatment for a complement-mediated disease. Such hapltoypes can be risk haplotypes. For example, and not to be limiting, a risk haplotype can be H2_62_A, H1_51_B, H2_51_B, or a complement thereof. Thus, described herein are methods of identifying a male Caucasian subject in need of treatment for a complement-mediated disease comprising determining in the male Caucasian subject the identity of one or more haplotypes described herein, wherein a risk haplotype can indicate that the male subject is in need of treatment. In one aspect, the method of identifying a male subject in need of treatment can further comprise administering a therapeutic composition to the male subject identified as being in need of treatment. In another aspect, the methods described herein can be used to identify a male Caucasian subject in need of more frequent screening for complement-mediated diseases, including, but not limited to, age-related macular degeneration. For example, identifying a male subject with risk haplotype can indicate that the male subject needs to be more closely and frequently monitored for the development of a complement-mediated disease such as AMD.

In another aspect, determining the identity of a certain haplotype or certain haplotypes in a male subject can indicate that the male subject is not in need of treatment for complement-mediated disease. Such haplotypes can be protective haplotypes. For example, and not to be limiting, a protective haplotype can be H11_62_A, H3_51_B, H14_51_B, or a complement thereof. Thus, described herein are methods for identifying a male Caucasian subject in need of treatment for a complement-mediated disease comprising determining in the male Caucasian subject the identity of one or more haplotypes described herein, wherein a protective haplotype can indicate that the male subject is not in need of treatment for a complement-mediated disease.

In yet another aspect, determining the identity of a certain haplotype or certain haplotypes in a male subject can indicate that the male subject is in need of continued screening for the development of a complement-mediated disease. Such haplotypes can be neutral haplotypes. For example, and not to be limiting, a neutral haplotype can be H1_62_A, H3_62_A, H4_62_A, H5_62_A, H6_62_A, H7_62_A, H8_62_A, H9_62_A, H10_62_A, H12_62_A, H13_62_A, H14_62_A, H15_62_A H4_51_B, H5_51_B, H6_51_B, H7_51_B, H8_51_B, H9_51_B, H10_51_B, H11_51_B, H12_51_B, H13_51_B, H15_51_B, H16_51_B, H17_51_B, H18_51_B, H19_51_B, H20_51_B, H21_51_B, H22_51_B, or a complement thereof. Thus, described herein are methods for identifying a male Caucasian subject in need of screening for the development of a complement-mediated disease comprising determining in the male Caucasian subject the identity of one or more haplotypes described herein, wherein a neutral haplotype can indicate a male subject in need of screening for the development of a complement-mediated disease. For example, and not to be limiting, identifying a male subject with a neutral haplotype can indicate that the male subject should be screened for the development of a complement-mediated disease, such as AMD, at an earlier age, and more often than would typically be done in the general population.

Also described herein are methods of identifying a Caucasian subject in need of treatment for a complement-mediated disease comprising determining in the subject the identity of one or more SNPs in the CFH-to-F13B locus. The SNPs can include, but are not limited to: (i) rs1061170, rs1410996, rs2274700, rs3753395, rs403846, or rs3753396 or (ii) a SNP in linkage disequilibrium with the SNPs of (i). In one aspect, the presence of one or more of the SNPs can indicate whether the subject is in need of treatment for a complement-mediated disease. In one aspect, an A at the rs1061170 SNP can indicate the subject is in need of treatment for a complement-mediated disease. In another aspect, an A at the rs1410996 SNP, an A at the rs2274700 SNP, a T at the rs3753395 SNP, or a G at the rs403846 SNP can indicate the subject is not in need of treatment for a complement-mediated disease. In yet another aspect, a G at the rs3753396 SNP can indicate the subject may be in need of treatment for a complement-mediated disease.

Also described herein are methods of identifying a Caucasian subject in need of treatment for a complement-mediated disease comprising determining in the subject the identity of a deletion tagging SNP in the CFH-to-F13B locus. The SNP can include, but is not limited to: (i) rs1214493 9 or (ii) a SNP in linkage disequilibrium with rs12144939. In one aspect, the presence of one or more of the SNPs can indicate whether the subject is in need of treatment for age-related macular degeneration. In another aspect, a T at the rs12144939 SNP can indicate the subject is not in need of treatment for age-related macular degeneration.

Also described herein are methods for identifying a Caucasian subject in need of treatment for age-related macular degeneration comprising determining in the Caucasian subject the identity of at least six SNPs in the CFH-to-F13B locus. The SNPs can include, but are not limited to: (i) rs35928059, rs800292, rs1061170, rs12144939, rs7546940, rs1409153, rs10922153, or rs698859, or (ii) a SNP in linkage disequilibrium with the SNPs of (i). In one aspect, the presence of at least six of the SNPs can indicate whether the subject is in need of treatment for age-related macular degeneration.

In one aspect, an A at rs35928059, a G at rs800292, a C at rs1061170, a G at rs12144939, a G at rs7546940, a G at rs1409153, a G at rs10922153, or an A at rs698859 can indicate the subject is in need of treatment for age-related macular degeneration. In another aspect, an A at rs35928059, a G at rs800292, a C at rs1061170, a G at rs12144939, a G at rs7546940, a C at rs1409153, a G at rs10922153, or a T at rs698859 can indicate the subject is in need of treatment for age-related macular degeneration. In yet another aspect, an A at rs35928059, a G at rs800292, a C at rs1061170, a G at rs12144939, a G at rs7546940, a C at rs1409153, a G at rs10922153, or a C at rs698859 can indicate the subject is in need of treatment for age-related macular degeneration. In still another aspect, an A at rs35928059, an A at rs800292, a T at rs1061170, a G at rs12144939, a G at rs7546940, an A at rs1409153, a T at rs10922153, or a G at rs698859 can indicate the subject is not in need of treatment for age-related macular degeneration. In another aspect, an A at rs35928059, a G at rs800292, a T at rs1061170, a T at rs12144939, a G at rs7546940, an A at rs1409153, a T at rs10922153, or a G at rs698859 can indicate the subject is not in need of treatment for age-related macular degeneration. In yet another aspect, an A at rs35928059, a G at rs800292, a T at rs1061170, a T atrs12144939, an A at rs7546940, an A at rs1409153, a T at rs10922153, or an A at rs698859 can indicate the subject is not in need of treatment for age-related macular degeneration. In still another aspect, an A at rs35928059, an A at rs800292, a T at rs1061170, a G at rs12144939, a G at rs7546940, a T at rs1409153, a T at rs10922153, or a C at rs698859 can indicate the subject is not in need of treatment for age-related macular degeneration. In another aspect, an A at rs35928059, a G at rs800292, a T at rs1061170, a T at rs12144939, a G at rs7546940, a T atrs1409153, a T atrs10922153, or a C at rs698859 can indicate the subject is not in need of treatment for age-related macular degeneration. In yet another aspect, an A at rs35928059, a G at rs800292, a T at rs1061170, a T atrs12144939, an A at rs7546940, a T atrs1409153, a T at rs10922153, or a T at rs698859 can indicate the subject is not in need of treatment for age-related macular degeneration. In still another aspect, an A at rs35928059, a G at rs800292, a T at rs1061170, a T at rs12144939, a G at rs7546940, a T atrs1409153, a T at rs10922153, or a T at rs698859 can indicate the subject is not in need of treatment for age-related macular degeneration.

Also described herein are methods of identifying a female Caucasian subject in need of treatment for a complement-mediated disease comprising determining in the female Caucasian subject the identity of at least six SNPs in the CFH-to-F13B locus. The SNPs can be, but are not limited to: (i) rs35928059, rs800292, rs1061170, rs12144939, rs7546940, rs1409153, rs10922153, or rs698859, or (ii) a SNP in linkage disequilibrium with the SNPs of (i). In one aspect, the presence of at least six of the SNPs can indicate whether the subject is in need of treatment for age-related macular degeneration.

In one aspect, an A at rs35928059, a G at rs800292, a C at rs1061170, a G at rs12144939, a G at rs7546940, a G at rs1409153, a G at rs10922153, or an G at rs698859 can indicate the subject is in need of treatment for age-related macular degeneration. In another aspect, an A at rs35928059, a G at rs800292, a C at rs1061170, a G at rs12144939, a G at rs7546940, a G at rs1409153, a G at rs10922153, or an A at rs698859 can indicate the subject is in need of treatment for age-related macular degeneration. In yet another aspect, an A at rs35928059, a G at rs800292, a C at rs1061170, a G at rs12144939, a G at rs7546940, a C at rs1409153, a G at rs10922153, or a C at rs698859 can indicate the subject is in need of treatment for age-related macular degeneration. In still another aspect, an A at rs35928059, a G at rs800292, a C at rs1061170, a G at rs12144939, a G at rs7546940, a C at rs1409153, a G at rs10922153, or a T at rs698859 can indicate the subject is in need of treatment for age-related macular degeneration. In another aspect, an A at rs35928059, an A at rs800292, a T at rs1061170, a G at rs12144939, a G at rs7546940, an A at rs1409153, a T at rs10922153, or a G at rs698859 can indicate the subject is not in need of treatment for age-related macular degeneration. In still another aspect, an A at rs35928059, a G at rs800292, a T at rs1061170, a T at rs12144939, a G at rs7546940, an A at rs1409153, a T at rs10922153, or a G at rs698859 can indicate the subject is not in need of treatment for age-related macular degeneration. In another aspect, an A at rs35928059, a G at rs800292, a T at rs1061170, a T at rs12144939, an A at rs7546940, an A at rs1409153, a T at rs10922153, or an A at rs698859 can indicate the subject is not in need of treatment for age-related macular degeneration. In yet another aspect, an A at rs35928059, an A at rs800292, a T at rs1061170, a G at rs12144939, a G at rs7546940, a T at rs1409153, a T at rs10922153, or a C at rs698859 can indicate the subject is not in need of treatment for age-related macular degeneration. In still another aspect, an A at rs35928059, a G at rs800292, a T atrs1061170, a T at rs12144939, a G at rs7546940, a T at rs1409153, a T at rs10922153, or a C at rs698859 can indicate the subject is not in need of treatment for age-related macular degeneration. In still another aspect, an A at rs35928059, a G at rs800292, a T at rs1061170, a T at rs12144939, an A at rs7546940, a T at rs1409153, a T at rs10922153, or a T at rs698859 can indicate the subject is not in need of treatment for age-related macular degeneration. In another aspect, an A at rs35928059, a G at rs800292, a T at rs1061170, a T at rs12144939, a G at rs7546940, a T at rs1409153, a T atrs10922153, or a T at rs698859 can indicate the subject is not in need of treatment for age-related macular degeneration.

Also described herein are methods of identifying a male Caucasian subject in need of treatment for a complement-mediated disease comprising determining in the male Caucasian subject the identity of at least six SNPs in the CFH-to-F13B locus. The SNPs can be, but are not limited to: (i) rs35928059, rs800292, rs1061170, rs12144939, rs7546940, rs1409153, rs10922153, or rs698859, or (ii) a SNP in linkage disequilibrium with the SNPs of (i). In one aspect, the presence of at least six of the SNPs can indicate whether the subject is in need of treatment for age-related macular degeneration.

In one aspect, an A at rs35928059, a G at rs800292, a C at rs1061170, a G at rs12144939, a G at rs7546940, a G at rs1409153, a G at rs10922153, or an A at rs698859 can indicate the subject is in need of treatment for age-related macular degeneration. In another aspect, an A at rs35928059, a G at rs800292, a C at rs1061170, a G at rs12144939, a G at rs7546940, a C at rs1409153, a G at rs10922153, or a C at rs698859 can indicate the subject is in need of treatment for age-related macular degeneration. In yet another aspect, an A at rs35928059, a G at rs800292, a C at rs1061170, a G at rs12144939, a G at rs7546940, a C at rs1409153, a G at rs10922153, or a T at rs698859 can indicate the subject is in need of treatment for age-related macular degeneration. In still another aspect, an A at rs35928059, a G at rs800292, a T atrs1061170, a T at rs12144939, an A at rs7546940, an A at rs1409153, a T at rs10922153, or an A at rs698859 can indicate the subject is not in need of treatment for age-related macular degeneration. In another aspect, an A at rs35928059, an A at rs800292, a T at rs1061170, a G at rs12144939, a G at rs7546940, a T at rs1409153, a T at rs10922153, or a C at rs698859 can indicate the subject is not in need of treatment for age-related macular degeneration. In yet another aspect, an A at rs35928059, a G at rs800292, a T at rs1061170, a T at rs12144939, a G at rs7546940, a T at rs1409153, a T at rs10922153, or a T at rs698859 can indicate the subject is not in need of treatment for age-related macular degeneration.

### 1. METHODS OF IDENTIFYING PATIENTS FOR CLINICAL TRIALS

Furthermore, described herein are methods of identifying a Caucasian subject that is appropriate for a complement-mediated disease clinical trial comprising determining in the Caucasian subject the identity of one or more major or minor haplotypes. The haplotypes can include, but are not limited to H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H1_51_A, H2_51_A, H3_51_A, H4_51_A, H5_51_A, H6_51_A, H7_51_A, H8_51_A, H9_51_A, H10_51_A, H11_51_A, H12_51_A, H13_51_A, H14_51_A, H15_51_A, H16_51_A, H17_51_A, H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, H20_51_B, H21_51_B, H22_51_B, or a complement thereof. As described herein, the presence of one or more of the haplotypes disclosed herein can indicate whether the subject is appropriate for a complement-mediated disease clinical trial. In one aspect, the complement-mediated disease can be age-related macular degeneration (AMD), and the clinical trial can be an age-related macular degeneration clinical trial. Thus, the methods described herein can be used to identify a Caucasian subject that is appropriate for an age-related macular degeneration clinical trial. As used herein, clinical trial means an experimental study conducted to evaluate the effectiveness and safety of a treatment or medical device by monitoring their effects on a subject or group of subjects. In one aspect, the clinical trial can be conducted to evaluate the effectiveness and safety of medications for a complement-mediated disease, for example, AMD.

In one aspect, determining the identity of a certain haplotype or certain haplotypes in a subject can indicate that the subject is appropriate for a complement-mediated disease clinical trial. Such hapltoypes can be risk haplotypes. For example, and not to be limiting, a risk haplotype can be H2_62_A, H2_51_A, H1_51_B, H2_51_B, or a complement thereof. Thus, described herein are methods for identifying a Caucasian subject that is appropriate for a complement-mediated disease clinical trial comprising determining in the Caucasian subject the identity of one or more haplotypes described herein, wherein a risk haplotype can indicate that the subject is appropriate for the clinical trial. In one aspect, the method of identifying a subject for the clinical trial can further comprise administering a therapeutic composition to the subject identified as being appropriate for the clinical trial.

In another aspect, determining the identity of a certain haplotype or certain haplotypes in a subject can indicate that the subject is not appropriate for a complement-mediated disease clinical trial. Such haplotypes can be protective haplotypes. For example, and not to be limiting, a protective haplotype can be H3_62_A, H5_62_A, H11_62_A, H3_51_A, H5_51_A, H10_51_A, H3_51_B, H5_51_B, H12_51_B, H14_51_B, or a complement thereof. Thus, described herein are methods for identifying a Caucasian subject that is appropriate for a complement-mediated disease clinical trial comprising determining in the Caucasian subject the identity of one or more haplotypes described herein, wherein a protective haplotype can indicate that the subject is not appropriate for a complement-mediated disease clinical trial. For example, a subject identified to have a protective haplotype described herein can be excluded from participation in a clinical trial designed to evaluate a treatment for a complement-mediated disease.

In yet another aspect, determining the identity of a certain haplotype or certain haplotypes in a subject can indicate that the subject may be appropriate for a complement-mediated disease clinical trial. Such haplotypes can be neutral haplotypes. For example, and not to be limiting, a neutral haplotype can be H1_62_A, H4_62_A, H6_62_A, H7_62_A, H8_62_A, H9_62_A, H10_62_A, H12_62_A, H13_62_A, H14_62_A, H15_62_A, H1_51_A, H4_51_A, H6_51_A, H7_51_A, H8_51_A, H9_51_A, H11_51_A, H12_51_A, H13_51_A, H14_51_A, H15_51_A, H16_51_A, H17_51_A, H4_51_B, H6_51_B, H7_51_B, H8_51_B, H9_51_B, H10_51_B, H11_51_B, H13_51_B, H15_51_B, H16_51_B, H17_51_B, H18_51_B, H19_51_B, H20_51_B, H21_51_B, H22_51_B, or a complement thereof. Thus, described herein are methods of identifying a Caucasian subject appropriate for a complement-mediated disease clinical trial comprising determining in the Caucasian subject the identity of one or more haplotypes described herein, wherein a neutral haplotype can indicate a subject that may be appropriate for a complement-mediated disease clinical trial.

Furthermore, described herein are methods of identifying a female Caucasian subject that is appropriate for a complement-mediated disease clinical trial comprising determining in the female Caucasian subject the identity of one or more major or minor haplotypes. The haplotypes can include, but are not limited to H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H1_51_B, H2_51_B, H3_51_B, H4_51_B, H5_51_B, H6_51_B, H7_51_B, H8_51_B, H9_51_B, H10_51_B, H11_51_B, H12_51_B, H_13_51_B, H14_51_B, H15_51_B, H16_51_B, H17_51_B, H18_51_B, H19_51_B, H20_51_B, or a complement thereof. As described herein, the presence of one or more of the haplotypes disclosed herein can indicate whether the female subject is appropriate for a complement-mediated disease clinical trial. In one aspect, the complement-mediated disease can be age-related macular degeneration (AMD), and the clinical trial can be an age-related macular degeneration clinical trial. Thus, the methods described herein can be used to identify a female Caucasian subject that is appropriate for an age-related macular degeneration clinical trial.

In one aspect, determining the identity of a certain haplotype or certain haplotypes in a female subject can indicate that the female subject is appropriate for a complement-mediated disease clinical trial. Such hapltoypes can be risk haplotypes. For example, and not to be limiting, a risk haplotype can be H1_62_A, H2_62_A, H1_51_B, H2_51_B, or a complement thereof. Thus, described herein are methods for identifying a female Caucasian subject that is appropriate for a complement-mediated disease clinical trial comprising determining in the female Caucasian subject the identity of one or more haplotypes described herein, wherein a risk haplotype can indicate that the female subject is appropriate for the clinical trial. In one aspect, the method of identifying a female subject for the clinical trial can further comprise administering a therapeutic composition to the subject identified as being appropriate for the clinical trial.

In another aspect, determining the identity of a certain haplotype or certain haplotypes in a female subject can indicate that the female subject is not appropriate for a complement-mediated disease clinical trial. Such haplotypes can be protective haplotypes. For example, and not to be limiting, a protective haplotype can be H3_62_A, H5_62_A, H11_62_A, H3_51_B, H5_51_B, H_12_51_B, H14_51_B, or a complement thereof. Thus, described herein are methods for identifying a female Caucasian subject that is appropriate for a complement-mediated disease clinical trial comprising determining in the female Caucasian subject the identity of one or more haplotypes described herein, wherein a protective haplotype can indicate that the female subject is not appropriate for a complement-mediated disease clinical trial. For example, a female subject identified to have a protective haplotype described herein can be excluded from participation in a clinical trial designed to evaluate a treatment for a complement-mediated disease.

In yet another aspect, determining the identity of a certain haplotype or certain haplotypes in a female subject can indicate that the subject may be appropriate for a complement-mediated disease clinical trial. Such haplotypes can be neutral haplotypes. For example, and not to be limiting, a neutral haplotype can be H4_62_A, H6_62_A, H7_62_A, H8_62_A, H9_62_A, H10_62_A, H12_62_A, H13_62_A, H14_62_A, H15_62_A, H4_51_B, H6_51_B, H7_51_B, H8_51_B, H9_51_B, H10_51_B, H11_5_B, H13_51_B, H15_51_B, H16_51_B, H17_51_B, H18_51_B, H19_51_B, H20_51_B, or a complement thereof. Thus, described herein are methods identifying a female Caucasian subject appropriate for a complement-mediated disease clinical trial comprising determining in the female Caucasian subject the identity of one or more haplotypes described herein, wherein a neutral haplotype can indicate a female that may be appropriate for a complement-mediated disease clinical trial.

Furthermore, described herein are methods of identifying a male Caucasian subject that is appropriate for a complement-mediated disease clinical trial comprising determining in the male Caucasian subject the identity of one or more major or minor haplotypes. The haplotypes can include, but are not limited to H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H18_51_B, H19_51_B, or a complement thereof. As described herein, the presence of one or more of the haplotypes disclosed herein can indicate whether the male subject is appropriate for a complement-mediated disease clinical trial. In one aspect, the complement-mediated disease can be age-related macular degeneration (AMD), and the clinical trial can be an age-related macular degeneration clinical trial. Thus, the methods described herein can be used to identify a male Caucasian subject that is appropriate for an age-related macular degeneration clinical trial.

In one aspect, determining the identity of a certain haplotype or certain haplotypes in a male subject can indicate that the male subject is appropriate for a complement-mediated disease clinical trial. Such hapltoypes can be risk haplotypes. For example, and not to be limiting, a risk haplotype can be H2_62_A, H1_51_B, H2_51_B, or a complement thereof. Thus, described herein are methods for identifying a male Caucasian subject that is appropriate for a complement-mediated disease clinical trial comprising determining in the male Caucasian subject the identity of one or more haplotypes described herein, wherein a risk haplotype can indicate that the male subject is appropriate for the clinical trial. In one aspect, the method of identifying a male subject for the clinical trial can further comprise administering a therapeutic composition to the male subject identified as being appropriate for the clinical trial.

In another aspect, determining the identity of a certain haplotype or certain haplotypes in a male subject can indicate that the male subject is not appropriate for a complement-mediated disease clinical trial. Such haplotypes can be protective haplotypes. For example, and not to be limiting, a protective haplotype can be H11_62_A, H3_51_B, H14_51_B, or a complement thereof. Thus, described herein are methods for identifying a male Caucasian subject that is appropriate for a complement-mediated disease clinical trial comprising determining in the male Caucasian subject the identity of one or more haplotypes described herein, wherein a protective haplotype can indicate that the male subject is not appropriate for a complement-mediated disease clinical trial. For example, a male subject identified to have a protective haplotype described herein can be excluded from participation in a clinical trial designed to evaluate a treatment for a complement-mediated disease.

In yet another aspect, determining the identity of a certain haplotype or certain haplotypes in a male subject can indicate that the subject may be appropriate for a complement-mediated disease clinical trial. Such haplotypes can be neutral haplotypes. For example, and not to be limiting, a neutral haplotype can be H1_62_A, H3_62_A, H4_62_A, H5_62_A, H6_62_A, H7_62_A, H8_62_A, H9_62_A, H10_62_A, H12_62_A, H13_62_A, H14_62_A, H15_62_A H4_51_B, H5_51_B, H6_51_B, H7_51_B, H8_51_B, H9_51_B, H10_51_B, H11_51_B, H12_51_B, H13_51_B, H15_51_B, H16_51_B, H17_51_B, H18_51_B, H19_51_B, H20_51_B, H21_51_B, H22_51_B, or a complement thereof. Thus, described herein are methods of identifying a male Caucasian subject appropriate for a complement-mediated disease clinical trial comprising determining in the male Caucasian subject the identity of one or more haplotypes described herein, wherein a neutral haplotype can indicate a male that may be appropriate for a complement-mediated disease clinical trial.

Also described herein are methods of identifying a Caucasian subject appropriate for complement-mediated disease clinical trial comprising determining in the subject the identity of one or more SNPs in the CFH-to-F13B locus. The SNPs can be, but are not limited to: (i) rs1061170, rs1410996, rs2274700, rs3753395, rs403846, or rs3753396 or (ii) a SNP in linkage disequilibrium with the SNPs of (i). In one aspect, the presence of one or more of the SNPs described herein can indicate whether the subject is appropriate for the clinical trial. In one aspect, an A at the rs1061170 SNP can indicate the subject is appropriate for the clinical trial. In another aspect, an A at the rs1410996 SNP, an A at the rs2274700 SNP, a T at the rs3753395 SNP, or a G at the rs403846 SNP can indicate the subject is not appropriate for the clinical trial. Thus, for example, when it is determined that a Caucasian subject has an A at the rs1410996 SNP, an A at the rs2274700 SNP, a T at the rs3753395 SNP, or a G at the rs403846 SNP, the subject can be excluded from a complement-mediated disease clinical trial. In yet another aspect, a G at the rs3753396 SNP can indicate the subject may be appropriate for the clinical trial.

Also described herein are methods of identifying a Caucasian subject appropriate for an a complement-mediated disease clinical trial comprising determining in the subject the identity of a deletion tagging SNP in the CFH-to-F13B locus. The SNP can include, but is not limited to: (i) rs12144939 or (ii) a SNP in linkage disequilibrium with rs12144939. In one aspect, the presence of one or more of the SNPs can indicate whether the subject is appropriate for the clinical trial. In another aspct, a T at the rs12144939 SNP can indicate the subject is not appropriate for the clinical trial.

Also described herein are methods of identifying a Caucasian subject appropriate for a complement-mediated disease clinical trial comprising determining in the Caucasian subject the identity of at least six SNPs in the CFH-to-F13B locus. The SNPs can be, but are not limited to: (i) rs35928059, rs800292, rs1061170, rs12144939, rs7546940, rs1409153, rs10922153, or rs698859, or (ii) a SNP in linkage disequilibrium with the SNPs of (i). In one aspect, the presence of at least six of the SNPs can indicate whether the subject is appropriate for the clinical trial.

In another aspect, an A at rs35928059, a G at rs800292, a C at rs1061170, a G at rs12144939, a G at rs7546940, a G at rs1409153, a G at rs10922153, or an A at rs698859 can indicate the subject is appropriate for the clinical trial. In yet another aspect, an A at rs35928059, a G at rs800292, a C at rs1061170, a G at rs12144939, a G at rs7546940, a C at rs1409153, a G at rs10922153, or a T at rs698859 can indicate the subject is appropriate for the clinical trial. In still another aspect, an A at rs35928059, a G at rs800292, a C at rs1061170, a G at rs12144939, a G at rs7546940, a C at rs1409153, a G at rs10922153, or a C at rs698859 can indicate the subject is appropriate for the clinical trial. In another aspect, an A at rs35928059, an A at rs800292, a T at rs1061170, a G at rs12144939, a G at rs7546940, an A at rs1409153, a T at rs10922153, or a G at rs698859 can indicate the subject is not appropriate for the clinical trial. In yet another aspect, an A at rs35928059, a G at rs800292, a T at rs1061170, a T at rs12144939, a G at rs7546940, an A at rs1409153, a T at rs10922153, or a G at rs698859 can indicate the subject is not appropriate for the clinical trial. In yet another aspect, an A at rs35928059, a G at rs800292, a T at rs1061170, a T at rs12144939, an A at rs7546940, an A at rs1409153, a T at rs10922153, or an A at rs698859 can indicate the subject is not appropriate for the clinical trial. In still another aspect, an A at rs35928059, an A at rs800292, a T at rs1061170, a G at rs12144939, a G at rs7546940, a T at rs1409153, a T at rs10922153, or a C at rs698859 can indicate the subject is not appropriate for the clinical trial. In another aspect, an A at rs35928059, a G at rs800292, a T at rs1061170, a T at rs12144939, a G at rs7546940, a T at rs1409153, a T at rs10922153, or a C at rs698859 can indicate the subject is not appropriate for the clinical trial. In yet another aspect, an A at rs35928059, a G at rs800292, a T at rs1061170, a T at rs12144939, an A at rs7546940, a T at rs1409153, a T at rs10922153, or a T at rs698859 can indicate the subject is not appropriate for the clinical trial. In still another aspect, an A at rs35928059, a G at rs800292, a T at rs1061170, a T at rs12144939, a G at rs7546940, a T at rs1409153, a T at rs10922153, or a T at rs698859 can indicate the subject is not appropriate for the clinical trial.

Also described herein are methods of identifying a female Caucasian subject appropriate for a complement-mediated disease clinical trial comprising determining in the female Caucasian subject the identity of at least six SNPs in the CFH-to-F13B locus. The SNPs can include, but are not limited to: (i) rs35928059, rs800292, rs1061170, rs12144939, rs7546940, rs1409153, rs10922153, or rs698859, or (ii) a SNP in linkage disequilibrium with the SNPs of (i). In one aspect, the presence of at least six of the SNPs can indicate whether the subject is appropriate for the clinical trial.

In another aspect, an A at rs35928059, a G at rs800292, a C at rs1061170, a G at rs12144939, a G at rs7546940, a G at rs1409153, a G at rs10922153, or an G at rs698859 can indicate the subject is appropriate for the clinical trial. In yet another aspect, an A at rs35928059, a G at rs800292, a C at rs1061170, a G at rs12144939, a G at rs7546940, a G at rs1409153, a G at rs10922153, or an A at rs698859 can indicate the subject is appropriate for the clinical trial. In still another aspect, an A at rs35928059, a G at rs800292, a C at rs1061170, a G at rs12144939, a G at rs7546940, a C at rs1409153, a G at rs10922153, or a C at rs698859 can indicate the subject is appropriate for the clinical trial. In another aspect, an A at rs35928059, a G at rs800292, a C at rs1061170, a G at rs12144939, a G at rs7546940, a C at rs1409153, a G at rs10922153, or a T at rs698859 can indicate the subject is appropriate for the clinical trial. In yet another aspect, an A at rs35928059, an A at rs800292, a T at rs1061170, a G at rs12144939, a G at rs7546940, an A at rs1409153, a T at rs10922153, or a G at rs698859 can indicate the subject is not appropriate for the clinical trial. In still another aspect, an A at rs35928059, a G at rs800292, a T at rs1061170, a T at rs12144939, a G at rs7546940, an A at rs1409153, a T at rs10922153, or a G at rs698859 can indicate the subject is not appropriate for the clinical trial. In another aspect, an A at rs35928059, a G at rs800292, a T at rs1061170, a T at rs12144939, an A at rs7546940, an A at rs1409153, a T at rs10922153, or an A at rs698859 can indicate the subject is not appropriate for the clinical trial. In yet another aspect, an A at rs35928059, an A at rs800292, a T at rs1061170, a G at rs12144939, a G at rs7546940, a T at rs1409153, a T at rs10922153, or a C at rs698859 can indicate the subject is not appropriate for the clinical trial. In still another aspect, an A at rs35928059, a G at rs800292, a T at rs1061170, a T at rs12144939, a G at rs7546940, a T at rs1409153, a T at rs10922153, or a C at rs698859 can indicate the subject is not appropriate for the clinical trial. In another aspect, an A at rs35928059, a G at rs800292, a T at rs1061170, a T at rs12144939, an A at rs7546940, a T at rs1409153, a T at rs10922153, or a T at rs698859 can indicate the subject is not appropriate for the clinical trial. In yet another aspect, an A at rs35928059, a G at rs800292, a T at rs1061170, a T at rs12144939, a G at rs7546940, a T at rs1409153, a T at rs10922153, or a T at rs698859 can indicate the subject is not appropriate for the clinical trial.

Also described herein are methods of identifying a male Caucasian subject appropriate for a complement-mediated disease clinical trial comprising determining in the male Caucasian subject the identity of at least six SNPs in the CFH-to-F13B locus. The SNPs can be, but are not limited to: (i) rs35928059, rs800292, rs1061170, rs12144939, rs7546940, rs1409153, rs10922153, or rs698859, or (ii) a SNP in linkage disequilibrium with the SNPs of (i). In one aspect, the presence of at least six of the SNPs can indicate whether the subject is appropriate for the clinical trial.

In another aspect, an A at rs35928059, a G at rs800292, a C at rs1061170, a G at rs12144939, a G at rs7546940, a G at rs1409153, a G at rs10922153, or an A at rs698859 can indicate the subject is appropriate for the clinical trial. In another aspect, an A at rs35928059, a G at rs800292, a C at rs1061170, a G at rs12144939, a G at rs7546940, a C at rs1409153, a G at rs10922153, or a C at rs698859 can indicate the subject is appropriate for the clinical trial. In still another aspect, an A at rs35928059, a G at rs800292, a C at rs1061170, a G at rs12144939, a G at rs7546940, a C at rs1409153, a G at rs10922153, or a T at rs698859 can indicate the subject is appropriate for the clinical trial. In another aspect, an A at rs35928059, a G at rs800292, a T at rs1061170, a T at rs12144939, an A at rs7546940, an A at rs1409153, a T at rs10922153, or an A at rs698859 can indicate the subject is not appropriate for the clinical trial. In yet another aspect, an A at rs35928059, an A at rs800292, a T at rs1061170, a G at rs12144939, a G at rs7546940, a T at rs1409153, a T at rs10922153, or a C at rs698859 can indicate the subject is not appropriate for the clinical trial. In still another aspect, an A at rs35928059, a G at rs800292, a T at rs1061170, a T at rs12144939, a G at rs7546940, a T at rs1409153, a T at rs10922153, or a T at rs698859 can indicate the subject is not appropriate for the clinical trial.

Also described herein are methods of enhancing clinical trials comprising choosing appropriate patient populations for those clinical trials. In one aspect, the methods can be used to ensure patients are identified to participate in clinical trials based upon whether or not they are responsive to the experimental therapeutic agent or agents being studied. The methods can comprise monitoring the condition of subjects receiving treatment for a complement-mediated disease such as AMD. A successful treatment outcome can be indicated by return of complement pathway activity, such as expression level, biochemical activity (e.g., enzymatic activity of a complement component), or serum auto antibodies against complement pathway associated molecules, from abnormal levels to or toward normal levels. In one aspect, the methods can comprise measuring an initial value for the level of abnormal activity (e.g., abnormal presence of an autoantibody, or abnormal levels of complement pathway molecules) before the subject has received treatment. Repeat measurements can then be made over a period of time. For example, and not to be limiting, that period of time can be about 1 day, 2 days, 5 days, 1 week, 2 weeks, 3 weeks, 1 month, 2 months, 3 months, 4 months, 6 months, 9 months, 1 year, or greater than 1 year. If the initial level is elevated relative to the mean level in a control population, a significant reduction in level in subsequent measurements can indicate a positive treatment outcome. Likewise, if the initial level of a measure marker is reduced relative to the mean in a control population, a significant increase in measured levels relative to the initial level can signal a positive treatment outcome. Subsequently measured levels are considered to have changed significantly relative to initial levels if a subsequent measured level differs by more than one standard deviation from the mean of repeat measurements of the initial level. If monitoring reveals a positive treatment outcome, that indicates a patient that can be chosen to participate in a clinical trial for that particular therapeutic agent or agents. If monitoring reveals a negative treatment outcome, that indicates a patient that should not be chosen to participate in a clinical trial for that particular therapeutic agent or agents.

### C. KITS

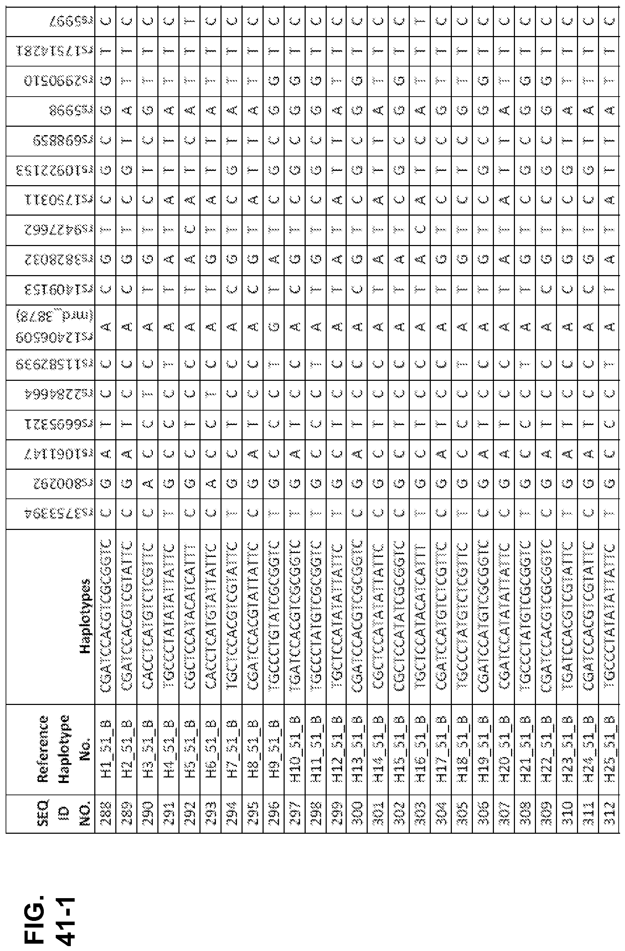

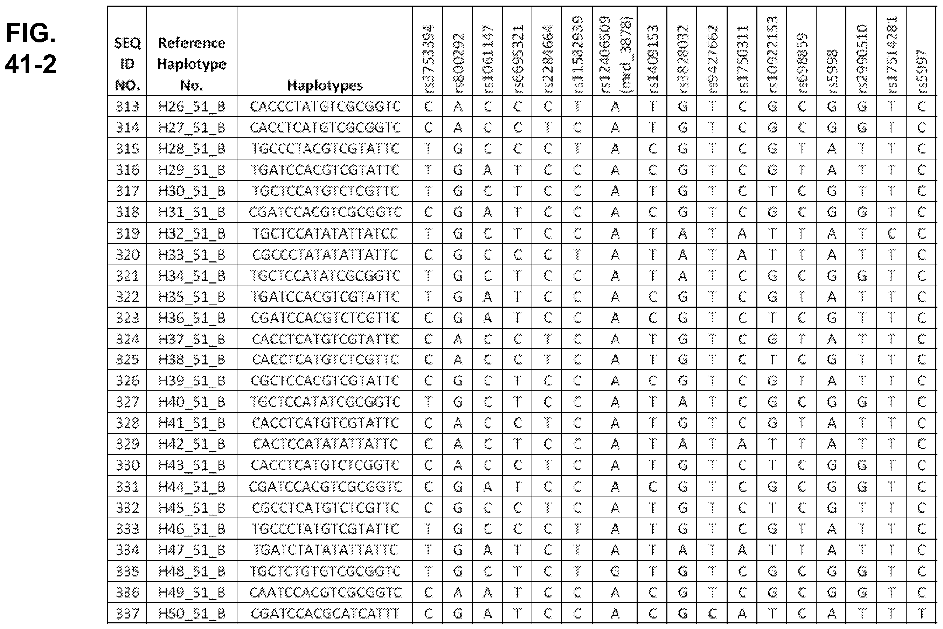

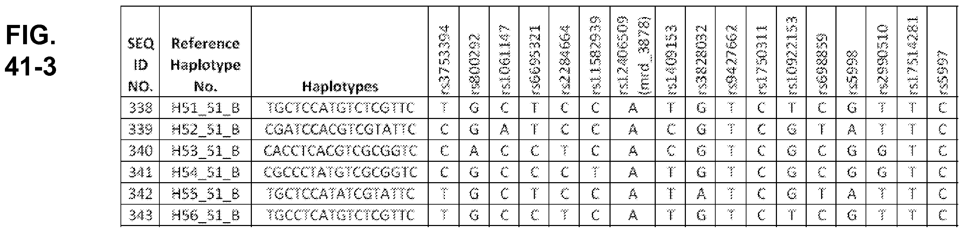

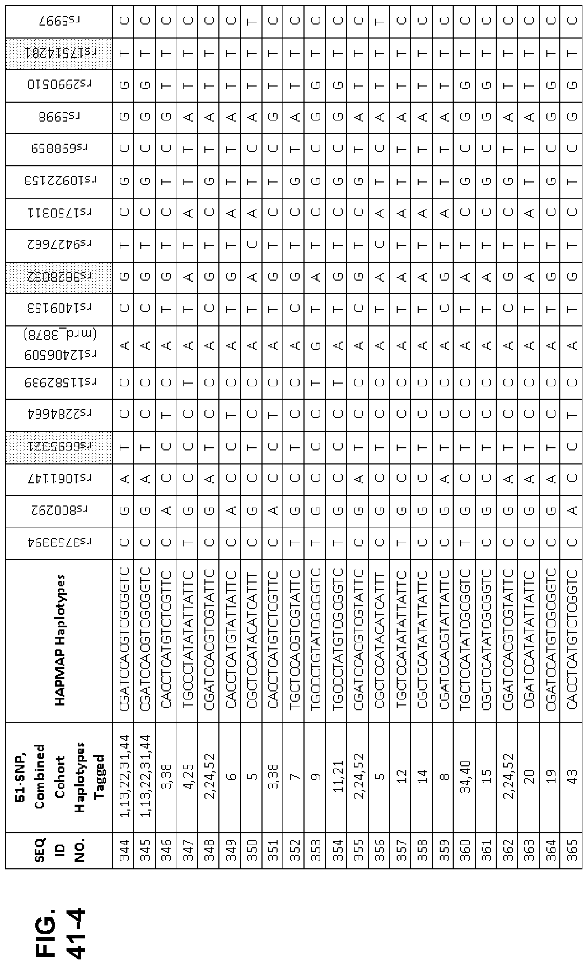

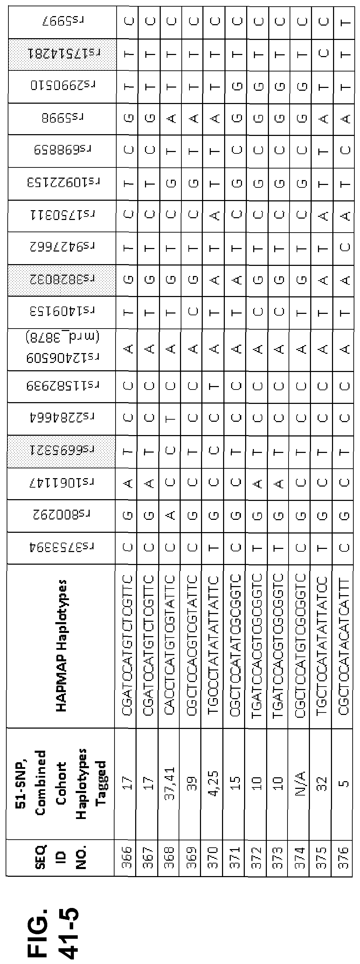

Also described herein are kits for utilizing the methods described herein. The kits described herein can comprise an assay or assays for detecting one or more haplotypes in a nucleic acid sample of a subject, wherein the one or more haplotypes are the haplotypes described herein. For example, and not to be limiting, the one or more haplotypes can be H1_62_A, H2_62_A, H3_62_A, H4_62_A, H5_62_A, H6_62_A, H7_62_A, H8_62_A, H9_62_A, H10_62_A, H11_62_A, H12_62_A, H13_62_A, H14_62_A, H15_62_A, H15_1_A, H2_51_A, H3_51_A, H4_51_A, H5_51_A, H6_51_A, H7_51_A, H8_51_A, H9_51_A, H_10_51_A, H11_51_A, H12_51_A, H13_51_A, H14_51_A, H15_51_A, H16_51_A, H17_51_A, H1_51_B, H2_51_B, H3_51_B, H4_51_B, H5_51_B, H6_51_B, H7_51_B, H8_51_B, H9_51_B, H10_51_B, H11_51_B, H12_51_B, H13_51_B, H14_51_B, H15_51_B, H16_51_B, H17_51_B, H1_51_B, H19_51_B, H20_51_B, H21_51_B, H22_51_B, or a complement thereof. In one aspect, the one or more haplotypes can be the haplotype tagging haplotypes described herein as well as the haplotype tagging haplotypes shown in Figure 41.

The kits described herein can further comprise amplification reagents for amplifying the CHF-to-F13B locus, or any of the genes or nucleic acids described herein.

### D. EXAMPLES

The following examples are put forth so as to provide those of ordinary skill in the art with a complete disclosure and description of how the compounds, compositions, articles, devices and/or methods claimed herein are made and evaluated, and are intended to be purely exemplary of the invention. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. Example 1: Patient Cohorts

DNA samples derived from well-characterized cohorts of study patients with and without AMD were used for the various studies described herein (see Abrera-Abeleda et al 2006; Allikmets et al 2009; Baird et al 2006; DeAngelis et al 2008; Gold et al 2006; Hageman et al, 2005, 2006, 2011; Lim et al 2012; Robman et al 2010; Wickremasinghe et al 2011; Zhang 2008). These collections consist of over 5,000 DNA samples from Caucasian patients with and without AMD. All cohorts were case-control cohorts, with the exception of two sib-pair cohorts, one highly discordant and one both discordant and concordant (657 subjects in 284 families). Index patients in the sib-pair cohort were aged 50 years or older and had CNV in at least one eye, as defined by sub-retinal hemorrhage, fibrosis or fluorescein angiographic presence of neovascularization documented at the time or, or prior to, enrolment in the study. The unaffected siblings had normal maculas at an age older than that at which the index patient was first diagnosed with CNV. All individuals in both the case-control and sib-pair cohorts were of European-American descent, over the age of sixty, and matched for age and ethnicity. Patients were examined and photographed by trained ophthalmologists. Urine, serum, plasma, DNA and extensive medical and ophthalmological data were available for the majority of these patients. Stereo fundus photographs were graded according to standardized classification systems as described in the references noted above. A large proportion of the samples in all cohorts were genotyped for variants and SNPs in the CFH to F13B locus, and other genes, as described herein.

DNA samples from several additional cohorts were also available: 1) early onset drusen cases (EOD) (103); 2) Caucasian newborns (1,870); 3) MPGN II (65); atypical hemolytic uremic syndrome (aHUS) (211); 4) glaucoma (330); 5) Alzheimer (1,214); 6) 45 families from the Utah Genetic Reference Project (UGRP); and 7) individuals from the Human Diversity Panels. Moreover, numerous other systemic and ocular diseases were represented in the collective set of samples.

### Example 2: Determination of Deletion Status and Extended Haplotypes Spanning the Chromosome 1q32 CFH-to-F13B Locus; Discovery Phase

### 2.1 Background

Recent genetic studies that have shown an association between age-related macular degeneration (AMD) and specific haplotypes of the complement factor H gene (CFH), which lies within the "regulators of complement activation" (RCA) gene cluster on chromosome 1q25-q31 (Hageman et al 2005). Although the understanding of nucleotide polymorphisms in loci associated with AMD has increased, characterization of the disease-associated CFH risk and protective haplotypes has not yet been assessed fully. Recent reports suggest that risk variants of the CFH gene in the Japanese and Chinese populations are either not associated with AMD, differ substantially from the Caucasian population and/or that the risk haplotype occurs at a substantially lower frequency (Lau et al 2006; Gotoh et al 2006).

Five CFH-related genes (CFHR1, CFHR2, CFHR3, CFHR4 and CFHR5), one CFHR pseudogene (LOC100289145) and one additional gene containing complement SCR domains (Factor 13B, or F13B) lie within the regulators of complement activation (RCA) locus on chromosome 1q32. A large, common deletion that encompasses both the CFHR1 and CFHR3 genes has been identified on one of the CFH protective haplotypes (Hageman et al 2006).

Due to the strong homology between these seven genes, substantial copy number variation with this 1q32 locus, and the lack of understanding of Complement-Mediated Disease causality within this locus, extended haplotypes encompassing CFH, the five CFHR genes, the CFHR pseudogene (LOC100289145) and Factor 13B (F13B) were determined, and their association with AMD was assessed.

### 2.2 Methods: Study Patient Samples

DNA samples from study patients, including sib pairs, with and without AMD were used. DNA samples from the 1,073 subjects employed for this study (referred to as '1,073 Cohort', 'GSH Cohort' or 'ParAllele Cohort') were of European-American decent and selected from a larger cohort (see Example 1 above). The average age of subjects was 76.2 years. Fifty-nine percent were female. 56% of subjects had age related maculopathy (ARM) or age-related macular degeneration (AMD) and 34.5% were controls. 0.6% of the patients in this cohort could not be graded.

### 2.3 Methods: Overview of Strategy for CFH-to-F13B Haplotype Determination and Validation in the Discovery Cohort

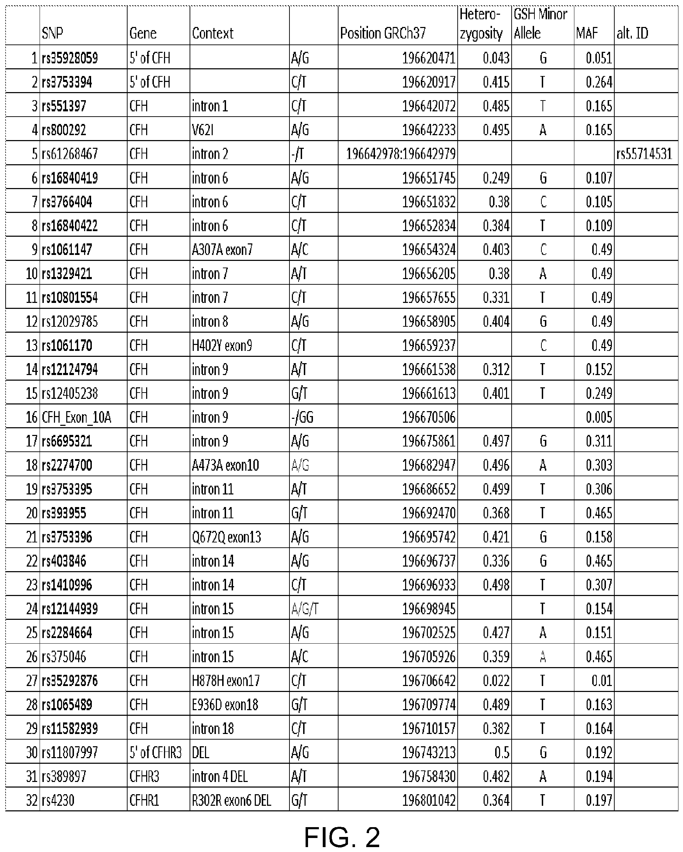

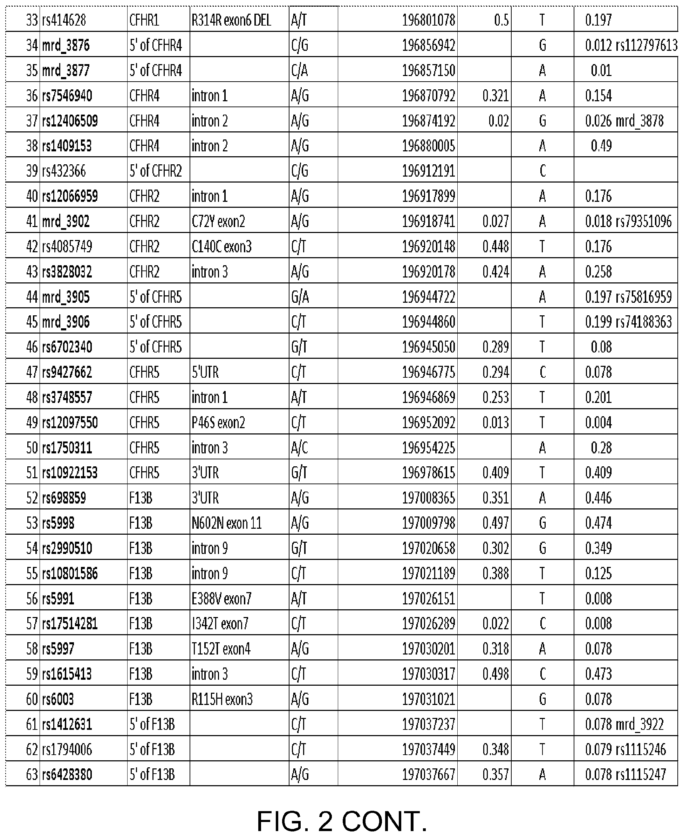

Sixty-three SNPs (Figure 2) spanning the CFH-to-F13B region were genotyped in 1,073 Caucasian individuals. Missing genotypes were imputed. The deletion status of the CFHR3/CFHR1 genes was assessed initially using single-strand conformation polymorphism (SSCP) and a qPCR assay developed to detect the deletion on single chromosomes. Genotypes were phased five independent times using PHASE v2.1.1, results were combined, and the most likely phased haplotypes for all 1,073 individuals were extracted. Haplotypes for all chromosomes were subsequently constructed (phased). Haplotype validity was confirmed in three-generation families. Haplotype and SNP disease associations were assessed using appropriate statistical assessment methods.

### 2.4 Methods: Single Nucleotide Polymorphism (SNPs) Genotyping

Sixty-three single nucleotide polymorphisms (SNPs) within the CFH-to-F13B locus, including non-genic regions both proximal and distal to the CFH and F13B genes (Figure 2), were genotyped using SSCP-based genotyping [5], Taqman based genotyping (ABI), Sanger sequencing, and molecular inversion probe genotyping (ParAllele BioScience) [6] in 1,073 human genomic DNA samples. SNPs were selected based on significance of association in a prior ParAllele Study (a study in which 1,162 DNA samples were genotyped using the Affymetrix 3K 'Iowa AMD Panel'; the final data yielded successful genotypes for 1,113 samples, with a final number of 3,266 successful assays out 3,308 assays attempted), as well as location to provide relatively dense coverage across the entire CFH-to-F13B locus. Missing values were multiply imputed during haplotype construction using PHASE v. 2.1.1 (Am. J. Hum. Gen. 68:978-989, 2001).

### 2.5 Methods: Identification of CFHR3/CFHR1 Deletion Chromosomes

The deletion status of the CFHR-3/CFHR-1 genes was assessed using SSCP and a qPCR assay developed to detect the deletion on single chromosomes. SSCP assay was used to identify all individuals homozygous for the CFHR3/CFHR1 deletion. Using a pair of PCR primers that amplify both CFH exon 22 and a homologous region in CFHR1 (see Hageman et al 2006 for primers), one of the resulting SSCP conformations (referred to as 'shift 4') was diagnostic of a subject for which both copies of CFHR1 were absent.

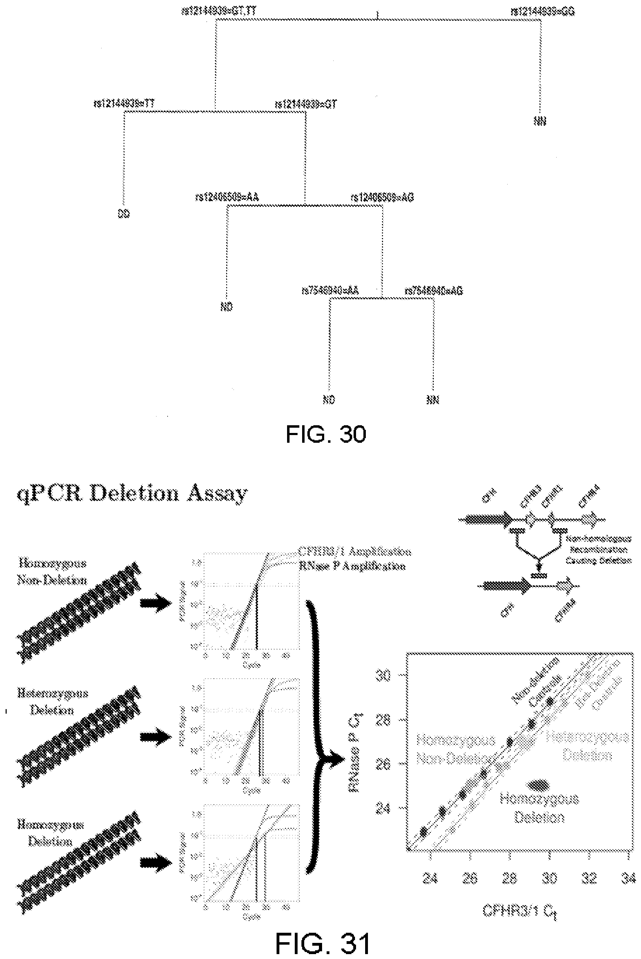

Real-time quantitative PCR (qPCR) was also used to determine the copy number state of the CFHR3 and CFHR1 genes (Figure 31). A set of five to six independent TaqMan assays measured simultaneously the amount of chromosomal DNA upstream of CFHR3 ('the deletion amplicon') using a 6-FAM fluorescent signal and the endogenous control amplicon of RNase P using a VIC tag (Applied Biosystems RNase P Control Reagent). The sequences of the primers and probe for the deletion assay were designed based on the mrd_3855 amplicon. They were designated mrd_3855-ANYF (TCT GCT CCT GCT CTT CTT TTC C SEQ ID NO. 285), mrd_3855-ANYR (CTC TAT GCT TGC TAC AAG AGA CAC A SEQ ID NO. 286), and mrd_3855-PROBE (6-FAM-AAC AGC ATG GAA TAT C-MGB SEQ ID NO. 287). The reference amplicon was of RNase P, a single copy gene, and reagents were purchased from Applied Biosystems (part number 4316844) with a VIC fluorescent tag attached. Thus, the FAM tag of the deletion amplicon and the VIC tag of the control amplicon could be detected simultaneously in the same rtPCR well. Applied Biosystems Fast Universal PCR Master Mix provided both the polymerase and ROX exogenous non-amplifying background fluorophore. An Applied Biosystems 7900HT Fast Real-Time PCR System was used for all experiments.

Following data acquisition, analysis was performed using a program written in the R statistical software language. The optimal fluorescence threshold for detection of the exponential amplification phase was determined using a set of serial dilutions of control samples containing one copy of CFHR3/CFHR1 per chromosome. Uncertainties were propagated at all stages to obtain a distribution of Ct values for all samples and all replicates. A sample was classified as having no deletion chromosomes (homozygous normal) if most sample replicates had Ct values for both the deletion amplicon and RNase P similar to the normal control. Samples heterozygous for the CFHR3/CFHR1 deletion had Ct values for the deletion amplicon approximately one cycle later than expected based on their RNase P Ct values. Finally, samples homozygous for the deletion had either no or very minor amplification of the deletion amplicon. A serial dilution of a normal control was run on each qPCR plate.

### 2.6 Methods: Deletion Prediction

Deletion status was modeled as a biallelic marker: deletion or no-deletion (normal) for each chromosome. Real-Time qPCR was capable of identifying individuals homozygous (DD) or heterozygous (ND) for the deletion and those not containing a deletion (NN). Based upon the deletion status and the genotyping call of CFH locus SNPs, a decision tree capable of predicting deletion status from genotyped SNPs was created using the rpart library in the R statistical software package. In addition to this predictive tree, the strategy that any individual who was heterozygous for one of the four SNPs screened in the CFHR3/CFHR1 gene region (rs11807997, rs389897, rs4230, or rs414628) cannot contain a deletion for CFHR3 and CFHR1 was also employed. All of these SNPs are in the central portion of the deletion region and none were present when the CFHR3 and CFHR1 genes were absent.

### 2.7 Methods: Haplotype Inference

The qPCR deletion assay was used to classify 536 (and 801 in a second assay) individuals as homozygous deletion (DD), heterozygous deletion (ND), or homozygous non-deletion (NN). All remaining individuals were classified according to their genotypes for SNPs in CFHR3 and CFHR1. All individuals containing more than one heterozygous SNP in CFHR3 or CFHR1 were assumed to be not consistent with a deletion on either chromosome (genotype NN). All other individuals were assigned to have one normal chromosome and one unknown chromosome (N?). All heterozygous deletion individuals were assigned one unknown allele for those SNPs between CFH and CFHR4, the deleted region; homozygous SNPs in this region (ex. CC) were replaced with one known and one unknown allele (C?) while heterozygous SNPs, being inconsistent with a heterozygous deletion, were replaced with two unknown alleles (??). All homozygous deletion individuals were assigned two unknown alleles (??) for each SNPs in the same region.

PHASE version 2.1.1 was used to impute missing genotypes and phase genotypes for each individual, care was taken to correctly handle the presence or deletion of CFHR3/1 SNPs as a group. Chromosomal positions for each SNP were obtained from NCBI dbSNP build 129 using the reference human genome (build 18). All individuals typed at SNPs in the CFHR3/CFHR1 region and seven distinct four-SNP haplotypes were imputed for the CFHR3/CFHR1 deletion region using the four SNPs in the deletion region spanning from rs11807997 through rs414628. Five independent runs of PHASE were used to confirm haplotype consistency and two haplotypes were assigned to each individual. Seven distinct four-SNP haplotypes were imputed for this region. Although the first two haplotypes were sufficient to phase 99% of the chromosomes, all seven imputed haplotypes were utilized later to avoid over-fitting of the phased genotypes.

Deletion status was subsequently treated as an octa-allelic SNP - one for each of the seven imputed CFHR-3/CFHR-1 haplotypes and an eighth allele to signify the deletion. The entire CFH locus was thus modeled as 58 bi-allelic and one octa-alleic SNP. Five independent runs of PHASE were used to impute and phase the genotypes. Five independent runs of PHASE were used to confirm phased genotype consistency. Following genotype phasing, the multi-allelic deletion status was expanded back into four separate tri-allelic SNPs, with a D allele representing the deletion haplotype. In some assessments, all minor haplotypes with frequency less than 1% were iteratively re-assigned to the most similar remaining haplotype.

PHASE provided probability-weighted, imputed, and phased genotypes for all individuals. Each individual run of PHASE produced multiple weighted estimates of each individual's phased genotypes (using the pairs output file from PHASE). The five independent runs of PHASE produced up to five different imputations of missing genotypes, all of which were then equally weighted. To assign a final pair of haplotypes to each individual, the following algorithm was employed: 1) Input was a collection of individuals where each individual had a set of potential diplotypes and its estimated probability (from PHASE pairs output files); 2) The active set was initialized to include all possible haplotypes; 3) Individual diplotypes were assigned based on the individual's diplotype probabilities with the constraint that diplotypes could only use haplotypes in the active set. It was possible for an individual to not be assigned a diplotype if all of the individual's potential diplotypes required haplotypes not in the active set; 4) Step #7 was skipped to if any individual was not assigned a diplotype; 5) The assigned individual diplotypes were saved as the current solution; 6) The least-used haplotype was removed from the active set and step #3 was returned to; 7) The current solution was outputted as the list of individual diplotypes, and thus haplotypes, present in the population.

Phased genotypes were also processed in some analyses using Beagle (version 3.0.1) to obtain two haplotype networks - one merging similar haplotypes in the 5' → 3' direction, the second in the 3' → 5' direction. All ten phased genotypes for each individual from the five independent runs of PHASE were used as input to account for uncertainty in the phasing or imputation of missing genotypes. The scale parameter for Beagle's haplotype merging process was set to 4.0. The directed acyclic graph haplotype output by Beagle indicated which SNPs form haplotype blocks and how those blocks were interrelated. Each chromosome was assigned a haplotype based on its path through the two networks leading to the rs4230 SNP. Haplotypes accounting for less than 1% of the chromosomes were not reported.

### 2.8 Methods: Haplotype Validation

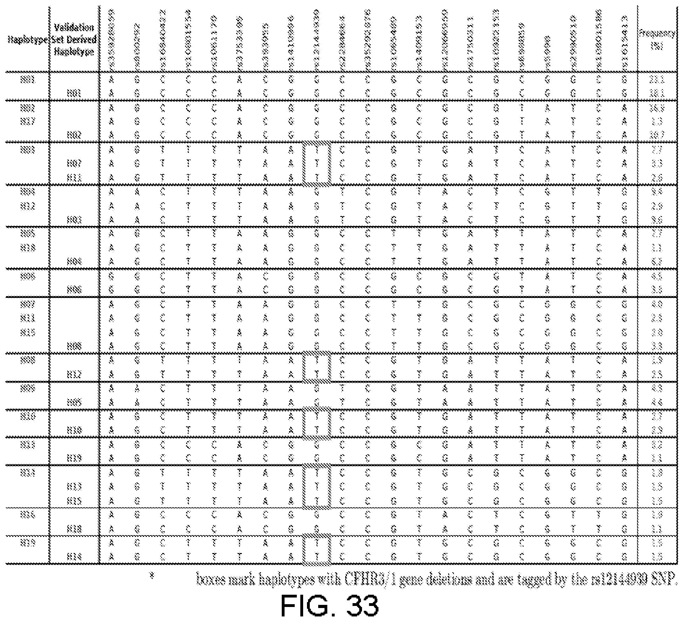

Reference control phased haplotypes were obtained from the members of the Utah Genetic Reference Project families (Ann. Rev. Genomics Hum. Genet. 9:347-358 2008). The UGRP family members consisted of over 600 individuals from 43 three-generation families that were also part of the original Utah CEPH (the Center d'Etude du Polymorphisme Humain) families. Sixty-six SNP genotypes were obtained on blood DNA samples from the entire UGRP members using the Affymetrix Genome-Wide Human SNP array 6.0. Genotype calling was carried out using the CRLMM calling algorithm from the Bioconductor 2.3 Oligo Package. Genotype calls whose confidence was less than 0.95 were replaced with missing values. SNP markers whose call rate was less than 95% were also dropped. Taqman SNP genotyping assays were also carried out to obtain genotypes for 23 additional SNP markers. PedCheck program was used to detect Mendelian inconsistencies and misinherited genotype calls were removed. Phased haplotypes were constructed based on the Mendelian inheritance model in each of the three-generation families. None of the haplotype inference programs were used. Haplotype frequencies were calculated on 136 unrelated UGRP individuals, who were mostly first generation grandparents, using Haploview program's phased haplotype input mode, Haps format (Figure 33).

### 2.9 Methods: AMD Disease Association

Association of haplotypes with AMD was assessed by Bayesian logistic regression in individual SNPs, in 536 individuals (median age = 79 yrs): 216 individuals without AMD and 320 individuals with AREDS categories 2, 3, geographic atrophy, or neovascular AMD. Complement-Mediated Disease association was determined using a Bayesian logistic regression model with Cauchy priors on the coefficients (Ann. Appl. Stat. 2:1360-1383, 2008). Single SNP associations in this case-control cohort were also assessed.

### 2.10 Results: Genotyping and Identification of Novel CFH Locus SNPs

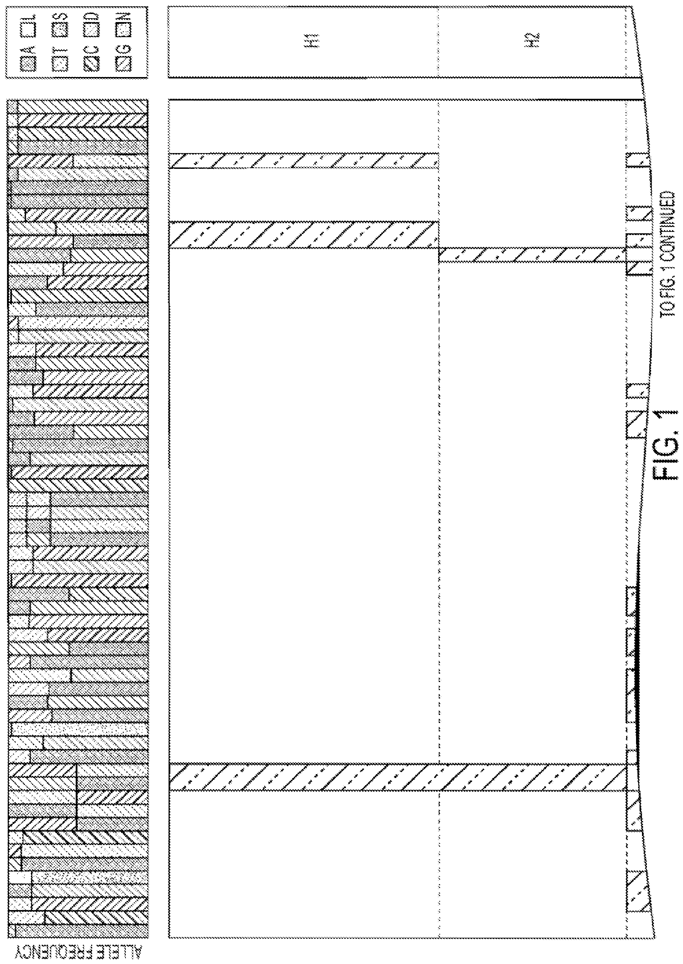

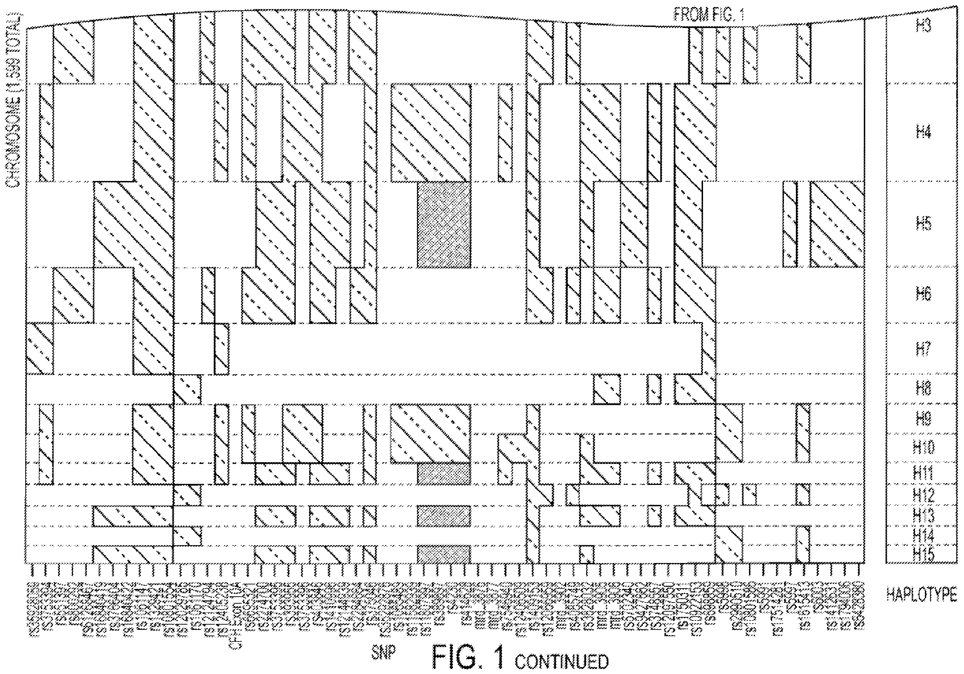

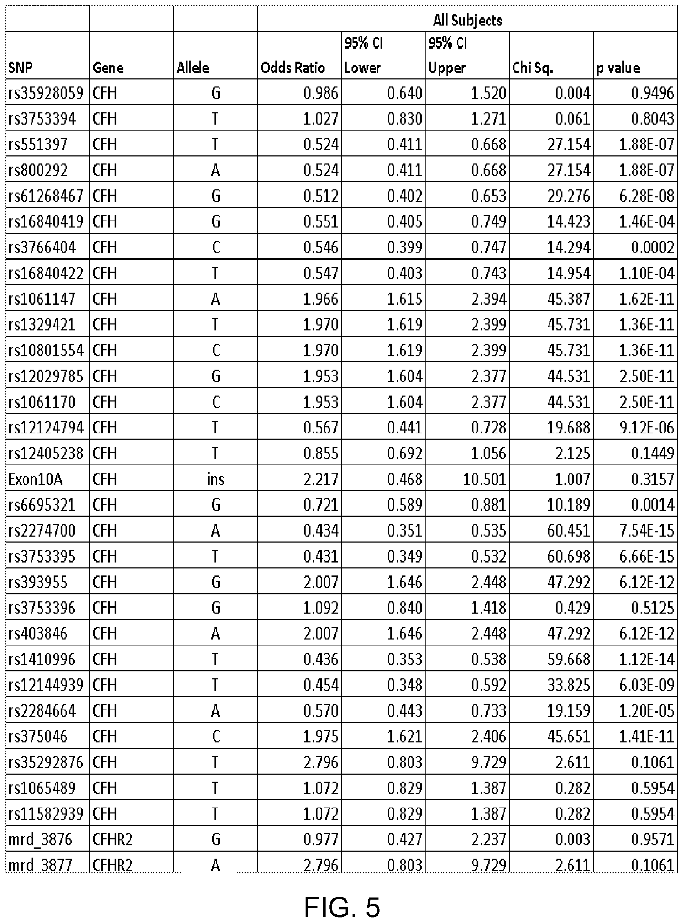

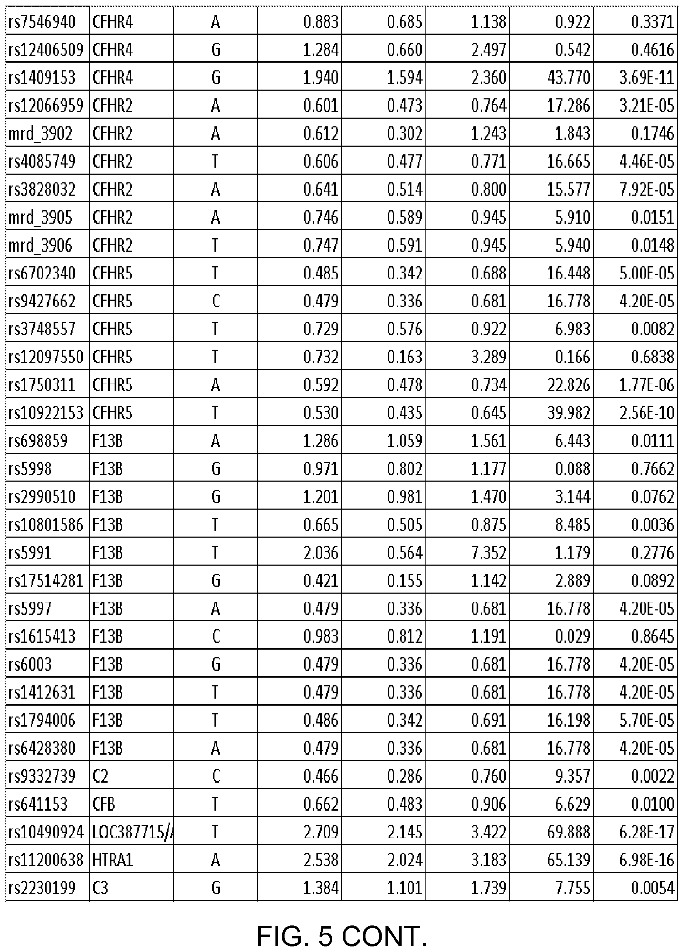

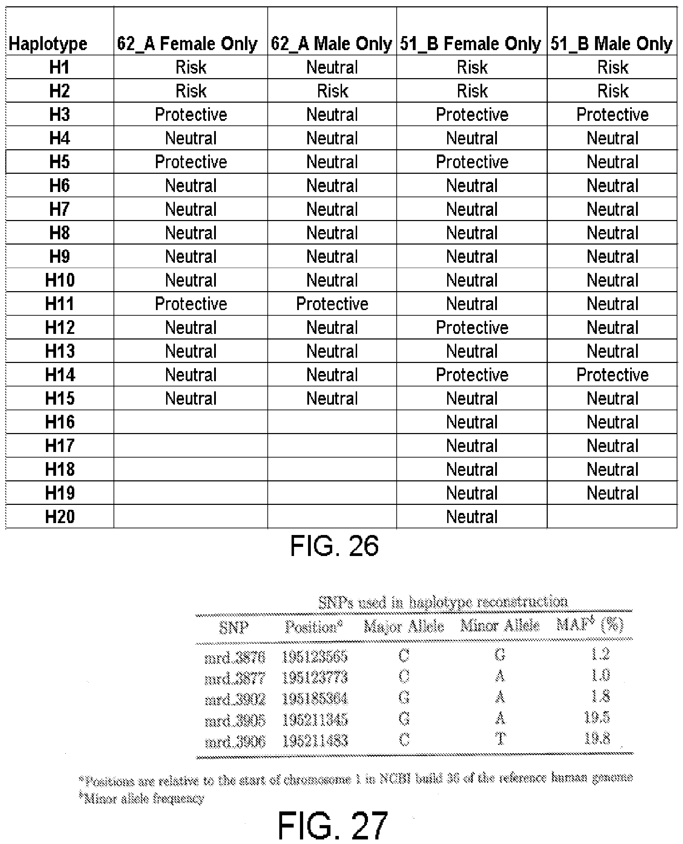

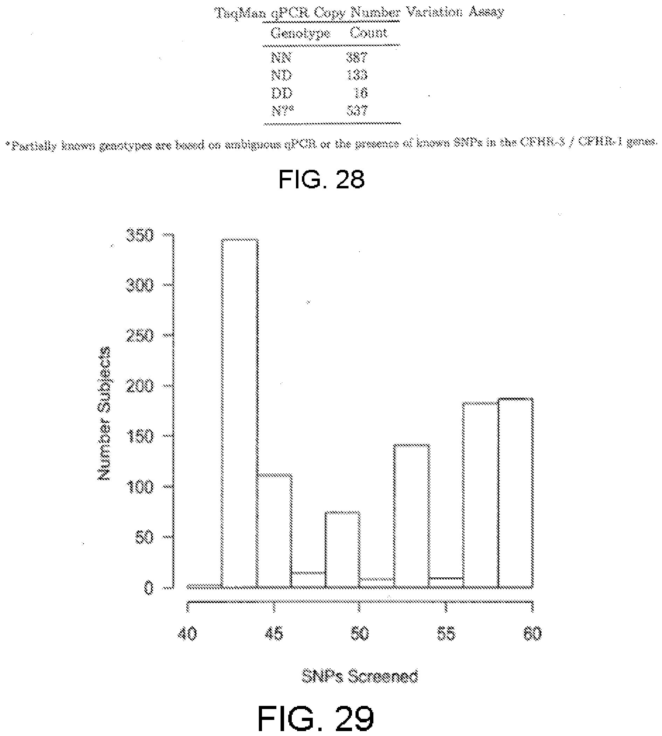

DNA from each subject was screened for 41 to 63 of the 63 SNPs employed (Figure 2 and Figure 29). Of the SNPs genotyped by ParAllele using MIP genotyping, five were unreported in the literature (Figure 27) at the time of their discovery. These were identified using mismatch repair detection (MRD) to amplify a variant-enriched pool of amplified genomic DNA. This pool of amplified DNA was then sequenced to identify rare alleles. The rare mrd_3876 and mrd_3877 SNPs were located within the promoter region of CFHR4. The minor allele of mrd_3876 was present in haplotype(s) that skewed toward risk for AMD, and the minor allele of mrd_3877 occurred in haplotype(s) that skewed significantly with AMD risk. The minor allele of mrd_3902, located within exon 2 of CFHR2, results in a Cys72Tyr coding mutation and occurred in haplotypes that appeared to skew toward protection for AMD. The minor alleles of SNPs mrd_3905 and mrd_3906 were more common (minor allele frequencies of approximately 20%) and were located within the promoter region of CFHR5; they did not appear to skew with AMD risk or protection (see Figures 1 and 5).

One additional novel SNP was identified within the CFH-to-F13B locus at the time these assays were conducted. This variant is located within CFH exon 10A - the last exon of the truncated form of complement factor H (Figure 2). This SNP was identified via SSCP and from sequencing is thought to be a one or two base-pair insertion into the 3' untranslated region of exon 10A. The minor allele, the 1-2 bp deletion, was present in only 1.2% of chromosomes.

### 2.11 Results: Deletion Chromosome Identification - TaqMan qPCR Copy Number Variation Assay

The results of the deletion assay are shown in FIG. 28. Homozygous deletion samples were verified by SSCP analysis. Of those samples that gave definitive copy number values, 28% had a deletion of one or both copies of CFHR3 and CFHR1. 15% of individual chromosomes did not contain these genes. After including imputed deletion status, 19% of individual chromosomes did not contain the deletion (absence of CFHR3 and CFHR1).

Experiments were also conducted to determine the reliability of this assay. As an example, one experiment produced 192 replicates for each of two DNA samples -- one homozygous non-deletion sample and a related heterozygous deletion sample. Each qPCR assay was perfomed using total genomic DNA between 100ng and 0.6ng. Based on these data, for a given amount of genomic DNA, an assay using heterozygous deletion DNA was predicted to have a Ct value 0.645 ± 0.002 cycles greater than an identical assay utilizing homozygous normal DNA. The expected distance from the line Ct,deletion = Ct,RNaseP for an individual sample was 0.16 cycles. Thus, the expected power for correctly classifying a heterozygous deletion of the CFHR3/CFHR1 genes using five to six qPCR replicates was 83%. This is similar to the 75% call rate shown in (Figure 28). Thus, to obtain a power of 95% with a 5% type-I error rate, nine replicates were run on each DNA sample.

### 2.12 Results: Deletion Prediction

Based upon measured (not imputed) genotypes, a decision tree (Figure 30) was made to predict deletion status from three SNPs. All homozygous deletion individuals had a TT genotype at SNP rs12144939 in CFH, although 17 heterozygous deletion individuals had genotype GG at this location. Thus, it was determined that deletion status in Caucasians can be predicted reliably by a 'T' at rs12144939 located within intron 15. The deletion status of the CFHR3/CFHR1 genes could be predicted with 97% specificity and 96% sensitivity based on the genotype of this single SNP. Fourteen percent of chromosomes in the discovery cohort contained this deletion. The inclusion of rs12406509 and rs7546940 (mrd_3878) slightly increased the sensitivity and specificity of the deletion prediction.

### 2.13 Results: Identification and Validation of 63 SNP-based CFH-to-F13B Haplotypes

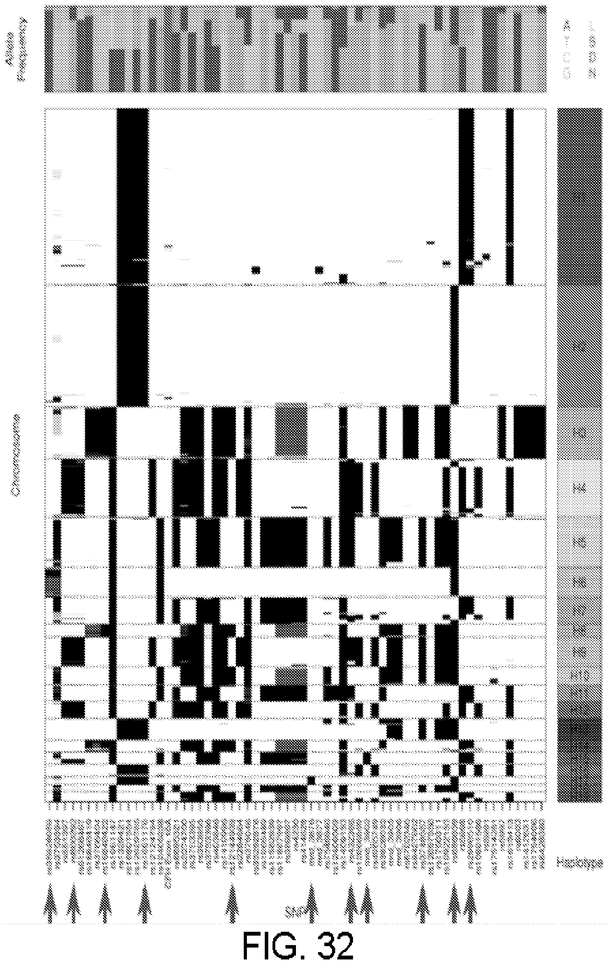

63 SNP-based haplotypes (Figure 32) spanning the extended CFH-to-F13B locus and including the CFHR3/CFHR1 deletion were determined. Nineteen major haplotypes (>1% frequencies) were identified within the approximately 260kb CFH-to-F13B locus based on haplotype construction using PHASE v.2.1.1 (Figure 32). Haplotype fidelity was confirmed in large, multi-generation Utah Genetic Reference Project families (Annu. Rev. Genomics Hum. Genet. 9:347-358 2008) (Figure 33). Haplotypes H1 and H2, the two most prevalent haplotypes (approximately 47% of the chromosomes in this cohort), contained the vast majority of the CFH Y402H variant. Five of the 19 haplotypes (H3, H8, H10, H14, and H19) contained the vast majority of all chromosomes with CFHR3/CFHR1 deletions. The CFH I62V protective variant was contained primarily in haplotypes H4, H9, and H12. Eleven SNPs (arrows in **Figure 32**) were sufficient to infer the nineteen haplotypes.

### 2.15 Results: Association of 63 SNP-based CFH-to-F13B Haplotypes with Disease

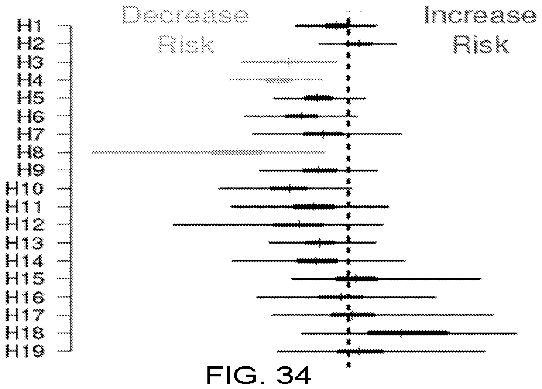

Preliminary assessment of AMD disease association showed significant associations of three of the nineteen major haplotypes with different AMD risk phenotypes (as a binary and quantitative trait) (p < 0.01), after computing bootstrap confidence intervals and controlling for age, sex, and smoking status. After controlling for age, gender, and smoking status, haplotypes H3, H4, and H8 were associated with significant decreased risk of developing AMD both as binary (p < 0.01) and as a quantitative trait (p < 0.05) (Figure 34).

### Example 3: Refined Assessment of Extended Haplotypes Spanning the Chromosome 1q32 CFH-to-F13B Locus

### 3.1 Methods: Study Cohorts

The same 1,073 subject cohort ('1,073 Cohort', 'GSH Cohort' or 'ParAllele Cohort') described in Example 2 was employed in studies to refine the understanding of extended CFH-to-F13B haplotypes and their association with AMD and other diseases. In addition, the highly discordant sib-pair cohort (657 subjects from 284 families), a combined AMD case-control cohort comprised of subjects ascertained in Utah (837 subjects), Iowa (1,555 subjects), Australia Melbourne (1,704 subjects) (this collective cohort is referred to as the 'Combined Cohort') and the UGRP cohort (604 subjects) were employed.

### 3.2 Methods: Genotyping

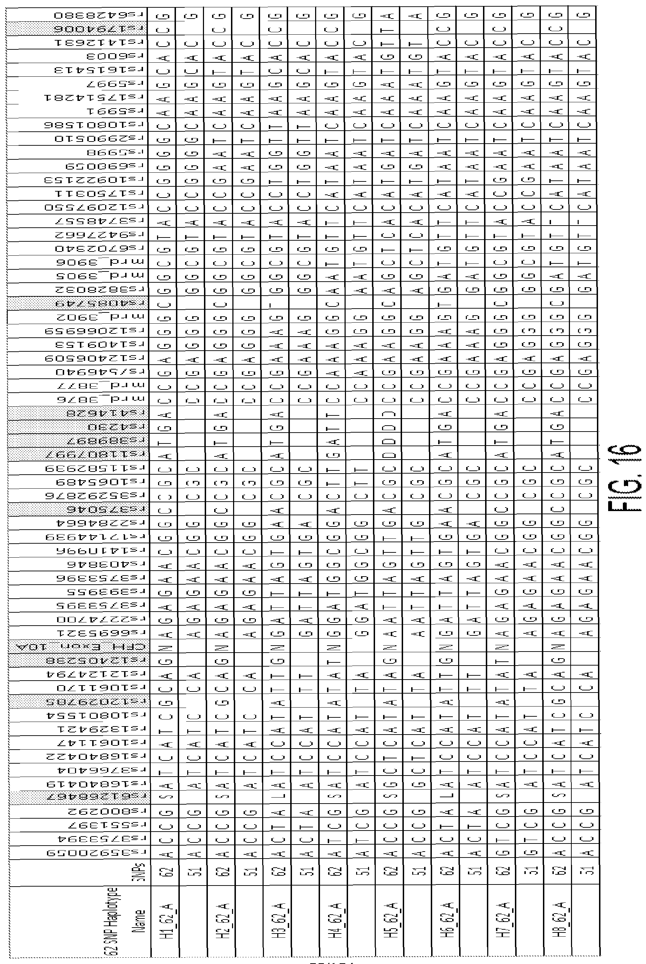

62 SNPs of the 63 SNPs employed in Example 2 were employed in the refined assessment. It was determined that one of the SNPs used to generate the 63 SNP haplotypes, rs432366, was not polymorphic in the location for which it was listed in the NCBI and other databases. Thus, this SNP was removed from all future analyses. Haplotype determinations and disease associations were reassessed based on the removal of rs432366. Genotype call rates exceeding 99% were obtained for 51 of the 62 variants and less than 99% for the remaining SNPs; missing genotypes were imputed using PHASE 2.1.1. Genotyping on all other cohorts was performed using either pre-designed or custom ordered Taqman assays (ABI) and, as such, limited to 51 SNPs (see bolded SNPs in Figure 2 and Figure 16).

### 3.3 Methods: Generation of 62 SNP-based and 51 SNP-based Haplotypes

PHASE 2.1.1 was employed to determine the SNP-based haplotypes present in the 1,073 patient cohort. Chromosomal positions used were from the NCBI dbSNP build 129 using the reference human genome (build 18). Following genotype phasing, the multi-allelic deletion status was expanded back into four separate tri-allelic SNPs (major, minor, and deleted).

In addition, 51 SNP-based haplotypes were generated for the highly discordant sib-pair cohort, the AMD case-control cohorts ascertained in Utah, Iowa, Australia ('Combined Cohort') and the UGRP cohort. Of the 62 SNPs employed for the 62 SNP-based haplotype determinations, the four SNPs lying within the CFHR3/CFHR1 deletion region in the 62 SNP-based set (rs11807997, rs389897, rs4230 and rs14628) were not used (instead, the deletion tagging SNP rs12144939 was used as a proxy) for generating the 51 SNP-based haplotypes. In addition, Taqman assays could not be designed to genotype 7 of the remaining 58 SNPs, so these were also not used for generating the 51 SNP-based haplotypes (see Figures 15 and 16 for SNPs employed to generate 51 SNP-based CFH-to-F13B haplotypes).

### 3.4 Methods: Validation of 51 SNP-based Haplotypes

To validate haplotypes and their frequencies, reference control phased haplotypes were obtained from the members of the Utah Genetic Reference Project (UGRP) families (Annu. Rev. Genomics Hum. Genet. 9:347-358, 2008). TaqMan SNP genotyping assays were used to obtain genotypes for the same 51 additional SNP markers previously genotyped in the GSH cohort (see above). PedCheck program was used to detect Mendelian inconsistencies and misinherited genotype calls were removed. Phased haplotypes were constructed based solely on the Mendelian inheritance model in each of the three-generation families. None of the haplotype inference programs were used. Haplotype frequencies were calculated on 136 unrelated UGRP individuals, who were mostly first generation grandparents, using Haploview program's phased haplotype input mode, Haps Format (Bioinformatics 21:263-265, 2005).

### 3.5 Methods: Disease Association Assessment Using Single Markers and 62 SNP-based and 51 SNP-based Haplotypes

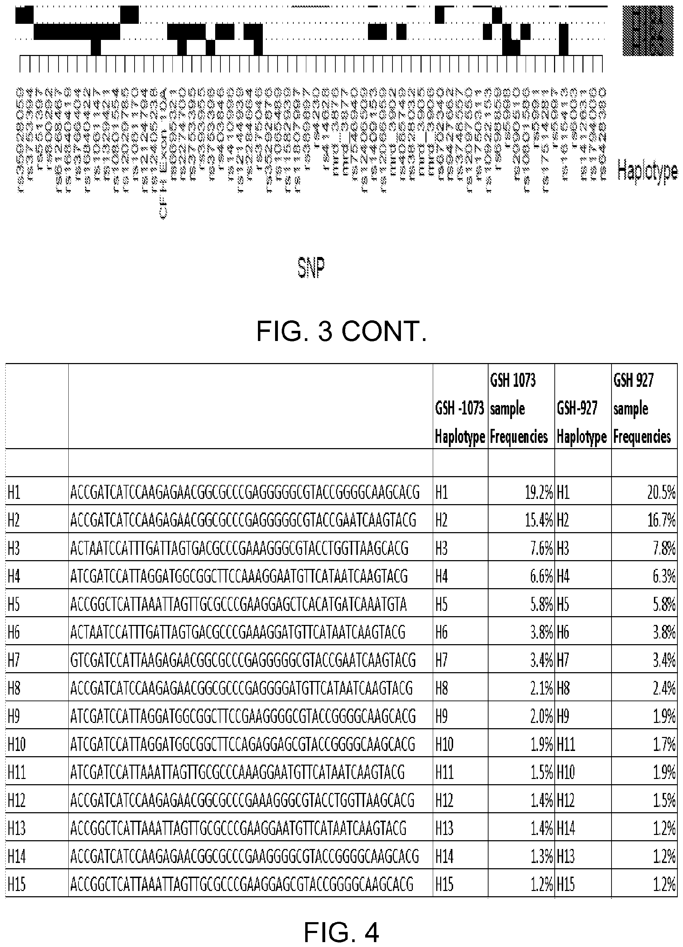

For determination of unrelated case/control disease (AMD) associations using 62 SNP-based haplotypes, only subjects 60 years or older in the 1,073 Cohort were used. Controls were defined as subjects with AMD grades 0, while cases consisted of all subjects with grades 1B-4C (as described in Example 1). Using these criteria, 927 samples from the GSH Cohort (the '927 GSH Cohort') were employed for analysis of disease association. Frequencies of extended haplotypes in the GSH 927 cohort used for disease association were nearly identical to those imputed for the entire 1,073 GSH Cohort (Figure 4). AMD disease association with the 62 SNP-based haplotypes was assessed using the Vassar 2x2 contingency table for Chi-2 based on allele frequencies.

51 SNPs were also genotyped in the Extremely Discordant Sib-pair Cohort (657 subjects from 284 families). Haploview (v4.2) was used to determine descriptive statistics using normal subjects (only one normal sibling per family for the Family Cohort), *i.e.* deviation from Hardy Weinberg Equilibrium (HWE) and the minor allele in the population. Tests for association of the minor allele with AMD were performed assuming an additive genetic model, and all AMD subtypes were compared to normals in the entire cohort, and then in males only and separately females only. For the Family Cohort, tests for association in the entire cohort were performed using Conditional Logistic Regression (CLR). In the analysis of males and females only, one subject of each gender was chosen per family (choosing the subject with the most severe disease), and tested for association with all AMD subtypes using logistic regression. Logistic regression was also used to assess 51 SNP-based haplotypes derived from the '927 GSH Cohort'. All analyses were performed in SAS (v9.1) and/or Haploview. Meta-Analysis was performed using Comprehensive Meta-Analysis 2.0.

### 3.6 Results: 62 SNP-based CFH-to-F13B Haplotypes Generated from the 1,073 Cohort

To generate haplotypes encompassing the CFH-to-F13B locus, 1,073 unrelated Caucasians of Northwestern European decent, were genotyped for 62 SNPs using a combination of assays, with missing genotypes imputed using PHASE2.1.1, as described above. Haplotypes for all samples were then determined using PHASE 2.1.1.

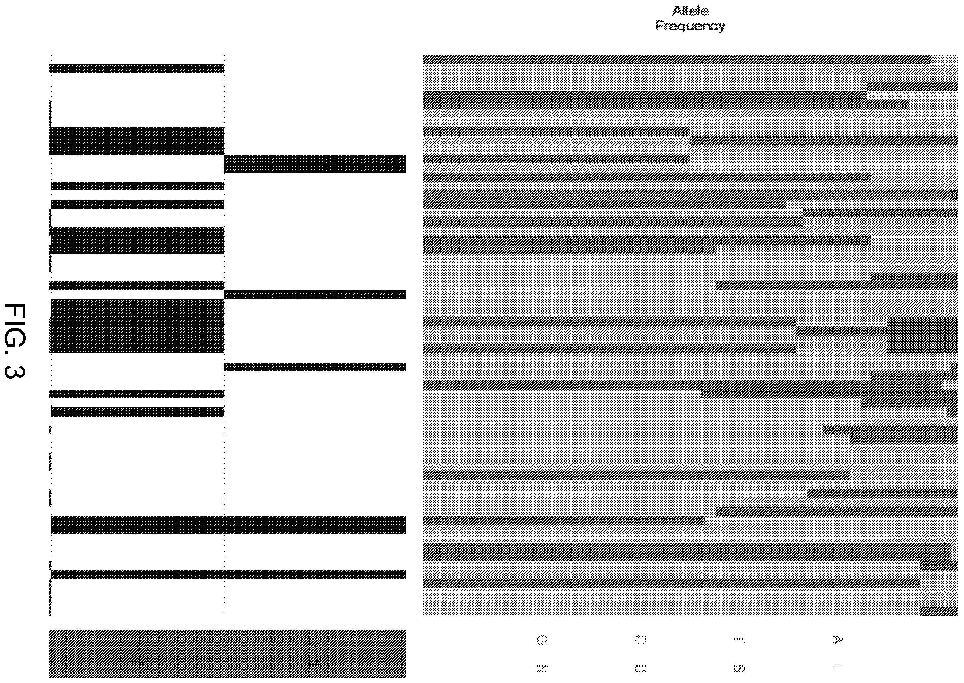

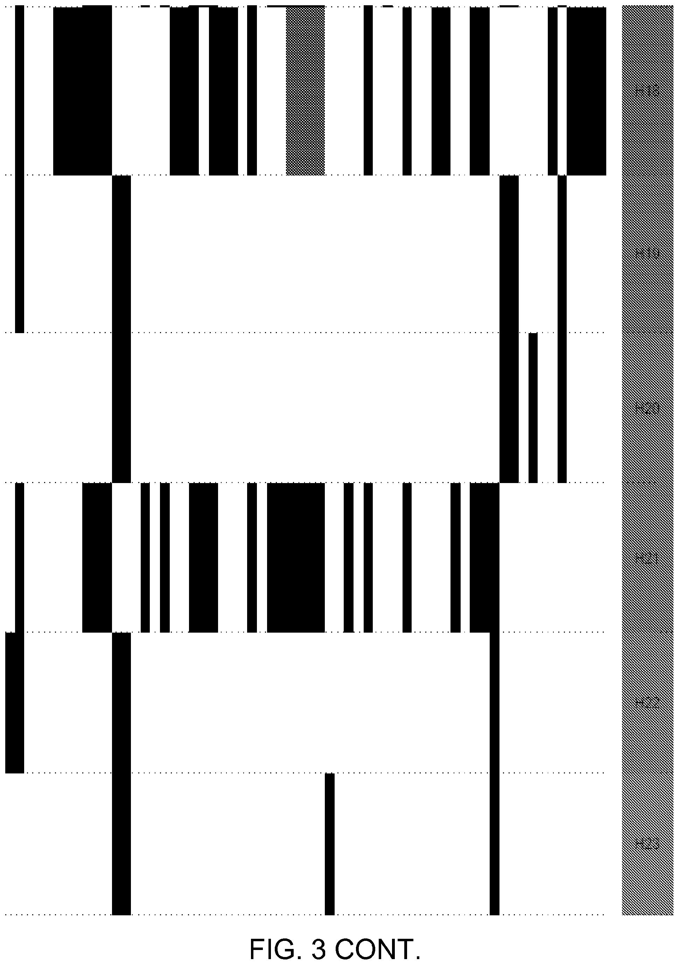

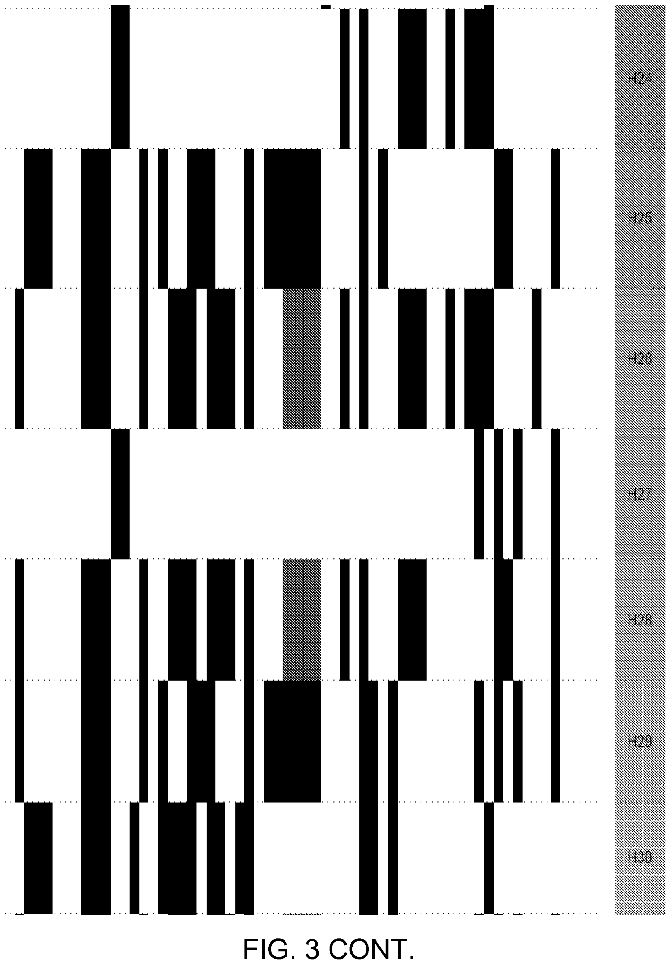

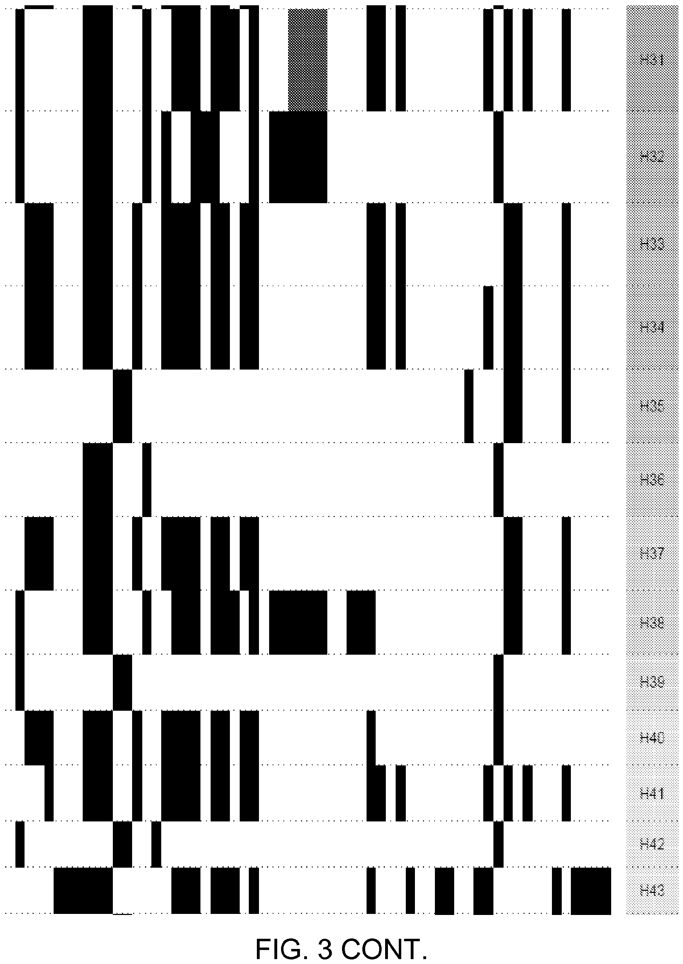

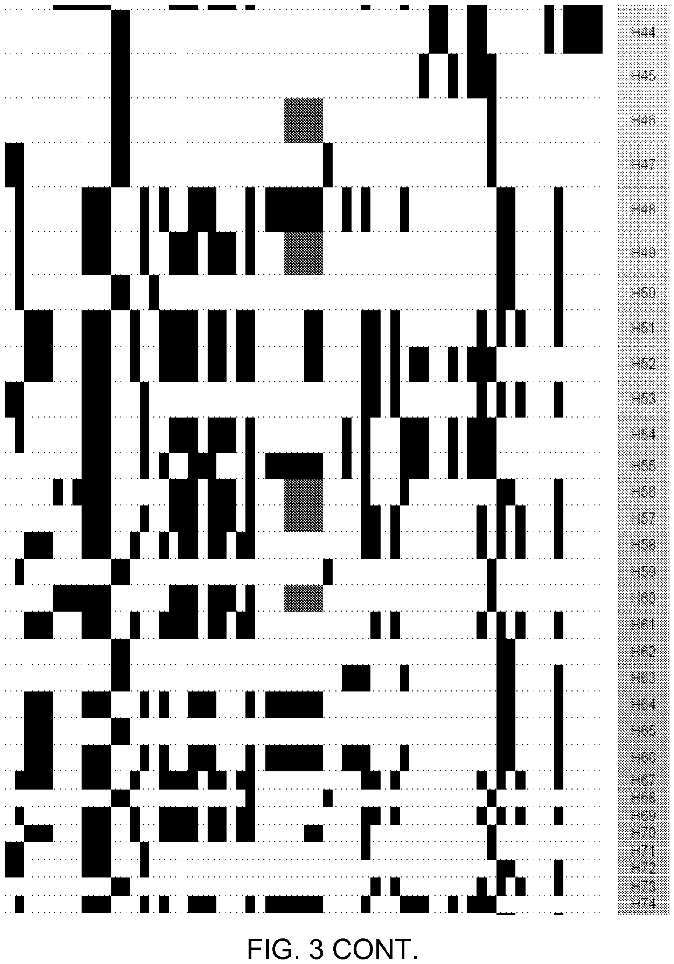

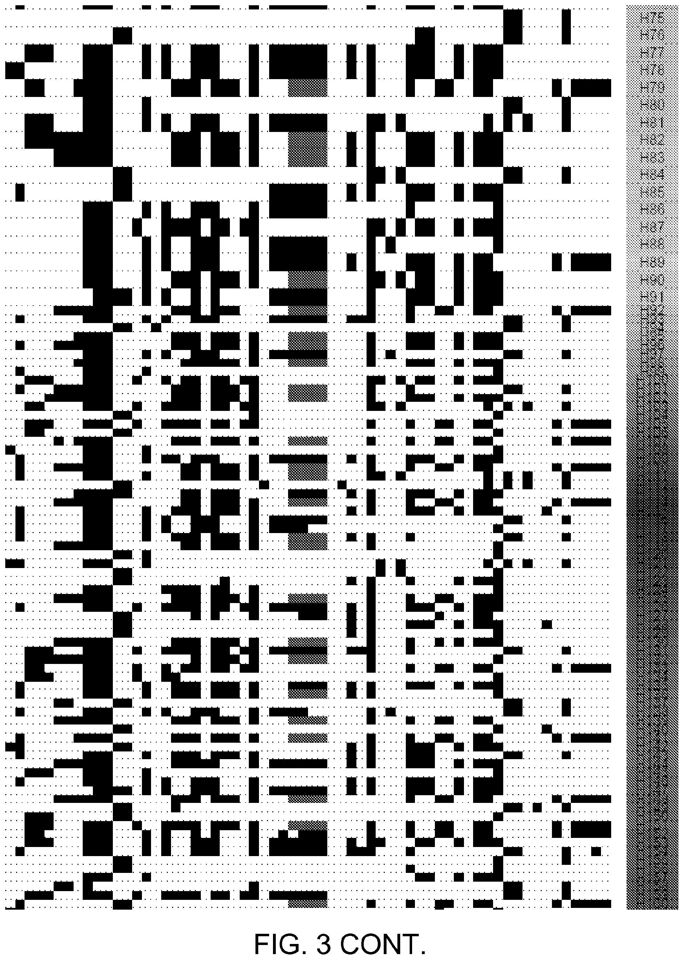

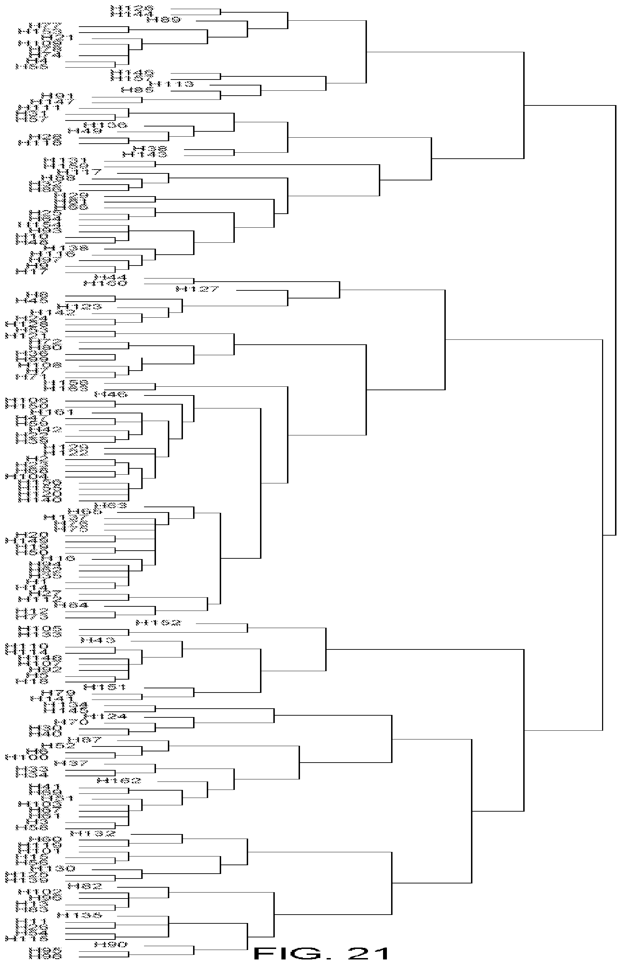

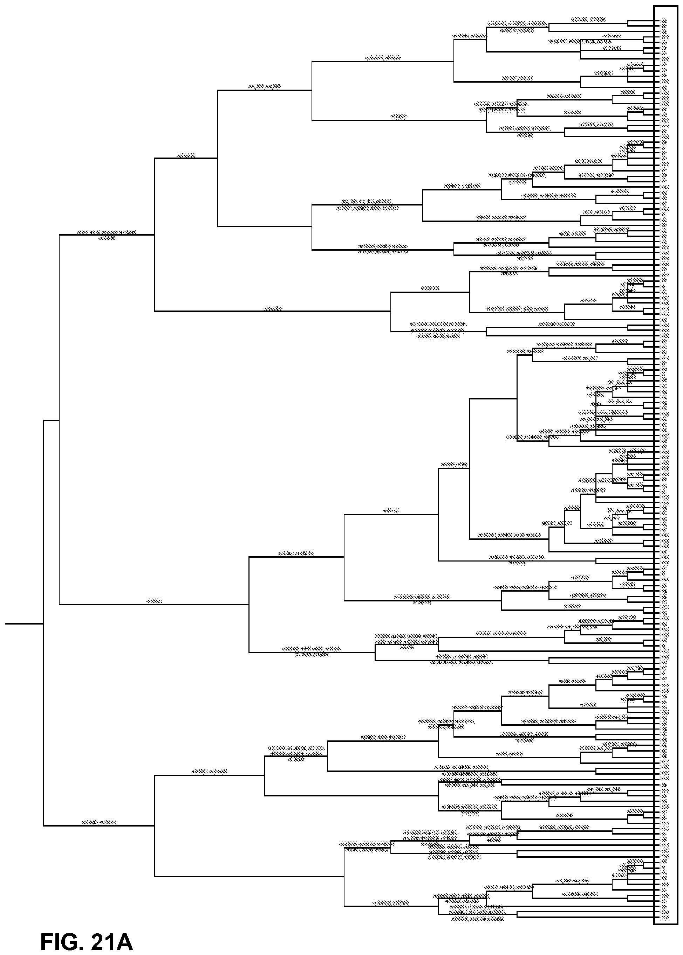

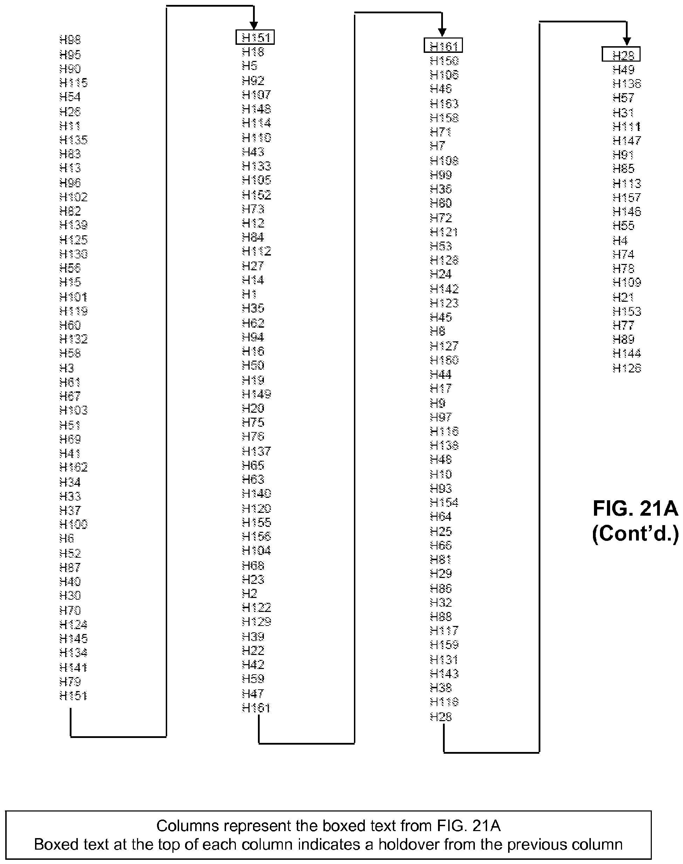

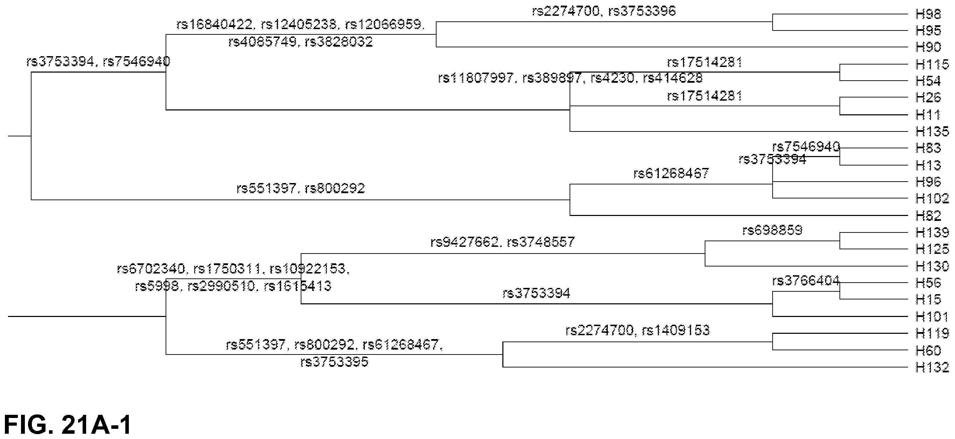

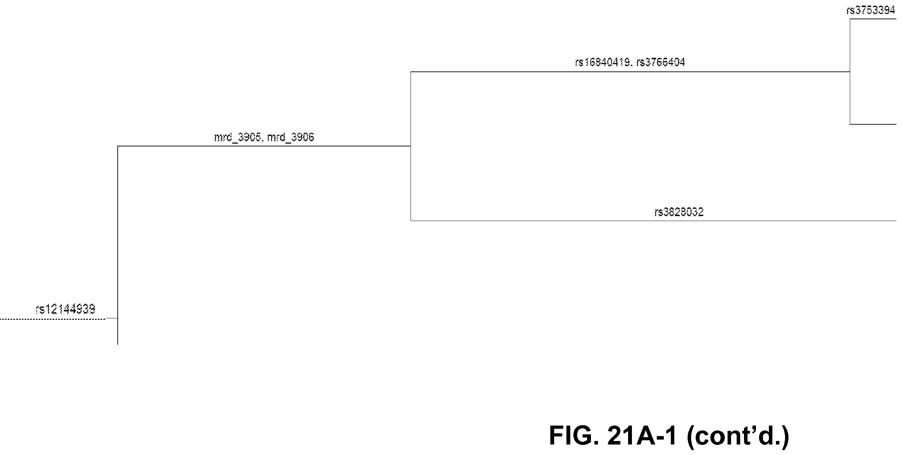

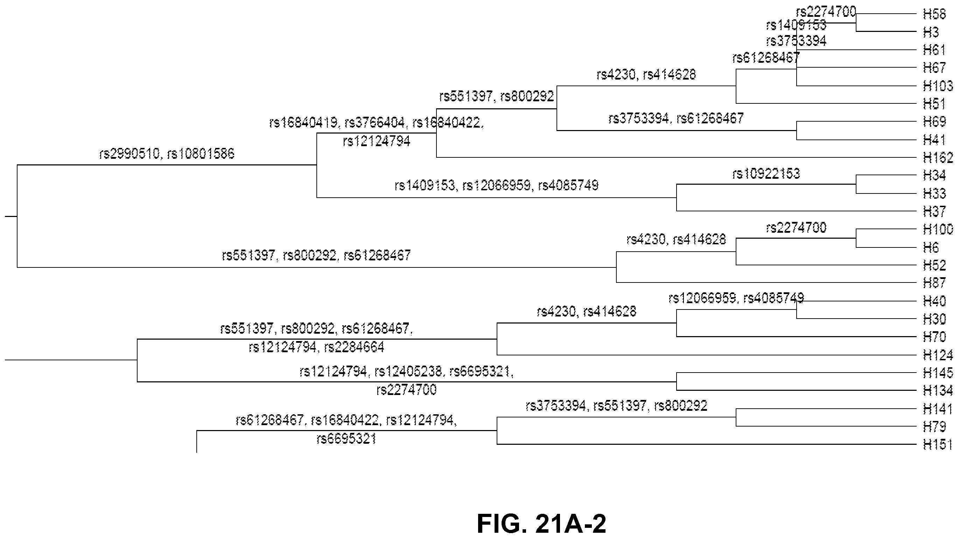

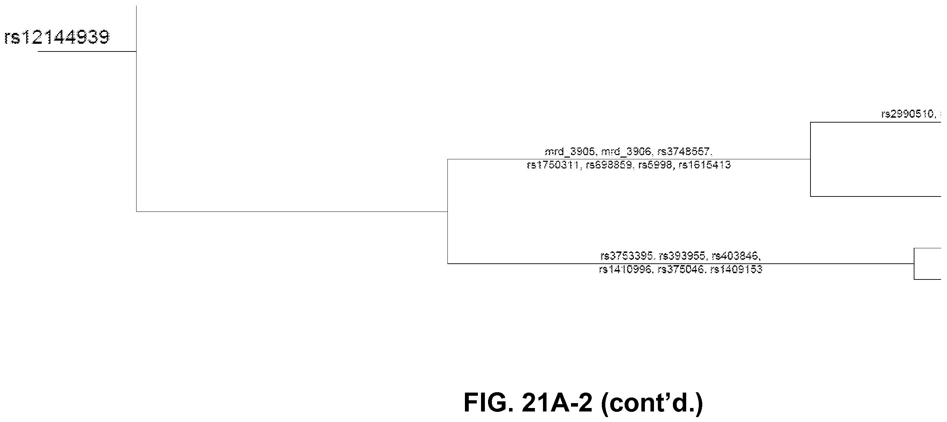

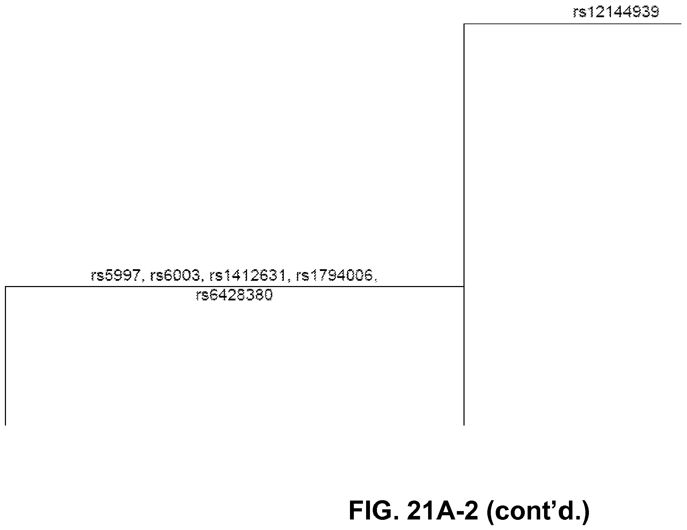

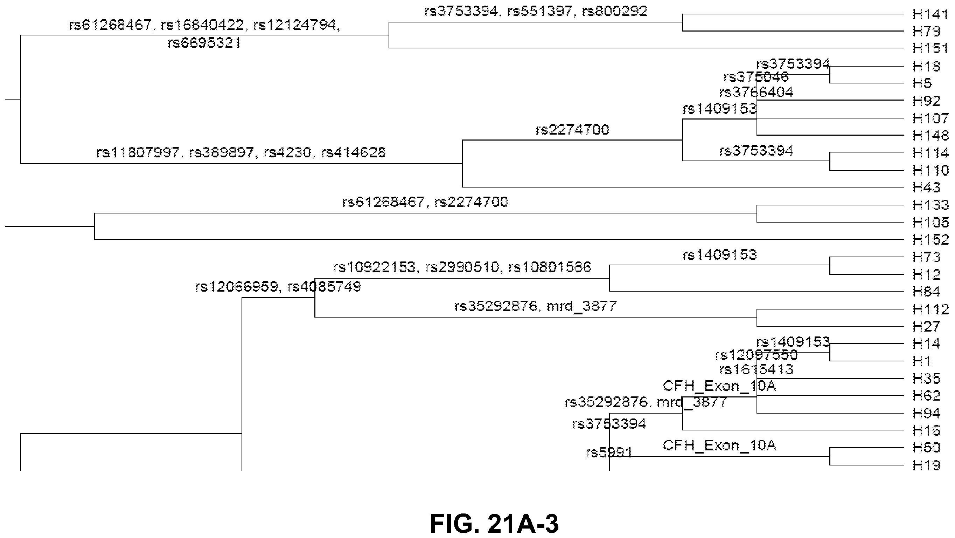

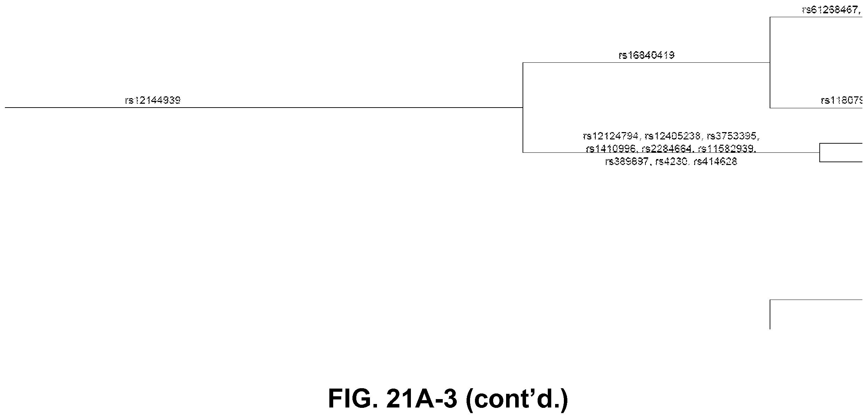

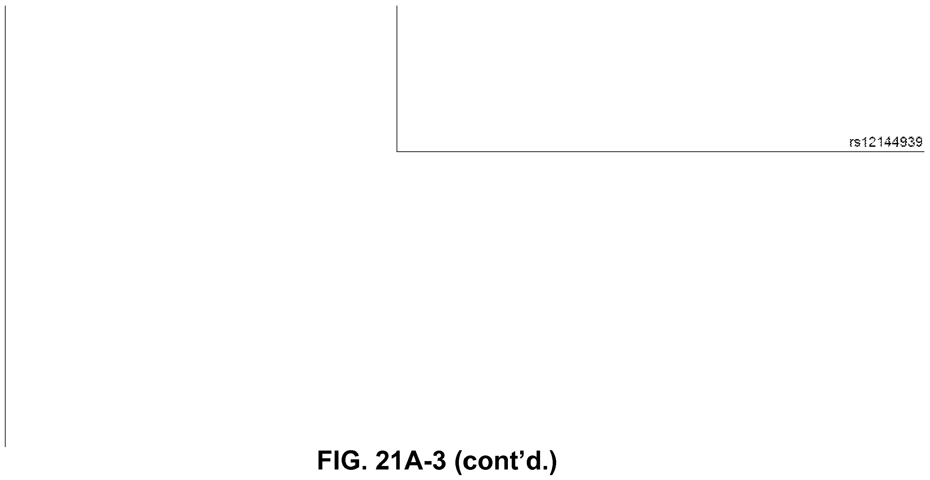

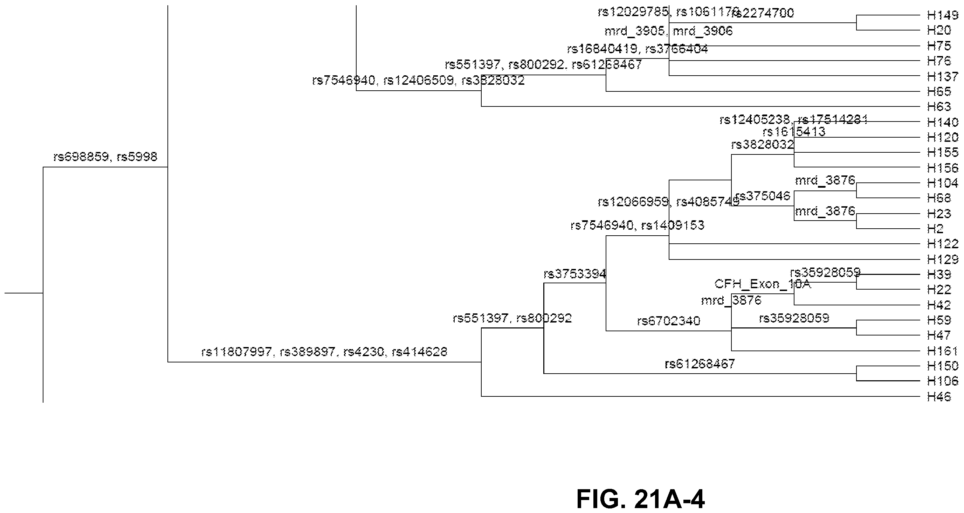

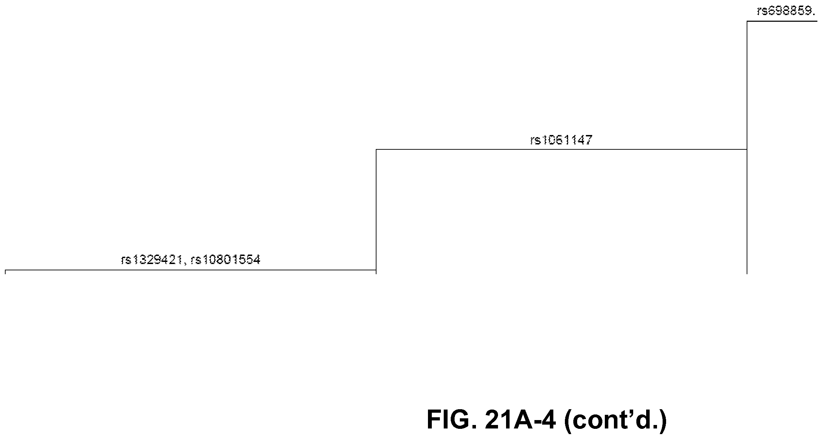

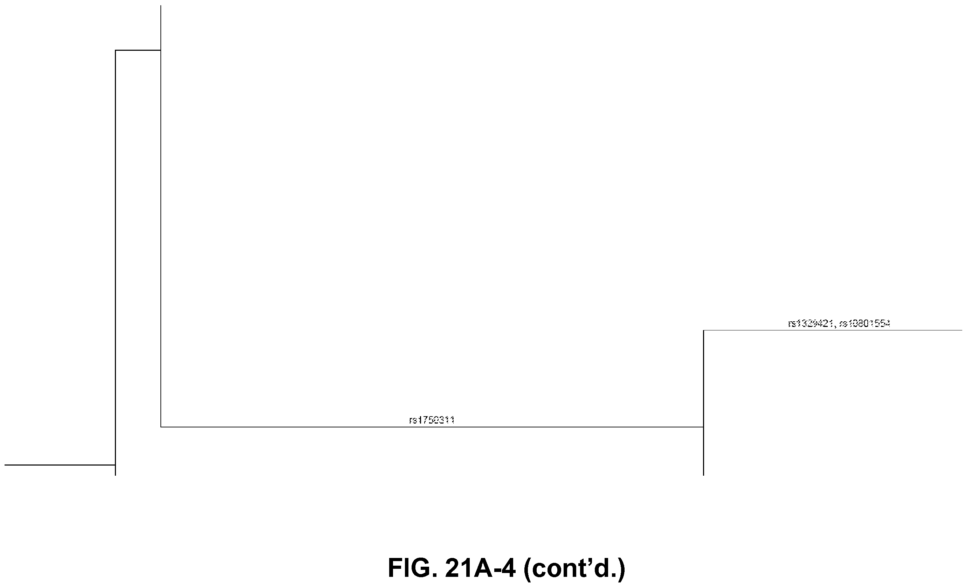

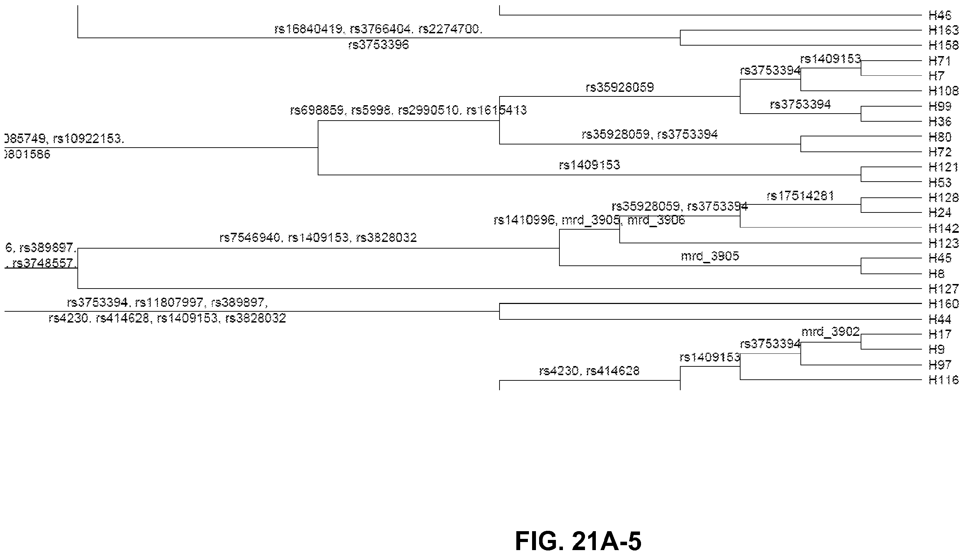

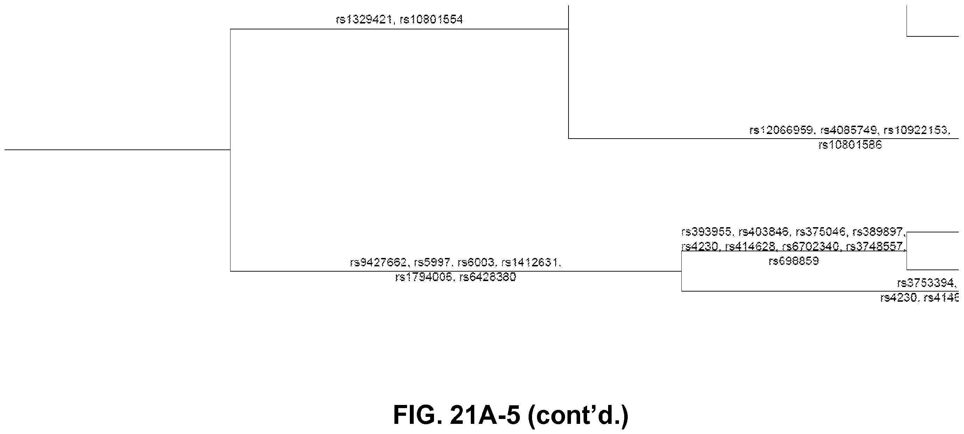

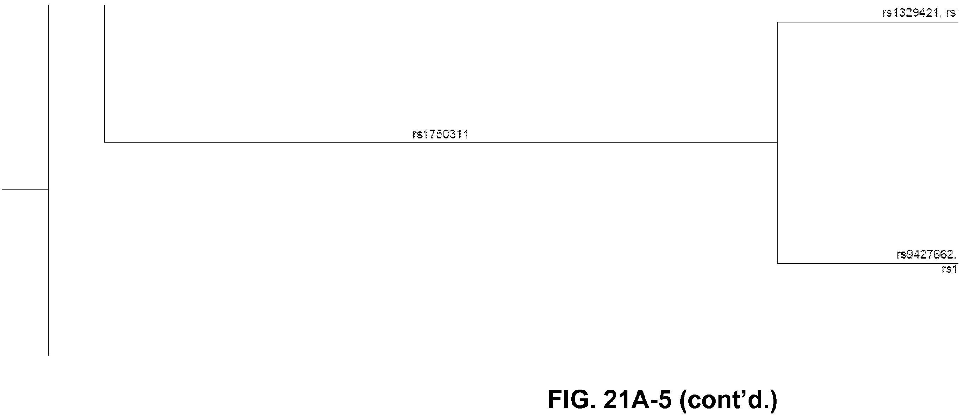

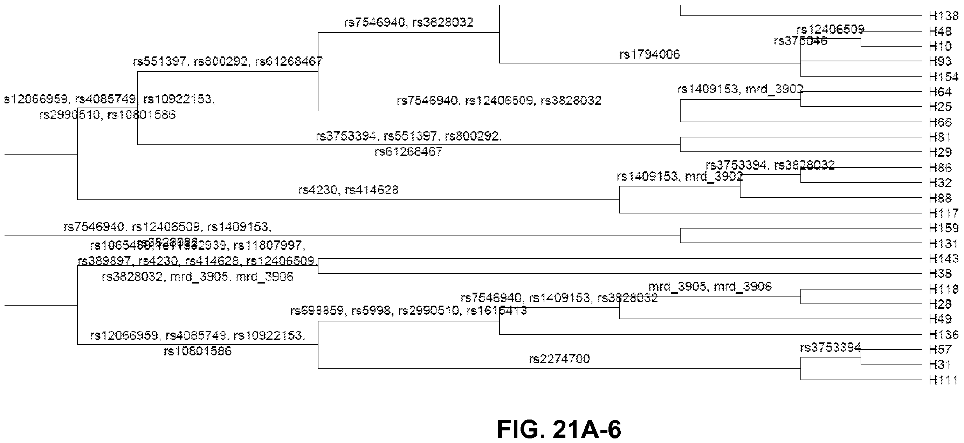

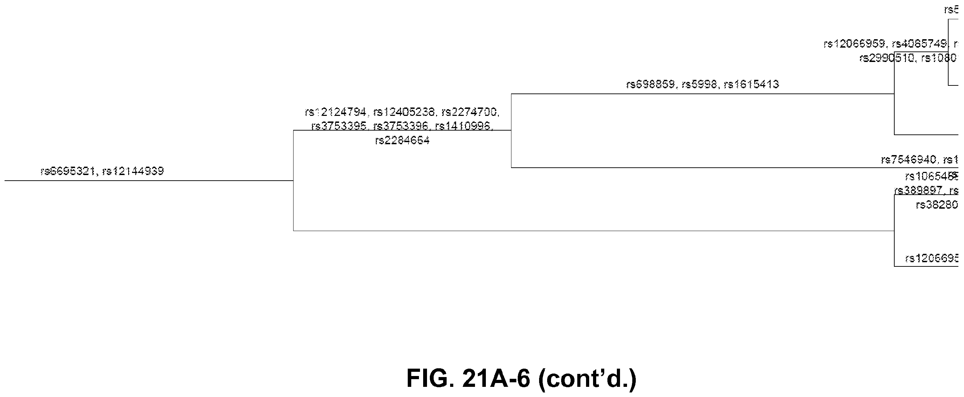

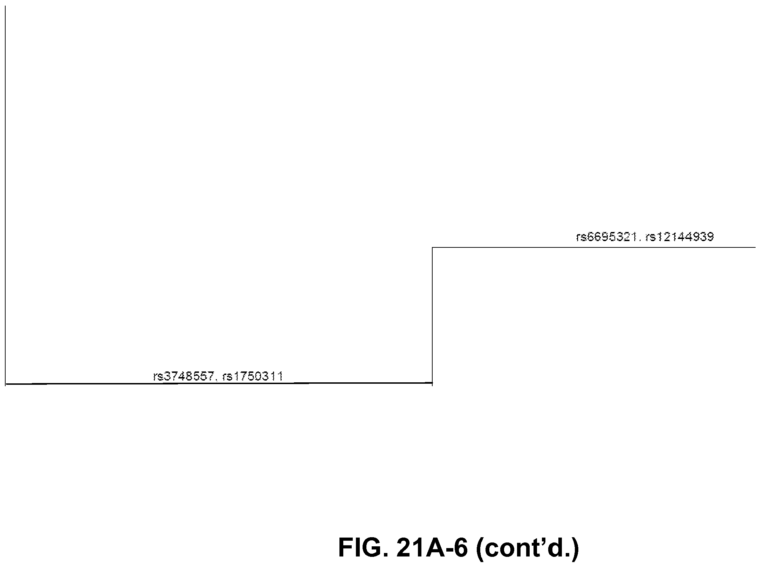

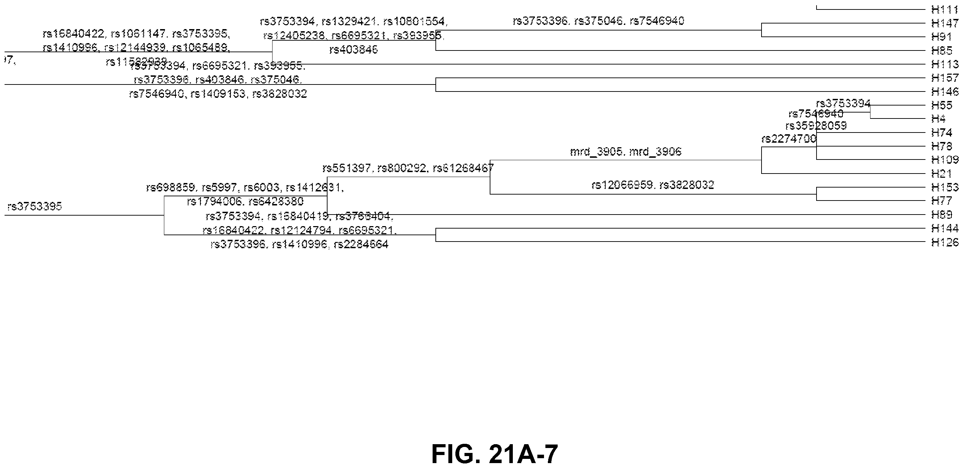

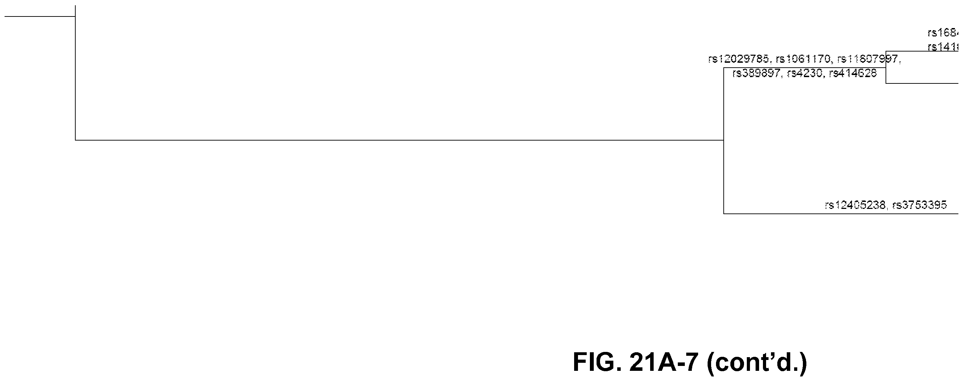

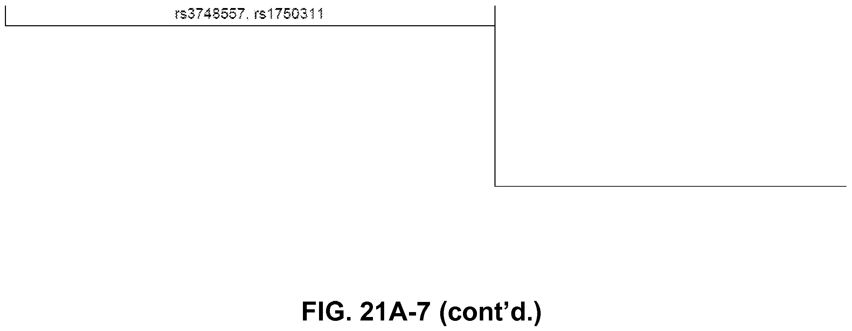

This analysis resulted in detection of 15 major haplotypes (H1_62_A through H15_62_A; Figure 1 and Figure 17) present at frequencies greater than 1% and 148 minor haplotypes, each present at less than 1% frequency (H16_62_A through H163_62_A; Figure 3 and Figure 17). In total, major haplotypes accounted for 1,599 total chromosomes, while the minor haplotypes contained 547 chromosomes, or 25.5% of the chromosomes in the cohort. The hierarchical relationships between the 163 62 SNP-based haplotypes is shown in Figure 21 (H1_62_A through H163_62_A). The 163 haplotypes were clustered based on the number of similar loci into this tree using the R statistical package hclust function using hierarchical clustering analysis. Similar haplotypes are the most related (closer together) and dissimilar haplotypes are further apart in this 'tree', shown in Figure 21. Importantly, three major clusters containing risk, protective and neutral haplotypes, respectively, were observed. The neutral haplotypes clustered at the left, or top, portion of the tree. Specifically, the neutral haplotype cluster spanned the region from H126_62_A to H163_62_A. The risk haplotypes clustered in the middle portion of the tree. Specifically, the risk haplotype cluster spanned the region from H46_62_A to H73_62_A. The protective haplotypes clustered at the right, or bottom, portion of the tree. Specifically, the protective haplotype cluster spanned the region from H152 62 A to H98_62_A.

The two most frequent of the 15 major haplotypes, H1_62_A (19.2% of total haplotypes) and H2_62_A (15.4% of total haplotypes) both contained the risk allele "C" for rs1061170 (402H). H1_62_A and H2_62_A were identical from the region proximal to CFH through CFHR2, based on the variants examined within this region; only in the F13B gene (beginning with rs698859 in the 3' UTR of F13B), do SNPs distinguish the H1_62_A and H2_62_A haplotypes (Figure 1). Other F13B SNPs that distinguish H1_62_A and H2_62_A include rs5998, rs2990510 and rs1615413. Three other major haplotypes contained the CFH 402H risk allele (H8, H12, H14). Two major haplotypes (H3_62_A and H6_62_A) contained the protective minor allele "A" for CFH rs800292. Additionally, 4 major haplotypes contained the CFHR3/CFHR1 deletion (H5_62_A, H11_62_A, H13_62_A, and H15_62_A; Figure 1). Four SNPs in near perfect LD (rs11807997, rs389897, rs4230, and rs414628) were located in the CFHR3/CFHR1 deletion region; they are shown as tri-allelic SNPs in Figure 1 (major allele shown in white, minor allele shown in line-filled boxes, and deleted CFHR3/CFHR1 shown in darkly shaded boxes).

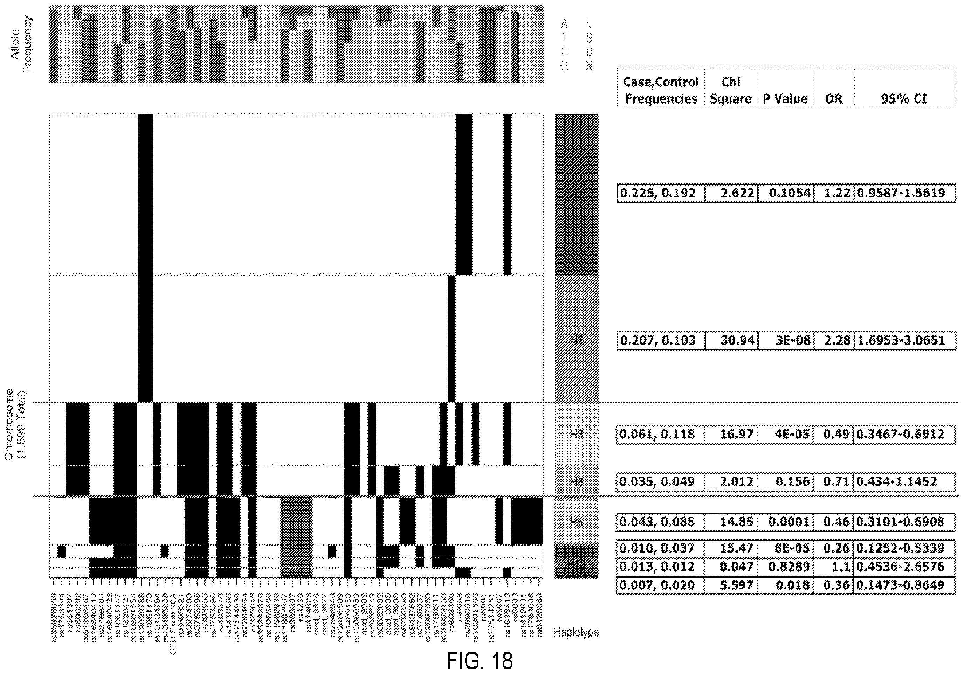

Extensive assessment of the 1,073 cohort (see Example 2), as well as families from the UGRP cohort, resulted in the identification of rs12144939 as a reliable surrogate "tag" for the CFHR3/CFHR1 deletion in >98% of Caucasian individuals, with the minor allele "T" accurately identifying an accompanying deletion. Surrogate proxies for this SNP that were in complete LD include rs6677604, rs16840522 and rs2019727. Major haplotypes containing the deletion of CFHR3/CFHR1 (H5 62 A, H11_62_A, H13_62_A and H15_62_A; Figure 1 and Figure 18), with the exception of H13_62_A, are highly protective against the development of AMD in this cohort (Figure 18).

Importantly, all major haplotypes containing either the protective allele at rs800292 (H5_62_A, H11_62_A, H13_62_A, and H15_62_A) or the CFHR3/CFHR1 deletion (H5_62_A, H11_62_A, H13_62_A, and H15_62_A; include rs11807997, rs389897, rs4230 and rs414628 and are tagged by rs12144939 or surrogate SNPs, as listed above) also contain the minor alleles of the rs2274700, rs3753395, rs393955, rs403846, and rs1410996 variants (Figure 1 and Figure 18). The variant rs1409153 is also in near complete LD with all major haplotypes associated with protection. Any of these SNPs, and any other SNP in near LD with these SNPs, singly or in combination, can serve as surrogates for the major protective haplotypes H3_62_A, H6_62_A, H5_62_A, H11_62_A, H13_62_A, and H15_62_A, as well as some of the minor protective haplotypes. Other SNPs within this region of DNA that are in near complete LD (and can serve as surrogates) were determined by generating an LD plot using all of the SNPs in HapMap lying within the CFH-to-CFHR4 region. This was done using the CEU cohort (CEU, Utah residents with Northern and Western European Ancestry from the CEPH Collection, is one of the 11 populations in HapMap Phase 3) genotypes. In this population, 7 SNPs were identified to be in complete or near complete LD with rs1410996, the most significantly associated variant in this "block." They were (in order of 5'-to-3' direction) rs10737680, rs7535263 (LD = 0.96), rs3753395, rs10922106, rs395998, rs1329428 and rs7540032. There are multiple other SNPs in this 22kb region that are in various levels of LD with rs 1410996.

### 3.7 Results: 51 SNP-based CFH-to-F13B Haplotypes in the 1,073 (927 Subset) Cohort

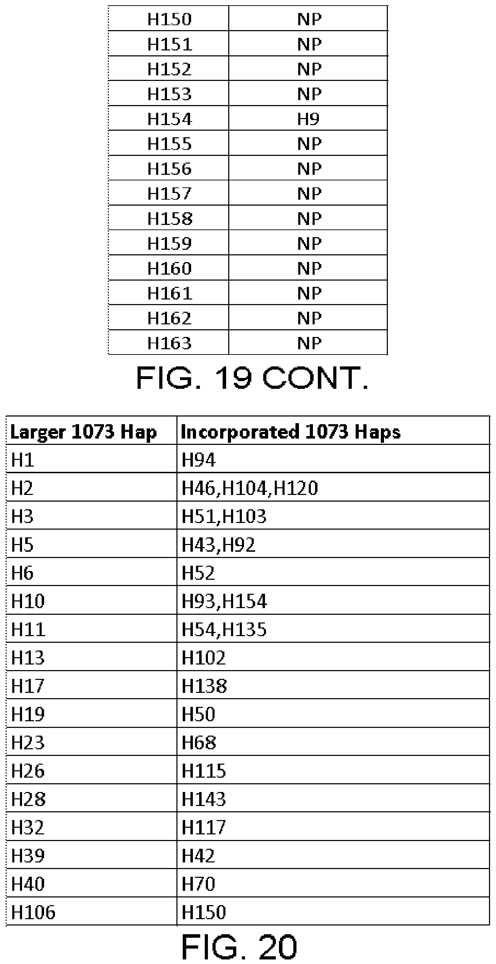

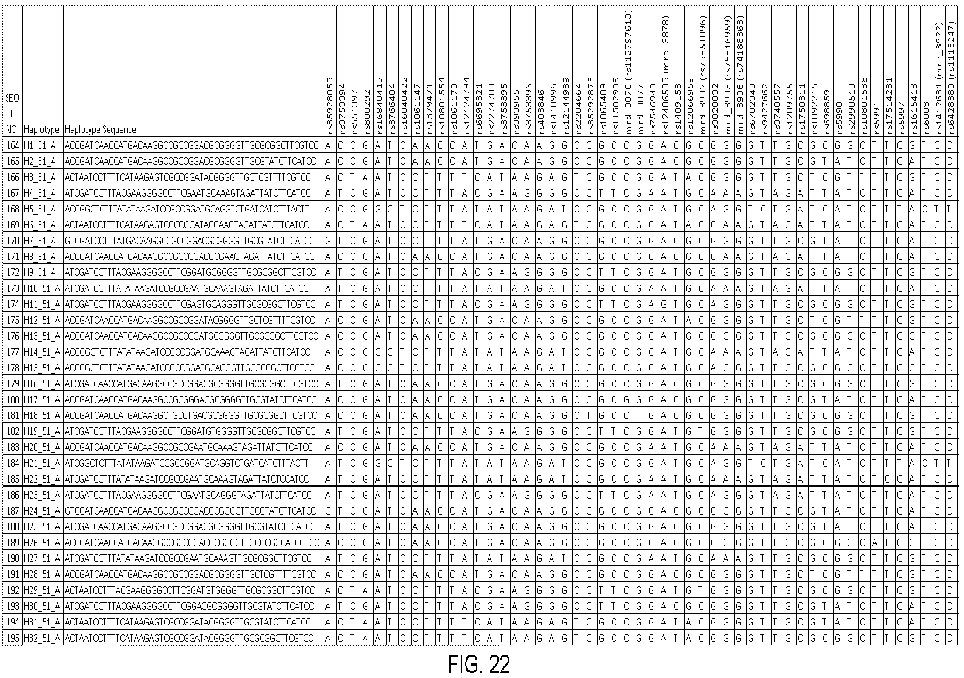

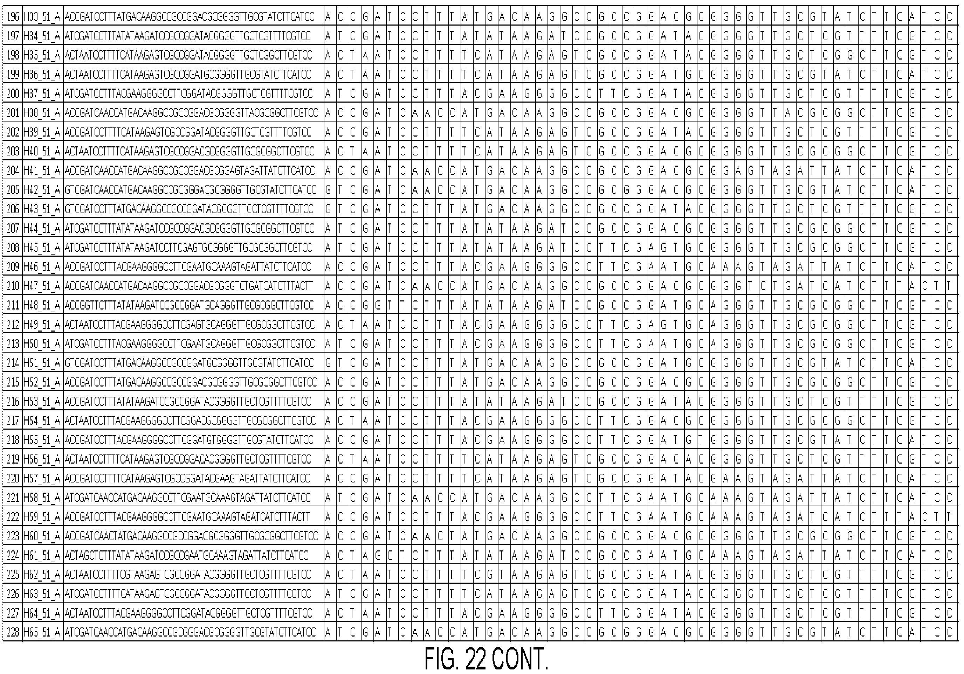

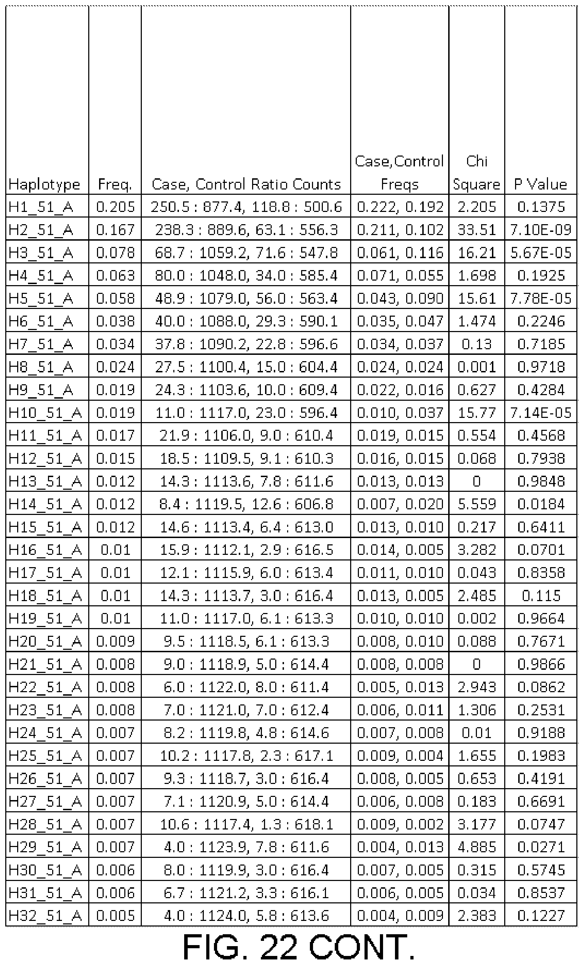

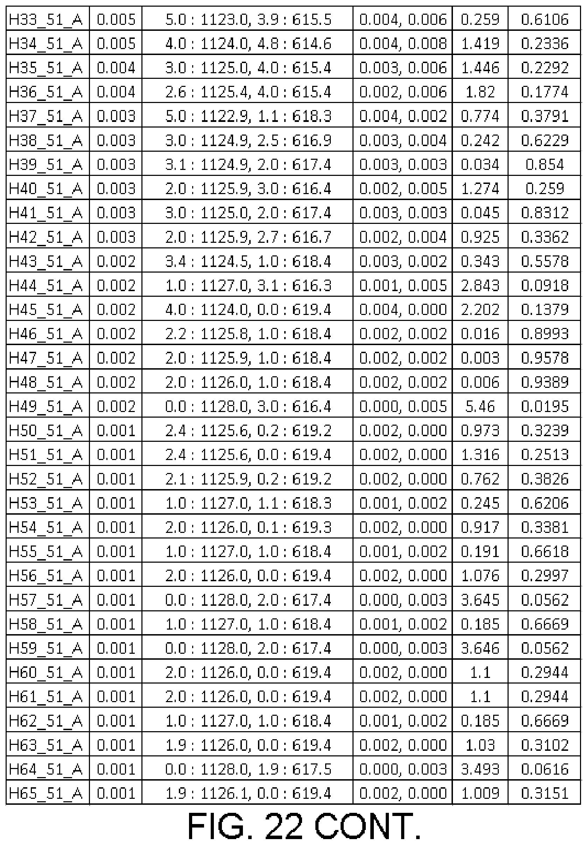

51 SNP-based haplotypes were also generated using the 1,073 (927 Subset) Cohort (Figure 22). Converting from 62 to 51 SNPs caused some of the 62 SNP-based haplotypes to collapse into the 51 SNP-based haploytpes (Figure 20). 17 major haplotypes occurred at frequencies greater than 1% in this cohort.

### 3.8 Results: Validation of 62 SNP-based and 51 SNP-based Haplotypes Using the UGRP Cohort

Because haplotypes were imputed, independent verification was sought that the haplotype structures and frequencies determined were real and representative of a Caucasian population. 62 SNP-based haplotypes were validated as described in see Example 2.

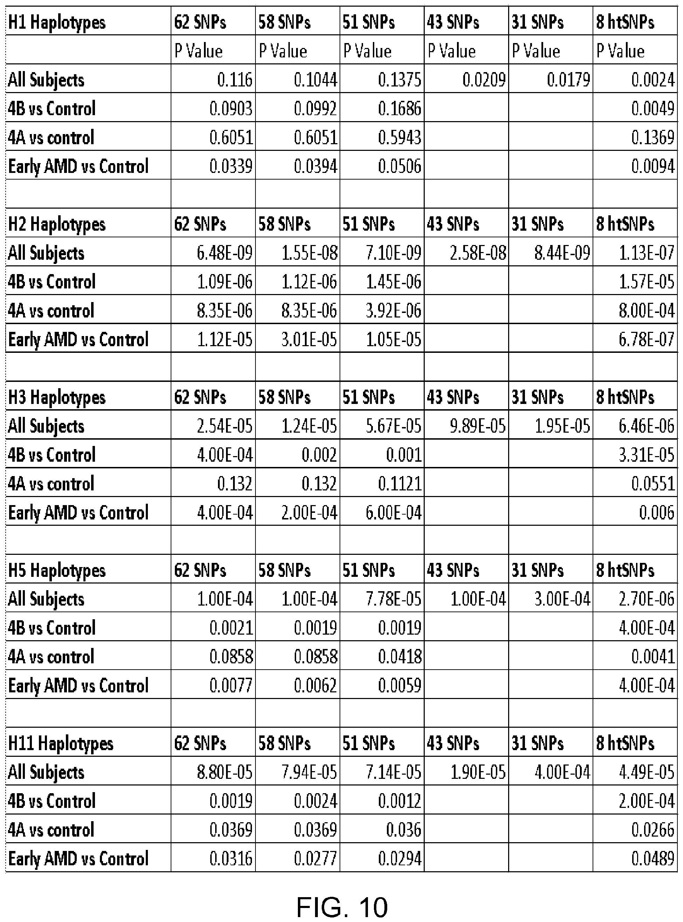

Using 136 unrelated individuals from the CEPH/UGRP reference cohort, non-imputed haplotypes were generated using 51 SNPs. Given the small size of the UGRP unrelated cohort, some differences in haplotype frequency between the GSH Cohort and the UGRP cohort was expected. However, when compared to the haplotypes shown in Figure 1, these differences were minor and haplotype frequencies were nearly similar (Figure 33), indicating that these haplotype frequencies were representative of aged Caucasian populations in general (Figure 10).

### 3.9 Results: Single SNP AMD Disease Associations in the 1,073 Cohort (927 Subset) Using 62 SNPs

Of the 1,073 samples in our original cohort, 927 were 60 years or older at diagnosis and had AMD (Grade 1B-4C) or did not have AMD and served as controls (Grade 0). A case/control analysis of risk was performed using logistic regression for single marker associations **(****Figure 5****).** Results showed patterns of association. rs1061170 was significantly associated with risk (p=2.5e-11, OR=1.953, 95% CI 1.604-2.377). The minor alleles of rs1410996, rs2274700, and rs3753395 [and other surrogates tagging the block of DNA containing variants that associate with the majority of protective haplotypes (chr1:196679455-196701284; Hg19)], were even more strongly associated than rs 1061170, but with protection from developing, rather than risk of developing AMD (p=1.12e-14, OR=0.436, 95% CI 0.353-0.538). Variants in the distal region of this locus including CFHR5 and F13B were also significantly associated with AMD, albeit at much lower levels (Figure 5).

### 3.10 Results: 62 SNP-based Haplotypes Associated with AMD Risk: 1,073 Cohort (927 Subset)

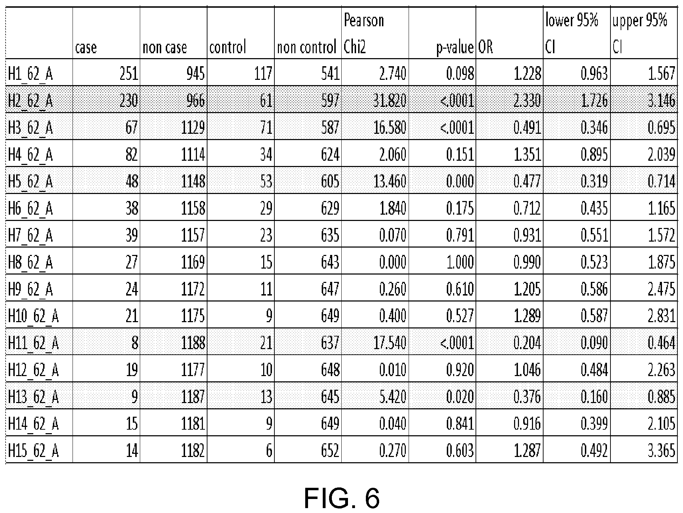

Disease associations were analyzed for the entire extended haplotypes using Haploview 4.2 (Figure 6) (Barrett JC, Bioinformatics, 2005 Jan 15).

Of the 15 major haplotypes present at a frequency greater than 1%, H2_62_A exhibited significant risk for AMD (Figure 6 and Figure 18; p<0.0001; note that values vary slightly between Figures 6 & 18 due to clinical reclassification of a few patients between the time these two analyses were conducted). H1_62_A, which also contains the 402H risk allele trended slightly toward risk for AMD (p=0.098) in this cohort, but was less significant than H1_62_A. Whereas four distal SNPs from the 62 SNP panel distinguish H1_62_A from H2_62_A, only one of these SNPs significantly associated with AMD in single marker assessment (rs698859, p=0.0111) (Figure 5). This indicated that additional features/elements in the haplotypes tagged by these SNPs are responsible either for risk in H2_62_A or lack of risk in H1_62_A. Given multiple haplotypes contain the risk allele at rs1061170 (H1_62_A, H2_62_A, H8_62_A, H12_62_A, and H14_62_A) and yet only one of these haplotypes associated significantly with risk in this cohort, there is likely a risk factor present in the H2_62_A haplotype that is lacking in all other non-H2_62_A 402H haploytpes. In fact, previous studies using less extended haplotypes that failed to include the F13B SNPs have attributed a significant risk of developing AMD to all patients containing the Y402H risk allele (42.3% of major haplotypes or 49% of all haplotypes in the GSH cohort), when in fact risk only associated with H2_62_A, or 16.5% of the cohort described herein. Thus, the haplotypes described herein represent a significant increase in the sensitivity of defining risk at the CFH locus.

Furthermore, the vast majority of AMD risk association, although not all, occurred in major haplotypes tagged by CFH 402H (H2_62_A, H8_62_A, H12_62_A, H14_62_A and H16_62_A; Figure 6 and Figure 18) in this cohort. CFH 402H, however, was not always associated with risk for AMD; H2_62_A was the most strongly associated haplotype with AMD risk, whereas major haplotypes H1_62_A, H8_62_A, H12_62_A, and H14_62_A were not significantly associated in this cohort. The minor haplotype H16_62_A trended toward risk in this cohort.

### 3.11 Results: 62 SNP-based Haplotypes Associated with AMD Protection: 1,073 Cohort (927 Subset)

In addition to risk, several major CFH-to-F13B haplotypes showed protection from AMD relative to the cohort as a whole. H3_62_A (includes the rs800292 A allele) was significantly protective (Figure 6). Just as H1_62_A and H2_62_A differed in their risk association, so to did H3_62_A (protection) with the other major haplotype containing the rs800292 A Allele, H6_62_A (neutral). Of the haplotypes containing CFHR3/CFHR1 deletions, H5_62_A and H11_62_A showed significant protection, while H13_62_A showed marginal protection and H15_62_A was neutral. H3_62_A and H6_62_A are identical until mrd_3905/rs75816959, which is in CFHR5. Similarly the CFHR3/CFHR1 deletion containing haplotypes H5_62_A, H13_62_A, and H15_62_A are identical until mrd_3905. Thus, placing these haplotypes in the context of extended haplotypes that included CFHR5 and F13B resulted in significantly refined association with AMD (Figures 6 & 18).

### 3.12 Results: 62 SNP-based Haplotypes Associated with Neutrality: GSH Cohort

Several major CFH-to-F13B haplotypes, including H4_62_A, H7_62_A, H10_62_A, H12_62_A, and H14_62_A, were neutral with respect to the development of AMD relative to the cohort as a whole. The minor allele of rs3753396 tagged many of these neutral haplotypes (Figure 1, Figure 3, Figure 6, and Figure 18). Other SNPs in LD with rs3753396 at a r2 value of 90 or greater and that can be employed as surrogates for rs3753396 include (listed proximal to distal): rs10489456, rs70620, rs742855, rs11799380, rs1065489, rs11582939, rs385390, rs421820, rs426736, rs370953 and rs371075.

### 3.13 Results: Gender Associations in 62 SNP-based Haplotypes: 1,073 Cohort (927 Subset)

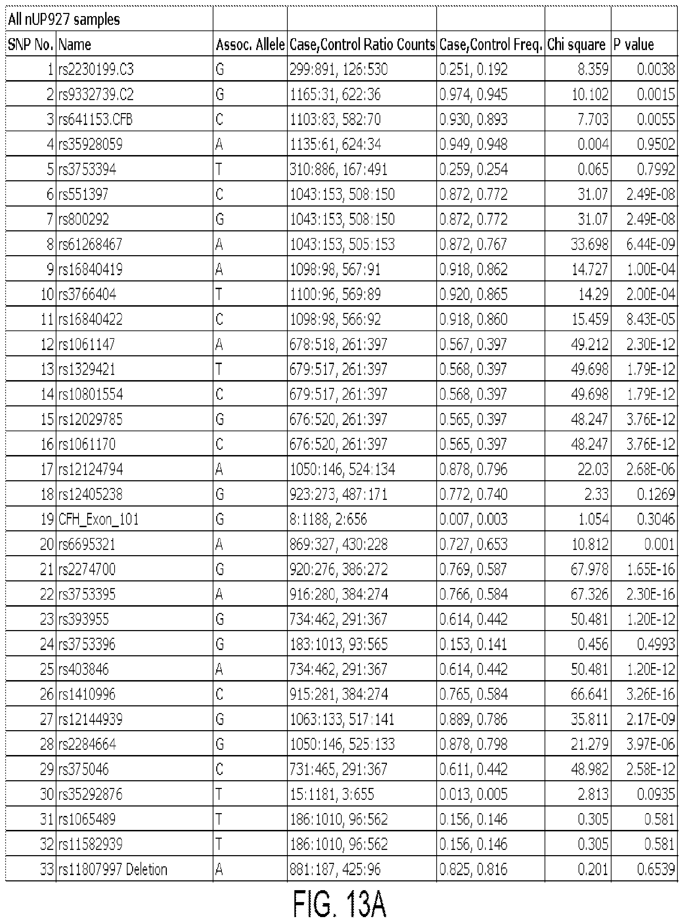

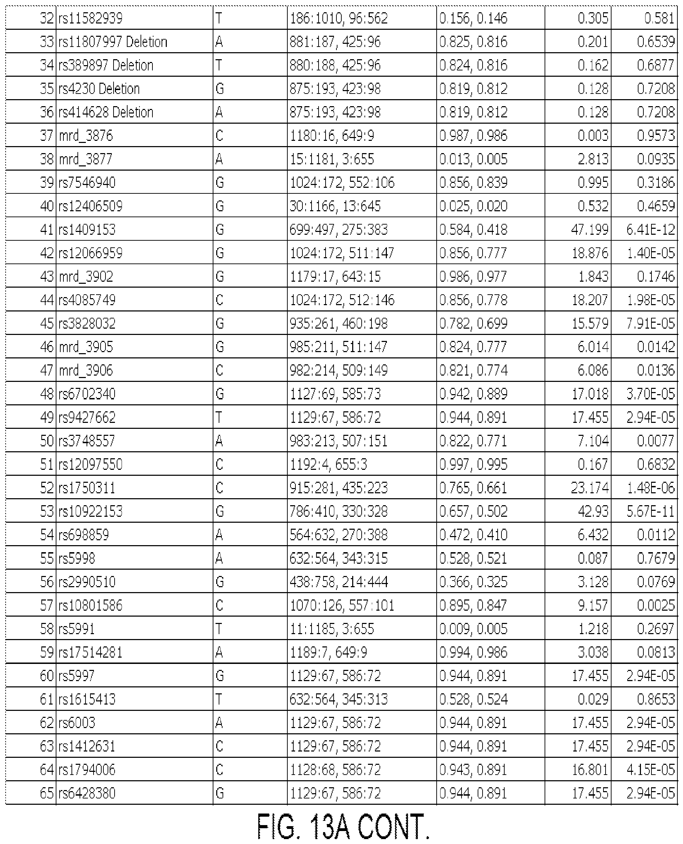

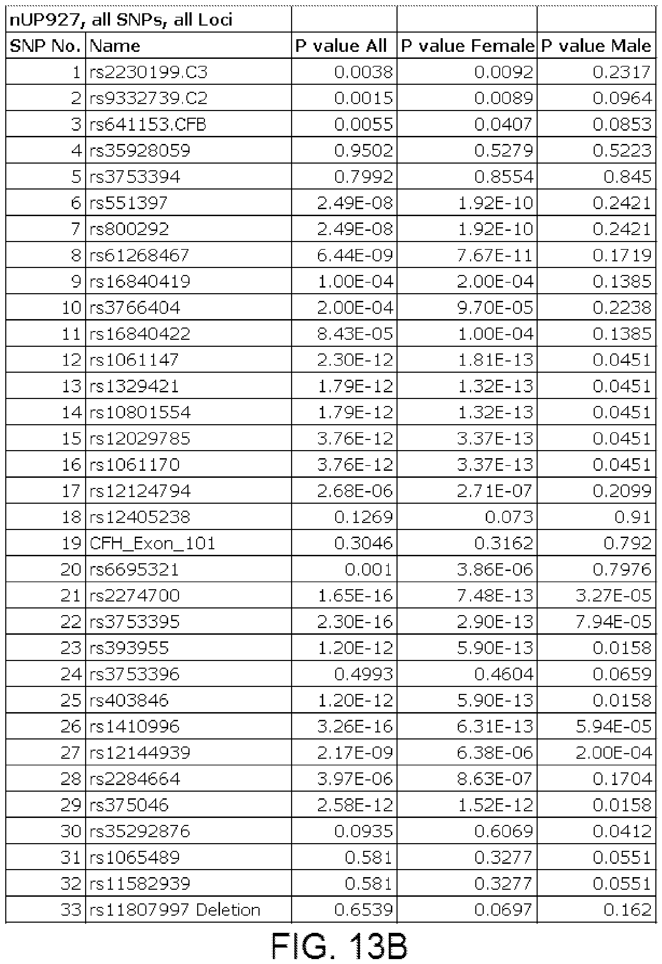

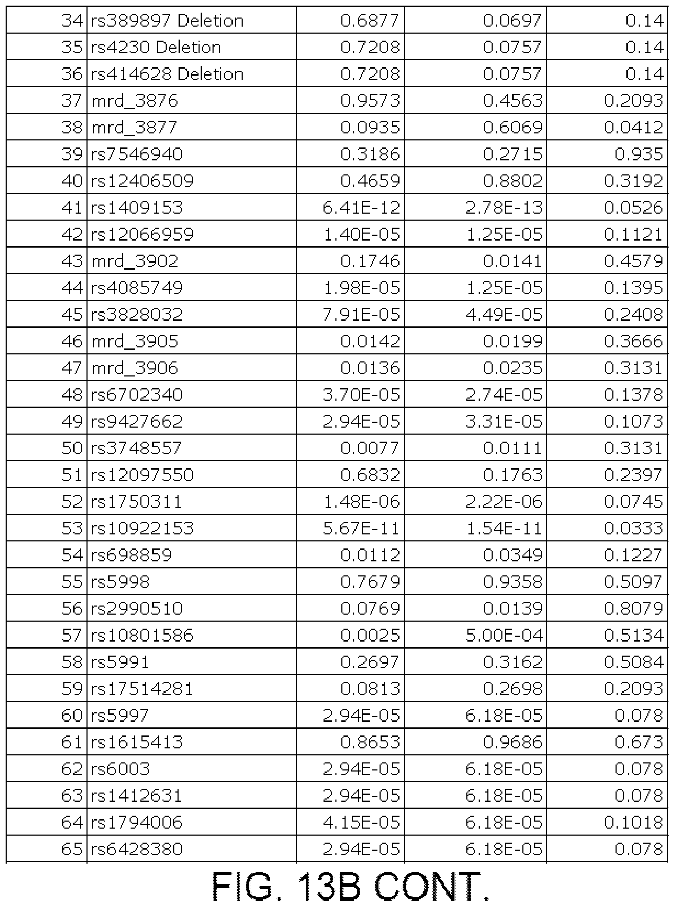

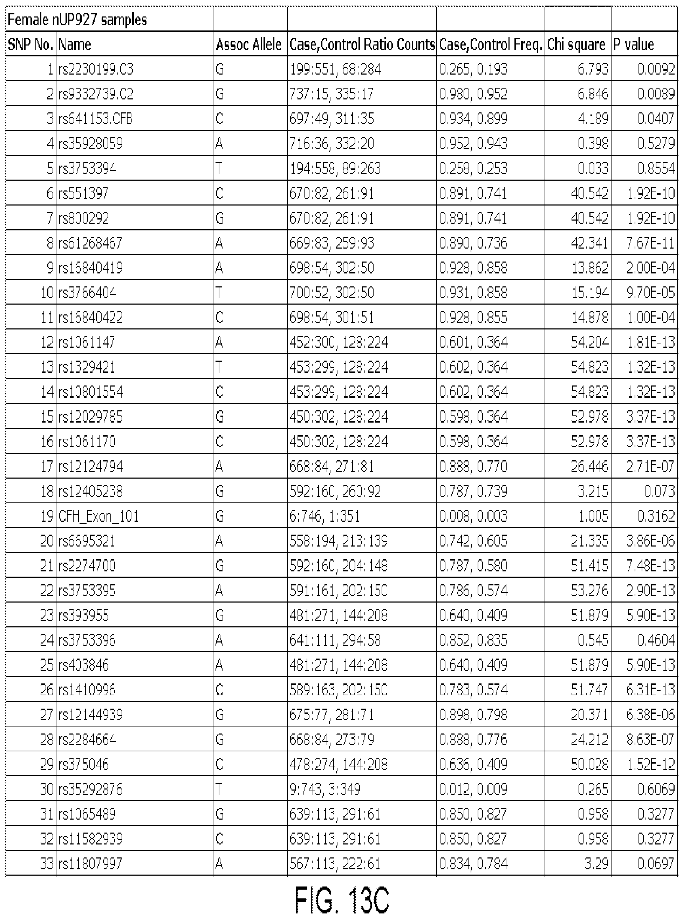

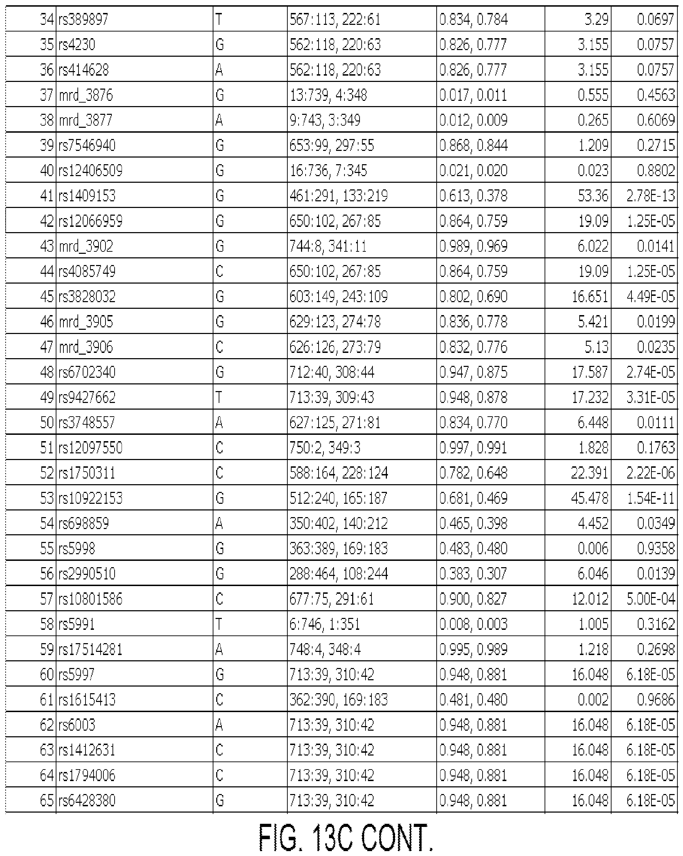

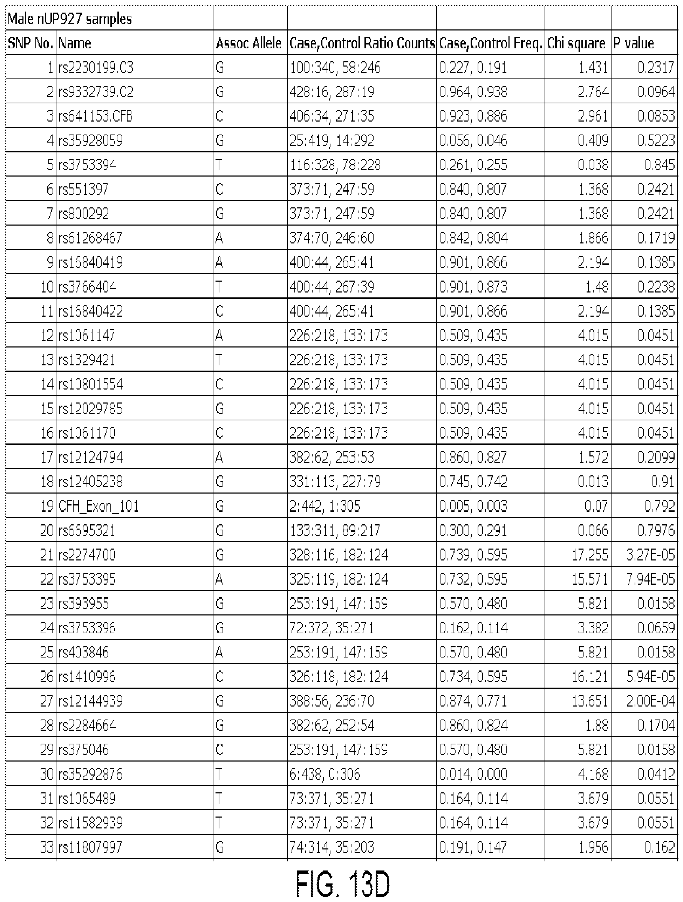

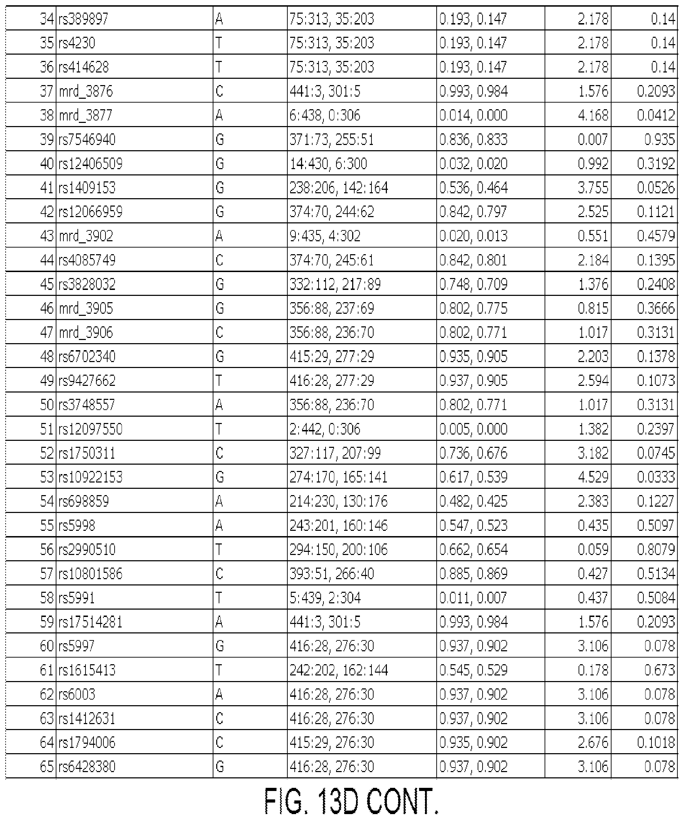

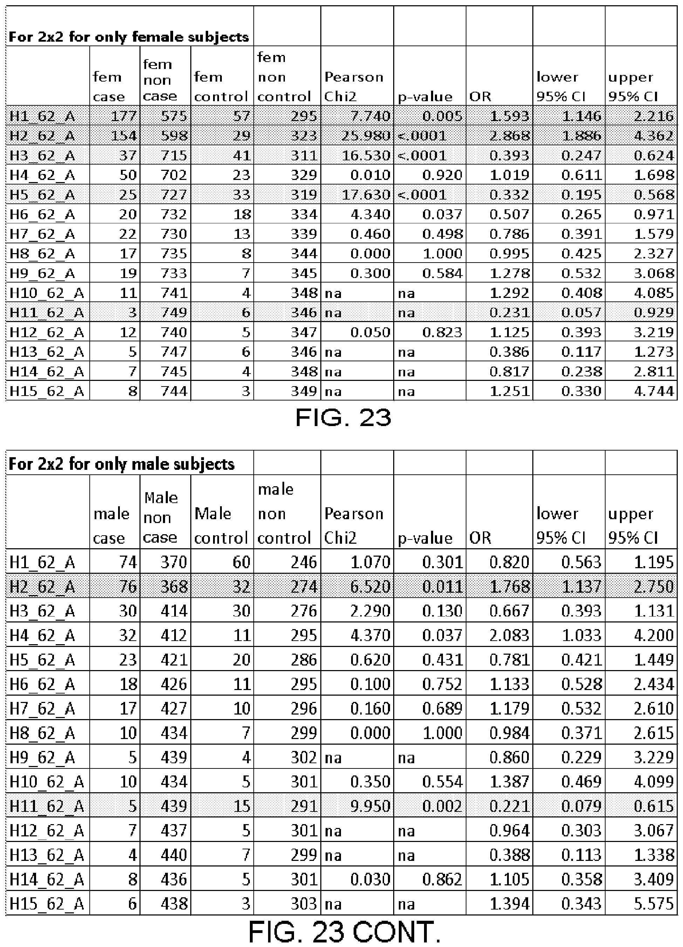

62 Single marker (SNPs) and haplotypes were examined for potential association with gender in the GSH case-control cohort. Associations were assessed using the entire cohort, the male sub-cohort only, and the female sub-cohort only (Figure 13 and Figure 23). The female cohort showed very similar levels of significance in the association of the 62 individual SNPs to AMD when compared to the '927 GSH Cohort (Figure 13). Male associations differed from the female associations. For example, association of rs800292, rs1061170 and rs1329421 was not observed in the male sub-cohort, a difference of approximately 11-orders of magnitude relative to females. Males did show lower levels of significant association with rs1410996, rs3753395 and rs393955, although also at levels up to 11-orders of magnitude less than was seen in the female cohort (Figure 13). The rs800292 A allele, previously associated with protection or reduced risk for AMD, also showed decreased association in the male sub-cohort. This data indicated that by controlling for sex, previous studies may have missed this stratification because of masking.

While the loss of association with AMD in males was significant throughout the CFH gene, one block of significant association remained, albeit at reduced levels. rs1410996 showed a p-value of 5.94e-5 (compared to 3.26e-16 in the full cohort and 6.31e-13 in females) (Figure 13). This SNP was found to be in close to 100% LD with several proxy SNPs (rs2274700, rs3753395, and rs10737680). When looking at the female sub-cohort, rs1410996 had a similar strength of association to AMD as does rs1061170. It was the association of rs1410996 in males, an association lacking at rs1061170, which accounted for the higher significance of rs1410996 (and its proxy SNPs rs2274700, rs3753395, and rs10737680). By analyzing males and females independently, a basis was identified for the disparity between risk at rs1061170 and rs1410996.

Studies suggested the male/female stratification seen at CFH was not a result of AMD disease in general, but of a specific mechanism of disease associated with CFH. Analysis of SNPs in C3 and C2/CFB showed much the same trend as with CFH: weaker but significant risk was found in both the full 927 cohort and the female-only cohort, while no risk was found in the male-only cohort. Thus, risk for AMD in males was much less associated with the complement system as a whole than in females.

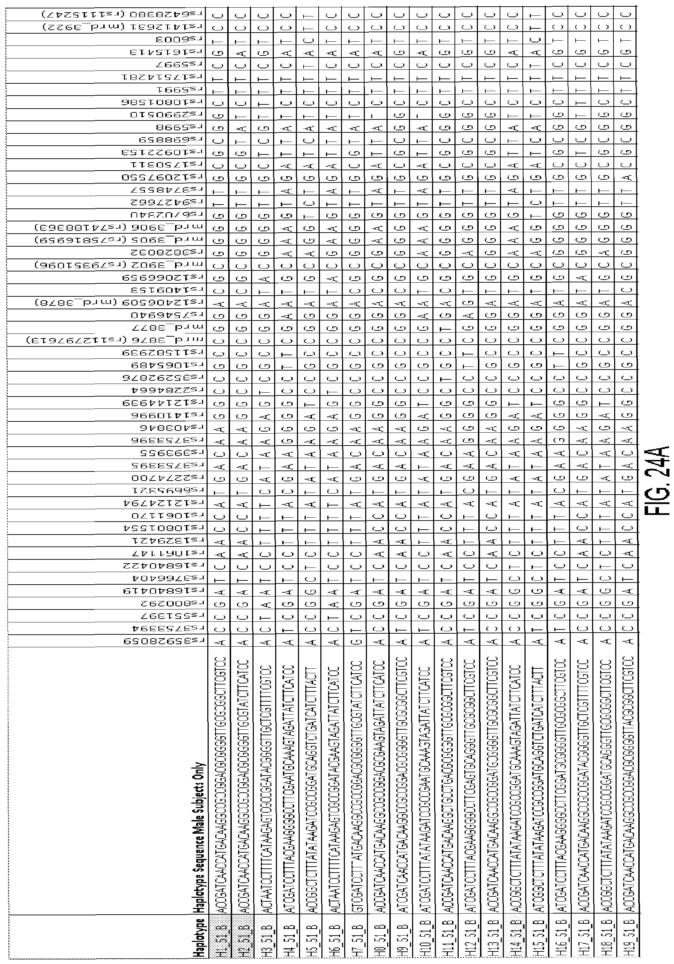

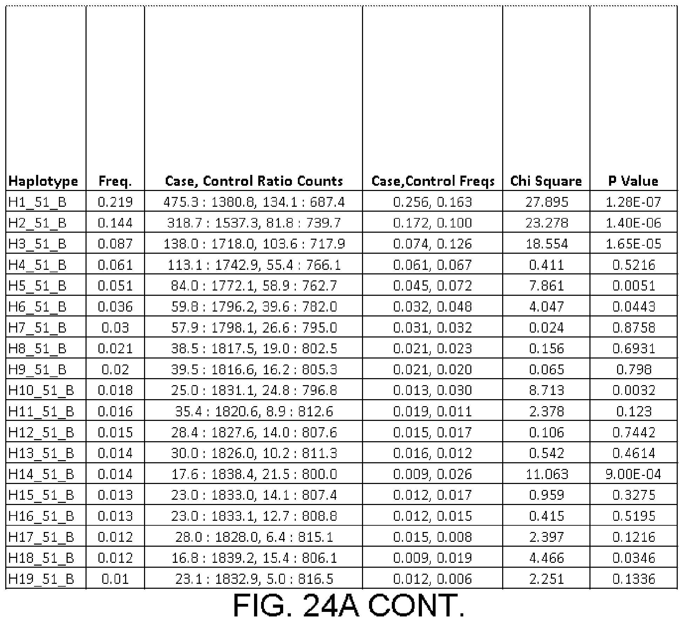

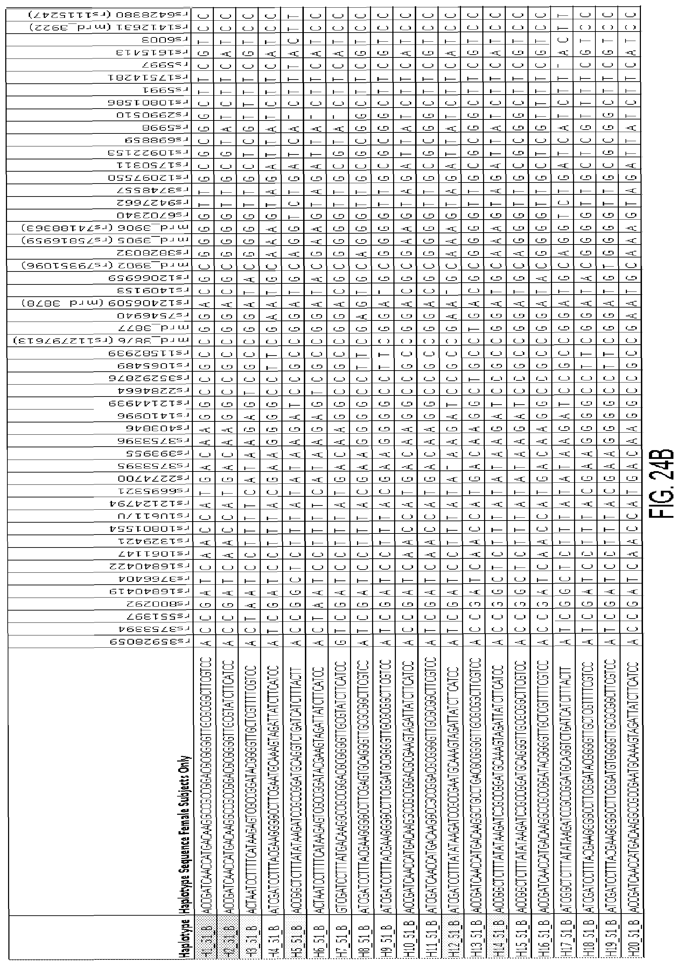

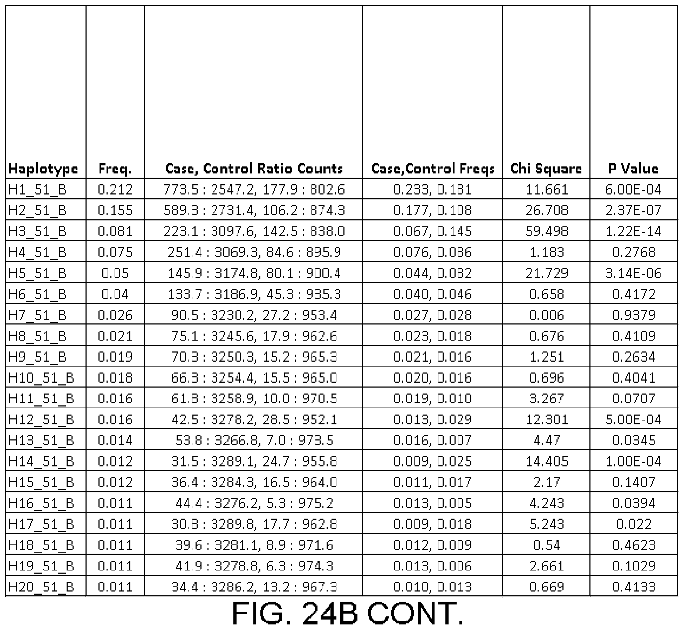

Haplotype disease associations were also examined independently in males and females. H2_62_A was barely significant in males but strongly significant in females (Figure 23). H2_51_B was more significant in males than H2_62_A, but still less significant than in females (Figure 24). H1_62_A was significant in females, not at all in males. Whereas, H1_51_B was significant in both males and females; however, it was again more significant in females (Figure 24). H11_62_A was significant in both males and females, but was more significant in males. H2_62_A, H3_62_A, H5_62_A, and H11_62_A were all significantly associated with AMD in all of the subjects (Figure 6) and also in the females (Figure 23). Females were slightly more significantly associated than the all-subj ects in H1_62_A and H6_62_A, and less significantly associated in H13_62_A, but these differences were fairly minor.

### 3.14 Results: 51 SNP-based Haplotypes Associated with AMD: 1,073 Cohort (927 Subset)

The association of haplotypes in the GSH 927 Cohort was also assessed using a 51 SNP-based haplotype analysis (Figure 22). The 51 SNPs included are depicted in Figure 22. The association of risk and protective haplotypes was similar to those observed in the 62 SNP-based haplotype analysis (Figure 6 and Figure 18; also see Figure 20 and Figure 22).

### 3.15 Results: 51 SNP-based Haplotypes and Their Association with AMD and AMD Phenotypes: 'Combined Cohort' and Extremely Discordant Sib-pair Cohort

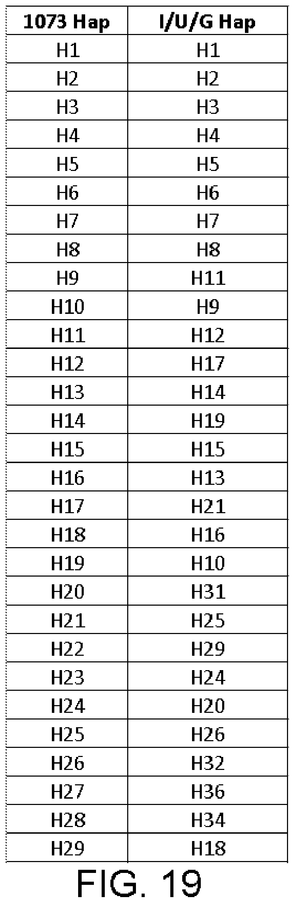

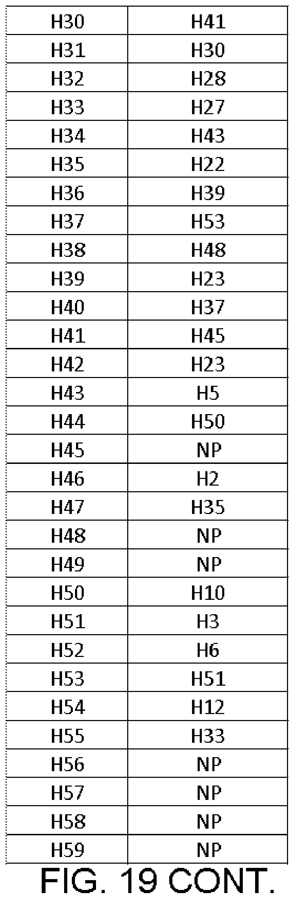

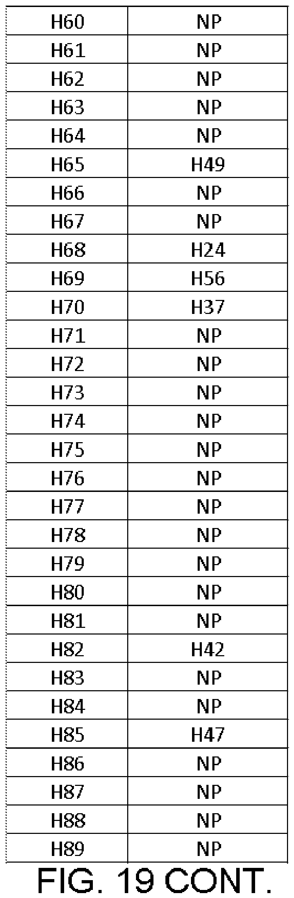

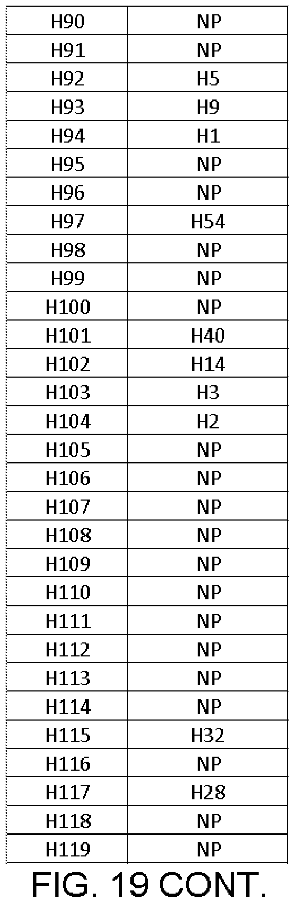

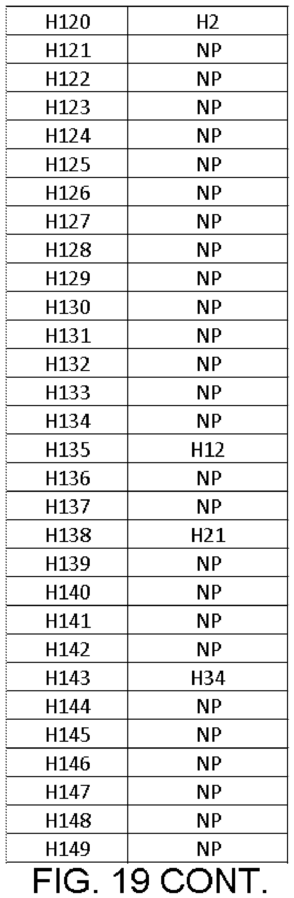

A large case-control cohort comprised of individuals with and without AMD from the Iowa, Utah, and Melbourne cohorts (the 'Combined Cohort') was employed to assess 51 SNP-based CFH-to-F13B haplotypes with AMD (controls versus all AMD) risk, protection, and neutrality. Fifty-six haplotypes were generated from the Combined Cohort, 18 of them occurred at frequencies above 1% (Figure 15). The relationships between the major 51 SNP-based and 62 SNP-based haplotypes are shown in Figure 16 and the relationship between 51 SNP-based haplotypes in the 1,073 and Combined Cohorts is depicted in Figure 19. Associations similar to those of 62 SNP-based (Hx_62_A) and 51 SNP-based assessments in the GSH 927 Cohort (Hx_51_A) were observed (Figure 15). H1_51_B, however, became more significant in this larger cohort.

Several minor CFH-to-F13B haplotypes showed risk and protection from AMD relative to the cohort as a whole. For example, H24_62_A and H28_62_A trended toward risk and H30_62_A (p=0.007) and H40_62_A trended toward protection (Figure 15).

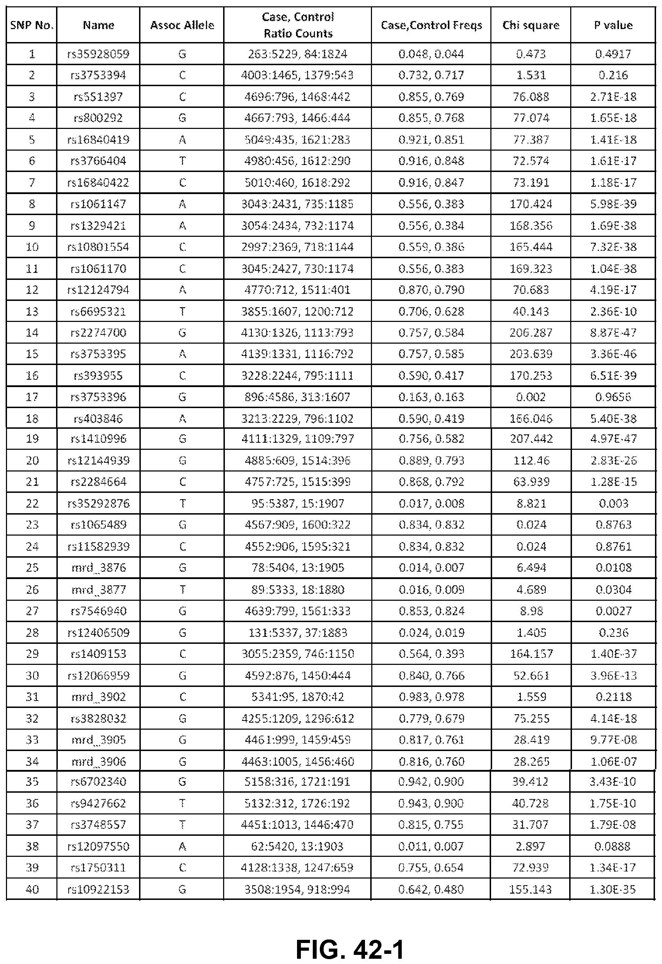

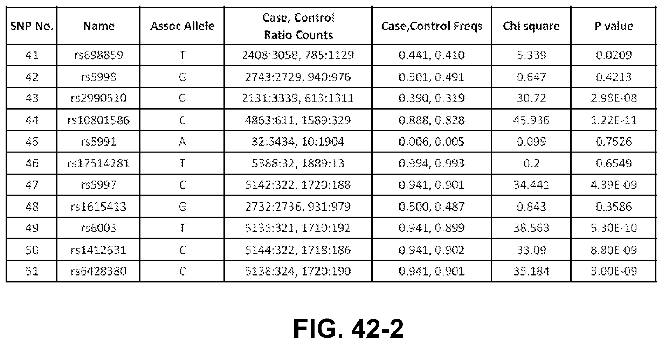

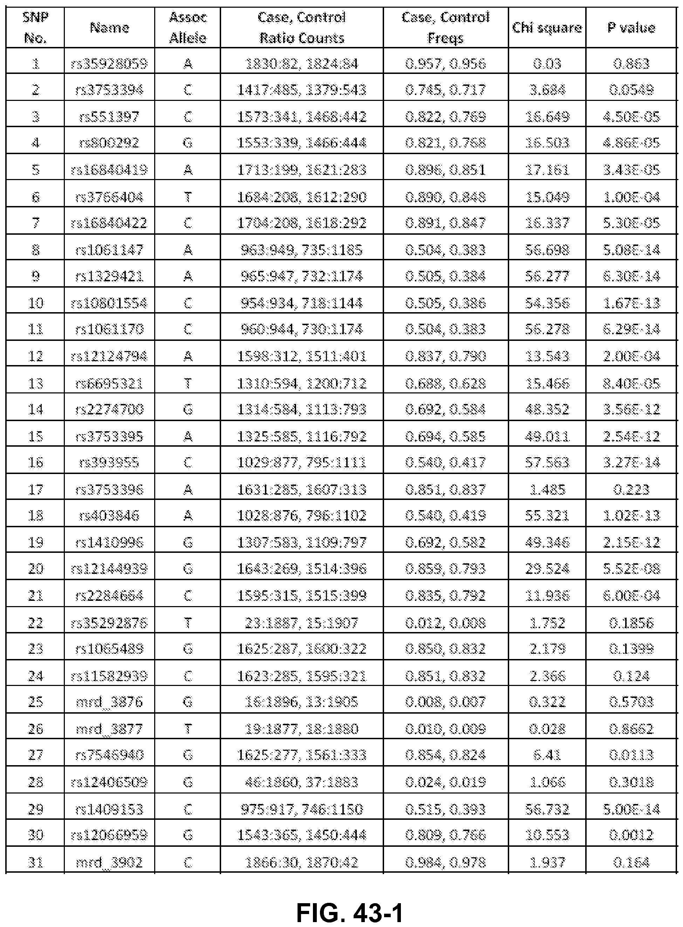

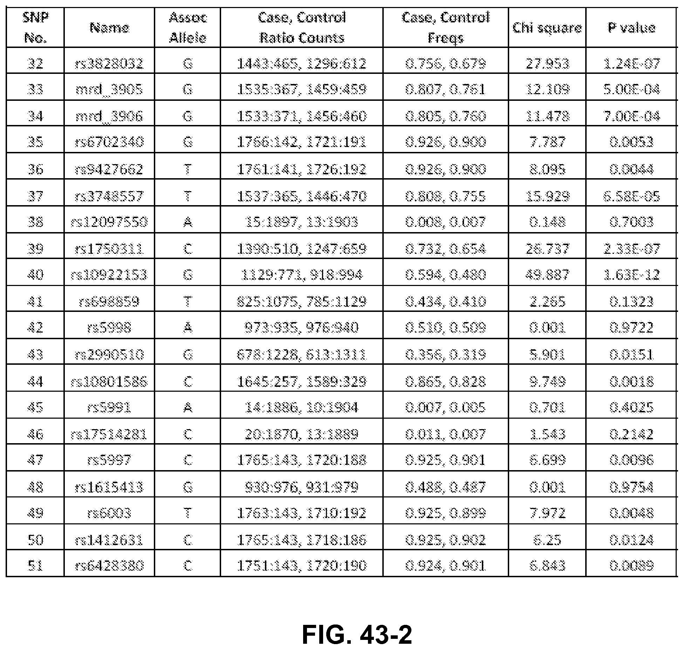

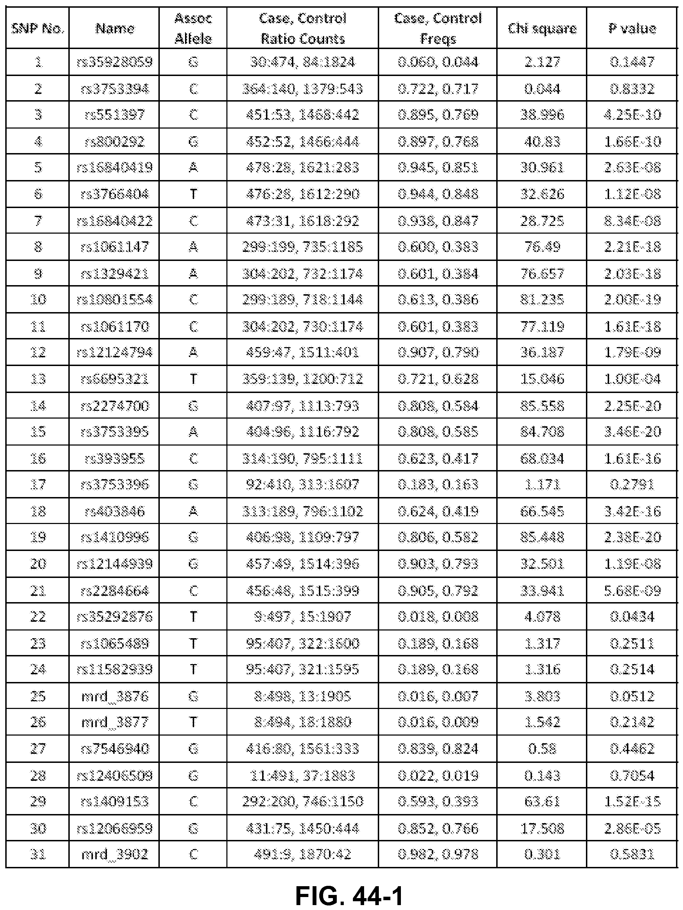

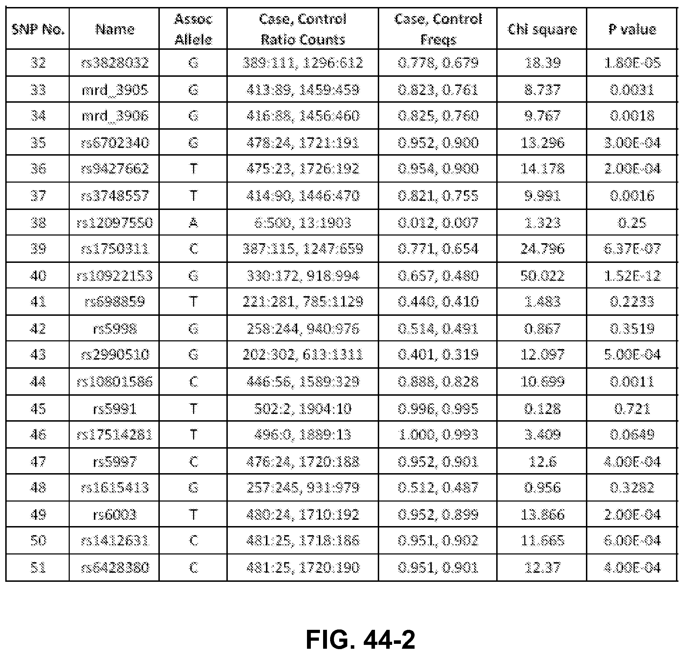

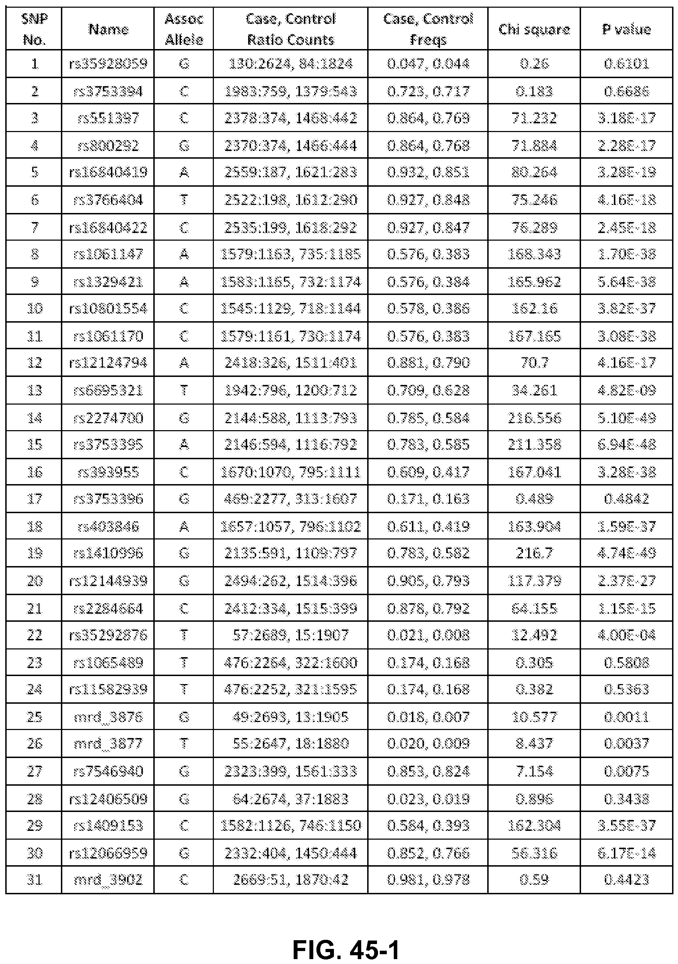

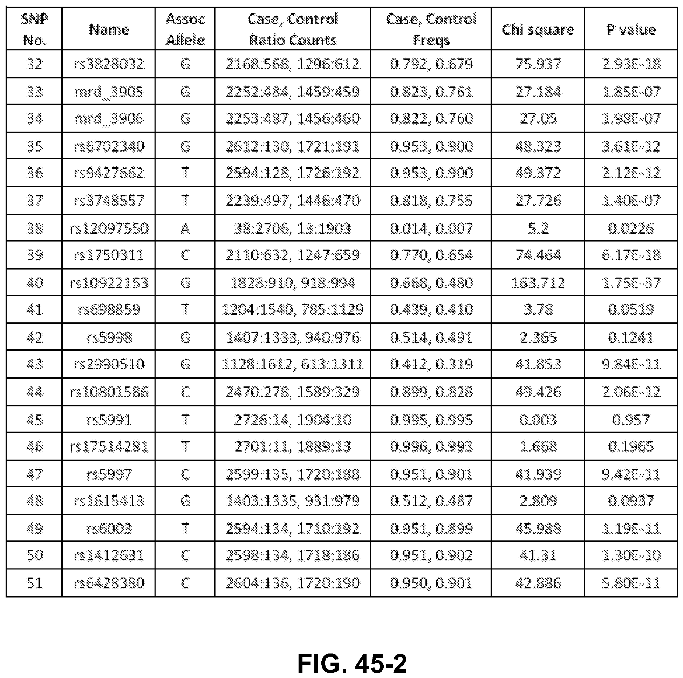

The 'Combined Cohort' was also employed to assess the association of single SNP associations of the 51 SNP panel and 51 SNP-based CFH-to-F13B haplotypes with the various phenotypes/stages of AMD, including comparisons of controls (stage 0) to all AMD (stages 1B-4C), early stage AMD (stages 1B-3), and the late stages of geographic atrophy (stage 4A) and CNV (stage 4B). Single marker associations in this cohort (Figure 42) did not differ from those of the 1,073 and highly discordant sib-pair cohorts when controls were compared to all AMD. Moreover, single marker associations with risk, protection and neutrality did not vary significantly when controls were compared to all AMD stages (Figure 42), early stage only (Figure 43), geographic atrophy only (Figure 44), or CNV only (Figure 45).

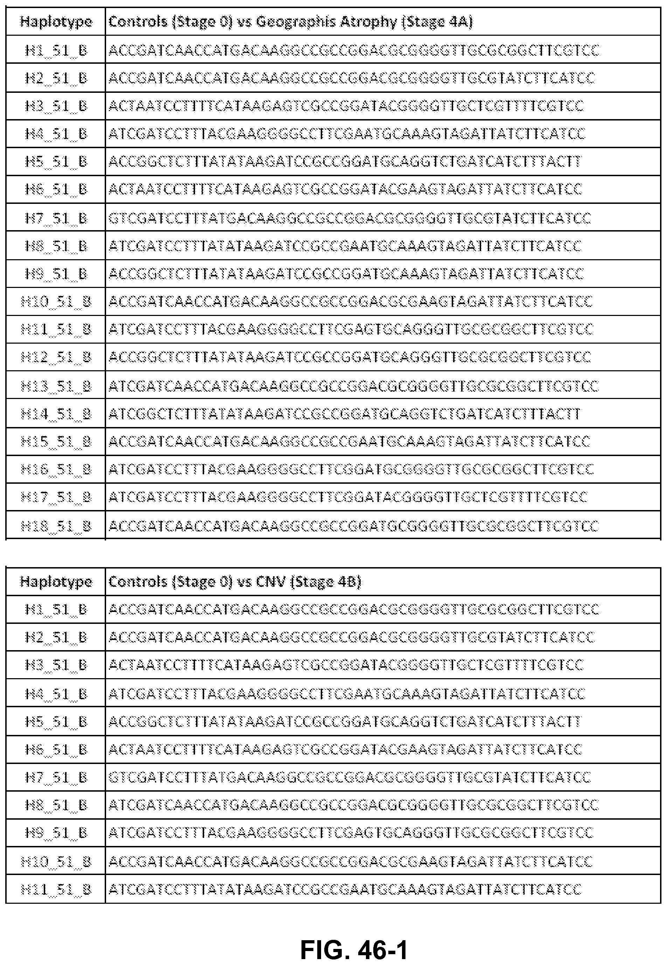

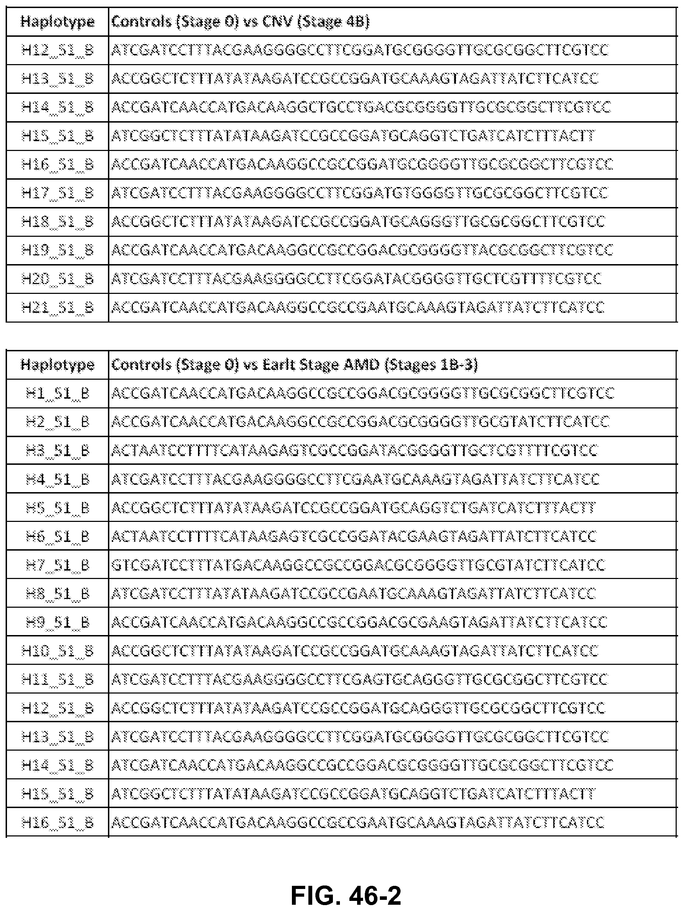

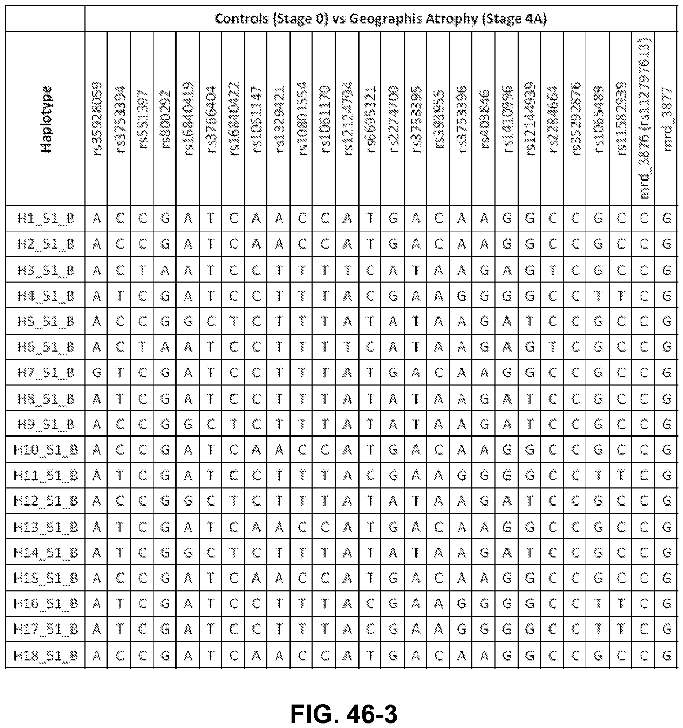

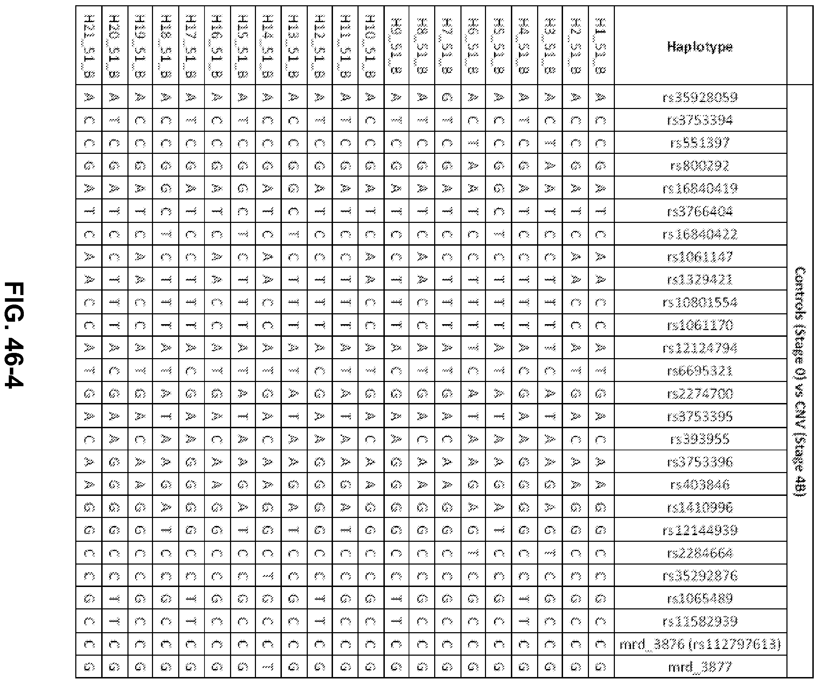

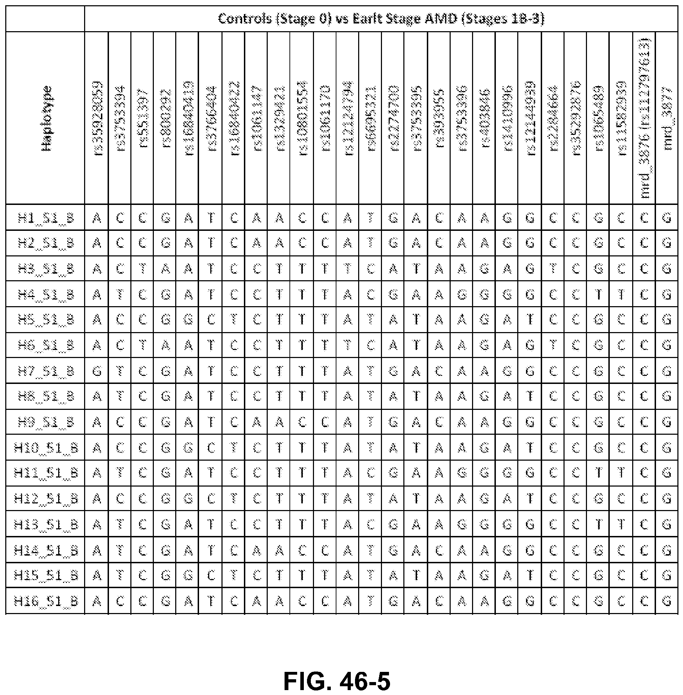

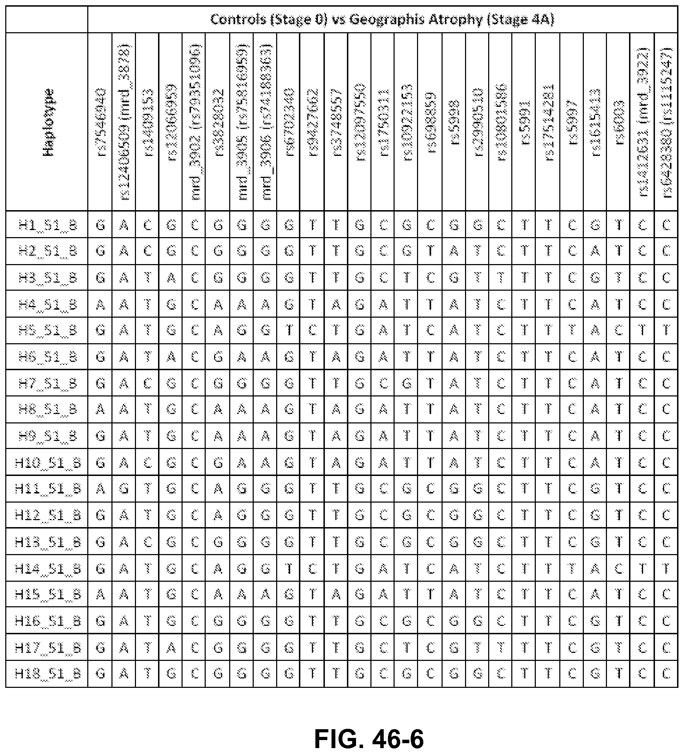

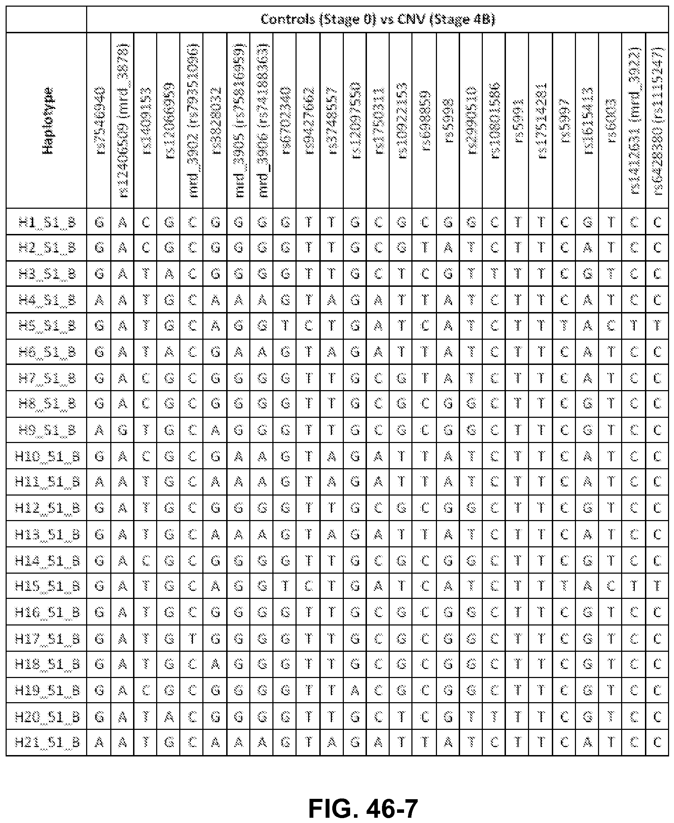

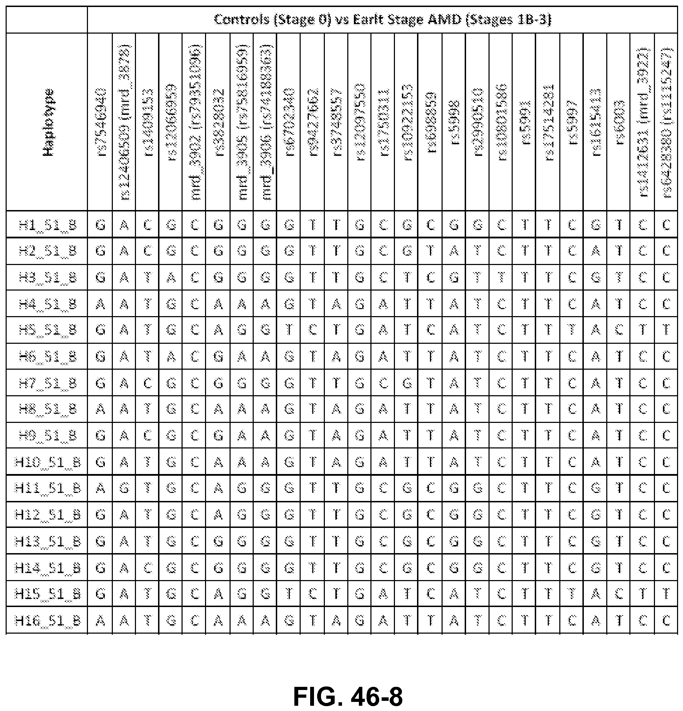

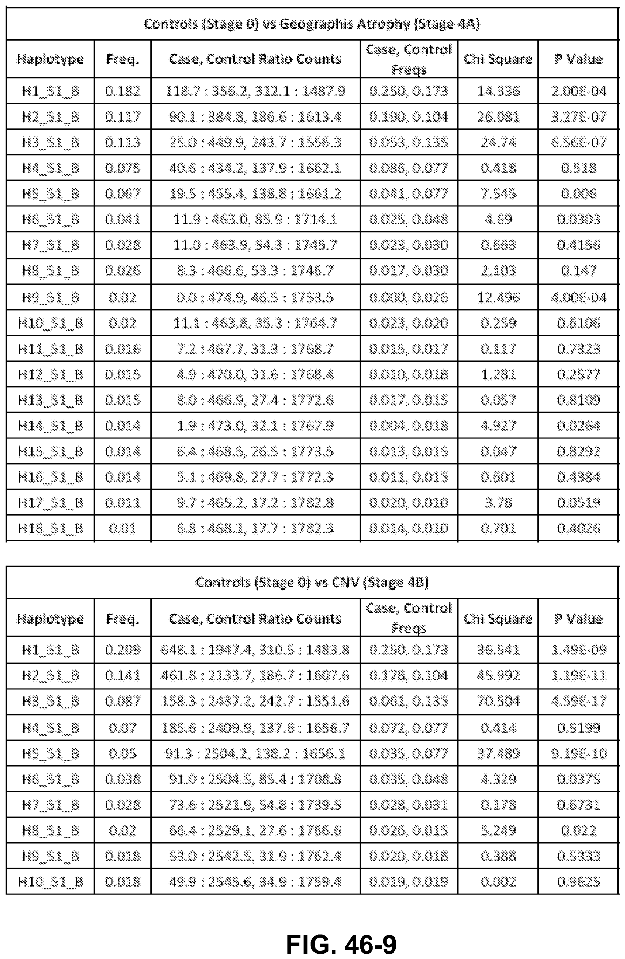

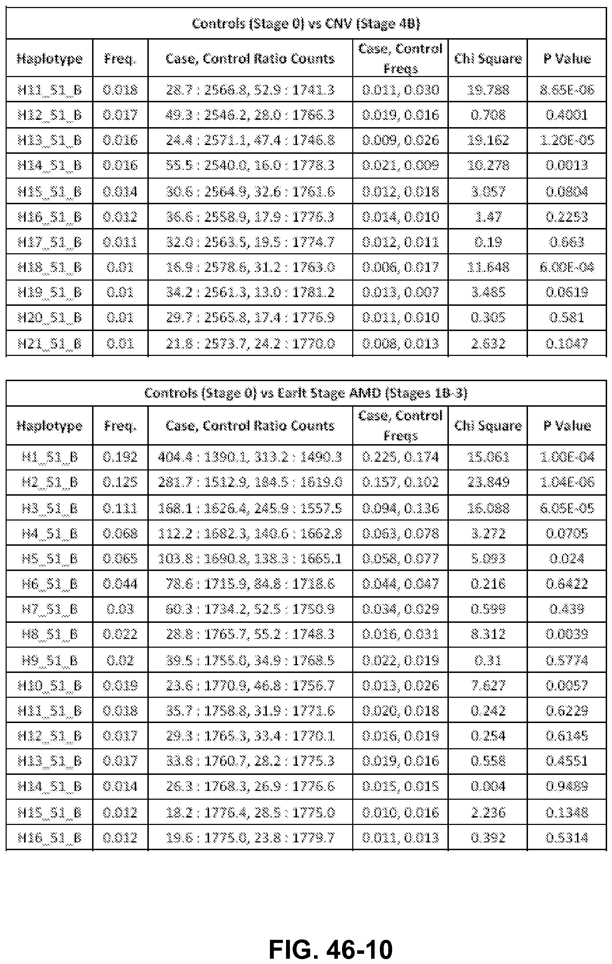

Haplotype associations with risk, protection and neutrality did not vary significantly between AMD phenotypes in the Combined Cohort (nor were they significantly different from analyses of the 1,073 cohort; Figure 20 and Figure 22) when controls were compared to early stage only, to geographic atrophy only, or to CNV only (Figure 46). Note that the haplotype order (based on frequencies) was not identical in the three analyses shown in Figure 46..

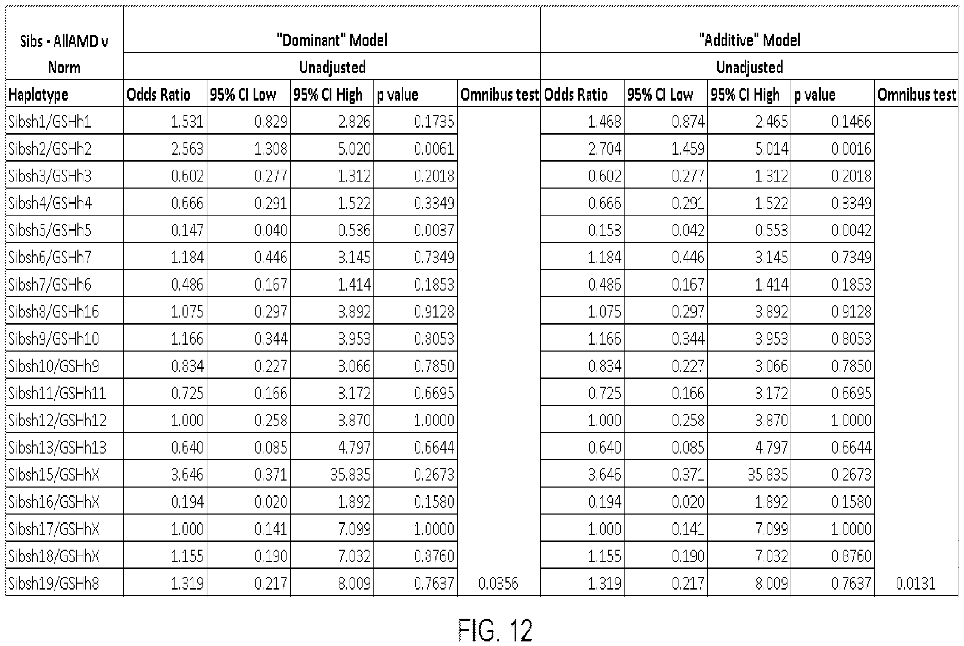

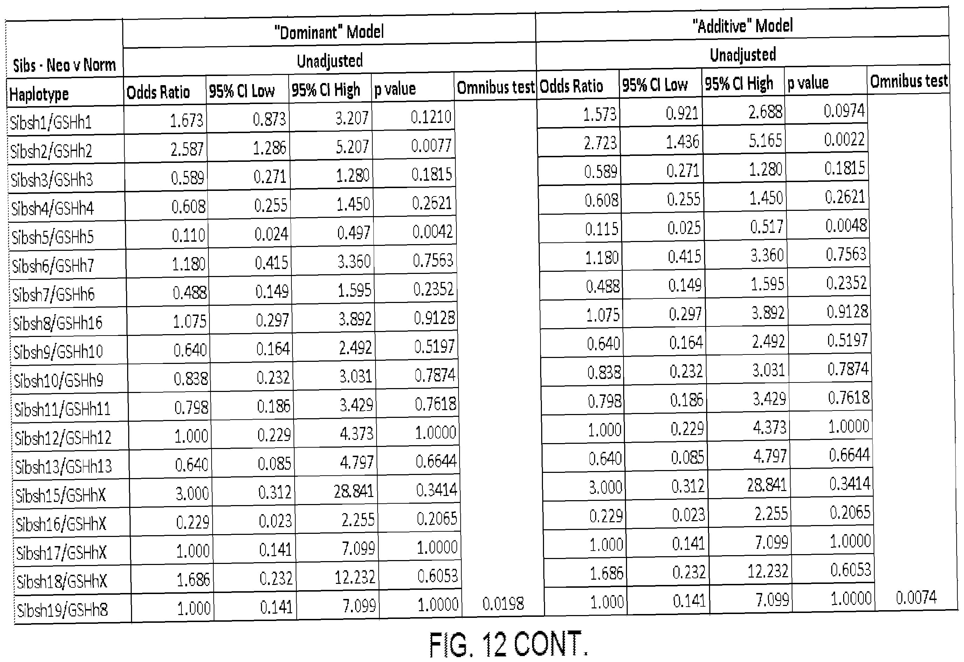

The differential association of H1_51_B and H2_51_B with AMD risk was verified using a highly discordant sib-pair cohort. 51 SNP-based haplotypes were generated for this cohort. Using logistic regression, a pattern was seen in which H1_51_A (as described in the GSH cohort) was not significantly associated with risk. The equivalent to H2_51_A, namely H2_51_B, wa highly associated with risk and wa the only haplotype that associates with risk (Figure 12). Of the remaining haplotypes, only H5_51_B (containing the CFHR3/CFHR1 deletion) associated with AMD: in this case as a protective haplotype (Figure 12). Thus, the difference in risk association between H1_51_A and H2_51_A was been replicated in a second independent cohort.

### 3.16 Results 51 SNP-based Diplotype Associations with AMD Risk, Protection and Neutrality: Combined Cohort

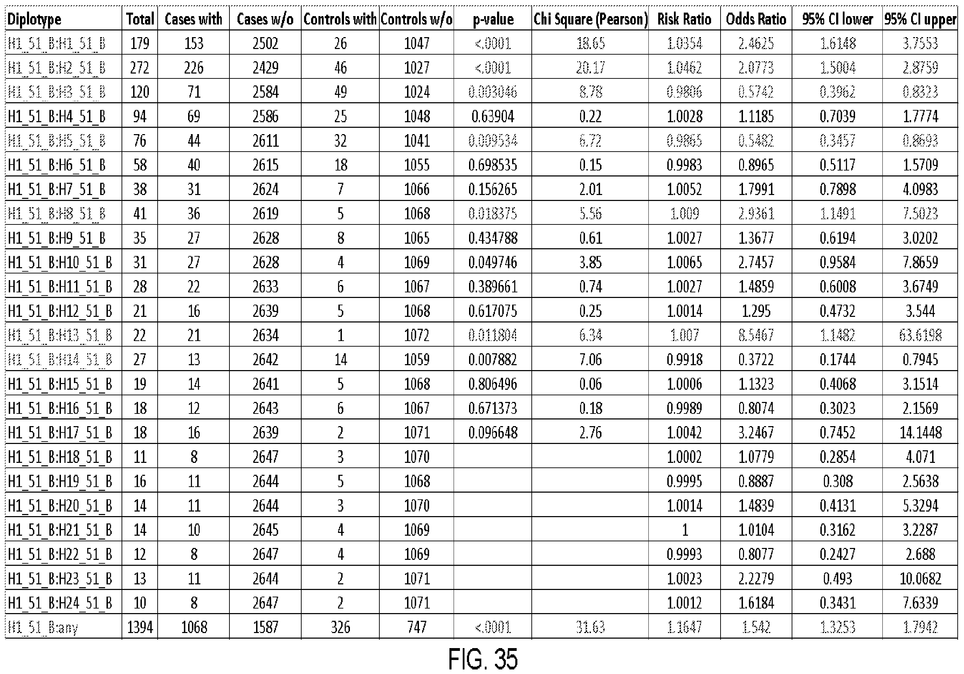

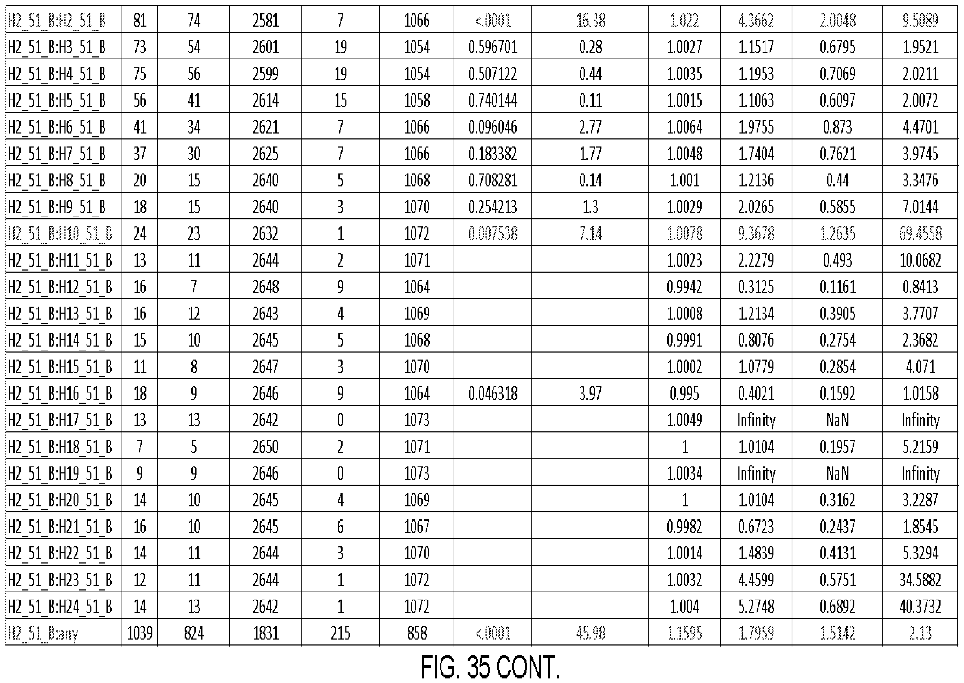

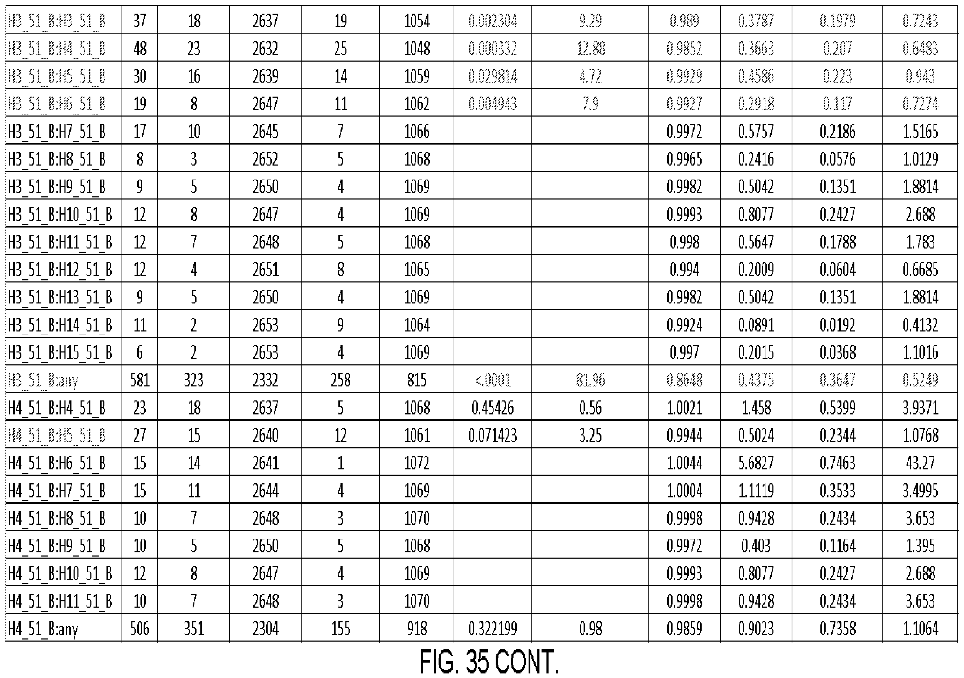

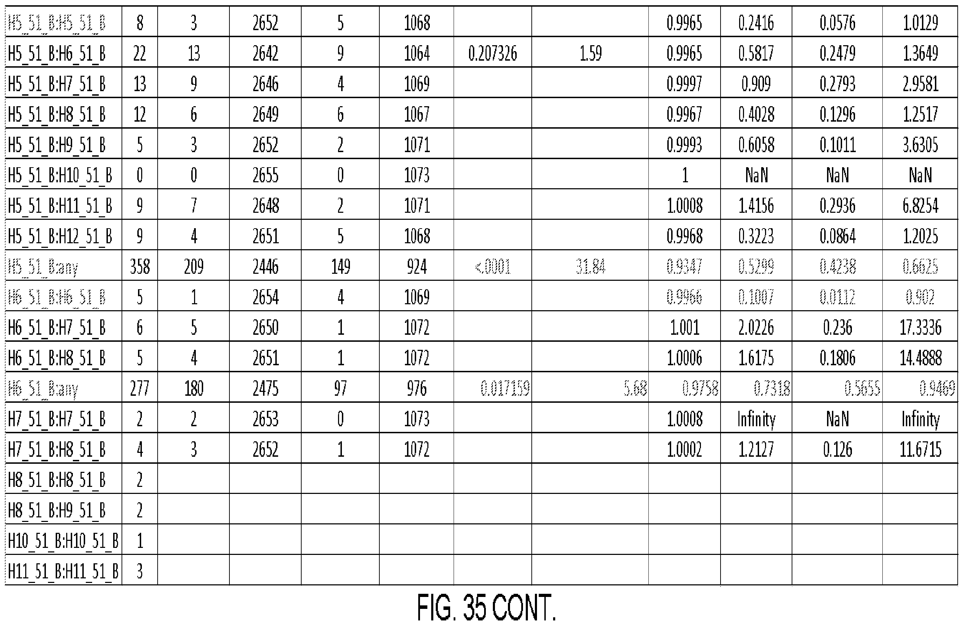

51 SNP-based diplotypes were phased and determined in the Combined Cohort using BEAGLE version 3.3.2 (S R Browning and B L Browning, 2007, Rapid and accurate haplotype phasing and missing data inference for whole genome association studies using localized haplotype clustering. Am J Hum Genet 81:1084-1097). Association with AMD risk, protection, and neutrality was assessed in the entire cohort (Figure 35), in males only (Figure 36) and in females only (Figure 37). Combinations of all diplotype combinations for which there was appropriate statistical power are shown in Figure 35; statistical power was sufficient to assess associations through haplotype 7.

A single H1 haplotype when combined with another H1 haplotype, or a H2, H8 or H13 haplotype was associated with increased risk for developing AMD whereas combination of H1 haplotype with a H3 or H5 haplotype was associated with protection. Only when a single H2 haplotype occurred with another H2 haplotype, a H1 haplotype or a H10 haplotype was there an association with risk. No combinations showing association with protection were observed, suggesting that the H2 haplotype is more strongly associated with risk than is H1. No risk association was observed with any combinations of a single H3, H4, H5, H6 or H7 haplotype in combination with any other haplotype. However, many of these combinations were protective, as depicted in Figure 35. The overall results for the male and female only groups were similar to those of the whole cohort, with some minor differences noted in males only, perhaps due to loss of statistical power (Figures 36 and 37).

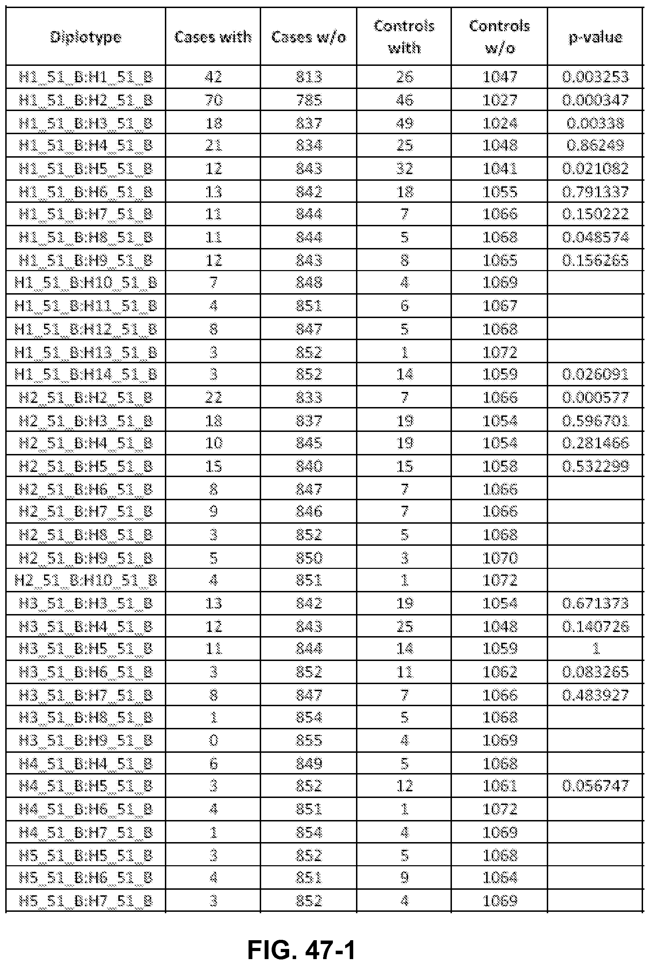

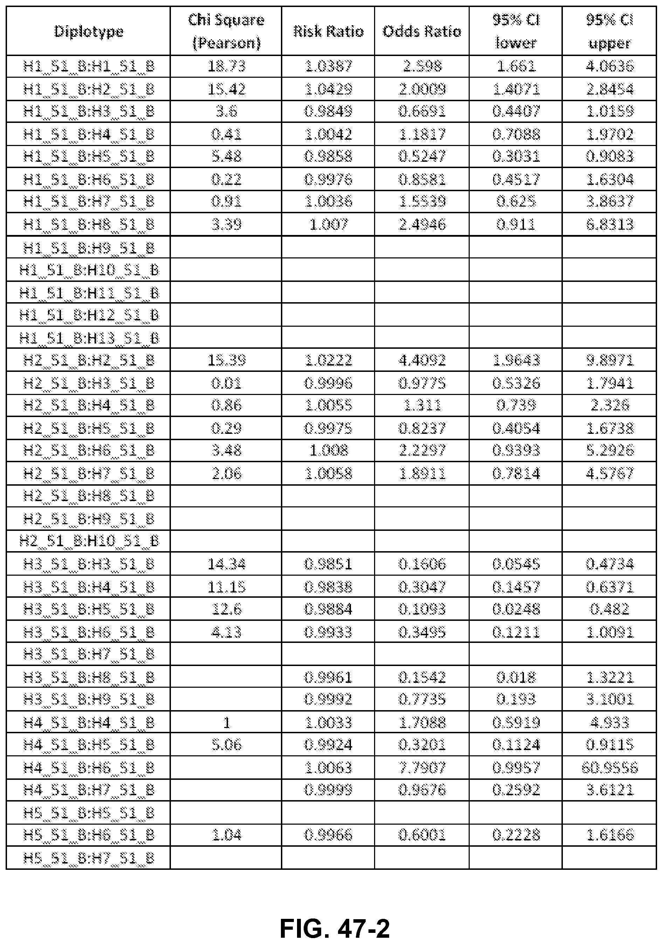

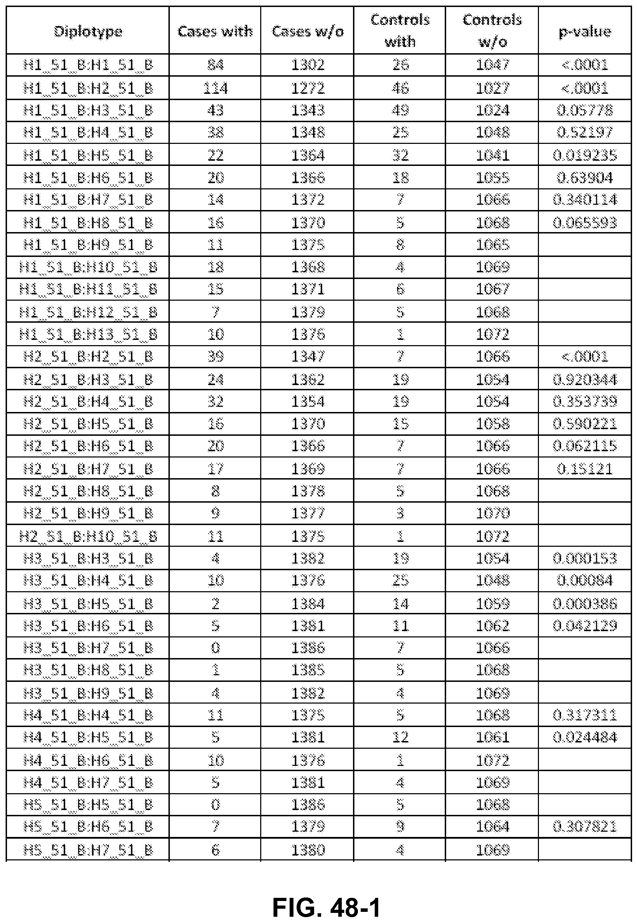

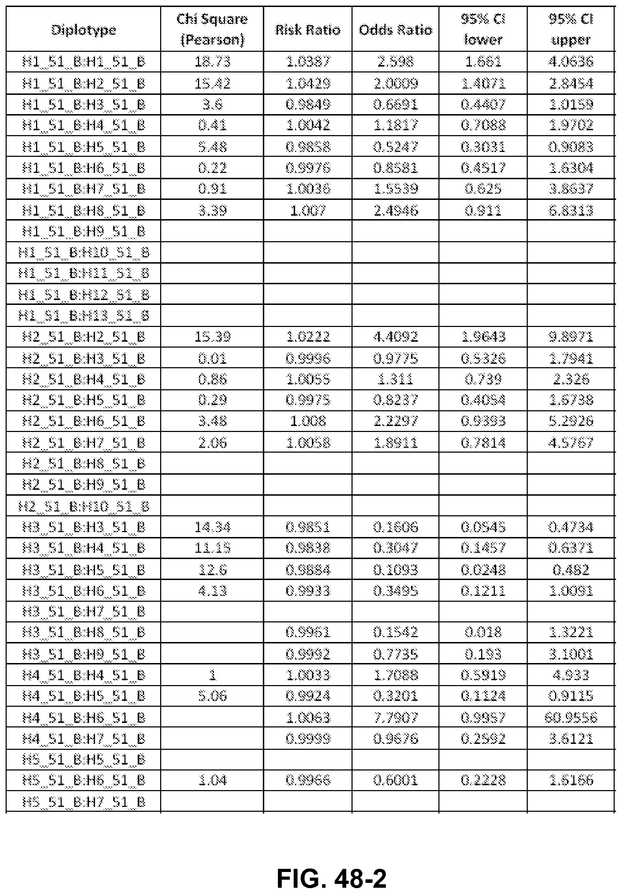

The 'Combined Cohort' was also employed to assess the association of 51 SNP-based CFH-to-F13B diplotypes with the various phenotypes/stages of AMD, including comparisons of controls (stage 0) to all AMD (stages 1B-4C), to early stage AMD (stages 1B-3), and to CNV only (stage 4B). Diplotype associations with risk, protection and neutrality did not vary significantly between the following groups: 1) controls compared to all AMD stages (Figure 35); and 2) controls compared to CNV only (Figure 48). Nor did these associations differ from those in other cohorts. However, early when controls were compared to early stage AMD only (Figure 47), the protective effect of all H3 combinations was greatly diminished, suggesting that H3 diplotype combinations may not be protective for phenotypes (drusen) associated with early AMD development or with specific types of drusen associated with early AMD development.

### 3.17 Results: Haplotype Tagging SNPs

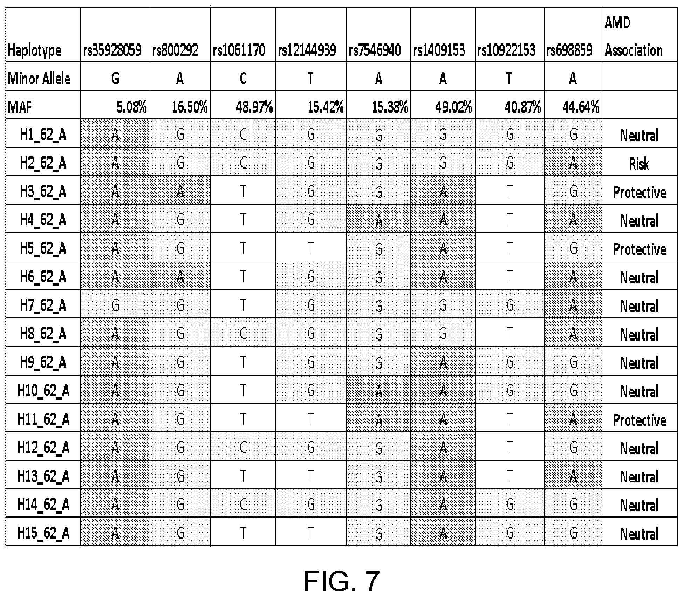

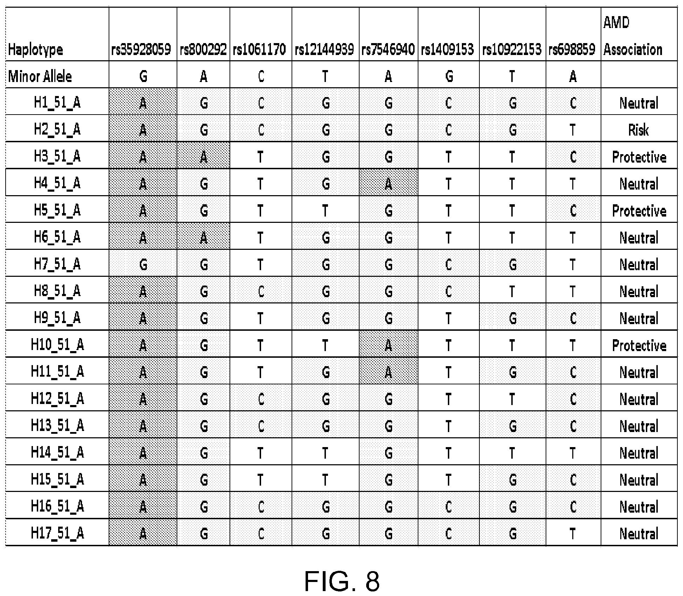

Each of the major 62 SNP-based haplotypes and each of the major 51 SNP-based haplotypes can be uniquely identified using a combination of eight haplotype tagging SNPs (htSNPs; Figure 7; note that rs800292 and rs35928059 are optional for tagging the 62 and 51 SNP-based haplotypes). Case/control analysis was repeated on the GSH 1,073 Cohort using only the eight htSNPs to define haplotypes using Haploview 4.2. Risk association with H1 differed by several orders of magnitude and was statistically significant (Figure 10; 8 htSNPs - H1 p-value = 0.0024; 62 SNPs - H1 p-value = 0.116; note that 62 SNP-based significance values are slightly different in Figure 10 as compared to Figure 6 because Haploview cannot handle the tri-allelic deletion SNPs) compared to the 62 SNP-based analysis. In order to show this effect more clearly, the 927 GSH cohort was reanalyzed in Haploview using progressively fewer SNPs in the following numbers: 1) all CFH locus SNPs (62); 2) all SNPs minus the four deletion-associated SNPs (58); 3) all SNPs for which Taqman assays were developed (51); 4) all SNPs for which MAF >5% frequency (43); 6) all SNPs with highly significant associations to AMD plus rs698859 (31); and 7) only the htSNPs (8) (Figure 10). As fewer SNPs were used to generate haplotypes, more minor haplotypes were recast as major haplotypes and H1 became significantly associated with risk for AMD. All other significant haplotypes showed only small changes in their association to AMD that did not affect the interpretation of risk.

### 3.18 Results: Extension of 51 SNP-based CFH-to-F13B Haplotypes to Genome Level Haplotypes; Association of HapMap Haplotypes with AMD

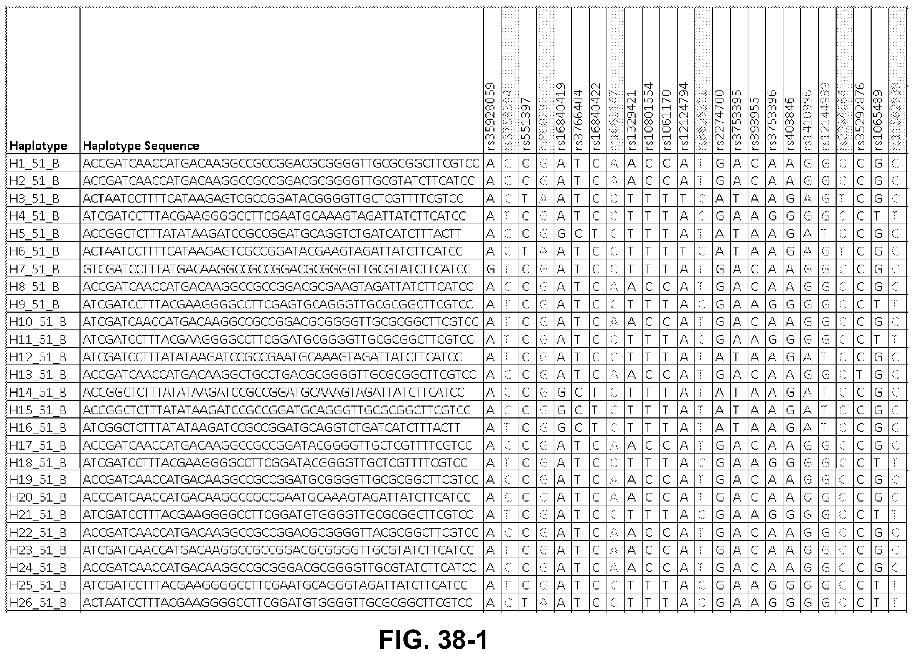

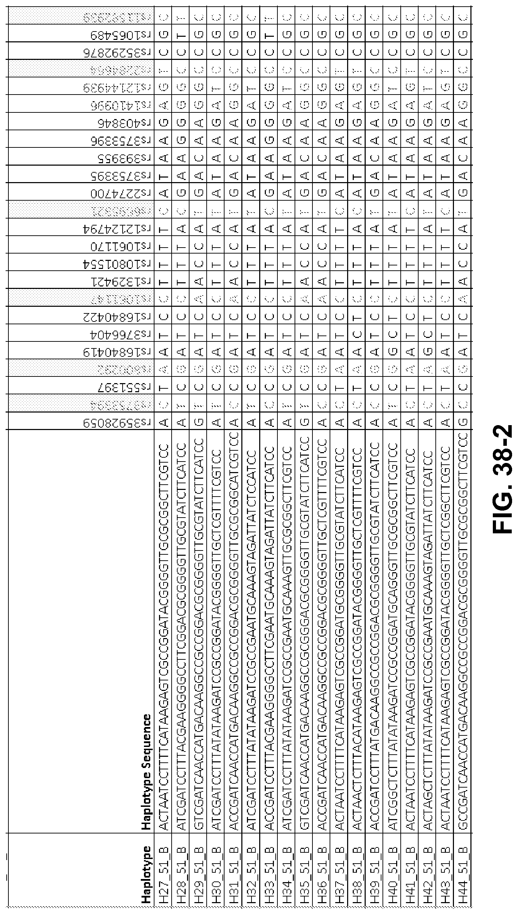

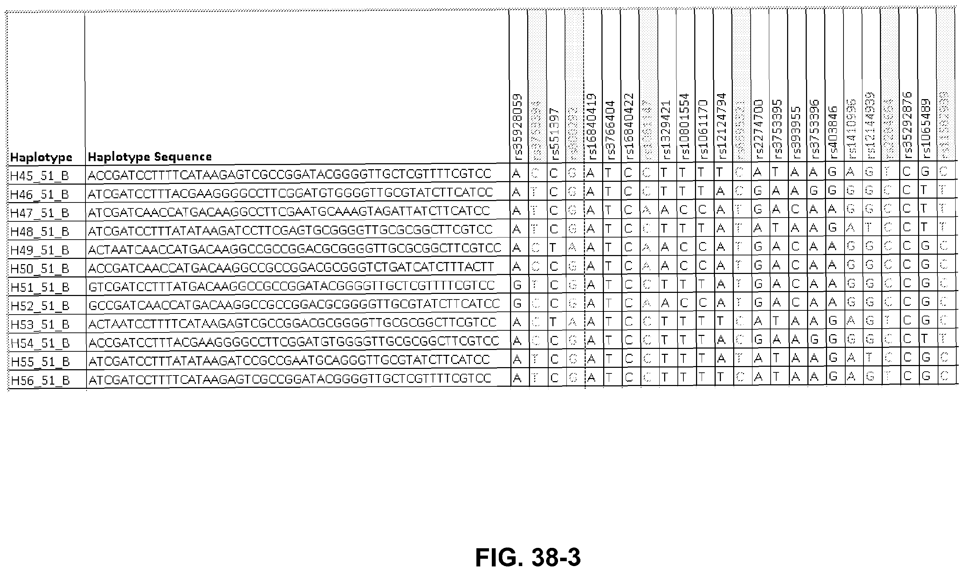

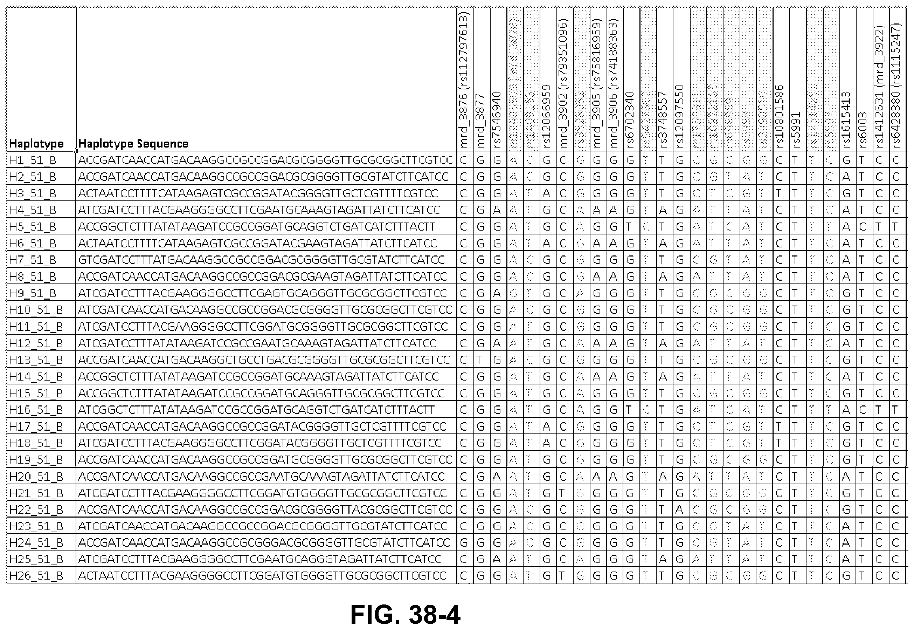

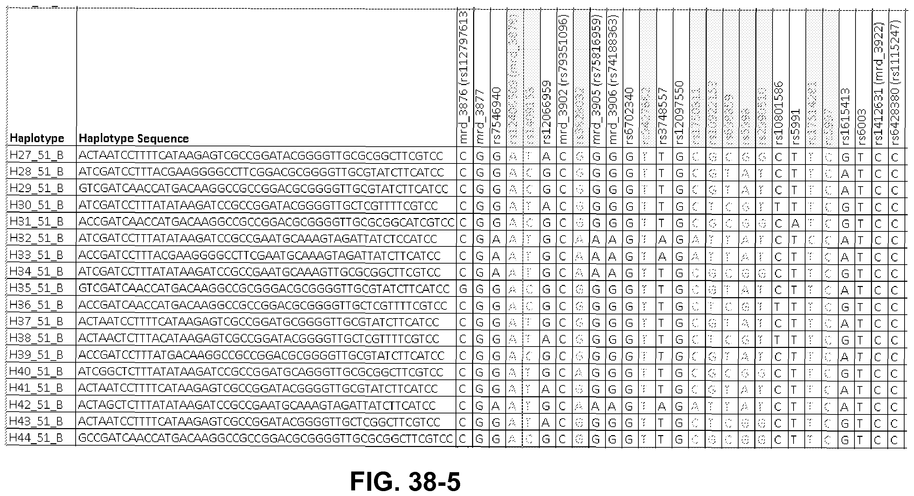

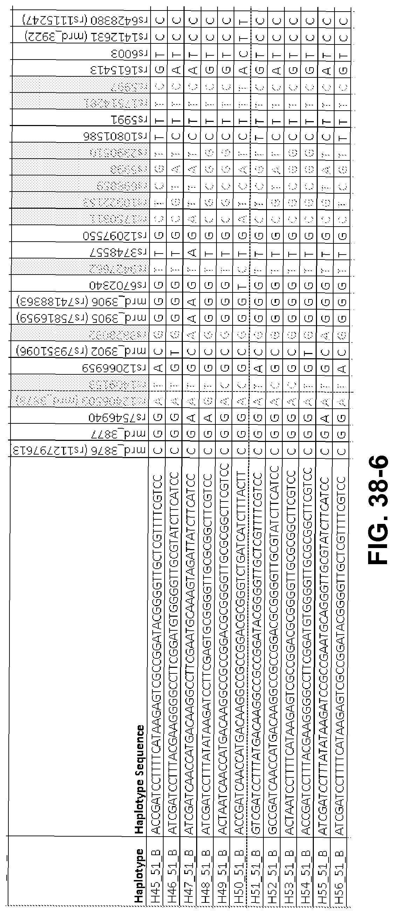

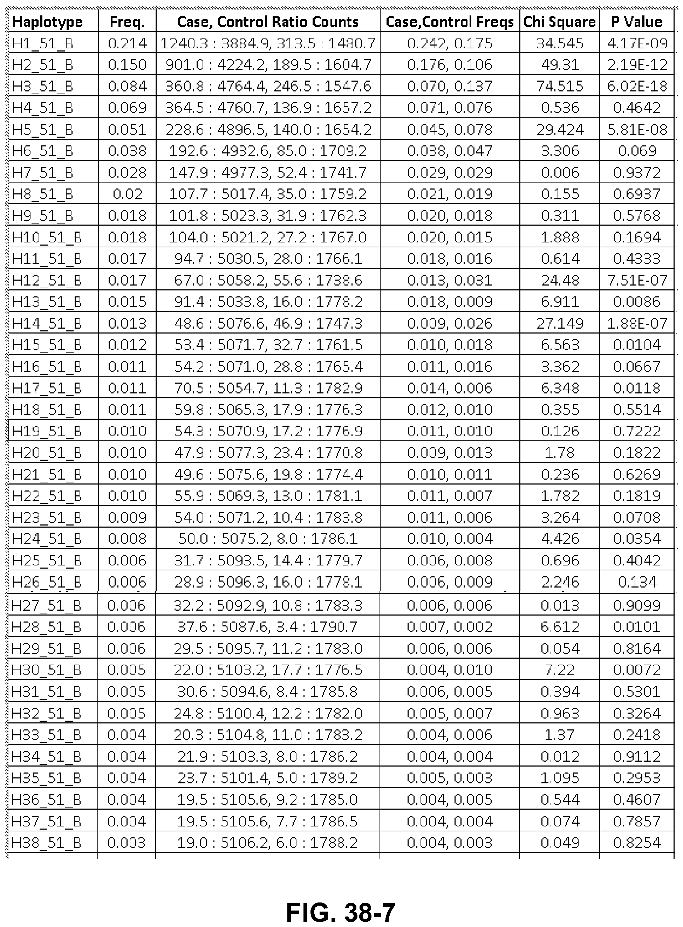

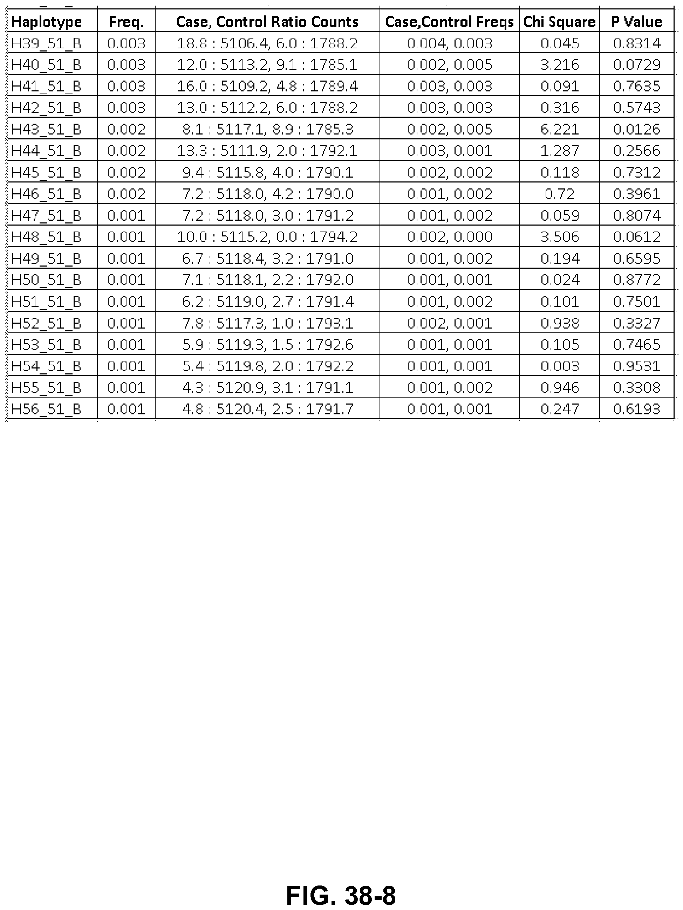

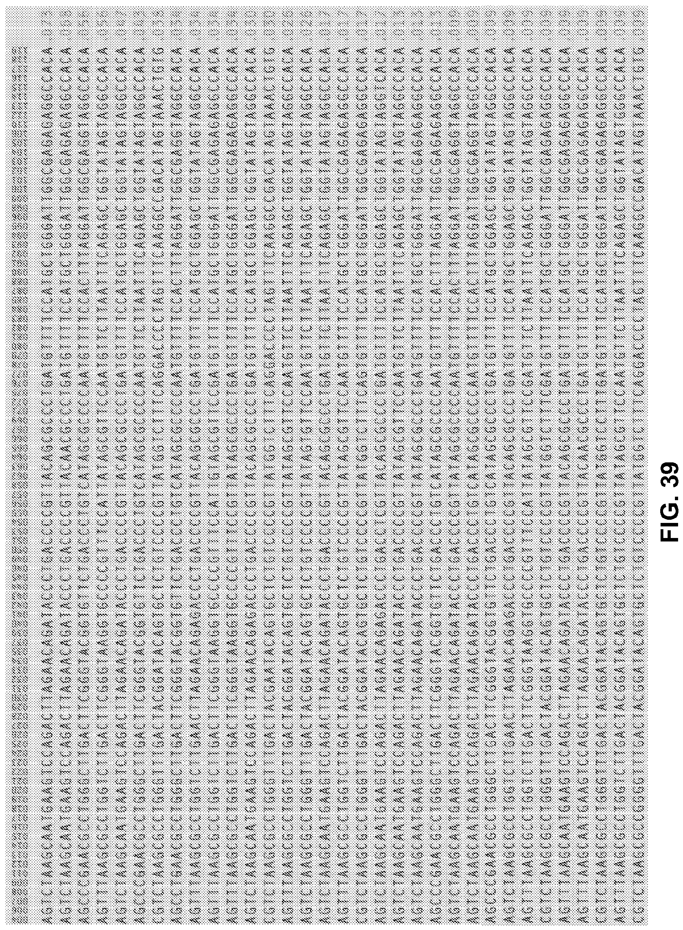

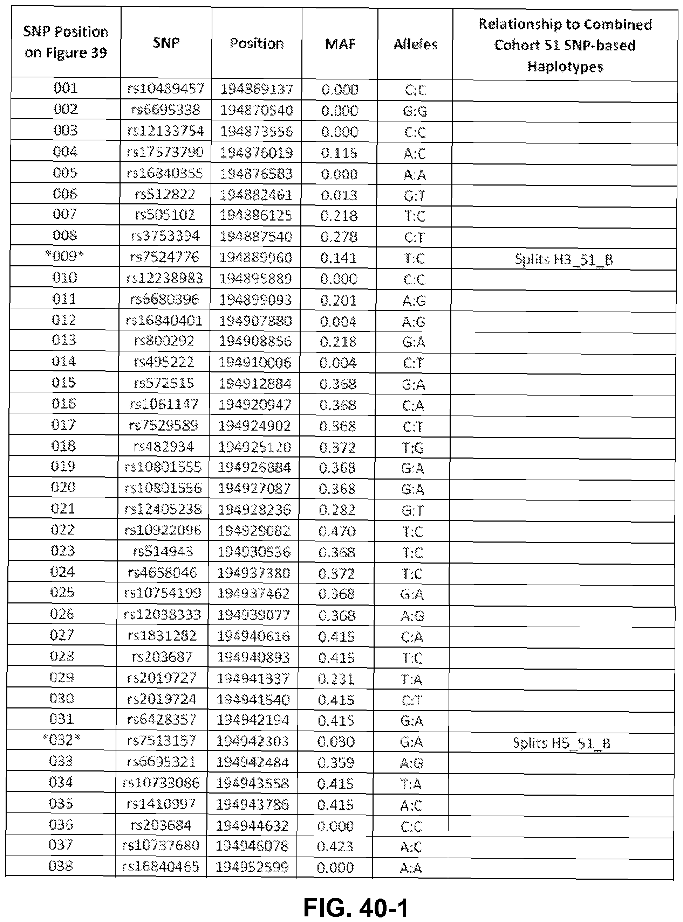

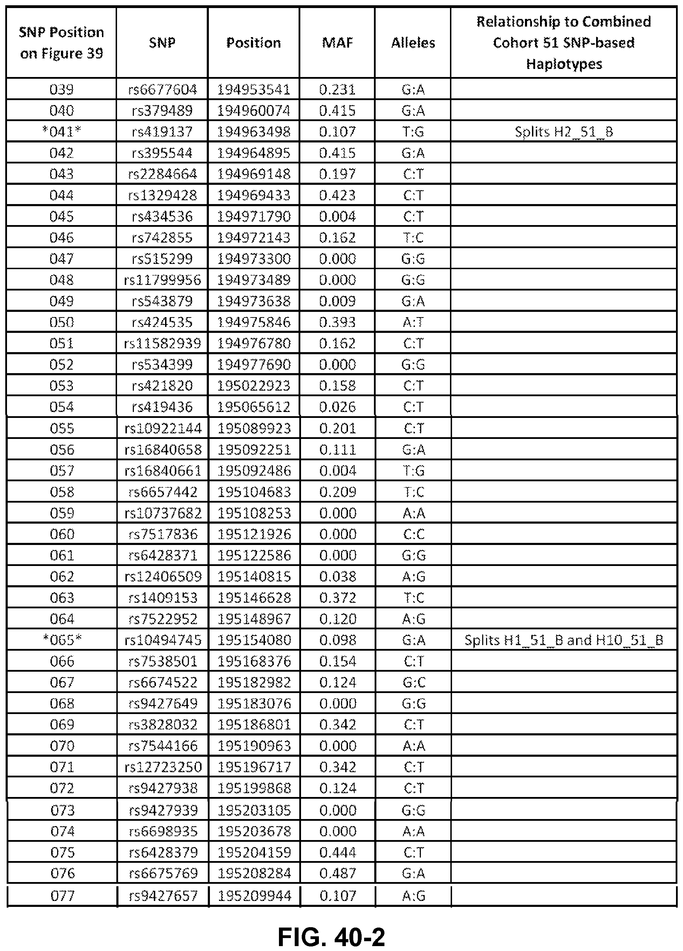

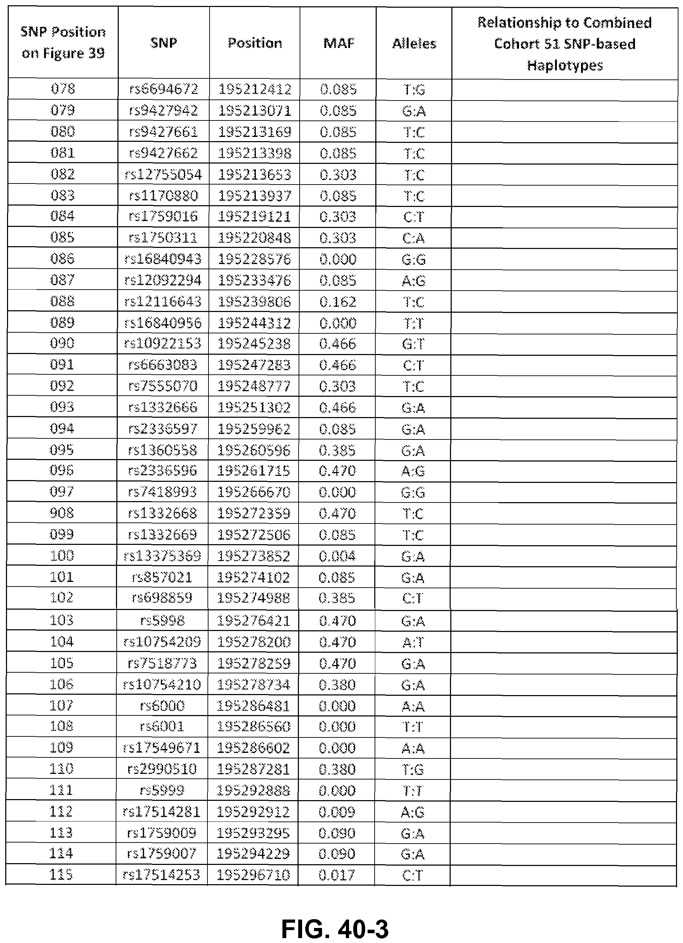

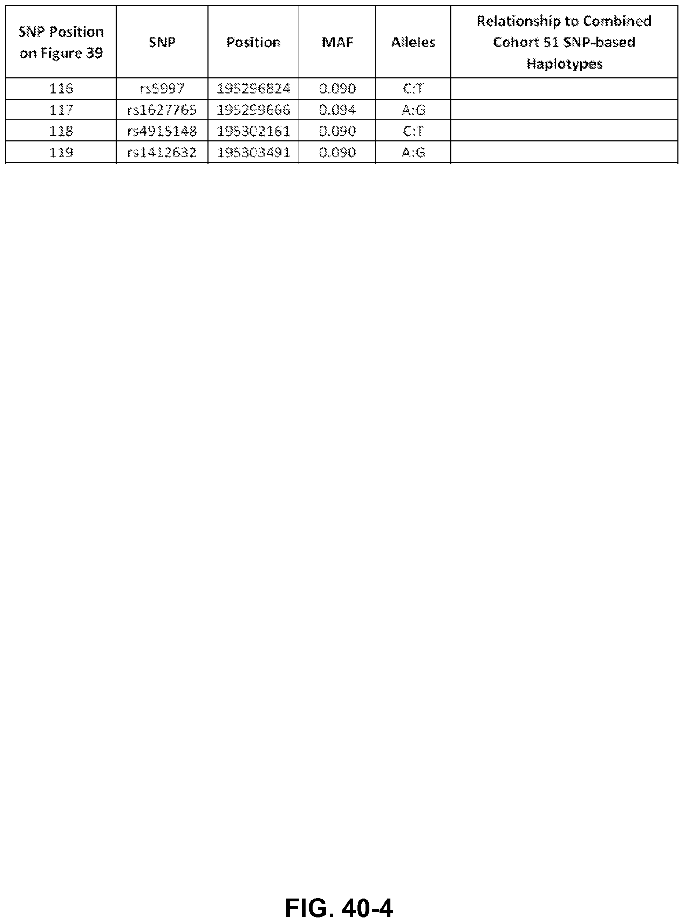

51 SNP-based haplotypes were compared to haplotypes generated using HapMap data in order to further refine haplotype association with AMD. The International HapMap Project database (http://hapmap.ncbi.nlm.nih.gov/) contains over 500 SNPs in the region encompassing the CFH-to-F13B locus (Hg18 chr1: 194869137-195303491). Genotypes from 180 Caucasian individuals (referred to as the CEU population) were downloaded for each of these 500 plus SNPs. Phased genotype data from the CEU set was downloaded, including the most informative SNPs (*i.e.* those that segregate in the CEU population) in this region of interest. Using Haploview (www.broadinstitute.org), haplotypes were constructed for the CEU dataset and their frequencies calculated (Figures 39 and 40). There is substantial overlap between the AMD case/control ('combined cohort') 51 SNP-based haplotypes and the HapMap haplotypes (Figures 38, 39 and 40). These overlapping SNPs were used as 'tagging' SNPs to match case/control haplotypes to their equivalent HapMap CEU haplotypes (Figures 38 and 41). AMD association with haplotype was previously established in the 51 SNP-based case/control ('combined') cohort (Figure 22); thus association could be extended to the equivalent HapMap haplotypes. In some instances, 51 SNP-based haplotypes overlapped with multiple HapMap haplotypes and vice versa due to the presence of HapMap SNPs that were not included in the 51 SNP-based haplotypes. Thus, in cases where HapMap haplotypes overlapped with multiple equivalent case/control haplotypes, key SNPs were identified that further refined the discriminative value of our major 51 SNP-based haplotypes. As an example, the 'combined cohort' haplotype H1 (Figures 39 and 41) matched the two most frequent of the major HapMap generated haplotypes (Figures 39 and 41). The only SNP that differentiated these two HapMap haplotypes was rs10494745. Other similar informative SNPs discovered in this analysis that will refine assessment of disease association are indicated with asterisks and their relationship to the Combined Cohort 51 SNP-based haplotypes are described in Figure 40. They are rs7524776, rs7513157, rs419137, and rs10494745.

### 3.19 Results: Extended Haplotype Blocks

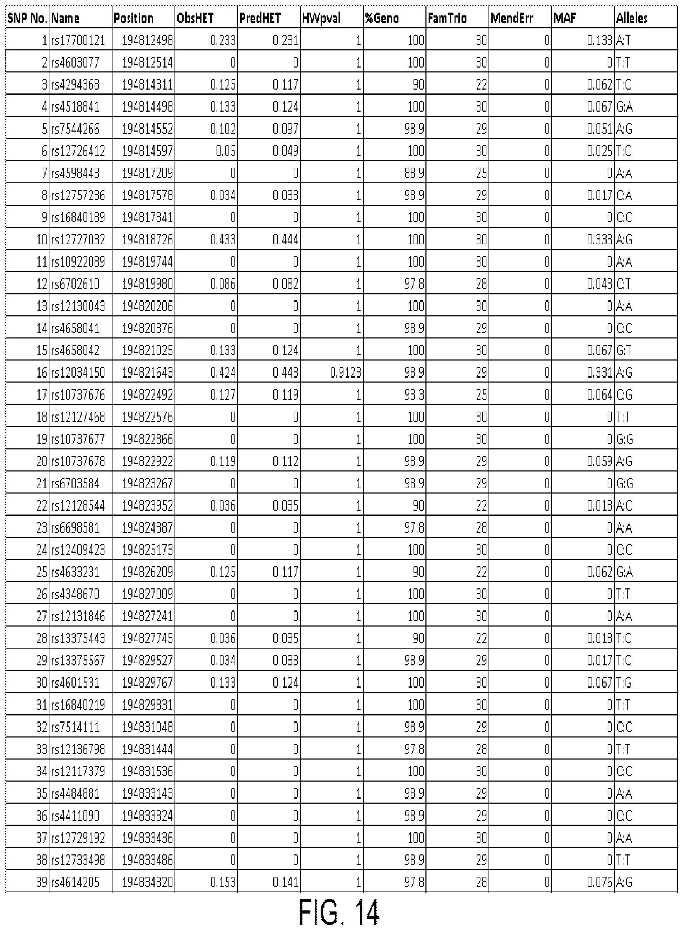

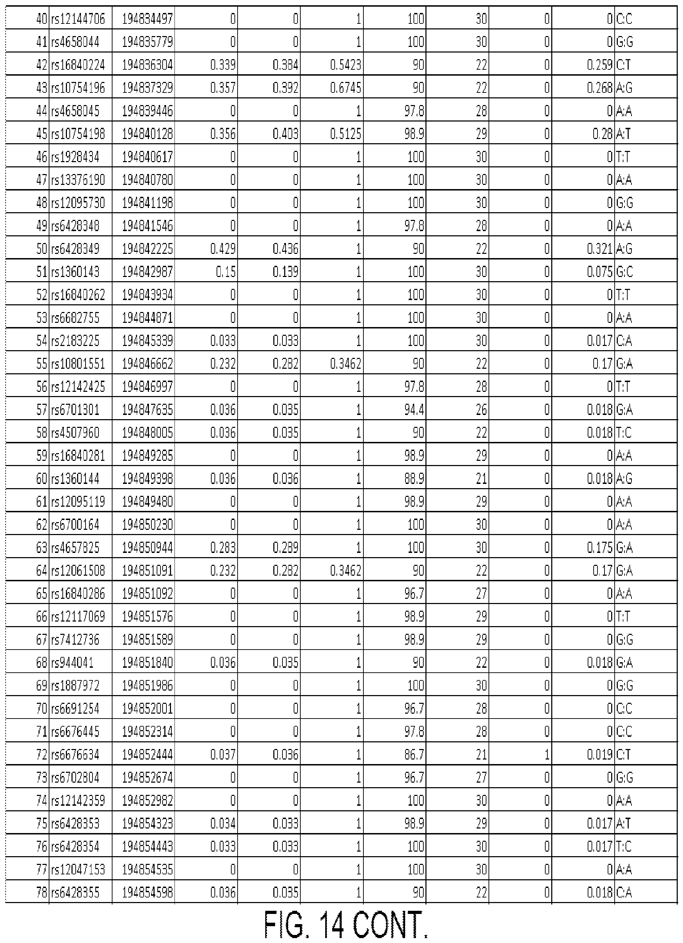

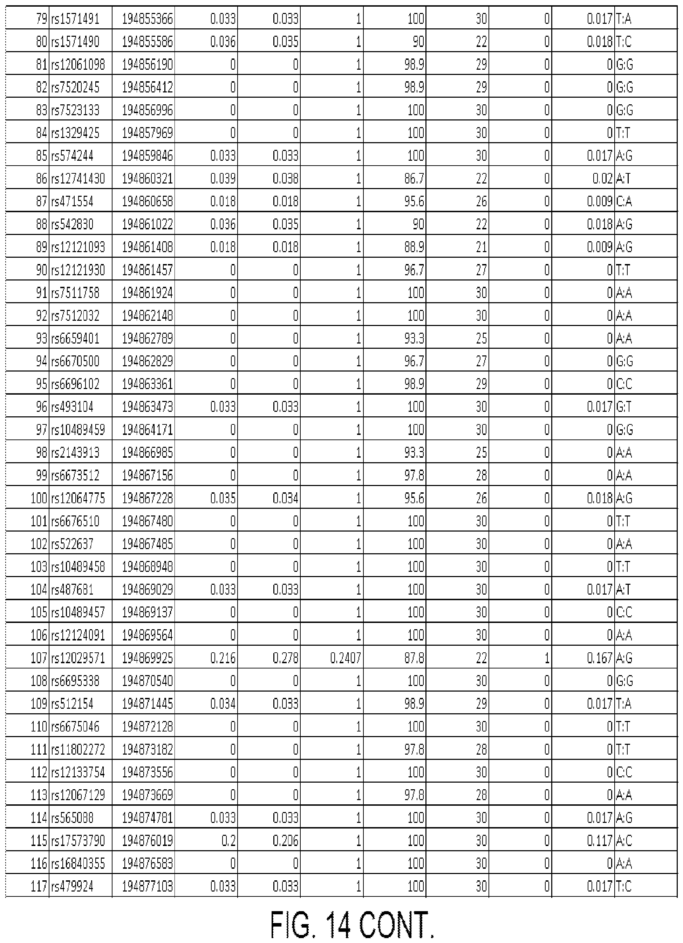

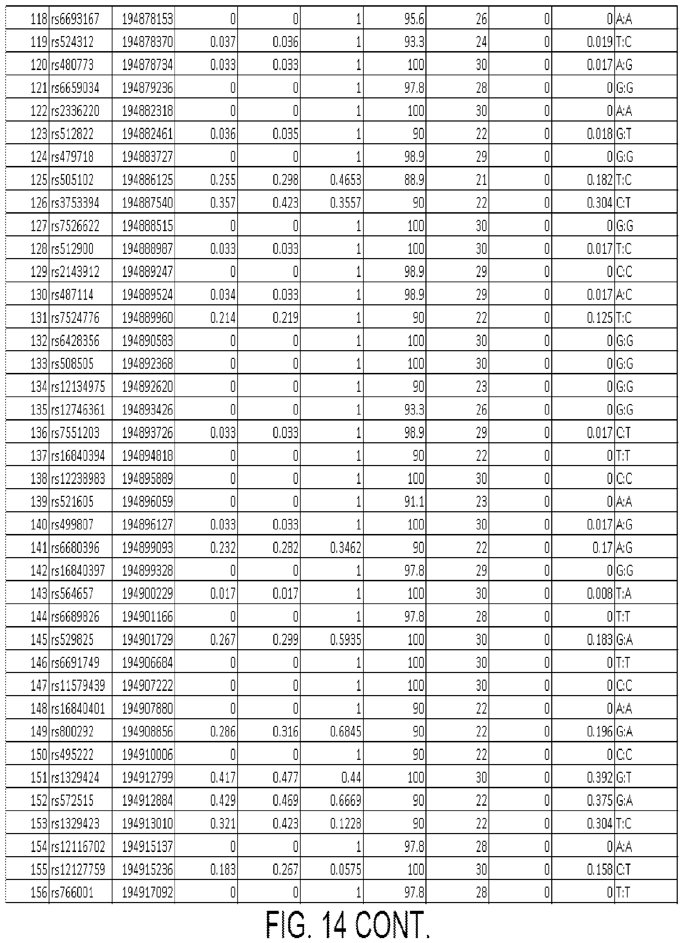

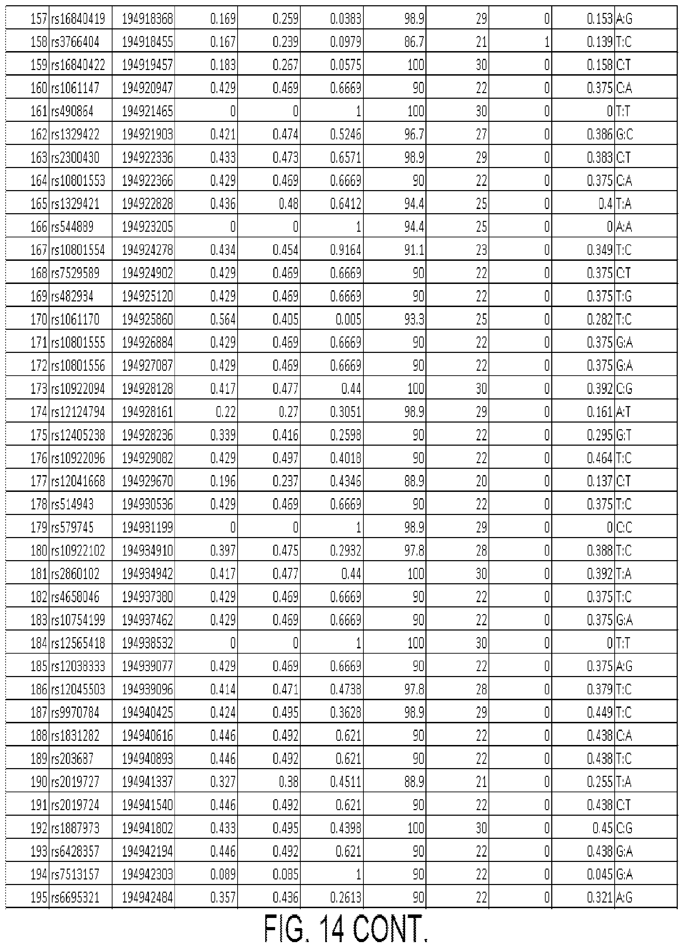

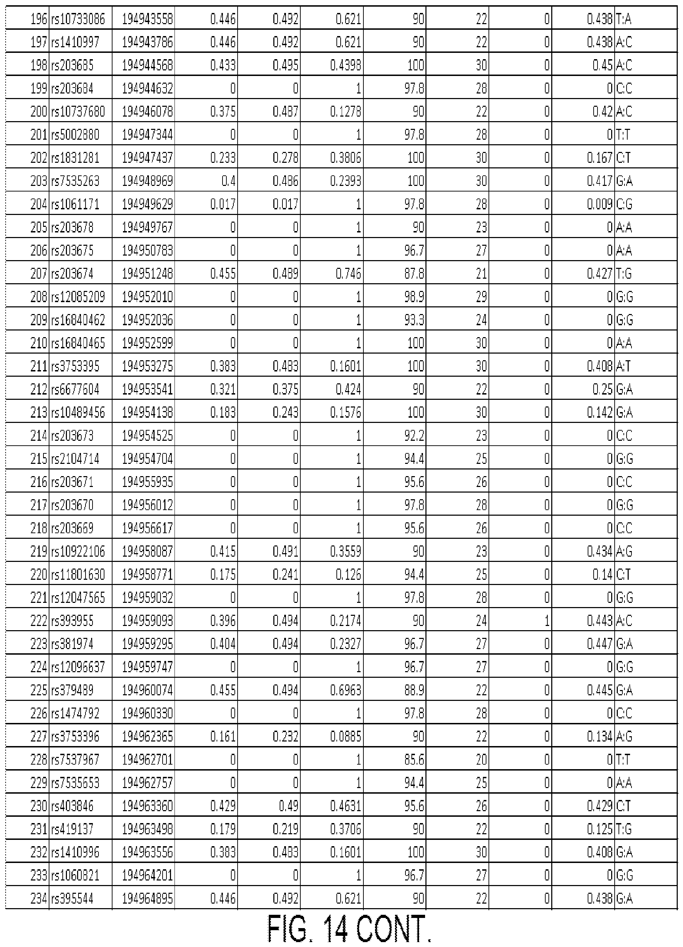

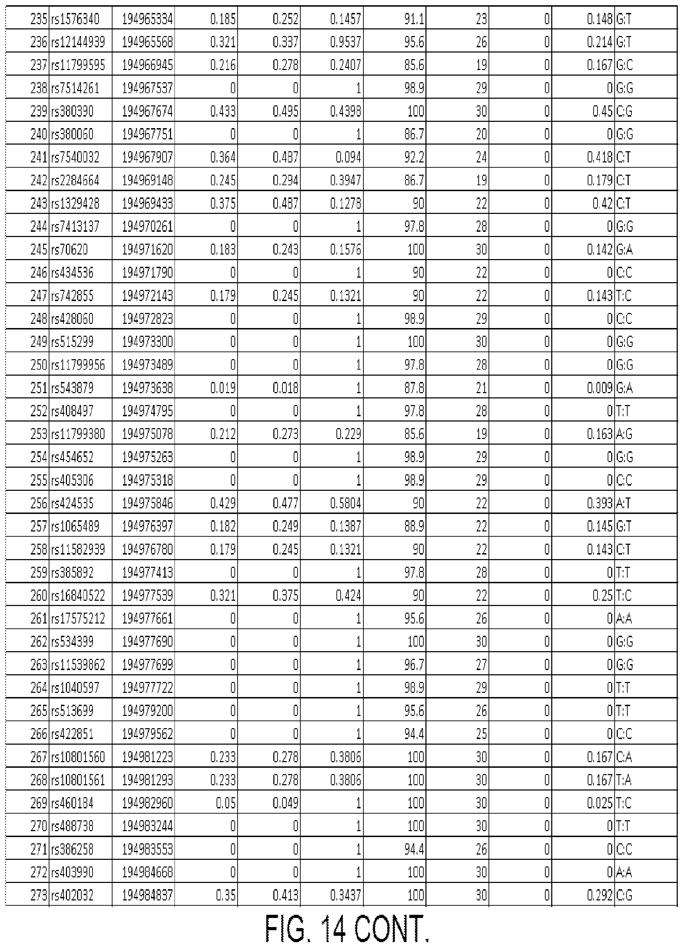

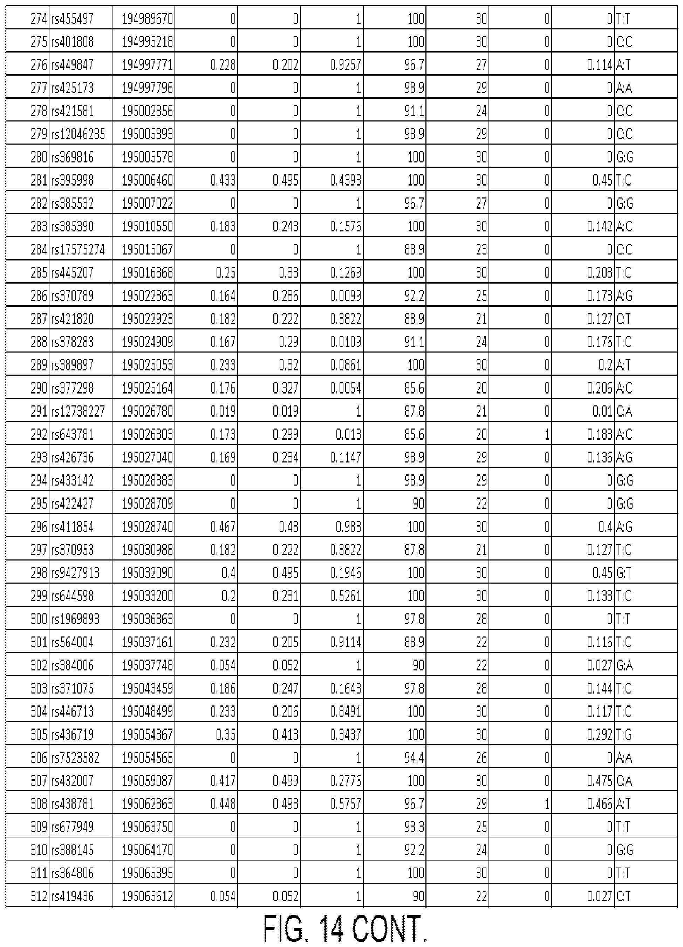

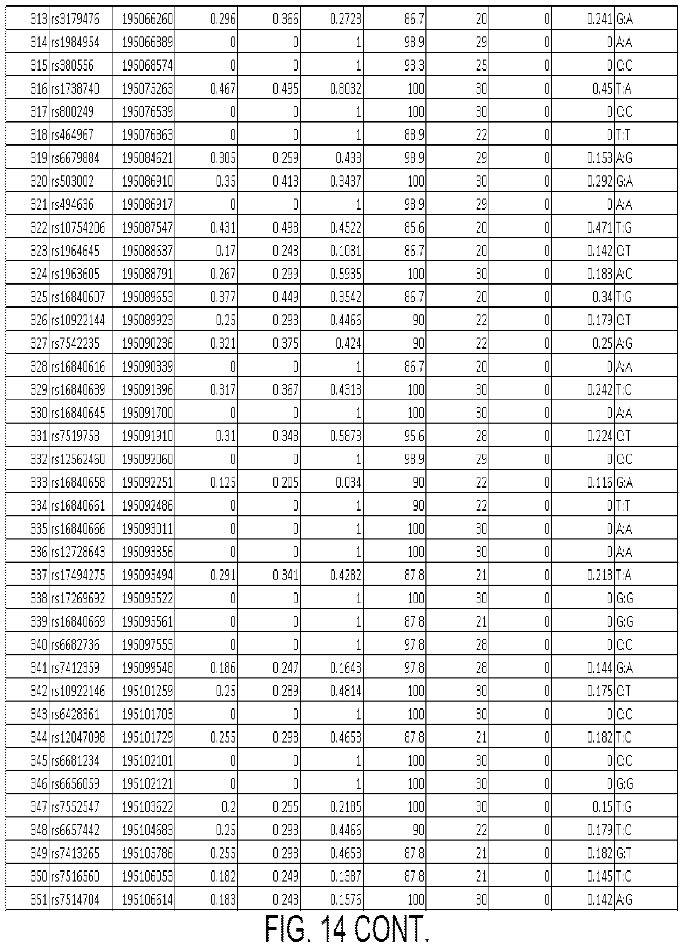

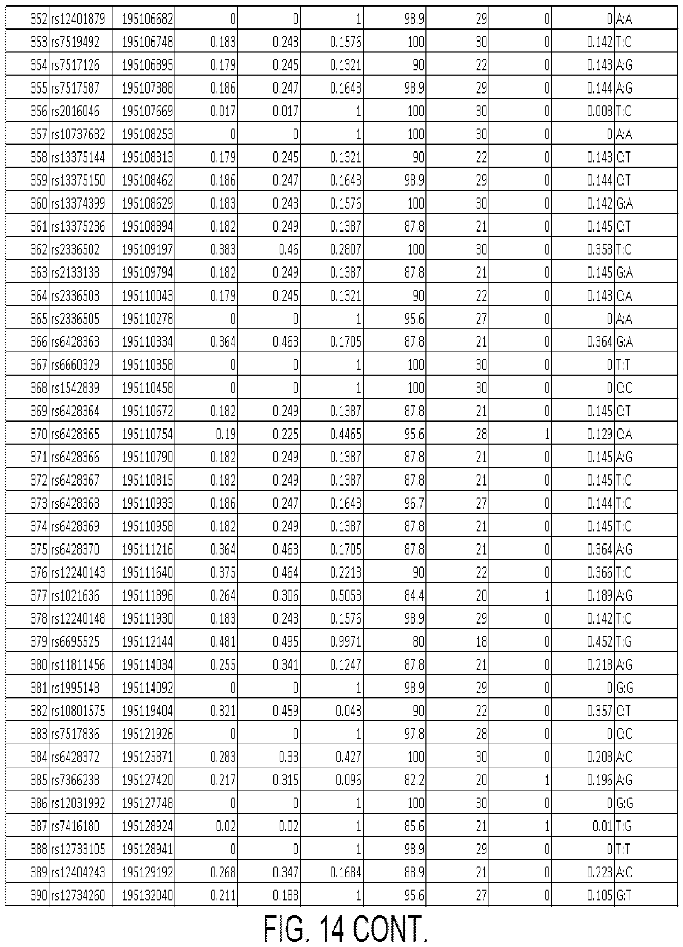

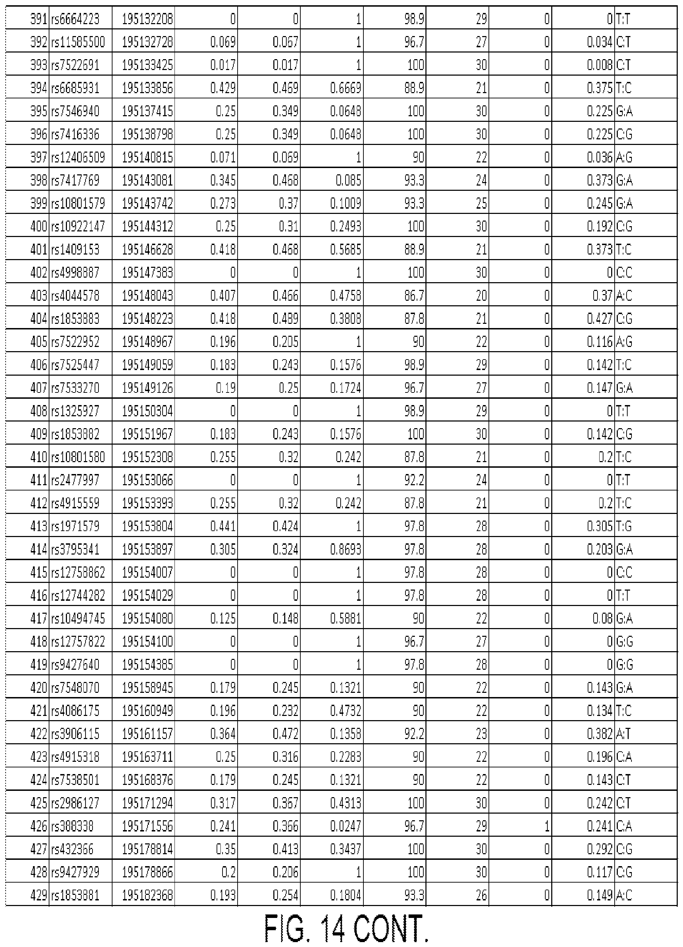

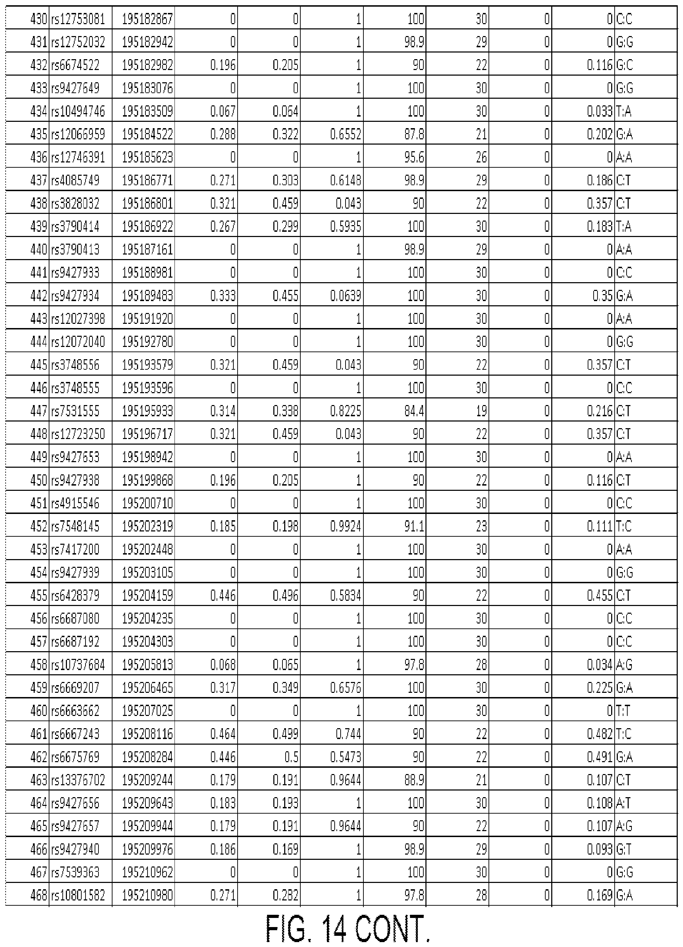

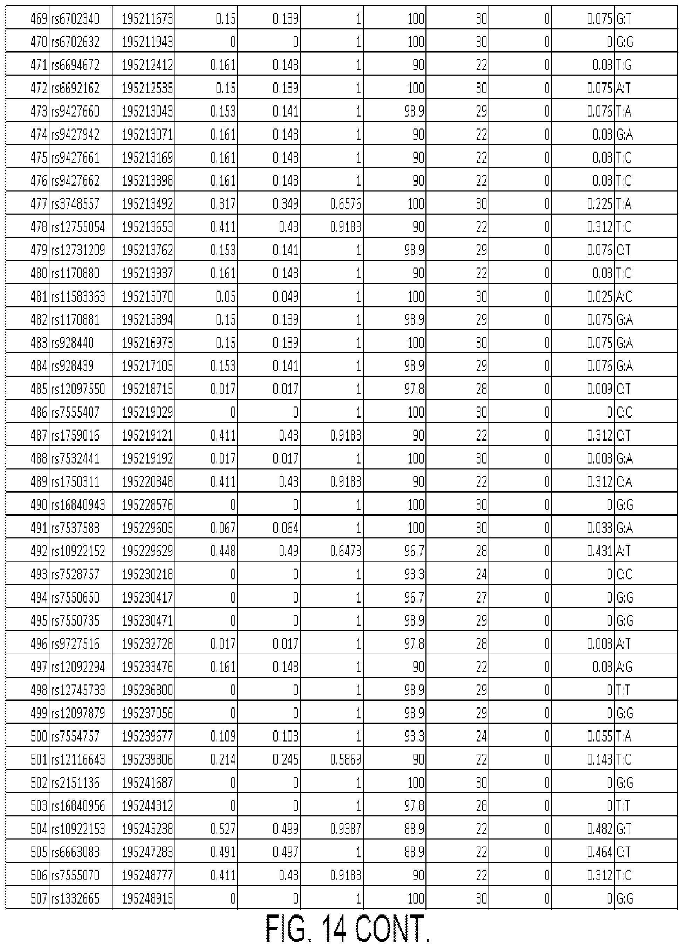

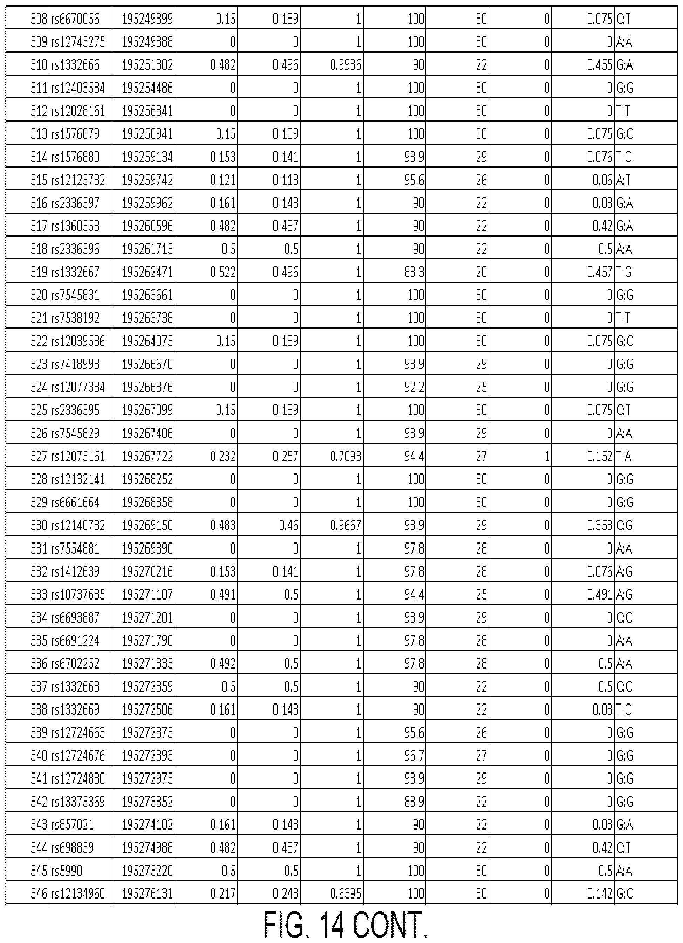

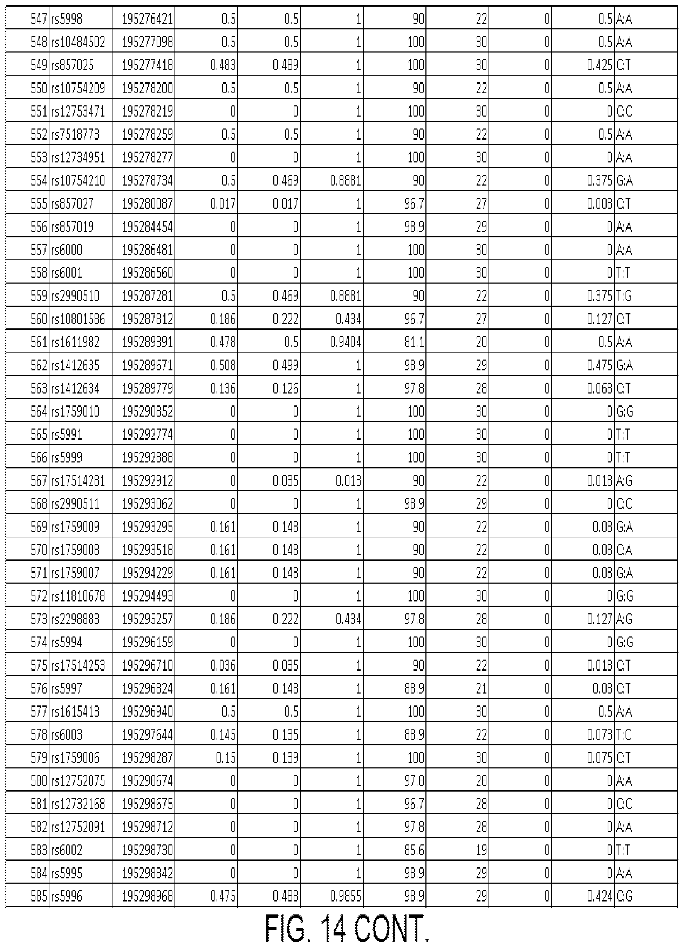

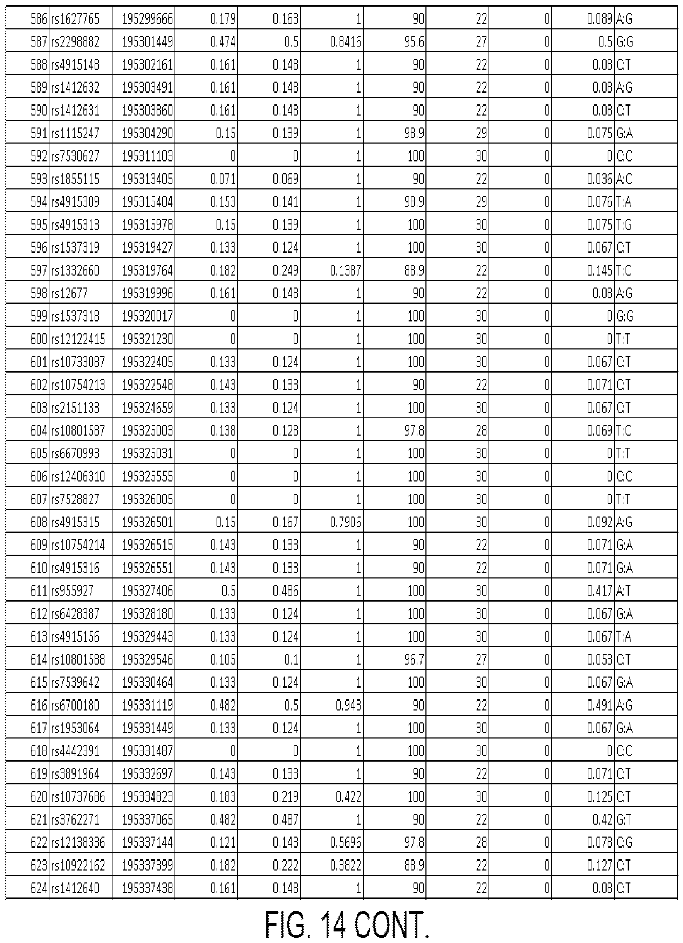

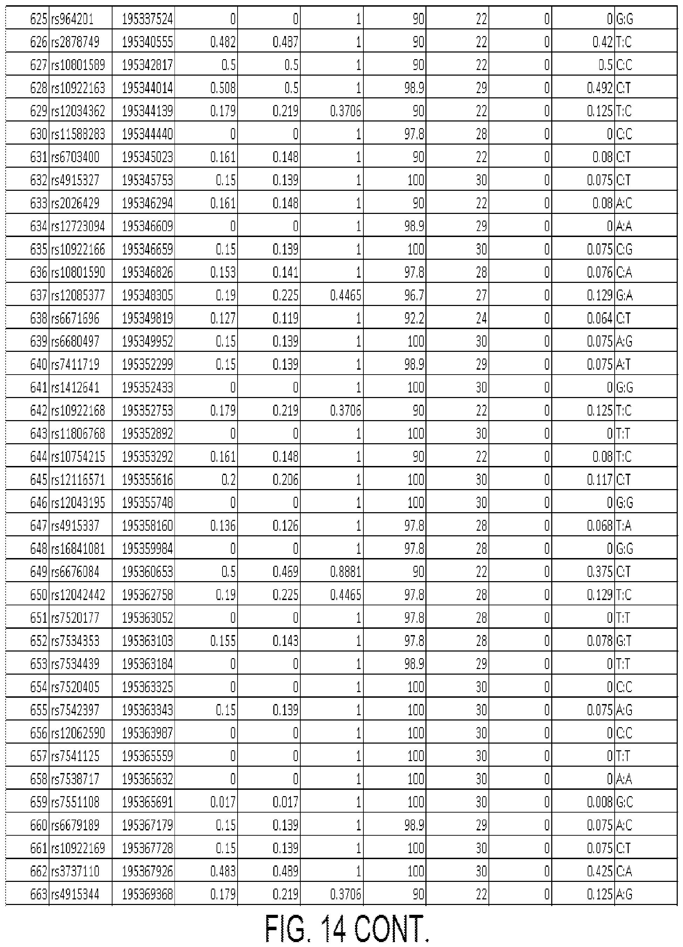

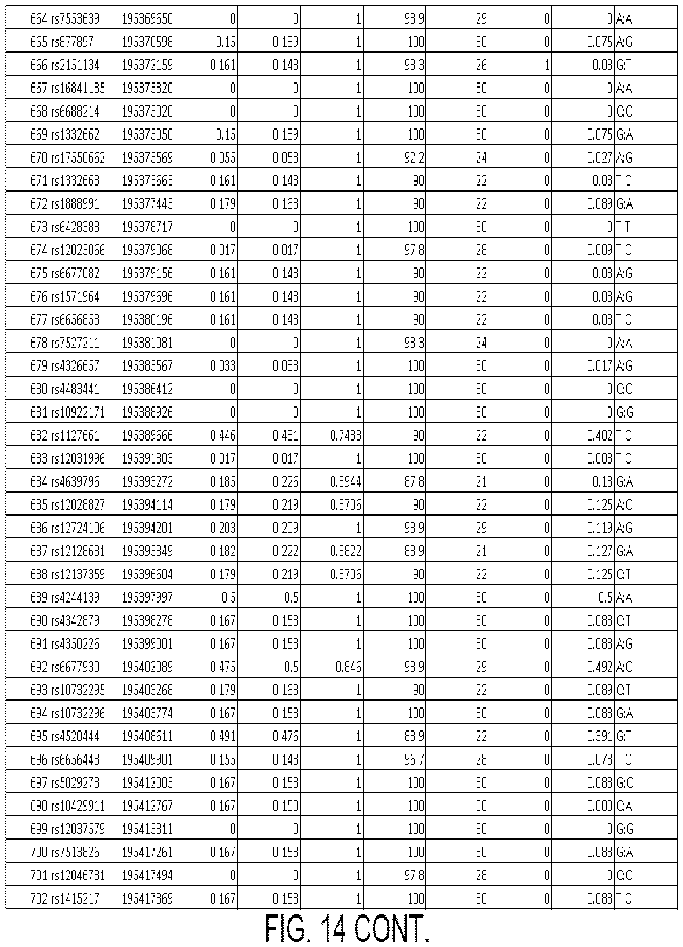

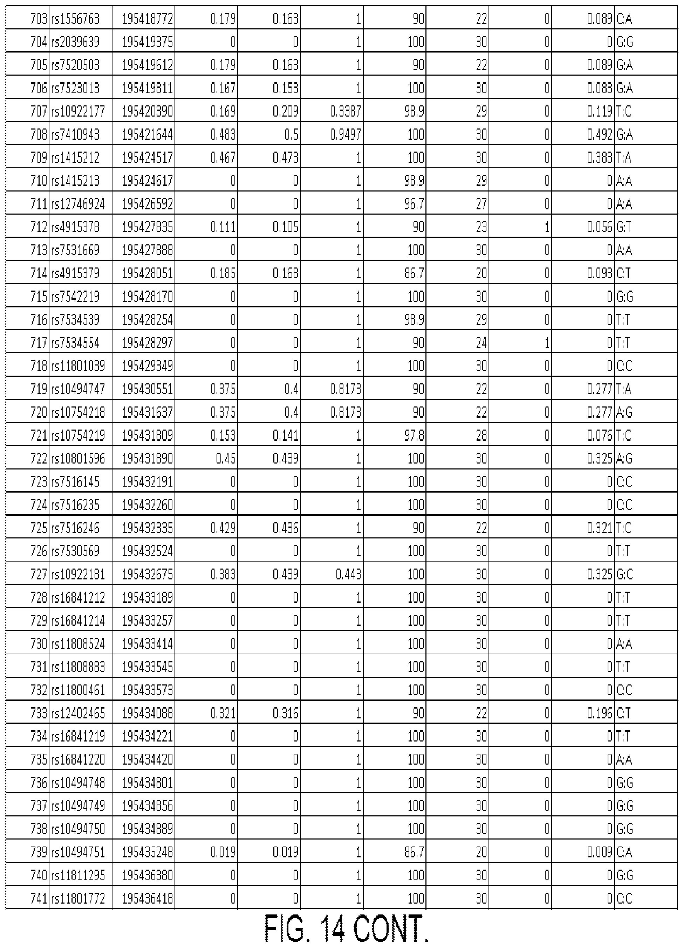

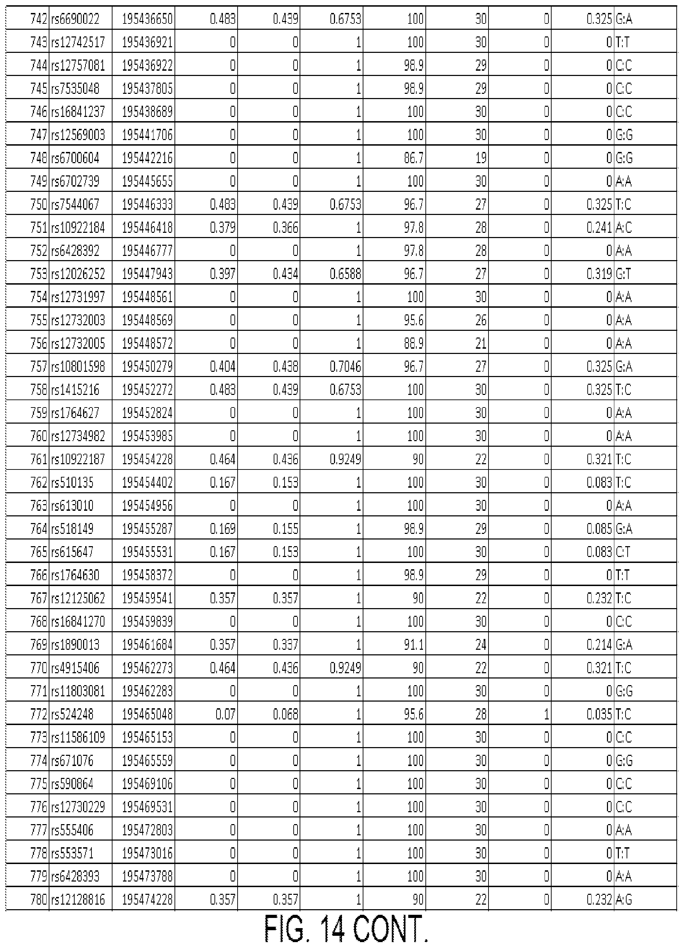

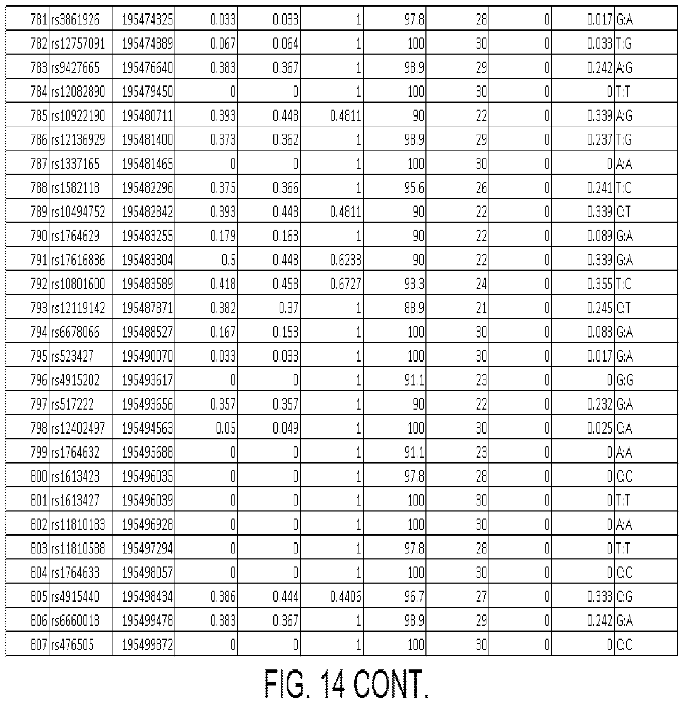

It was determined whether there were SNPs proximal and distal to the boundaries defined by the 62 SNP-based haplotypes that are in LD with the defined major haplotype blocks. All Hapmap SNPs for Caucasians lying between Chr1:194,814,311 and 195,431,890 were downloaded with subject information and analyzed in Haploview (Figures 14). Default (Gabriel et al.) blocks encompassed the entire region. The second block, defined by rs10801551 (194,848,662) through rs800292 (194,908,856) encompassed rs35928059, the most 5' SNP employed in the 62-SNP set. This suggests that the rs35928059 is in LD with SNPs as far proximal as rs10801551. Similarly, at the distal 3' end of our locus, rs6428380, the most 3' SNP in the 62_A SNP set, lies between rs4915148 (195,302,161) and rs1537319 (195,319,427), suggesting that the distal SNPs within this block are likely in LD with rs1537319.

### 3.20 Results: Characterization of CFHR3/CFHR1 Deletion Boundaries

Quantitative PCR (qPCR) was performed on multiple CEPH individuals representing each deletion tagging SNP genotype. Three qPCR primer sets were designed to amplify both duplicated regions equally, and two additional probes were designed in the sequence between the segmental duplications. All experiments were performed using QuantiFast SYBR RT-PCR Master Mix (Qiagen) and the MyiQ Single Color Real-Time PCR Detection System (BioRad). Non-deletion homozygotes showed the presence of both segmental duplications, as well as normal amplification of probes between the duplications. Heterozygotes showed intermediate values for probes within the duplications and a one-copy deletion in the region between the duplications. Individuals homozygous for the deletion tagging allele showed the presence of only one segmental duplication region and no amplification of probes located between the duplicated regions. Thus this qPCR assay can be used to reliably identify individuals as homozygous R3/R1 deletion, heterozygous R3/R1 deletion, or homozygous R3/R1 non-deletion.

### 3.21 Results: Development of Alternative CFHR3/CFHR1 Deletion Status Assays and Confirmation of rs12144939 as a CFHR3/CFHR1 Deletion Tagging SNP

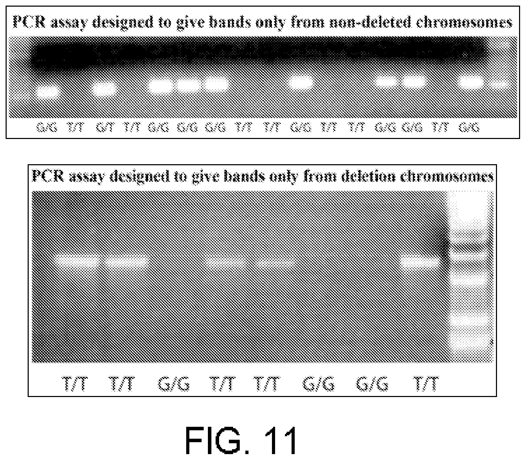

Two additional PCR assays were developed to confirm presence of deletion or presence on non-deletion chromosomes (Figure 11). These assays were not designed to distinguish heterozygotes, but to determine status of homozygous deletion or non-deletion samples. Using the sequence variant signatures found in deletion homozygotes, primers were designed to generate a 3.7kb PCR product only when the deletion is present, either in heterozygous or homozygous form. Reactions were performed using AccuPrime High Fidelity polymerase, buffers, and protocols (Invitrogen). Samples from twelve unrelated CEPH individuals and five African individuals were amplified and run on a 1% agarose gel. All homozygous deletion and heterozygous individuals produced a 3.7kb band in the assay designed to amplify across the deletion, while none of the homozygote non-deletion individuals amplified. In addition, primers were designed and used to amplify a small (200bp) PCR product only when the deletion is not present. Reactions were performed on eighty-eight CEPH individuals. All amplified well except for deletion homozygotes, which failed without exception. Thus these two assays can be used to unambiguously identify individuals homozygous either for the CFHR3-R1 deletion or for non-deletion status and in combination can determine heterozygotes.

### 3.21 Confirmation of the Deletion Tagging SNP

A predesigned Taqman CNV assay was run on all 604 CEPH individuals in order to confirm deletion status. This assay was located on chr1:195011298-195011397, just proximal to the *CFHR3* gene. Assays were run on a 7900HT Fast Real-Time PCR System (Applied Biosystems) and analyzed using CopyCaller software (Applied Biosystems). Copy number calls were compared against rs12144939 (deletion tagging SNP) genotypes. In all cases where a clear call was made by CopyCaller software, copy number calls were consistent with what would be predicted by rs12144939, with the exception of one family in which six individuals were heterozygous for the tagging SNP but were called homozygous non-deletion in the CNV analysis. This confirmed that in Caucasians, rs12144939 tagged the deletion with near 100% reliability. Thus, CFHR1/R3 deletion status can now be obtained using the three assays described above. Analysis of the 1000-genome data indicates that the following SNPs accurately tag rs12144939, and therefore the CFHR3/CFHR1 deletion: rs6689009, rs35253683, rs731557, and rs60642321.

### E. REFERENCES

1. Abrera-Abeleda MA, Nishimura C, Smith JL, Sethi S, McRae JL, Murphy BF, Silvestri G, Skerka C, Jözsi M, Zipfel PF, Hageman GS, Smith RJ. Variations in the complement regulatory genes factor H (CFH) and factor H related 5 (CFHR5) are associated with membranoproliferative glomerulonephritis type II (dense deposit disease). J Med Genet. 2006 Jul;43(7):582-9.
2. Allikmets R, Bergen AA, Dean M, Guymer RH, Hageman GS, Klaver CC, Stefansson K, Weber BH; International Age-related Macular Degeneration Genetics Consortium. Geographic atrophy in age-related macular degeneration and TLR3. N Engl J Med. 2009 May 21;360(21):2252.
3. Baird PN, Islam FM, Richardson AJ, Cain M, Hunt N, Guymer R. Analysis of the Y402H variant of the complement factor H gene in age-related macular degeneration. Invest Ophthalmol Vis Sci. 2006 Oct;47(10):4194-4198.
4. Breiman, Friedman, Olshen, and Stone. Classification and Regression Trees. Wadsworth, 1984.
5. S. R. Browning. Multilocus association mapping using variable-length Markov chains. Am J Hum Genet, 78:903-913, 2006.
6. DeAngelis MM, Ji F, Adams S, Morrison MA, Harring AJ, Sweeney MO, Capone A Jr, Miller JW, Dryja TP, Ott J, Kim IK. Alleles in the HtrA serine peptidase 1 gene alter the risk of neovascular age-related macular degeneration. Ophthalmology. 2008 Jul;115(7):1209-1215.e7.
7. W. S. De Jong, D. M. De Jong, and M. Bodis. A fluorogenic 50 nuclease (taqman) assay to assess dosage of a marker tightly linked to red skin color in autotetraploid potato. Theor Appl Genet, 107:1384-1390, 2003.
8. H. Fakhrai-Rad, J. Zheng, T. D. Willis, K. Wong, K. Suyenaga, M. Moorhead, J. Eberle, Y. R. Thorstenson, T. Jones, R. W. Davis, E. Namsaraev, and M. Faham. Snp discovery in pooled samples with mismatch repair detection. Genome Res, 14:1404-1412, 2004.
9. Gold B, Merriam JE, Zernant J, Hancox LS, Taiber AJ, Gehrs K, Cramer K, Neel J, Bergeron J, Barile GR, Smith RT; AMD Genetics Clinical Study Group, Hageman GS, Dean M, Allikmets R. Variation in factor B (BF) and complement component 2 (C2) genes is associated with age-related macular degeneration. Nat Genet. 2006 Apr;38(4):458-62.
10. Gotoh N, Yamada R, Hiratani H, Renault V, Kuroiwa S, Monet M, et al. No association between complement factor H gene polymorphism and exudative age-related macular degeneration in Japanese. Hum Genet. 2006 Aug;120(1):139-43.
11. Hageman GS, Gehrs K, Lejnine S, Bansal AT, Deangelis MM, Guymer RH, Baird PN, Allikmets R, Deciu C, Oeth P, Perlee LT. Clinical validation of a genetic model to estimate the risk of developing choroidal neovascular age-related macular degeneration. Hum Genomics. 2011 Jul;5(5):420-40.
12. Hageman GS, Anderson DH, Johnson LV, Hancox LS, Taiber AJ, Hardisty LI, Hageman JL, Stockman HA, Borchardt JD, Gehrs KM, Smith RJ, Silvestri G, Russell SR, Klaver CC, Barbazetto I, Chang S, Yannuzzi LA, Barile GR, Merriam JC, Smith RT, Olsh AK, Bergeron J, Zernant J, Merriam JE, Gold B, Dean M, Allikmets R. A common haplotype in the complement regulatory gene factor H (HF1/CFH) predisposes individuals to age-related macular degeneration. Proc Natl Acad Sci U S A. 2005 May 17;102(20):7227-7232.
13. Hageman GS, Hancox LS, Taiber AJ, Gehrs KM, Anderson DH, Johnson LV, Radeke MJ, Kavanagh D, Richards A, Atkinson J, Meri S, Bergeron J, Zernant J, Merriam J, Gold B, Allikmets R, Dean M; AMD Clinical Study Group. Extended haplotypes in the complement factor H (CFH) and CFH-related (CFHR) family of genes protect against age-related macular degeneration: characterization, ethnic distribution and evolutionary implications. Ann Med. 2006;38(8):592-604.
14. P. Hardenbol, J. Banr, M. Jain, M. Nilsson, E. A. Namsaraev, G. A. Karlin-Neumann, H. Fakhrai-Rad, M. Ronaghi, T. D. Willis, U. Landegren, and R. W. Davis. Multiplexed genotyping with sequence-tagged molecular inversion probes. Nat Biotechnol, 21:673-678, 2003.
15. Lau LI, Chen SJ, Cheng CY, Yen MY, Lee FL, Lin MW, et al. Association of the Y402H polymorphism in complement factor H gene and neovascular age-related macular degeneration in Chinese patients. Invest Ophthalmol Vis Sci. 2006 Aug;47(8):3242-6.
16. Lim JH, Wickremasinghe SS, Xie J, Chauhan DS, Baird PN, Robman LD, Hageman GS, Guymer RH. Delay to treatment and visual outcomes in patients treated with anti-vascular endothelial growth factor for age-related macular degeneration. Am J Ophthalmol. 2012 Apr;153(4):678-686.
17. R Development Core Team. R: A Language and Environment for Statistical Computing. R Foundation for Statistical Computing, Vienna, Austria, 2006, ISBN 3-900051-07-0.
18. Robman L, Baird PN, Dimitrov PN, Richardson AJ, Guymer RH. C-reactive protein levels and complement factor H polymorphism interaction in age-related macular degeneration and its progression. Ophthalmol. 2010 Oct;117(10):1982-1988.
19. M. N. Stephens and P. Donnelly. A comparison of bayesian methods for haplotype reconstruction. Am J Hum Genet, 73:1162-1169, 2003.
20. M. N. Stephens, N. J. Smith, and P. Donnelly. A new statistical method for haplotype reconstruction from population data. Am J Hum Genet, 68:978-989, 2001.
21. Wickremasinghe SS, Xie J, Lim J, Chauhan DS, Robman L, Richardson AJ, Hageman G, Baird PN, Guymer R. Variants in the APOE gene are associated with improved outcome after anti-VEGF treatment for neovascular AMD. Invest Ophthalmol Vis Sci. 2011 Jun 8;52(7):4072-9.
22. Zhang H, Morrison MA, Dewan A, Adams S, Andreoli M, Huynh N, Regan M, Brown A, Miller JW, Kim IK, Hoh J, Deangelis MM. The NEI/NCBI dbGAP database: genotypes and haplotypes that may specifically predispose to risk of neovascular age-related macular degeneration. BMC Med Genet. 2008 Jun 9;9:51.

## Claims

1. An *in vitro* method of (i) determining a Caucasian subject's susceptibility to having or developing age-related macular degeneration, (ii) identifying a Caucasian subject in need of treatment for age-related macular degeneration, or (iii) identifying a Caucasian subject in need of a prophylactic treatment for age-related macular degeneration, wherein the method comprises determining in the Caucasian subject the identity of the haplotype H2_62_A or a complement thereof, the haplotype H2_62_A being defined in Fig. 7, and
wherein the presence of the haplotype H2_62_A indicates the subject's susceptibility for having or developing age-related macular degeneration; whether the subject is in need of treatment for age-related macular degeneration; or whether the subject is in need of prophylactic treatment for age-related macular degeneration, respectively.

2. An *in vitro* method of identifying a Caucasian subject appropriate for an age-related macular degeneration clinical trial, wherein the method comprises determining in the Caucasian subject the identity of the haplotype H2_62_A or a complement thereof, the haplotype H2_62_A being defined in Fig. 7,
wherein the presence of the haplotype H2_62_A indicates whether the subject is appropriate for the clinical trial.

3. The use of the haplotype H2_62_A, or a complement thereof, in a method of identifying a Caucasian subject appropriate for an age-related macular degeneration clinical trial, the haplotype H2_62_A being defined in Fig. 7, wherein the use comprises determining in the Caucasian subject the identity of the haplotype H2_62_A or a complement thereof, wherein the presence of the haplotype H2_62_A indicates whether the subject is appropriate for the clinical trial.

4. The use of the haplotype H2_62_A according to claim 3, wherein method further comprises determining in the Caucasian subject the identity of one or more additional haplotypes, wherein the one or more additional haplotypes of H2_51_A, H1_51_B, and H2_51_B, or a complement thereof, the haplotypes being defined in Fig. 8 and Fig. 9; and wherein the presence of one or more of the additional haplotypes indicates whether the subject is appropriate for the clinical trial.

5. The use of the haplotype H2_62_A according to claim 3, wherein the method further comprises determining in the Caucasian subject
(a) the identity of one or more SNPs in the CFH-to-F13B locus, wherein the one or more SNPs is/are rsl410996, rs2274700, rs3753395, rs403846, and rs3753396; or
(b) the identity of a deletion tagging SNP in the CFH-to-F13B locus, wherein the SNP is rsl2144939.

6. The method of claims 1 or 2, wherein the method further comprises determining in the Caucasian subject the identity of one or more additional haplotypes, wherein the one or more additional haplotypes are H2_51_A, H1_51_B, and H2_51_B, or a complement thereof, the haplotypes being defined in Fig. 8 and Fig. 9; and
wherein the presence of one or more of the additional haplotypes indicates the subject's susceptibility for having or developing age-related macular degeneration; whether the subject is in need of treatment for age-related macular degeneration; whether the subject is in need of prophylactic treatment for age-related macular degeneration; or whether the subject is appropriate for the clinical trial; respectively.

7. The method of any of claims 1, 2 or 6, or the use of the haplotype H2_62_A according to any of claims 3 to 5, wherein the subject is female.

## Patentansprüche

1. *In-vitro*-Verfahren zum (i) Bestimmen der Anfälligkeit eines kaukasischen Subjekts, altersbedingte Makuladegeneration zu haben oder zu entwickeln, (ii) Identifizieren eines kaukasischen Subjekts, das einer Behandlung von altersbedingter Makuladegeneration bedarf, oder (iii) Identifizieren eines kaukasischen Subjekts, das einer prophylaktischen Behandlung von altersbedingter Makuladegeneration bedarf, wobei das Verfahren Bestimmen der Identität des Haplotyps H2_62_A oder eines Komplements davon in dem kaukasischen Subjekt aufweist, wobei der Haplotyp H2_62_A in Fig. 7 definiert ist, und
wobei das Vorhandensein des Haplotyps H2_62_A die Anfälligkeit des Subjekts anzeigt, eine altersbedingte Makuladegeneration zu haben oder zu entwickeln, ob das Subjekt einer Behandlung einer altersbedingten Makuladegeneration bedarf bzw. ob das Subjekt einer prophylaktischen Behandlung einer altersbedingten Makuladegeneration bedarf.

2. In-vitro-Verfahren zum Identifizieren eines kaukasischen Subjekts, das für eine klinische Studie zur altersbedingten Makuladegeneration geeignet ist, wobei das Verfahren Bestimmen der Identität des Haplotyps H2_62_A oder eines Komplements davon in dem kaukasischen Subjekt aufweist, wobei der Haplotyp H2_62_A in Fig. 7 definiert ist,
wobei das Vorhandensein des Haplotyps H2_62_A anzeigt, ob das Subjekt für die klinische Studie geeignet ist.

3. Verwendung des Haplotyps H2_62_A oder eines Komplements davon in einem Verfahren zum Identifizieren eines kaukasischen Subjekts, das für eine klinische Studie zur altersbedingten Makuladegeneration geeignet ist, wobei der Haplotyp H2_62_A in Fig. 7 definiert ist, wobei die Verwendung Bestimmen der Identität des Haplotyps H2_62_A oder eines Komplements davon in dem kaukasischen Subjekt aufweist, wobei das Vorhandensein des Haplotyps H2_62_A anzeigt, ob das Subjekt für die klinische Studie geeignet ist.

4. Verwendung des Haplotyps H2_62_A nach Anspruch 3, wobei das Verfahren weiterhin Bestimmen der Identität eines oder mehrerer zusätzlicher Haplotypen in dem kaukasischen Subjekt aufweist, wobei der eine oder die mehreren zusätzlichen Haplotypen H2_51_A, H1_51_B und H2 51_B oder ein Komplement davon sind, wobei die Haplotypen in Fig. 8 und Fig. 9 definiert sind, und
wobei das Vorhandensein eines oder mehrerer der zusätzlichen Haplotypen anzeigt, ob das Subjekt für die klinische Prüfung geeignet ist.

5. Verwendung des Haplotyps H2_62_A nach Anspruch 3, wobei das Verfahren weiterhin Bestimmen im kaukasischen Subjekt aufweist
(a) der Identität eines oder mehrerer SNPs im CFH-bis-F13B-Locus, wobei das eine oder die mehreren SNPs rs1410996, rs2274700, rs3753395, rs403846 und rs3753396 ist/sind; oder
(b) der Identität eines Deletions-kennzeichnenden SNPs im CFH-bis-F13B-Locus, wobei das SNP rsl2144939 ist.

6. Verfahren nach Anspruch 1 oder 2, wobei das Verfahren weiterhin Bestimmen der Identität eines oder mehrerer zusätzlicher Haplotypen im kaukasischen Subjekt aufweist, wobei der eine oder die mehreren zusätzlichen Haplotypen H2_51_A, H1_51_B und H2_51_B oder ein Komplement davon sind, wobei die Haplotypen in Fig. 8 und Fig. 9 definiert sind, und
wobei das Vorhandensein eines oder mehrerer der zusätzlichen Haplotypen die Anfälligkeit des Subjekts anzeigt, eine altersbedingte Makuladegeneration zu haben oder zu entwickeln, ob das Subjekt einer Behandlung einer altersbedingten Makuladegeneration bedarf, ob das Subjekt einer prophylaktischen Behandlung einer altersbedingten Makuladegeneration bedarf, bzw. oder ob das Subjekt für die klinische Studie geeignet ist.

7. Verfahren nach einem der Ansprüche 1, 2 oder 6 oder Verwendung des Haplotyps H2_62_A nach einem der Ansprüche 3 bis 5, wobei das Subjekt weiblich ist.

## Revendications

1. Procédé *in vitro* (i) de détermination de la sensibilité d'un sujet caucasien au fait de contracter ou de développer une dégénérescence maculaire liée à l'âge, (ii) d'identification d'un sujet caucasien ayant besoin d'un traitement pour la dégénérescence maculaire liée à l'âge, ou (iii) d'identification d'un sujet caucasien ayant besoin d'un traitement prophylactique pour la dégénérescence maculaire liée à l'âge, dans lequel le procédé comprend la détermination chez le sujet caucasien de l'identité de l'haplotype H2_62_A ou d'un complément de celui-ci, l'haplotype H2_62_A étant défini à la figure 7, et
dans lequel la présence de l'haplotype H2_62_A indique respectivement la sensibilité du sujet au fait de contracter ou de développer une dégénérescence maculaire liée à l'âge ; si le sujet a besoin d'un traitement pour la dégénérescence maculaire liée à l'âge ; ou si le sujet a besoin d'un traitement prophylactique pour la dégénérescence maculaire liée à l'âge.

2. Procédé *in vitro* d'identification d'un sujet caucasien approprié pour une expérimentation clinique de dégénérescence maculaire liée à l'âge, dans lequel le procédé comprend la détermination chez le sujet caucasien de l'identité de l'haplotype H2_62_A ou d'un complément de celui-ci, l'haplotype H2_62_A étant défini à la figure 7,
dans lequel la présence de l'haplotype H2_62_A indique si le sujet est approprié pour l'expérimentation clinique.

3. Utilisation de l'haplotype H2_62_A, ou d'un complément de celui-ci, dans un procédé d'identification d'un sujet caucasien approprié pour une expérimentation clinique de dégénérescence maculaire liée à l'âge, l'haplotype H2_62_A étant défini à la figure 7, dans laquelle l'utilisation comprend la détermination chez le Caucasien de l'identité de l'haplotype H2_62_A ou d'un complément de celui-ci, dans laquelle la présence de l'haplotype H2_62_A indique si le sujet est approprié pour l'expérimentation clinique.

4. Utilisation de l'haplotype H2_62_A selon la revendication 3, dans laquelle le procédé comprend en outre la détermination chez le sujet caucasien de l'identité d'un ou plusieurs haplotypes supplémentaires, dans laquelle le ou les plusieurs haplotypes supplémentaires de H2_51_A, H1_51_B et H2_51_B, ou un complément de ceux-ci, les haplotypes étant définis à la figure 8 et à la figure 9 ; et dans laquelle la présence d'un ou plusieurs des haplotypes supplémentaires indique si le sujet est approprié pour l'expérimentation clinique.

5. Utilisation de l'haplotype H2_62_A selon la revendication 3, dans laquelle le procédé comprend en outre la détermination chez le sujet caucasien
(a) de l'identité d'un ou plusieurs SNP dans le lieu géométrique CFH-à-F13B, dans laquelle le ou les plusieurs SNPS est/sont rs1410996, rs2274700, rs3753395, rs403846 et rs3753396 ; ou
(b) de l'identité d'un marquage de délétion SNP dans le lieu géométrique CFH-à-F13B, dans laquelle le SNP est rs12144939.

6. Procédé selon les revendications 1 ou 2, dans lequel le procédé comprend en outre la détermination chez le sujet caucasien de l'identité d'un ou plusieurs haplotypes supplémentaires, dans lequel le ou les plusieurs haplotypes supplémentaires sont H2_51_A, H1_51_B et H2_51_B, ou un complément de ceux-ci, les haplotypes étant définis à la figure 8 et à la figure 9 ; et
dans lequel la présence d'un ou plusieurs des haplotypes supplémentaires indique respectivement la sensibilité du sujet pour le fait de contracter ou de développer une dégénérescence maculaire liée à l'âge ; si le sujet a besoin d'un traitement pour la dégénérescence maculaire liée à l'âge ; si le sujet a besoin d'un traitement prophylactique pour la dégénérescence maculaire liée à l'âge ; ou si le sujet est approprié pour l'expérimentation clinique.

7. Procédé selon l'une quelconque des revendications 1, 2 ou 6, ou utilisation de l'haplotype H2_62_A selon l'une quelconque des revendications 3 à 5, dans lequel le sujet est de sexe féminin.
